# EUROPEAN PATENT SPECIFICATION

(11) **EP 1 703 905 B1**
(45) Date of publication and mention of the grant of the patent: **12.11.2008**
(21) Application number: 05701855.8
(22) Date of filing: 13.01.2005
(51) Int. Cl.: A61K 31/404, C07D 209/42, C07D 401/04, C07D 401/06, C07D 403/04, C07D 405/04, C07D 409/04, C07D 413/04, A61P 29/00

(54) **INDOLE DERIVATIVES AND USE THEREOF AS KINASE INHIBITORS IN PARTICULAR IKK2 INHIBITORS**
INDOL-DERIVATE UND IHRE VERWENDUNG ALS KINASE-HEMMER, INSBESONDERE IKK2-HEMMER
DERIVES D'INDOLE ET UTILISATION DE CEUX-CI COMME INHIBITEURS DE KINASE, NOTAMMENT DES INHIBITEURS DE IKK2

(30) Priority: 15.01.2004 GB 0400895
(43) Date of publication of application: 27.09.2006
(73) Proprietor: SMITHKLINE BEECHAM CORPORATION, Philadelphia, PA 19101 (US)
(72) Inventor: BALDWIN, Ian, Robert, Stevenage Hertfordshire SG1 2NY (GB); BAMBOROUGH, Paul, Stevenage Hertfordshire SG1 2NY (GB); CHRISTOPHER, John, Andrew, Stevenage Hertfordshire SG1 2NY (GB); KERNS, Jeffrey, K, King of Prussia, Pennsylvania 19406 (US); LONGSTAFF, Timothy, Stevenage Hertfordshire SG1 2NY (GB); MILLER, David, Drysdale, Stevenage Hertfordshire SG1 2NY (GB)
(74) Representative: Giddings, Peter John
(86) International application number: PCT/GB2005/000085
(87) International publication number: WO 2005/067923

(56) References cited:
- WO-A-03/037886

## Description

The present invention relates to indole carboxamide derivatives, compositions and- medicaments containing the same, as well as processes for the preparation and use of such compounds, compositions and medicaments. Such indole carboxamide derivatives may be useful in the treatment of diseases associated with inappropriate IKK2 (also known as IKKβ) activity, in particular in the treatment and prevention of disease states mediated by IKK2 mechanisms including inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, ankylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

An important large family of enzymes is the protein kinase enzyme family. Currently, there are about 500 different known protein kinases. However, because three to four percent of the human genome is a code for the formation of protein kinases, there may be many thousands of distinct and separate kinases in the human body. Protein kinases serve to catalyze the phosphorylation of an amino acid side chain in various proteins by the transfer of the γ-phosphate of the ATP-Mg²⁺ complex to said amino acid side chain. These enzymes control the majority of the signaling processes inside cells, thereby governing cell function, growth, differentiation and destruction (apoptosis) through reversible phosphorylation of the hydroxyl groups of serine, threonine and tyrosine residues in proteins. Studies have shown that protein kinases are key regulators of many cell functions, including signal transduction, transcriptional regulation, cell motility, and cell division. Several oncogenes have also been shown to encode protein kinases, suggesting that kinases play a role in oncogenesis. These processes are highly regulated, often by complex intermeshed pathways where each kinase will itself be regulated by one or more kinases. Consequently, aberrant or inappropriate protein kinase activity can contribute to the rise of disease states associated with such aberrant kinase activity. Due to their physiological relevance, variety and ubiquitousness, protein kinases have become one of the most important and widely studied family of enzymes in biochemical and medical research.

The protein kinase family of enzymes is typically classified into two main subfamilies: Protein Tyrosine Kinases and Protein Serine/Threonine Kinases, based on the amino acid residue they phosphorylate. The serine/threonine kinases (PSTK), includes cyclic AMP- and cyclic GMP-dependent protein kinases, calcium and phospholipid dependent protein kinase, calcium- and calmodulin-dependent protein kinases, casein kinases, cell division cycle protein kinases and others. These kinases are usually cytoplasmic or associated with the particulate fractions of cells, possibly by anchoring proteins. Aberrant protein serine/threonine kinase activity has been implicated or is suspected in a number of pathologies such as rheumatoid arthritis, psoriasis, septic shock, bone loss, many cancers and other proliferative diseases. Accordingly, serine/threonine kinases and the signal transduction pathways which they are part of are important targets for drug design. The tyrosine kinases phosphorylate tyrosine residues. Tyrosine kinases play an equally important role in cell regulation. These kinases include several receptors for molecules such as growth factors and hormones, including epidermal growth factor receptor, insulin receptor, platelet derived growth factor receptor and others. Studies have indicated that many tyrosine kinases are transmembrane proteins with their receptor domains located on the outside of the cell and their kinase domains on the inside. Much work is also under progress to identify modulators of tyrosine kinases as well.

Nuclear factor κB (NF-κB) belongs to a family of closely related dimeric transcription factor complexes composed of various combinations of the Rel/NF-κB family of polypeptides. The family consists of five individual gene products in mammals, RelA (p65), NF-κB1 (p50/ p105), NF-κ82 (p49/ p100), c-Rel, and RelB, all of which can form hetero- or homodimers. These proteins share a highly homologous 300 amino acid "Rel homology domain" which contains the DNA binding and dimerization domains. At the extreme C-terminus of the Rel homology domain is a nuclear translocation sequence important in the transport of NF-κB from the cytoplasm to the nucleus. In addition, p65 and cRel possess potent transactivation domains at their C-terminal ends.

The activity of NF-κB is regulated by its interaction with a member of the inhibitor IκB family of proteins. This interaction effectively blocks the nuclear localization sequence on the NF-κB proteins, thus preventing migration of the dimer to the nucleus. A wide variety of stimuli activate NF-κB through what are likely to be multiple signal transduction pathways. Included are bacterial products (LPS), some viruses (HIV-1, HTLV-1), inflammatory cytokines (TNFα, IL-1), environmental and oxidative stress and DNA damaging agents. Apparently common to all stimuli however, is the phosphorylation and subsequent degradation of IκB. IκB is phosphorylated on two N-terminal serines by the recently identified IκB kinases (IKK-α and IKK-β). IKK-β is also known as IKK2. Site-directed mutagenesis studies indicate that these phosphorylations are critical for the subsequent activation of NF-κB in that once phosphorylated the protein is flagged for degradation via the ubiquitin-proteasome pathway. Free from IκB, the active NF-κB complexes are able to translocate to the nucleus where they bind in a selective manner to preferred gene-specific enhancer sequences. Included in the genes regulated by NF-κB are a number of cytokines and chemokines, cell adhesion molecules, acute phase proteins, immunoregualtory proteins, eicosanoid metabolizing enzymes and anti-apoptotic genes.

It is well-known that NF-κB plays a key role in the regulated expression of a large number of pro-inflammatory mediators including cytokines such as TNF, IL-1β, IL-6 and IL-8, cell adhesion molecules, such as ICAM and VCAM, and inducible nitric oxide synthase (iNOS). Such mediators are known to play a role in the recruitment of leukocytes at sites of inflammation and in the case of iNOS, may lead to organ destruction in some inflammatory and autoimmune diseases.

The importance of NF-κB in inflammatory disorders is further strengthened by studies of airway inflammation including asthma, in which NF-κB has been shown to be activated. This activation may underlie the increased cytokine production and leukocyte infiltration characteristic of these disorders. In addition, inhaled steroids are known to reduce airway hyperresponsiveness and suppress the inflammatory response in asthmatic airways. In light of the recent findings with regard to glucocorticoid inhibition of NF-κB, one may speculate that these effects are mediated through an inhibition of NF-κB.

Further evidence for a role of NF-κB in inflammatory disorders comes from studies of rheumatoid synovium. Although NF-κB is normally present as an inactive cytoplasmic complex, recent immunohistochemical studies have indicated that NF-κB is present in the nuclei, and hence active, in the cells comprising rheumatoid synovium. Furthermore, NF-κB has been shown to be activated in human synovial cells in response to stimulation with TNF-α or IL-1β. Such a distribution may be the underlying mechanism for the increased cytokine and eicosanoid production characteristic of this tissue. See Roshak, A. K., et al., J. Biol. Chem., 271, 31496-31501 (1996). Expression of IKK-β has been shown in synoviocytes of rheumatoid arthritis patients and gene transfer studies have demonstrated the central role of IKK-β in stimulated inflammatory mediator production in these cells. See Aupperele et al. J. Immunology 1999. 163:427-433 and Aupperle et al. J. Immunology 2001;166:2705-11. More recently, the intra-articular administration of a wild type IKK-β adenoviral construct was shown to cause paw swelling while intra-articular administration of dominant-negative IKKβ inhibited adjuvant-induced arthritis in rat. See Tak et al. Arthritis and Rheumatism 2001, 44:1897-1907.

The NF-κB/Rel and IκB proteins are also likely to play a key role in neoplastic transformation and metastasis. Family members are associated with cell transformation *in vitro* and *in vivo* as a result of over expression, gene amplification, gene rearrangements or translocations. In addition, rearrangement and/or amplification of the genes encoding these proteins are seen in 20-25% of certain human lymphoid tumors. Further, NF-κB is activated by oncogenic ras, the most common defect in human tumors and blockade of NF-κB activation inhibits ras mediated cell transformation. In addition, a role for NF-κB in the regulation of apoptosis has been reported strengthening the role of this transcription factor in the regulation of tumor cell proliferation. TNF, ionizing radiation and DNA damaging agents have all been shown to activate NF-κB which in turn leads to the upregulated expression of several anti-apoptotic proteins. Conversely, inhibition of NF-κB has been shown to enhance apoptotic-killing by these agents in several tumor cell types. As this likely represents a major mechanism of tumor cell resistance to chemotherapy, inhibitors of NF-κB activation may be useful chemotherapeutic agents as either single agents or adjunct therapy. Recent reports have implicated NF-κB as an inhibitor of skeletal cell differentiation as well as a regulator of cytokine-induced muscle wasting (Guttridge et al. Science; 2000; 289: 2363-2365.) further supporting the potential of NFκB inhibitors as novel cancer therapies.

Several NF-κB inhibitors are described in C. Wahl, et al. J. Clin. Invest. 101(5), 1163-1174 (1998), R. W. Sullivan, et al. J. Med. Chem. 41, 413-419 (1998), J. W. Pierce, et al. J. Biol Chem. 272, 21096-21103 (1997).

The marine natural product hymenialdisine is known to inhibit NF-κB. Roshak, A., et al., JPET, 283, 955-961 (1997). Breton, J. J and Chabot-Fletcher, M. C., JPET, 282, 459-466 (1997).

Additionally, patent applications have been filed on aminothiophene inhibitors of the IKK2, see Callahan, et al., WO 2002030353; Baxter, et al., WO 2001058890, Faull, et al., WO 2003010158; Griffiths, et al., WO2003010163; Fancelli, et al., WO 200198290; imidazole inhibitors of IKK2, see Callahan, et al., WO 200230423; anilinophenylpyrimidine inhibitors of IKK2, see Kois, et al., WO 2002046171; β-carboline inhbitors of IKK2, see Ritzeler, et al, WO 2001068648, Ritzeler, et al, EP 1134221; Nielsch, et al. DE 19807993; Ritzeler, et al., EP 1209158; indole inhbitors of IKK2, see Ritzeler, et al., WO 2001030774; benzimidazole inhibitors of the IKK2, see Ritzeler, et al., DE 19928424; Ritzeler et al, WO 2001000610; aminopyridine inhibitors of IKK2, see Lowinger, et al, WO 2002024679; Murata, et al, WO 2002024693; Murata, et al., WO 2002044153; pyrazolaquinazoline inhibitors of IKK2, see Beaulieu, et al., WO 2002028860; Burke et al, WO 2002060386, Burke, et al. US 20030022898; quinoline inhibitors of IKK2, Browner, et al., WO2002041843, Browner, et al., US 20020161004 and pyridylcyanoguanidine inhibitors of IKK2, see Bjorkling, et al., WO 2002094813, Binderup et al, WO 2002094322 and Madsen, et al., WO 200294265.The natural products staurosporine, quercetin, K252a and K252b have been shown to be IKK2 inhibitors, see Peet, G. W. and Li, J. J. Biol. Chem., 274, 32655-32661 (1999) and Wisniewski, D., et al., Analytical Biochem. 274, 220-228 (1999). Synthetic inhibitors of IKK2 have also been described, see Burke, et al. J. Biol. Chem., 278, 1450-1456 (2003) and Murata, et al., Bioorg. Med. Chem. Lett., 13, 913-198 (2003) have described IKK2 inhibitors.

The present inventors have identified novel indole carboxamide compounds, which are inhibitors of kinase activity, in particular IKK2 activity. Such indole carboxamide derivatives are therefore of potential benefit in the treatment of disorders associated with inappropriate kinase, suitably inappropriate IKK2 activity, in particular in the treatment and prevention of disease states mediated by IKK2 mechanisms including inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, ankylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

In one aspect of the present invention, there is provided a compound of Formula (I): 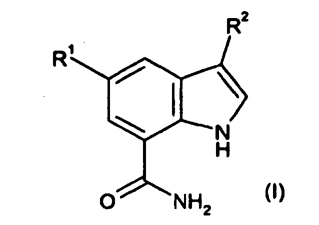 wherein R¹ represents H, halogen, or a group -YZ;
Y represents a bond (i.e. is absent), C₁₋₆ alkylene or C₂₋₆ alkenylene;
Z represents an aryl or heteroaryl group each comprising 5-14 ring members, said aryl or heteroaryl being optionally substituted by one or more substituents independently selected from halogen, OH, C₁₋₆ alkyl, C₁₋₆ haloalkyl, C₁₋₆ alkoxy, C₁₋₆ haloalkoxy, CN, C₁₋₆ hydroxyalkyl, phenyl, O-(CH₂)₁₋₆-phenyl, NHSO₂R³, NHCOR³, CONR⁴R⁵, SO₂NR⁴R⁵;
R³, R⁴ and R⁵ independently represent H or C₁₋₆ alkyl;
R² represents H, halogen or a group -Y¹Z¹**;**
Y¹ represents a bond (i.e. is absent), C₁₋₆ alkylene, C₂₋₆ alkenylene;
Z¹ represents a 6 membered aryl, 5 or 6 membered heteroaryl, 5-7 membered heterocyclyl, C₅₋₇ cycloalkyl, C₅₋₇ cycloalkenyl, each of which may be optionally substituted by one or more substituents independently selected from SO₂R⁶, NHSO₂R⁶, 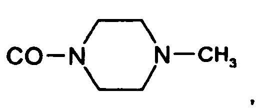 COR⁷, NR⁷R⁸, SO₂NR⁷R⁸, C₁₋₆ alkyl, C₁₋₆ haloalkyl, C₁₋₆ alkoxy, C₁₋₆ haloalkoxy, halogen, CONR⁷R⁸, NHCOR⁷, or phenyl (directly attached or attached by a C₁₋₆alkylene, CONH, C₂₋₆ alkenylene spacer and optionally substituted by one or more substituent selected from 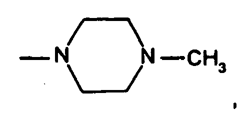 C₁₋₆ alkyl, C₁₋₆ alkoxy, C₁₋₆ haloalkyl, C₁₋₆ haloalkoxy, OH, halogen);
R⁶ represents H, C₁₋₆ alkyl, -(CH₂)ₙ phenyl or -(CH₂)ₙ napthyl (where n is 0 or 1 and each of which phenyl or naphthyl may be optionally substituted by one or more substitutents independently selected from C₁₋₆ alkyl, C₁₋₆ alkoxy, halogen, NR⁷R⁸, C₁₋₆ haloalkyl, C₁₋₆ haloalkoxy), CN or -(O)ₚ phenyl (where p is 0 or 1 and the phenyl is optionally substituted by one or more substitutents independently selected from halogen, C₁₋₆ alkyl or C₁₋₆ alkoxy)):
R⁷ and R⁸ independently represents C₁₋₆ alkyl, H, C₁₋₆ alkylene NR⁹R¹⁰;
R⁹ and R¹⁰ independently represents C₁₋₆ alkyl, H;
with the proviso R¹ and R² do not both represent H;
or a salt thereof.

In a second aspect of the present invention, there is provided a compound of formula (I), or a salt thereof for use in therapy, in particular in the treatment of a disorder associated with inappropriate kinase, in particular inappropriate IKK2 activity.

In a third aspect of the present invention, there is provided a pharmaceutical composition comprising a therapeutically effective amount of a compound of formula (I) or a salt thereof and one or more of pharmaceutically acceptable carriers, diluents and excipients.

In a fourth aspect of the present invention, there is provided the use of a compound of formula (I), or a salt thereof in the preparation of a medicament for use in the treatment of a disorder mediated by inappropriate IKK2 activity.

In a fifth aspect there is provided the use of a compound of formula (I) or a salt thereof in the manufacture of a medicament for the treatment of inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, ankylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein "a compound of the invention" or "a compound of formula (I)" means a compound of formula (I) or a pharmaceutically acceptable salt thereof.

As used herein the term "alkyl" refers to a straight- or branched-chain hydrocarbon radical having the specified number of carbon atoms, so for example, as used herein, the terms "C₁-C₃ alkyl" and "C₁-C₆ alkyl" refer to an alkyl group, as defined above, containing at least 1, and at most 3 or 6 carbon atoms respectively. Examples of such branched or straight-chained alkyl groups useful in the present invention include, but are not limited to, methyl, ethyl, n-propyl, isopropyl, isobutyl, n-butyl, t-butyl, n-pentyl, isopentyl, and n-hexyl.

As used herein, the term "alkylene" refers to a straight or branched chain divalent hydrocarbon radical having the specified number of carbon atom, so for example, as used herein, the terms "C₁-C₃ alkylene" and "C₁-C₆ alkylene" refer to an alkylene group, as defined above, which contains at least 1, and at most 3 or 6, carbon atoms respectively. Examples of "C₁-C₆ alkylene" and "C₁-C₆ alkylene" groups useful in the present invention include, but are not limited to, methylene, ethylene, n-propylene, n-butylene, isopentylene, and the like.

As used herein, the term "alkenyl" (and "alkenylene") refers to straight or branched hydrocarbon chains containing the specified number of carbon atoms and at least one and up to 3 carbon-carbon double bonds. Examples include ethenyl (and ethenylene) and propenyl (and propenylene).

As used herein, the term "halogen" refers to fluorine (F), chlorine (Cl), bromine (Br), or iodine (I) and the term "halo" refers to the halogen radicals: fluoro (-F), chloro (-Cl), bromo(-Br), and iodo(-I).

As used herein, the term "C₁-C₆ haloalkyl" refers to a straight or branched chain alkyl group as defined above containing at least 1, and at most 6 carbon atoms respectively substituted with at least one halo group, halo being as defined herein. Examples of such branched or straight chained haloalkyl groups useful in the present invention include, but are not limited to, methyl, ethyl, propyl, isopropyl, isobutyl and n-butyl substituted independently with one or more halos, *e.g.*, fluoro, chloro, bromo and iodo.

As used herein, the term "cycloalkyl" refers to a non-aromatic cyclic hydrocarbon ring containing the specified number of carbon atoms so, for example, the term "C₅-C₇ cycloalkyl" refers to a non-aromatic cyclic hydrocarbon ring having from five to seven carbon atoms. Exemplary "C₅-C₇ cycloalkyl" groups useful in the present invention include, but are not limited to, cyclopentyl, cyclohexyl and cycloheptyl.

As used herein, the term "cycloalkenyl" refers to a non-aromatic monocyclic carboxycyclic ring having the specified number of carbon atoms and up to 3 carbon-carbon double bonds. "Cycloalkenyl" includes by way of example cyclopentenyl and cyclohexenyl.

As used herein, the term "heterocyclic" or the term "heterocyclyl" refers to a non-aromatic heterocyclic ring containing the specified number ring atoms being saturated or having one or more degrees of unsaturation, containing one or more heteroatom substitutions selected from O and/or N. Such a ring may be optionally fused to one or more other "heterocyclic" ring(s) or cycloalkyl ring(s). Examples of "heterocyclic" moieties include, but are not limited to, tetrahydrofuran, pyran, 1,4-dioxane, 1,3-dioxane, piperidine, piperazine, 2,4-piperazinedione, pyrrolidine, imidazolidine, pyrazolidine, morpholine, thiomorpholine, tetrahydrothiopyran, tetrahydrothiophene, and the like.

As used herein, the term "aryl" refers to monocyclic carbocyclic groups and fused bicyclic carbocyclic groups having the specified number of carbon atoms and having at least one aromatic ring. Examples of aryl groups include phenyl and naphthyl.

As used herein, the term "heteroaryl" refers to an aromatic monocyclic ring, or to a fused bicyclic or tricyclic ring system wherein at least one ring is aromatic, having the specified number of ring atoms and containing at least one heteratom selected from N, O, and/or S. Examples of "heteroaryl" groups used herein include furanyl, thiophenyl, pyrrolyl, imidazolyl, pyrazolyl, triazolyl, tetrazolyl, thiazolyl, oxazolyl, isoxazolyl, oxadiazolyl, oxopyridyl, thiadiazolyl, isothiazolyl, pyridyl, pyridazyl, pyrazinyl, pyrimidyl, quinolinyl, isoquinolinyl, benzofuranyl, benzothiophenyl, indolyl, indazolyl.

As used herein, the term "alkoxy" refers to the group RₐO-, where Rₐ is alkyl as defined above and the terms "C₁-C₃ alkoxy" and "C₁-C₆ alkoxy" refer to an alkoxy group as defined herein wherein the alkyl moiety contains at least 1, and at most 3 or 6, carbon atoms. Exemplary "C₁₋C₃ alkoxy" and "C₁₋C₆ alkoxy" groups useful in the present invention include, but are not limited to, methoxy, ethoxy, n-propoxy, isopropoxy, n-butoxy, and t-butoxy.

As used herein the term "hydroxyalkyl refers to the group -RₐOH, where Rₐ is an alkylene group as defined above.

As used herein, the term "haloalkoxy" refers to the group RₐO-, where Rₐ is haloalkyl as defined above and the term "C₁-C₆ haloalkoxy" refers to a haloalkoxy group as defined herein wherein the haloalkyl moiety contains at least 1, and at most 6, carbon atoms. Exemplary C₁-C₆ haloalkoxy groups useful in the present invention include, but is not limited to, trifluoromethoxy.

In one embodiment R¹ is -YZ.

In one embodiment Y is a bond (i.e. is absent) or -CH = CH-. In a particular aspect, Y is a bond.

In one embodiment, Z is phenyl (which may be unsubstituted or substituted once or twice with substituents independently selected from C₁₋₃ alkoxy, CN, OH, phenyl, -OCH₂ phenyl NHSO₂R³, NHCOR³, CONR⁴R⁵, SO₂NR⁴R⁵, halogen, C₁₋₃ hydroxyalkyl, C₁₋₄ alkyl) or a heteroaryl group selected from benzofuranyl, quinolinyl, 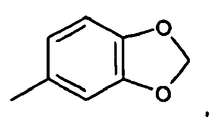 pyrimidinyl, thiophenyl, benzothiophenyl, isoxazolyl, pyridinyl (each of which may be optionally substituted by one or two groups independently selected from C₁₋₃ alkyl, C₁₋₃ alkoxy, halogen.

In a further embodiment, Z is phenyl (which is unsubstituted or substituted once by a substituent selected from phenyl, OCH₂ phenyl, NHSO₂CH₃, NHCOCH₃, CONH₂, CON(CH₃)₂, Cl, F, OCH₃, CN, OH, CH₂OH, CH₃, C(CH₃)₃) or a heterocyclic group selected from benzofuranyl, quinolinyl, 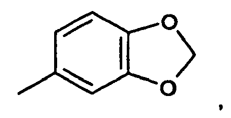 pyrimidinyl, thiophenyl, benzothiophenyl, isoxazolyl, pyridinyl (each of which, is substituted or is substituted once by a group selected from -OCH₃, CH₃, F).

In a further embodiment, Z is phenyl (which is unsubstituted or substituted once by a substituent selected from phenyl, OCH₂ phenyl, NHSO₂CH₃, NHCOCH₃, CONH₂, CON(CH₃)₂, Cl, F, OCH₃, CN, OH, CH₂OH, CH₃, C(CH₃)₃).

In a further embodiment Z is phenyl.

In one embodiment, R² is H or Y¹Z¹.

In one embodiment, R² is Y¹Z¹.

In one embodiment Y¹ is a bond, (i.e. is absent), or C₁₋₃ alkylene.

In one embodiment Y¹ is a bond.

In one embodiment Z' is H, halogen, phenyl (unsubstituted or substituted by one substituent selected from NHSO₂R⁶, CONR⁷R⁸, CF₃, C₁₋₃ alkoxy, SO₂R⁶, NHCOR⁷, SO₂NR⁷R⁸, NR⁷R⁸) or a 6 membered heterocyclic group which contains one nitrogen atom (which is unsubstituted or substituted one time by a group selected from C₁₋₃ alkyl, -CH₂ phenyl, SO₂R⁶, CONR⁷R⁸).

In one embodiment Z¹ is a 6 membered heterocyclyl optionally substituted by one or more substituents as described above. Preferably Z¹ is a 6 membered heterocyclyl substituted by -SO₂R⁶ wherein R⁶ is as defined above. Most preferably the 6 membered heterocyclyl is 4-piperidyl.

In a further aspect of the present invention, there is provided a compound of Formula (Ia): 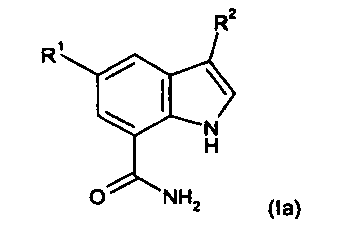 wherein R¹ represents H, halogen, or a group -YZ;
Y represents a bond (i.e. is absent), C₁₋₆ alkylene or C₂₋₆ alkenylene;
Z represents an aryl or heteroaryl group each comprising 5-14 ring members, said aryl or heteroaryl being optionally substituted by one or more substituents independently selected from halogen, OH, C₁₋₆ alkyl, C₁₋₆ haloalkyl, C₁₋₆ alkoxy, C₁₋₈ haloalkoxy, CN, C₁₋₆ hydroxyalkyl, phenyl, O-C₁₋₆ alkyl-phenyl, NHSO₂R³, NHCOR³, CONR⁴R⁵, SO₂NR⁴R⁵;
R³, R⁴ and R⁵ independently represent H or C₁₋₆ alkyl;
R² represents H, halogen or a group -Y¹Z¹;
Y¹ represents a bond (i.e. is absent), C₁₋₆ alkylene, C₂₋₆ alkenylene;
Z¹ represents a 6 membered aryl, 5 or 6 membered heteroaryl, 5 - 7 membered heterocyclyl, C₅₋₇ cycloalkyl, C₅₋₇ cycloalkenyl, each of which may be optionally substituted by one or more substituents independently selected from SO₂R⁶, NHSO₂R⁶, COC₁₋₆, alkyl, C₁₋₆ alkyl, C₁₋₆ haloalkyl, C₁₋₆ alkoxy, C₁₋₆ haloalkoxy, halogen, CONR⁷R⁸, NHCOR⁷ or phenyl (directly attached or attached by a C₁₋₆alkylene, CONH, C₂₋₆ alkenylene spacer and optionally substituted by one or more substituent selected from 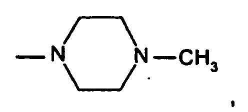 C₁₋₆ alkyl, C₁₋₆ alkoxy, C₁₋₆ haloalkyl, C₁₋₆ haloalkoxy, OH, halogen);
R⁶ represents H C₁₋₆ alkyl, phenyl (optionally substituted by C₁₋₆ alkyl, halogen, C₁₋₆ haloalkyl, C₁₋₆ haloalkoxy, C₁₋₆ alkoxy);
R⁷ and R⁸ independently represents C₁₋₆ alkyl, H, C₁₋₆ alkylene NR⁹R¹⁰;
R⁹ and R¹⁰ independently represents C₁₋₆ alkyl, H;
with the proviso R¹ and R² do not both represent H;
or a salt thereof.

While embodiments for each variable have generally been listed above separately for each variable, compounds of this invention includes those in each variable in formula (I) may be independently selected from each described embodiment for each variable. Therefore, this invention is intended to include all combinations of embodiments for each variable. Examples are described below.

Thus, in one embodiment, R¹ is YZ and R² is H or Br, wherein YZ includes all combinations of embodiments described above for Y and Z.

In a further embodiment, R¹ is phenyl or Br and R² is Y¹Z¹ wherein Y¹Z¹ includes all combinations of embodiments described above for Y¹ and Z¹.

In a further embodiment R¹ is YZ and R² is Y¹Z¹ wherein YZ and Y¹Z¹ include all combinations of embodiments described above for YZ and Y¹Z¹.

Specific examples of compounds of the present invention include the following:
5-phenyl-1H-indole-7-carboxamide;
5-(4-biphenylyl)-1H-indole-7-carboxamide;
5-{4-[(phenylmethyl)oxy]phenyl}-1H-indole-7-carboxamide;
5-{4-[(methylsulfonyl)amino]phenyl}-1H-indole-7-carboxamide;
5-[4-(acetylamino)phenyl]-1H-indole-7-carboxamide;
5-[3-(aminocarbonyl)phenyl]-1H-indole-7-carboxamide;
5-(4-chlorophenyl)-1H-indole-7-carboxamide;
5-[3-(acetylamino)phenyl]-1H-indole-7-carboxamide;
5-[3-(aminosulfonyl)phenyl]-1H-indole-7-carboxamide;
5-{3-[(dimethylamino)carbonyl]phenyl}-1H-indole-7-carboxamide;
5-(3-fluorophenyl)-1H-indole-7-carboxamide;
5-[3-(methyloxy)phenyl]-1H-indole-7-carboxamide;
5-(3-cyanophenyl)-1H-indole-7-carboxamide;
5-(3-hydroxyphenyl)-1H-indole-7-carboxamide;
5-(3-quinolinyl)-1H-indole-7-carboxamide;
5-(1-benzofuran-4-yl)-1H-indole-7-carboxamide;
5-(1,3-benzodioxol-5-yl)-1H-indole-7-carboxamide;
5-[(E)-2-phenylethenyl]-1H-indole-7-carboxamide;
5-(5-pyrimidinyl)-1H-indole-7-carboxamide;
5-(3-biphenylyl)-1H-indole-7-carboxamide;
5-(1-benzofuran-2-yl)-1H-indole-7-carboxamide;
5-(1-benzothien-2-yl)-1*H*-indole-7-carboxamide;
5-[3-(hydroxymethyl)phenyl]-1H-indole-7-carboxamide;
5-(2-naphthalenyl)-1H-indole-7-carboxamide;
5-(4-fluorophenyl)-1H-indole-7-carboxamide;
5-[6-(methyloxy)-3-pyridinyl]-1H-indole-7-carboxamide;
5-[4-(hydroxymethyl)phenyl]-1H-indole-7-carboxamide;
5-(3-chlorophenyl)-1H-indole-7-carboxamide;
5-(2-methylphenyl)-1H-indole-7-carboxamide;
5-{3-[(phenylmethyl)oxy]phenyl}-1H-indole-7-carboxamide;
5-(2-chlorophenyl)-1H-indole-7-carboxamide;
5-(3,5-dimethyl-4-isoxazolyl)-1H-indole-7-carboxamide;
5-{2-[(phenylmethyl)oxy]phenyl}-1H-indole-7-carboxamide;
5-(5-quinolinyl)-1H-indole-7-carboxamide;
5-(1-naphthalenyl)-1H-indole-7-carboxamide;
3-bromo-5-phenyl-1H-indole-7-carboxamide;
3-iodo-5-phenyl-1H-indole-7-carboxamide;
3,5-diphenyl-1H-indole-7-carboxamide;
3-{4-[(methylsulfonyl)amino]phenyl}-5-phenyl-1H-indole-7-carboxamide;
5-phenyl-3-(3-pyridinyl)-1*H*-indole-7-carboxamide;
3-(4-{[(2-aminoethyl)amino]carbonyl}phenyl)-5-phenyl-1*H*-indole-7-carboxamide;
3-[4-({[4-(methyloxy)-3-(4-methyl-1-piperazinyl)phenyl]amino}carbonyl)phenyl]-5-phenyl-1H-indole-7-carboxamide formate;
5-phenyl-3-[3-(trifluoromethyl)phenyl]-1H-indole-7-carboxamide;
5-bromo-3-iodo-1H-indole-7-carboxamide;
3-(1-ethyl-3-piperidinyl)-5-phenyl-1H-indole-7-carboxamide;
5-phenyl-3-(3-piperidinyl)-1H-indole-7-carboxamide;
5-phenyl-3-[1-(phenylmethyl)-3-piperidinyl-1H-indole-7-carboxamide;
3-(1-cyclohexen-1-yl)-5-phenyl-1H-indole-7-carboxamide;
3-cyclohexyl-5-phenyl-1 H-indole-7-carboxamide;
3-{1-[3-(methyloxy)phenyl]ethenyl}-5-phenyl-1H-indole-7-carboxamide;
5-phenyl-3-[1-(phenylmethyl)-1,2,3,6-tetrahydro-4-pyridinyl]-1H-indole-7-carbox-amide;
5-phenyl-3-(4-piperidinyl)-1H-indole-7-carboxamide;
3-{1-[(4-chlorophenyl)sulfonyl]-4-piperidinyl}-5-phenyl-1H-indole-7-carboxamide;
5-phenyl-3-[1-(propylsulfonyl)-4-piperidinyl-1H-indole-7-carboxamide;
3-(1-acetyl-4-piperidinyl)-5-phenyl-1H-indole-7-carboxamide;
3-[1-(N,N-dimethyl-β-alanyl)-4-piperidinyl]-5-phenyl-1H-indole-7-carboxamide;
3-(1-ethyl-4-piperidinyl)-5-phenyl-1H-indole-7-carboxamide formate;
3-(1-methylpyrrolidin-2-yl)-5-phenyl-1*H*-indole-7-carboxamide;
3-[1-(ethylsulfonyl)pyrrolidin-3-yl]-5-phenyl-1*H*-indole-7-carboxamide;
3-[4-(methylsulfonyl)phenyl]-5-phenyl-1*H*-indole-7-carboxamide;
3-[3-(acetylamino)phenyl]-5-phenyl-1*H*-indole-7-carboxamide;
3-[4-(ethylsulfonyl)phenyl]-5-phenyl-1*H*-indole-7-carboxamide;
**3-**[3-(methylsulfonyl)phenyl]-5-phenyl-1*H*-indole-7-carboxamide;
3-(hexahydro-1*H*-azepin-4-yl)-5-phenyl-1*H*-indole-7-carboxamide;
3-[1-(ethylsulfonyl)hexahydro-1*H*-azepin-4-yl]-5-phenyl-1*H*-indole-7-carbox-amide;
5-phenyl-3-[2-(4-pyridinyl)ethyl]-1*H*-indole-7-carboxamide;
3-{[1-(ethylsulfonyl)-4-piperidinylidene]methyl}-5-phenyl-1*H*-indole-7-carboxamide;
3-[1-(ethylsulfonyl)-4-piperidinyl]-5-[4-(hydroxymethyl)phenyl]-1*H*-indole-7-carboxamide;
5-phenyl-3-(3-piperidinylmethyl)-1*H*-indole-7-carboxamide;
5-phenyl-3-[2-(4-piperidinyl)ethyl]-1*H*-indole-7-carboxamide;
3-{2-[1-(ethylsulfonyl)-4-piperidinyl]ethyl}-5-phenyl-1*H*-indole-7-carboxamide;
3-{[1-(ethylsulfonyl)-3-piperidinyl]methyl}-5-phenyl-1*H*-indole-7-carboxamide;
3-{[1-(ethylsulfonyl)-4-piperidinyl]methyl}-5-phenyl-1*H*-indole-7-carboxamide;
3-{1-[(2)sulfonyl]-4-piperidinyl)-5-phenyl-1*H*-indole-7-carboxamide;
3-{1-[(4-fluorophenyl)sulfonyl]-4-piperidinyl}-5-phenyl-1*H*-indole-7-carboxamide;
3-{1-[(4-methylphenyl)sulfonyl]-4-piperidinyl}-5-phenyl-1*H*-indole-7-carboxamide;
3-{phenylsulfonyl-4-piperidinyl}-5-phenyl-1*H*-indole-7-carboxamide;
3-(1-{[4-(methyloxy)phenyl]sulfonyl}-4-piperidinyl)-5-phenyl-1*H*-indole-7-carboxamide;
3-[1-(ethanesulfonyl)-4-piperidinyl]-5-phenyl-1*H*-indole-7-carboxamide;
3-{1-[(2-propanesulfonyl]-4-piperidinyl}-5-phenyl-1*H*-indole-7-carboxamide;
5-phenyl-3-[1-(propanesulfonyl)-1,2,3,6-tetrahydro-4-pyridinyl]-1*H*-indole-7-carboxamide;
5-phenyl-3-(1-{[4-(trifluoromethyl)phenyl]sulfonyl}-4-piperidinyl)-1*H*-indole-7-carboxamide;
3-{1-[(2,4-dichlorophenyl)sulfonyl]-4-piperidinyl)-5-phenyl-1*H*-indole-7-carboxamide;
3-{1-[(3,4-dichlorophenyl)sulfonyl]-4-piperidinyl}-5-phenyl-1*H*-indole-7-carboxamide;
3-{1-[(ethylamino)carbonyl]-4-piperidinyl}-5-phenyl-1*H*-indole-7-carboxamide;
3-{1-[(4-1-piperazinyl)carbonyl]-4-piperidinyl}-5-phenyl-1*H*-indole-7-carboxamide;
5-(4-chlorophenyl)-3-[1-(propanesulfonyl)-4-piperidinyl]-1*H*-indole-7-carboxamide;
3-{1-[(4-fluorophenyl)sulfonyl]-4-piperidinyl}-5-(4-chlorophenyl)-1*H*-indole-7-carboxamide;
5-{4-[(methylsulfonyl)amino]phenyl}-3-[1-(phenylmethyl)-1,2,3,6-tetrahydro-4-pyridinyl]-1*H*-indole-7-carboxamide;
5-{4-[(methylsulfonyl)amino]phenyl}-3-(4-piperidinyl)-1*H*-indole-7-carboxamide;
5-bromo-3-[1-(ethanesulfonyl)-4-piperidinyl)-1*H*-indole-7-carboxamide;
3-[1-(ethanesulfonyl)-4-piperidinyl]-5-{4-[(methylsulfonyl)amino]phenyl}-1*H*-indole-7-carboxamide;
3-[1-(ethanesulfonyl)-4-piperidinyl]-5-(3-methylphenyl)-1*H*-indole-7-carboxamide;
3-[1-(ethylsulfonyl)-4-piperinyl]-5-(2-thienyl)-1*H*-indole-7-carboxamide;
3-[1-(ethylsulfonyl)-4-piperidinyl]-5-(3-thienyl)-1*H*-indole-7-carboxamide;
3-[4-(methylsulfonyl)phenyl]-5-phenyl-1H-indole-7-carboxamide;
3-{4-[(dimethylamino)sulfonyl]phenyl}-5-phenyl-1H-indole-7-carboxamide;
3-{3-[(methylsulfonyl)amino]phenyl}-5-phenyl-1H-indole-7-carboxamide;
3-[1-(ethylsulfonyl)-4-piperidinyl]-5-{3-[(methylsulfonyl)amino]phenyl}-1*H*-indole-7-carboxamide;
5-[4-(acetylamino)phenyl]-3-[1-(ethylsulfonyl)-4-piperidinyl]-1*H*-indole-7-carboxamide;
5-{4-[(dimethylamino)sulfonyl]phenyl}-3-[1-(ethylsulfonyl)-4-piperidinyl]-1*H*-indole-7-carboxamide;
5-[3-(acetylamino)phenyl]-3-[1-(ethylsulfonyl)-4-piperidinyl]-1*H*-indole-7-carbox-amide;
3-[1-(ethylsulfonyl)-4-piperidinyl]-5-(1*H*-pyrazol-4-yl)-1*H*-indole-7-carboxamide;
3-[1-(ethylsulfonyl)-4-piperidinyl]-5-[3-(hydroxymethyl)phenyl]-1*H*-indole-7-carboxamide;
5-(2,4-difluorophenyl)-3-[1-(ethylsulfonyl)-4-piperidinyl]-1*H*-indole-7-carboxamide;
3-[1-(ethylsulfonyl)-4-piperidinyl]-5-[4-(methyloxy)phenyl]-1*H*-indole-7-carboxamide;
3-[1-(ethylsulfonyl)-4-piperidinyl]-5-(4-fluoro-2-methylphenyl)-1*H*-indole-7-carboxamide;
3-[1-(ethylsulfonyl)-4-piperidinyl]-5-(4-fluorophenyl)-1*H*-indole-7-carboxamide;
5-(4-biphenylyl)-3-[1-(ethylsulfonyl)-4-piperidinyl]-1*H*-indole-7-carboxamide;
5-[4-(1,1-dimethylethyl)phenyl]-3-[1-(ethylsulfonyl)-4-piperidinyl]-1*H*-indole-7-carboxamide;
3-[1-(ethylsulfonyl)-4-piperidinyl]-5-(4-methylphenyl)-1*H*-indole-7-carboxamide;
3-[1-(ethylsulfonyl)-4-piperidinyl]-5-(4-pyridinyl)-1*H*-indole-7-carboxamide;
3-[1-(ethylsulfonyl)-4-piperidinyl]-5-(6-fluoro-3-pyridinyl)-1*H*-indole-7-carboxamide;
3-[1-(ethylsulfonyl)-4-piperidinyl]-5-(6-methyl-3-pyridinyl)-1*H*-indole-7-carboxamide;
3-[1-(ethylsulfonyl)-4-piperidinyl]-5-(4-methyl-3-pyridinyl)-1*H*-indole-7-carboxamide;
3-[1-(ethylsulfonyl)-4-piperidinyl]-5-[6-(methyloxy)-3-pyridinyl]-1*H*-indole-7-carboxamide;
5-phenyl-3-(*N*-acetyl-3-piperidinylmethyl)-1*H*-indole-7-carboxamide;
5-[3-(ethyloxy)phenyl]-3-[1-(ethylsulfonyl)-4-piperidinyl]-1*H*-indole-7-carboxamide;
3-[1-(ethylsulfonyl)-4-piperidinyl]-5-(2-fluorophenyl)-1*H*-indole-7-carboxamide;
3-[1-(ethylsulfonyl)-4-piperidinyl]-5-[3-(trifluoromethyl)phenyl]-1*H*-indole-7-carboxamide;
3-[1-(ethylsulfonyl)-4-piperidinyl]-5-[4-(trifluoromethyl)phenyl]-1*H*-indole-7-carboxamide;
3-[1-(ethylsulfonyl)-4-piperidinyl]-5-(3-fluorophenyl)-1*H*-indole-7-carboxamide;
5-(3,5-dichlorophenyl)-3-[1-(ethylsulfonyl)-4-piperidinyl]-1*H*-indole-7-carboxamide;
5-(3,4-difluorophenyl)-3-[1-(ethylsulfonyl)-4-piperidinyl]-1*H*-indole-7-carboxamide;
3-bromo-5-{3-[(dimethylamino)carbonyl]phenyl)-1*H*-indole-7-carboxamide;
5-[2,6-bis(methyloxy)phenyl]-3-bromo-1*H*-indole-7-carboxamide;
3-bromo-5-(4-fluoro-2-methylphenyl)-1*H*-indole-7-carboxamide;
3-bromo-5-[5-fluoro-2-(methyloxy)phenyl]-1*H*-indole-7-carboxamide;
3-bromo-5-(3-quinolinyl)-1*H*-indole-7-carboxamide trifluoroacetate;
3-bromo-5-(5-quinolinyl)-1*H*-indole-7-carboxamide trifluoroacetate;
5-[2,5-bis(methyloxy)phenyl]-3-bromo-1*H*-indole-7-carboxamide;
3-bromo-5-(2-fluorophenyl)-1*H*-indole-7-carboxamide;
5-[2,4-bis(methyloxy)phenyl]-3-bromo-1*H*-indole-7-carboxamide;
3-bromo-5-[2-(methyloxy)-3-pyridinyl]-1*H*-indole-7-carboxamide trifluoroacetate;
3-bromo-5-[2,3,4-tris(methyloxy)phenyl]-1*H*-indole-7-carboxamide;
3-{1-[(4-chloro-2,5-dimethylphenyl)sulfonyl]-4-piperidinyl}-5-phenyl-1*H*-indole-7-carboxamide;
3-(1-{[5-bromo-2-(methyloxy)phenyl]sulfonyl}-4-piperidinyl)-5-phenyl-1*H*-indole-7-carboxamide;
3-{1-[(5-fluoro-2-methylphenyl)sulfonyl]-4-piperidinyl}-5-phenyl-1*H*-indole-7-carboxamide;
3-{1-[(3-fluorophenyl)sulfonyl]-4-piperidinyl}-5-phenyl-1*H*-indole-7-carboxamide;
5-phenyl-3-(1-{[2,4,6-tris(1-methylethyl)phenyl]sulfonyl}-4-piperidinyl)-1*H*-indole-7-carboxamide;
3-(1-{[4-(1,1-dimethylpropyl)phenyl]sulfonyl}-4-piperidinyl)-5-phenyl-1*H*-indole-7-carboxamide;
3-{1-[(2-methylphenyl)sulfonyl]-4-piperidinyl}-5-phenyl-1*H*-indole-7-carboxamide;
3-{1-[(2-iodophenyl)sulfonyl]-4-piperidinyl}-5-phenyl-1*H*-indole-7-carboxamide;
3-{1-[(4-pentylphenyl)sulfonyl]-4-piperidinyl}-5-phenyl-1*H*-indole-7-carboxamide;
5-phenyl-3-{1-[(4-propylphenyl)sulfonyl]-4-piperidinyl}-1*H*-indole-7-carboxamide;
3-{1-[(2,4-difluorophenyl)sulfonyl]-4-piperidinyl}-5-phenyl-1*H* indole-7-carboxamide;
3-{1-[(2,5-dimethylphenyl)sulfonyl]-4-piperidinyl}-5-phenyl-1*H*-indole-7-carboxamide;
3-{1-[(4-ethylphenyl)sulfonyl]-4-piperidinyl}-5-phenyl-1*H*-indole-7-carboxamide;
3-{1-[(3-methylphenyl)sulfonyl]-4-piperidinyl}-5-phenyl-1*H*-indole-7-carboxamide;
3-{1-[(4-([2-(methyloxy)phenyl]oxy}phenyl)sulfonyl]-4-piperidinyl}-5-phenyl-1*H*-indole-7-carboxamide;
3-{1-[(4-{[4-(methyloxy)phenyl]oxy}phenyl)sulfonyl]-4-piperidinyl}-5-phenyl-1*H*-indole-7-carboxamide;
3-[1-({3-[(4-fluorophenyl)oxy]phenyl}sulfonyl)-4-piperidinyl]-5-phenyl-1*H*-indole-7-carboxamide;
3-{1-[(3-{[2-(methyloxy)phenyl]oxy}phenyl)sulfonyl]-4-piperidinyl}-5-phenyl-1*H*-indole-7-carboxamide;
3-[1-({4-[(4-chlorophenyl)oxy]phenyl}sulfonyl)-4-piperidinyl]-5-phenyl-1*H*-indole-7-carboxamide;
3-{1-[(3-{[4-(methyloxy)phenyl]oxy}phenyl)sulfonyl]-4-piperidinyl}-5-phenyl-1*H*-indole-7-carboxamide;
5-phenyl-3-(1-{[3-(phenyloxy)phenyl]sulfonyl}-4-piperidinyl)-1*H*-indole-7-carboxamide;
3-[1-({3-[(4-chlorophenyl)oxy]phenyl}sulfonyl)-4-piperidinyl]-5-phenyl-1*H*-indole-7-carboxamide;
3-[1-({4-[(2-methylphenyl)oxy]phenyl}sulfonyl)-4-piperidinyl]-5-phenyl-1*H*-indole-7-carboxamide;
3-{1-[(4'-chloro-4-biphenylyl)sulfonyl]-4-piperidinyl}-5-phenyl-1*H*-indole-7-carboxamide;
3-[1-({3-[(2-methylphenyl)oxy]phenyl}sulfonyl)-4-piperidinyl]-5-phenyl-1*H*-indole-7-carboxamide;
3-[1-({3-[(2-chlorophenyl)oxy]phenyl}sulfonyl)-4-piperidinyl]-5-phenyl-1*H*-indole-7-carboxamide;
3-{1-[(5-chloro-1-naphthalenyl)sulfonyl]-4-piperidinyl}-5-phenyl-1*H*-indole-7-carboxamide;
3-(1-{[4'-(methyloxy)-3-biphenylyl]sulfonyl}-4-piperidinyl)-5-phenyl-1*H*-indole-7-carboxamide;
3-[1-(3-biphenylylsulfonyl)-4-piperidinyl]-5-phenyl-1*H*-indole-7-carboxamide;
3-(1-{[(4-fluorophenyl)methyl]sulfonyl}-4-piperidinyl)-5-phenyl-1*H*-indole-7-carboxamide;
3-{1-[(5-chloro-2-naphthalenyl)sulfonyl]-4-piperidinyl}-5-phenyl-1*H*-indole-7-carboxamide;
3-(1-{[4'-(methyloxy)-4-biphenylyl]sulfonyl}-4-piperidinyl)-5-phenyl-1*H*-indole-7-carboxamide;
5-(2-fluorophenyl)-1*H*-indole-7-carboxamide;
5-(3-{[(2,2-dimethylpropyl)amino]carbonyl}phenyl)-1*H*-indole-7-carboxamide;
5-(3-{[(1-methylethyl)amino]carbonyl}phenyl)-1*H*-indole-7-carboxamide;
5-(4-{[(2,2-dimethylpropyl)amino]carbonyl}phenyl)-1*H*-indole-7-carboxamide;
5-{4-[(propylamino)carbonyl]phenyl}-1*H*-indole-7-carboxamide;
5-(4-{[(1-methylethyl)amino]carbonyl}phenyl)-1*H*-indole-7-carboxamide;
5-{4-[(diethylamino)carbonyl]phenyl}-1*H*-indole-7-carboxamide ;
3-[1-(methylsulfonyl)-1,2,3,6-tetrahydro-4-pyridinyl]-5-phenyl-1*H*-indole-7-carboxamide;
3-(3-oxocyclopentyl)-5-phenyl-1*H*-indole-7-carboxamide;
5-phenyl-3-{3-[(phenylmethyl)amino]cyclopentyl}-1*H*-indole-7-carboxamide;
3-(3-aminocyclopentyl)-5-phenyl-1*H*-indole-7-carboxamide;
3-{3-[(ethylsulfonyl)amino]cyclopentyl}-5-phenyl-1*H*-indole-7-carboxamide;
5-bromo-3-[1-(propylsulfonyl)-4-piperidinyl]-1*H*-indole-7-carboxamide;
5-bromo-3-(3-pyridinyl)-1H-indole-7-carboxamide;
5-bromo-3-[1-(methylsulfonyl)-1,2,3,6-tetrahydro-4-pyridinyl]-1*H*-indole-7-carboxamide;
3-[(4-hydroxyphenyl)methyl]-5-phenyl-1H-indole-7-carboxamide;
5-bromo-1H-indole-7-carboxamide;
5-(4-chlorophenyl)1H-indole-7-carboxamide;
5-bromo-3(4-piperidinyl)1H-indole-7-carboxamide;
or a salt thereof.

As used herein, the term "optionally" means that the subsequently described event(s) may or may not occur, and includes both event(s), which occur, and events that do not occur.

As used herein, the term "substituted" refers to substitution with the named substituent or substituents, multiple degrees of substitution being allowed unless otherwise stated.

Certain of the compounds described herein may contain one or more chiral atoms, or may otherwise be capable of existing as two enantiomers. The compounds of this invention include mixtures of enantiomers as well as purified enantiomers or enantiomerically enriched mixtures. Also included within the scope of the invention are the individual isomers of the compounds represented by formula (I) above as well as any wholly or partially equilibrated mixtures thereof. The present invention also covers the individual isomers of the compounds represented by the formulas above as mixtures with isomers thereof in which one or more chiral centers are inverted. Also, it is understood that any tautomers and mixtures of tautomers of the compounds of formula (I) are included within the scope of the compounds of formula (I).

The present invention also covers salt of the compounds of formula (I). Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in the compound of formula (I). Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

While it is possible that, for use in therapy, therapeutically effective amounts of a compound of formula (I), as well as salts thereof, may be administered as the raw chemical, it is possible to present the active ingredient as a pharmaceutical composition accordingly, the invention further provides a pharmaceutical composition, which comprises a compound of formula (I) and salts thereof, and one or more pharmaceutically acceptable carriers, diluents, or excipients. The compounds of the formula (I) and salts thereof, are as described above. The carrier(s), diluent(s) or excipient(s) must be acceptable in the sense of being compatible with the other ingredients of the formulation and not deleterious to the recipient thereof. In accordance with another aspect of the invention there is also provided a process for the preparation of a pharmaceutical composition including admixing a compound of the formula (I), or salts thereof, with one or more pharmaceutically acceptable carriers, diluents or excipients.

Pharmaceutical compositions may be presented in unit dose forms containing a predetermined amount of active ingredient per unit dose. Such a unit may contain, for example, 0.5mg to 1g, preferably 1mg to 700mg, more preferably 5mg to 100mg of a compound of the formula (I), depending on the condition being treated, the route of administration and the age, weight and condition of the patient, or pharmaceutical compositions may be presented in unit dose forms containing a predetermined amount of active ingredient per unit dose. Preferred unit dosage compositions are those containing a daily dose or sub-dose, as herein above recited, or an appropriate fraction thereof, of an active ingredient. Furthermore, such pharmaceutical compositions may be prepared by any of the methods well known in the pharmacy art.

Pharmaceutical compositions may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture, as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, com sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging,steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit compositions for oral administration can be microencapsulated. The formulation can also be prepared to prolong or sustain the release as for example by coating or embedding particulate material in polymers, wax or the like.

The compounds of formula (I), and salts thereof, can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of formula (I) and salts thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide -phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils.

For treatments of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical compositions adapted for topical administrations to the eye include eye drops wherein the active ingredient is dissolved or suspended in a suitable carrier, especially an aqueous solvent.

Pharmaceutical compositions adapted for topical administration in the mouth include lozenges, pastilles and mouth washes.

Pharmaceutical compositions adapted for rectal administration may be presented as suppositories or as enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists, which may be generated by means of various types of metered, dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the compositions may include other agents conventional in the art having regard to the type of composition in question, for example those suitable for oral administration may include flavouring agents.

A therapeutically effective amount of a compound of the present invention will depend upon a number of factors including, for example, the age and weight of the animal, the precise condition requiring treatment and its severity, the nature of the formulation, and the route of administration, and will ultimately be at the discretion of the attendant physician or veterinarian. However, an effective amount of a compound of formula (I) for the treatment of neoplastic growth, for example colon or breast carcinoma, will generally be in the range of 0.1 to 100 mg/kg body weight of recipient (mammal) per day and more usually in the range of 1 to 10 mg/kg body weight per day. Thus, for a 70kg adult mammal, the actual amount per day would usually be from 70 to 700 mg and this amount may be given in a single dose per day or more usually in a number (such as two, three, four, five or six) of sub-doses per day such that the total daily dose is the same. An effective amount of a salt thereof, may be determined as a proportion of the effective amount of the compound of formula (I) per se. It is envisaged that similar dosages would be appropriate for treatment of the other conditions referred to above.

The compounds of formula (I) and salts thereof, are believed to have utility in inflammatory and tissue repair disorders, particularly rheumatoid arthritis, inflammatory bowel disease, asthma and COPD (chronic obstructive pulmonary disease);osteoarthritis,osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, ankylosing spondylitis, tissue and organ rejection, Alzheimer's disease, stroke, atherosclerosis, restenosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia as a result of inhibition of the protein kinase IKK2.

The present invention thus also provides compounds of formula (I) and pharmaceutically acceptable salts thereof, for use in medical therapy, and particularly in the treatment of disorders mediated by IKK2 activity.

The inappropriate IKK2 activity referred to herein is any IKK2 activity that deviates from the normal IKK2 activity expected in a particular mammalian subject. Inappropriate IKK2 activity may take the form of, for instance, an abnormal increase in activity, or an aberration in the timing and or control of IKK2 activity. Such inappropriate activity may result then, for example, from overexpression or mutation of the protein kinase leading to inappropriate or uncontrolled activation.

The present invention is directed to regulating, modulating, or inhibiting IKK2 for the prevention and/or treatment of disorders related to unregulated IKK2 activity. In particular, the compounds of the present invention can also be used in the treatment of certain forms of renal and cardiovascular disease as well as congestive heart failure.

A further aspect of the invention provides the use of a compound of formula (I) or a pharmaceutically acceptable salt thereof in the preparation of a medicament for use in the treatment of a mammal suffering from a disorder mediated by IKK2 activity. In a preferred embodiment, the disorder is a susceptible cancer.

A further aspect of the present invention provides the use of a compound of formula (I), or a pharmaceutically acceptable salt thereof, in the preparation of a medicament for the treatment of a disorder characterized by inappropriate IKK2 activity.

Particular disorders characterised by inappropriate IKK2 activity include inflammatory and tissue repair disorders, particularly rheumatoid arthritis, inflammatory bowel disease, asthma and COPD (chronic obstructive pulmonary disease);osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, ankylosing spondylitis, tissue and organ rejection, Alzheimer's disease, stroke, atherosclerosis, restenosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia as a result of inhibition of the protein kinase IKK2.

Particular disorders are an inflammatory or tissue repair disorder, most particularly rheumatoid arthritis, inflammatory bowel disease, asthma, and COPD (chronic obstructive pulmonary disease).

In a still further aspect, the disorder is selected from the group consisting of autoimmune diseases; tissue and organ rejection, Alzheimer's disease, stroke, atherosclerosis, restenosis, diabetes, glomerulonephritis, osteoarthritis, osteoporosis, and Ataxia Telangiestasia. Most particularly the disorder is an autoimmune disease including systemic lupus eythematosus, multiple sclerosis, psoriatic arthritis, or ankylosing spondylitis, diabetes.

In a still further aspect, the disease is cachexia or cancer, more particularly Hodgkins disease.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the Working Examples.

Compounds of general formula (I) may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (T. W. Green and P. G. M. Wuts (1991) Protecting Groups in Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of Formula (I). Those skilled in the art will recognize if a stereocenter exists in compounds of Formula (I). Accordingly, the present invention includes both possible stereoisomers and includes not only racemic compounds but the individual enantiomers as well. When a compound is desired as a single enantiomer, it may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

Compounds of Formula I can be prepared for example according to Scheme 1 depicted below and the Examples section following: 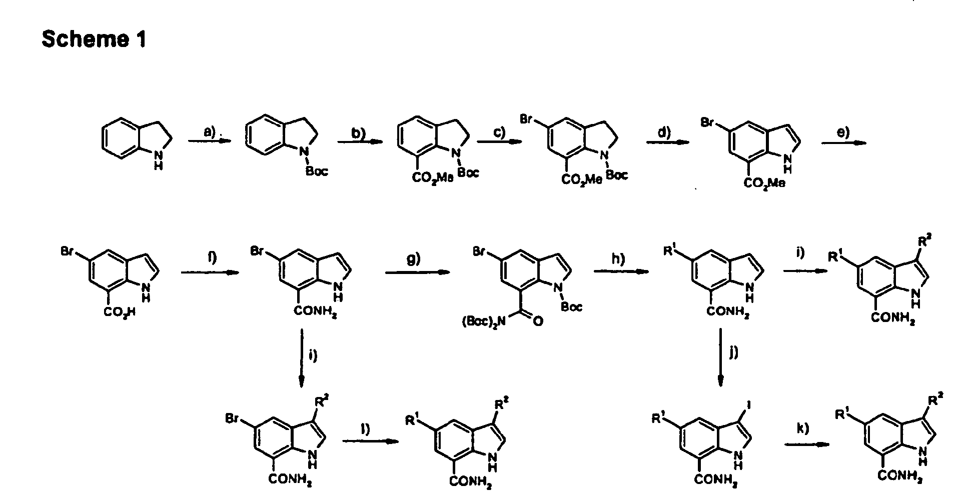
a) O[CO₂C(CH₃)₃]₂, THF, rt.
b)
   i) s-Buthyl lithium, TMEDA.
   ii) MeOC(O)CI, -78°C to r.
c) NBS, CH₂Cl₂, rt.
d)
   i) TFA, rt.
   ii) MnO₂, THF, rt.
e) LiOH(aq), MeOH, rt.
f) HATU, NH₃, DIPEA, Dioxane-CH₂Cl₂, rt.
g) O[CO₂C(CH₃)₃]_{2,} DMAP, CH₂,Cl₂, rt.
h) i) ArB(OH)₂, K₂CO₃, PdCl₂(dppf).CH₂Cl₂, DMF.
   ii) HCl, EtOH, 150°C.
i) i) represents a ketone or aldehyde condensation reaction which produces a compound where R² includes an alkyl derivative directly attached to the ring. KOH or NaOMe, MeOH, 65°.
j) NIS, CH₂Cl₂, rt.
k)
   i) represents a Suzuki coupling to introduce R² including aryl or heteroaryl moieties, e.g. O[CO₂C(CH₃)₃]₂, DMAP, CH₂Cl₂, rt.
   ii) ArylB(OH)₂, K₂CO₃, PdCl₂(dppf).CH₂Cl_{2,} DMF, 8.
   iii) HCl, EtOH, 150°C.
l) i) ArB(OH)₂, K₂CO₃ (or) CS₂CO₃, (Ph₃P)₄Pd, Dioxane, water.

Compounds of Formula I represented by example 57 may be prepared according to Scheme 2. 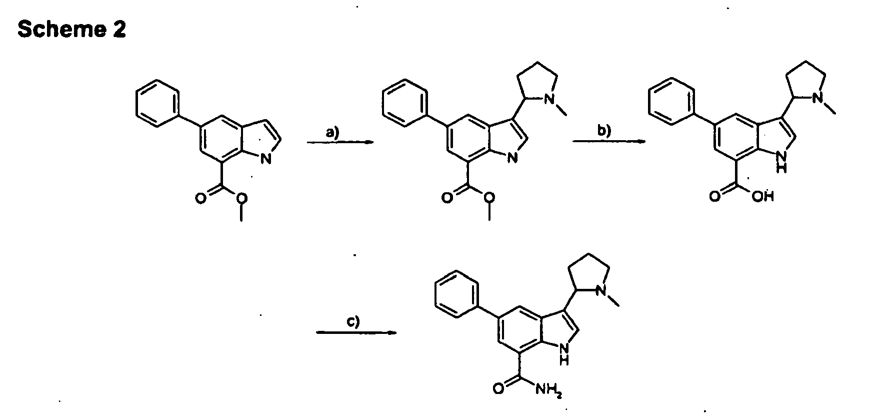
a).
   i) 1-methyl-2-pyrrolidine, POCl₃, 15°C to 80°C
   ii) EtOH, NaBH4
b). LiOH.2H₂O, MeOH, HCl, reflux
c). *O*-(7-Azabenzatriazol-1-yl)-*N,N,N',N'*-tetramethyluroniumhexafluorophosphate, NH₃, dioxane

Alternatively, compounds of Formula I represented by example 58 may be prepared according to Scheme 3. 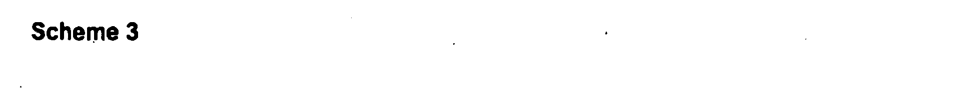 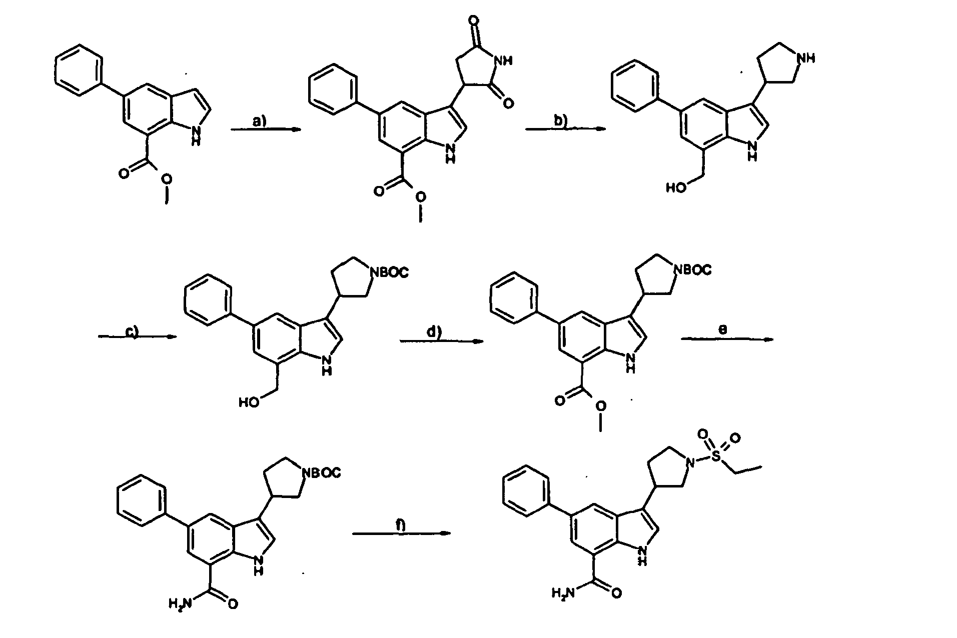
a) HOAc, H₃PO₄, maleimide, reflux
b) LAH, THF, reflux
c) Et₃N, (BOC)₂O, Dichloromethane
d)
   i) THF, MnO₂
   ii) Ag₂O, NaCN, MeOH, HCl
e)
   i) LiOH.2H₂O, MeOH
   ii) O-(7-Azabenzatriazol-1-yl)-*N,N,N'N*'-tetramethyluronium - hexafluorophosphate, NH₃, MeOH
f) i) TFA, THF
ii) CISO₂Et, Et₃N, DCM

Reaction conditions will be apparent to a skilled person and Examples of suitable conditions are further exemplified in the Examples Section below. It will also be evident from the above scheme that a compound of formula (I) may be converted into another compound of formula (I) using conventional procedures. It will also be evident to the skilled artisan that the reaction sequence may be rearranged from that depicted in the general scheme. Thus incorporation of substituent R² may occur before R¹. Alternatively, R¹ may be installed prior to the conversion of the carboxylic acid to the amide.

Certain embodiments of the present invention will now be illustrated by way of example only. The physical data given for the compounds exemplified is consistent with the assigned structure of those compounds.

### EXAMPLES

As used herein the symbols and conventions used in these processes, schemes and examples are consistent with those used in the contemporary scientific literature, for example, the *Journal of the American Chemical Society* or the *Journal of Biological Chemistry*. Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); . |
| µL (microliters); | psi (pounds per square inch); |
| M (molar); | mM (millimolar); |
| i. v. (intravenous); | Hz (Hertz); |
| MHz (megahertz); | mol (moles); |
| mmol (millimoles); | rt (room temperature); |
| min (minutes); | h (hours); |
| mp (melting point); | TLC (thin layer chromatography); |
| Tᵣ (retention time); | RP (reverse phase); |
| MeOH (methanol); | *i*-PrOH (isopropanol); |
| TEA (triethylamine); | TFA (trifluoroacetic acid); |
| TFAA (trifluoroacetic anhydride); | THF (tetrahydrofuran); |
| DMSO (dimethylsulfoxide); | AcOEt (ethyl acetate); |
| DME (1,2-dimethoxyethane); | DCM (dichloromethane); |
| DCE (dichloroethane); | DMF (*N,N*-dimethylformamide); |
| DMPU (*N,N*'-dimethylpropyleneurea); | CDI (1,1-carbonyldiimidazole); |
| IBCF (isobutyl chloroformate); | HOAc (acetic acid); |
| HOSu (*N*-hydroxysuccinimide); | HOBT (1-hydroxybenzotriazole); |
| mCPBA (meta-chloroperbenzoic acid; | |
| EDC (1-[3-dimethylamino) propyl]-3-ethylcarbodiimide hydrochloride); | |
| BOC (*tert*-butyloxycarbonyl); FMOC (9-fluorenylmethoxycarbonyl); | |
| DCC (dicyclohexylcarbodiimide); | CBZ (benzyloxycarbonyl); |
| Ac (acetyl); | atm (atmosphere); |
| TMSE (2-(trimethylsilyl)ethyl); | TMS (trimethylsilyl); |
| TIPS (triisopropylsilyl); | TBS (*t*-butyldimethylsilyl); |
| DMAP (4-dimethylaminopyridine); | BSA (bovine serum albumin) |
| ATP (adenosine triphosphate); | HRP (horseradish peroxidase); |
| DMEM (Dulbecco's modified Eagle medium); | |
| HPLC (high pressure liquid chromatography); | |
| BOP (bis(2-oxo-3-oxazolidinyl)phosphinic chloride); | |
| TBAF (tetra-*n*-butylammonium fluoride); | |
| HBTU(O-Benzotriazole-1-yl-N,N,N',N'-tetramethyluroniumhexafluoro phosphate). | |
| HEPES (4-(2-hydroxyethyl)-1-piperazine ethane sulfonic acid); | |
| DPPA (diphenylphosphoryl azide); | |
| fHNO₃ (fuming HNO₃); and | |
| EDTA (ethylenediaminetetraacetic acid). | |
| TMEDA (N,N,N',N'-tetramethyl-1,2-ethanediamine) | |
| NBS (N-bromosuccinimide) | |
| HATU (O-(7azabenzobenzotriazol-1-yl)-N,N,N',N'-tetramethyluronium | |
| hexafluorophosphate) | |
| DIPEA (diisopropylethylamine) | |
| dppf (1,1'-bis(diphenylphosphino)ferrocene) | |
| NIS (N-iodsuccinimide) | |
| LAH (Lithium Aluminum Hydride) | |

All references to ether are to diethyl ether; brine refers to a saturated aqueous solution of NaCl. Unless otherwise indicated, all temperatures are expressed in °C (degrees Centigrade). All reactions are conducted under an inert atmosphere at room temperature unless otherwise noted.

¹H NMR spectra were recorded on a Brucker DPX400, a Brucker DPX250, a Brucker AC400, or a Varian Inova 400. Chemical shifts are expressed in parts per million (ppm, δ units). Splitting patterns describe apparent multiplicities and are designated as s (singlet), d (doublet), t (triplet), q (quartet), quint (quintet), m (multiplet), br (broad).

Low-resolution mass spectra (MS) were recorded on a JOEL JMS-AX505HA, JOEL SX-102, or a SCIEX-APIiii spectrometer; LC-MS were recorded on Waters ZQ or PE Sciex Single Quadrupole LC/MS API-150 spectrometers.

Preparative HPLC refers to methods where the material was purified by high performance liquid chromatography on a HPLC ABZ+ 5µm column (10 cm x 21.2 mm i.d.) with 0.1% formic acid in water and 0.05% formic acid in acetonitrile utilising gradient elution at a flow rate of 8 ml/min and UV detection at 254nM.

Unless otherwise stated, silica flash column chromatography and Combiflash refers to the purification of material using Redisep^{™} pre-packed silica flash columns on an ISCO sq16x machine with the stated solvent systems.

Reverse phase HPLC method A refers to methods where the materials were purified by high performance liquid chromatography on an HPLC S-5 µm column (75×30 mm i.d.) utilizing gradient elution with the stated solvent systems and UV detection at 254 nm.

Reverse phase HPLC method B refers to methods where the materials was purified by high performace liquid chromatography on a HPLC Luna C18 (2) 100A column (50x21.2 mm i.d.) utilizing gradient elution with the stated solvent system and UV detection at 254 nm.

LC-MS Experimental Conditions for PE Sciex Single Quadrupole LC/MS API-150:

| | |
|---|---|
| Liquid Chromatograph: | |
| System: | Shimadzu LC system with SCL-10A Controller and dual UV detector |
| Autosampler: | Leap CTC with a Valco six port injector |
| Column: | Aquasil/Aquasil (C18 40x1 mm) |
| Inj. Volume (µL): | 2.0 |
| Solvent A: | H2O, 0.02% TFA |
| Solvent B: | MeCN, 0.018% TFA |
| Gradient: | linear |
| Channel A: | UV 214 nm |
| Channel B: | ELS |

| Step | Time (min) | | Dura.(min) | | Flow (µL/min) | Sol.A | Sol.B |
|---|---|---|---|---|---|---|---|
| 0 | 0.00 | 0.00 | 300.00 | 95.00 | 5.00 | | |
| 1 | 0.00 | 0.01 | 300.00 | 95.00 | 5.00 | | |
| 2 | 0.01 | 3.20 | 300.00 | 10.00 | 90.00 | | |
| 3 | 3.21 | 1.00 | 300.00 | 10.00 | 90.00 | | |
| 4 | 4.21 | 0.10 | 300.00 | 95.00 | 5.00 | | |
| 5 | 4.31 | 0.40 | 300.00 | 95.00 | 5.00 | | |

| | |
|---|---|
| Mass Spectrometer: | PE Sciex Single Quadrupole LC/MS API-150 |
| Polarity: | Positive |
| Acquisition mode: | Profile |

### Intermediates

Some of the compounds listed below as "Intermediates" also fall within the scope of Formula (I) and are thus also compounds of the invention as well as useful in preparing further compounds of Formula (I).

### (1) 1,1-dimethylethyl 2,3-dihydro-1H-indole-1-carboxylate

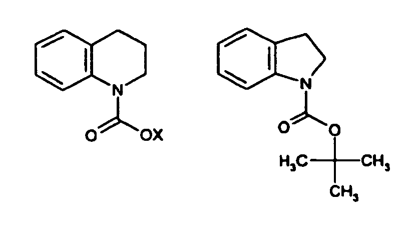

Indoline (10 g, 84 mmol) was dissolved in tetrahydrofuran (100 mL) and di-tert-butylcarbonate (22 g, 0.1 mol) was added. The mixture was left stirring for 16 hours at room temperature under an inert nitrogen atmosphere. The tetrahydrofuran was removed *in vacuo* and the crude product purified by vacuum distillation to give the title compound (15.1 g) as a clear pale pink oil that crystallised upon standing (temperature: 160-162°C, pressure 1-0.1 mm Hg).

1H NMR (400 MHz, DMSO-D6) δ ppm 1.50 (s, 9 H) 3.04 (t, *J*=8.7 Hz, 2 H) 3.89 (t, *J*=8.8 Hz, 2 H) 6.91 (td, *J*=7.3, 0.8 Hz, 1 H) 7.13 (t, *J*=7.5 Hz, 1 H) 7.18 (d, J=7.3 Hz, 1 H) 7.5-7.8 (bs, 1 H) r.t. 3.44 min.

### (2) 1-(1,1-dimethylethyl) 7-methyl 2,3-dihydro-1H-indole-1,7-dicarboxylate

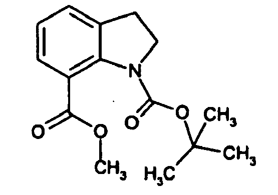

1,1-dimethylethyl 2,3-dihydro-1H-indole-1-carboxylate (5 g, 22.8 mmol) and *N,N,N',N'-*tetramethyl-1,2-ethanediamine (4.6 mL, 30.5 mmol) was dissolved in dry diethyl ether (300 mL) and cooled to -78°C in an acetone/dry ice bath. Sec-butyl lithium (1.4 M solution in cyclohexane, 17.6 mL, 24.6 mmol) was added dropwise over 10 minutes and the reaction left stirring for 90 minutes at this temperature. Methyl chloroformate (8.8 mL, 10.8 g, 0.1 mol) was added to the mixture and the reaction was allowed to warm up to room temperature over 1 hour. Water was added carefully to the mixture and the organic layer separated and washed 3 times with more water. The organic layer was dried over magnesium sulfate, filtered and concentrated *in vacuo* to give the title compound (4.91 g) as a gummy yellow solid.

1 H NMR (400 MHz, DMSO-D6) δ ppm 1.44 (s, 9 H) 3.06 (t, *J*=8.2 Hz, 2 H) 3.69 (s, 3 H) 4.02 (t, *J*=8.3 Hz, 2 H) 7.06 (t, *J*=7.5 Hz, 1 H) 7.35 (d, *J*=7.5 Hz, 1 H) 7.39 (dd, *J*=7.4, 1.1 Hz, 1 H) MS m/z 278 (M+1)⁺ r.t. 3.18 min.

### (3) 1-(1,1-dimethylethyl) 7-methyl 5-bromo-2,3-dihydro-1H-indole-1,7-dicarboxylate

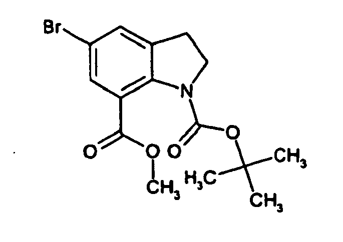

1-(1,1-dimethylethyl) 7-methyl 2,3-dihydro-1H-indole-1,7-dicarboxylate (3.1 g, 11.2 mmol) and N-bromosuccinimide (2.0 g, 11.2 mmol) were dissolved in dry dichloromethane (100 mL) and stirred under a nitrogen atmosphere at room temperature for 16 hours. The reaction was partitioned with sodium hydroxide solution (2 M), separated and washed with more sodium hydroxide solution. The organic layer was dried over magnesium sulfate and concentrated *in vacuo* to give the title compound as a gummy red solid (3.55 g).

1 H NMR (400 MHz, DMSO-D6) δ ppm 1.41 (s, 9 H) 3.09 (t, *J*=8.3 Hz, 2 H) 3.70 (s, 3 H) 4.02 (t, *J*=8.3 Hz, 2 H) 7.46 (s, 1 H) 7.60 (s, 1 H) MS m/z 356/358 (1:1 ratio) (M+1)⁺ r.t. 3.52 min.

### (4) methyl 5-bromo-2,3-dihydro-1H-indole-7-carboxylate

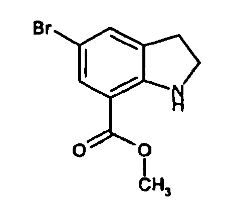

1-(1,1-dimethylethyl) 7-methyl 5-bromo-2,3-dihydro-1H-indole-1,7-dicarboxylate (9 g, 25 mmol) was dissolved in trifluoroacetic acid (6 mL) and stirred at room temperature for 16 hours. Dichloromethane and sodium hydroxide solution (2 M) were added and the organic layer washed twice with sodium hydroxide solution until the aqueous layer pH > 7. The organic layer was then concentrated *in vacuo* to give the title compound as a brown solid (6.5 g).

1H NMR (400 MHz, DMSO-D6) δ ppm 2.99 (t, *J*=8.5 Hz, 2 H) 3.61 (t, *J*=8.4 Hz, 2 H) 3.78 (s, 3 H) 6.72 (s, 1 H) 7.28 (d, *J*=1 Hz, 1H) 7.46 (d, *J*=2 Hz, 1 H) MS m/z 256/258 (1:1 ratio) (M+1)⁺ r.t. 3.32 min.

### (5) methyl 5-bromo-1H-indole-7-carboxylate

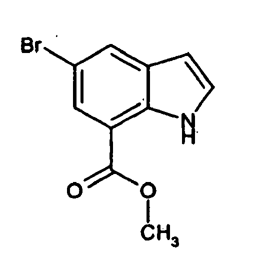

Methyl 5-bromo-2,3-dihydro-1H-indole-7-carboxylate (6.5 g, 25 mmol) was dissolved in tetrahydrofuran (100 mL). Activated manganese dioxide (5µm particle size, 22 g, 0.25 mol) was added and the mixture stirred at room temperature for 16 hours. A further 22 g of activated manganese dioxide was added and the reaction stirred for 96 hours. The reaction was then filtered through celite and concentrated *in vacuo* to give the title compound (5.1 g) as a beige solid.

1H NMR (400 MHz, DMSO-D6) δ ppm 3.94 (s, 3 H) 6.58 (d, *J*=3 Hz, 1 H) 7.48 (d, *J*=3 Hz, 1 H) 7.8 (d, *J*=2 Hz, 1 H) 8.07 (d, *J*=1.8 Hz, 1 H) 11.39 (bs, 1 H) MS m/z 252/254 (1:1 ratio) (M-1) r.t. 3.41 min.

### (6) 5-bromo-1H-indole-7-carboxylic acid

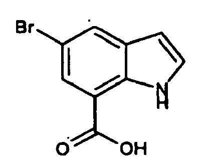

5-bromo-1H-indole-7-carboxylate (5 g, 19.7 mmol) was dissolved in methanol (200 mL) and a solution of lithium hydroxide (0.99 g, 41 mmol) in water (10 mL) was added. The mixture was heated at reflux for 50 hours. The methanol was removed *in vacuo* and the residue diluted with aqueous hydrochloric acid (2 M). The resulting precipitate was filtered off and dried in a heated vacuum pistol to give the title compound as a beige solid (4.7 g).

1H NMR (400 MHz, DMSO-D6) δ ppm 6.54 (dd, *J*=2.0, 3.2 Hz, 1 H) 7.42 (t, *J*=2.8 Hz, 1 H) 7.77 (d, *J*=2 Hz, 1 H) 8.03 (d, *J*=1.8 Hz, 1 H) 11.27 (s, 1 H) 13.1-13.7 (bs, 1 H) MS m/z 238/240 (1:1 ratio) (M-1) r.t. 3.41 min.

### (7) 5-bromo-1H-indole-7-carboxamide

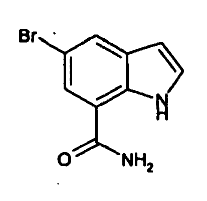

5-bromo-1H-indole-7-carboxylic acid (20 g, 83.5 mmol), *O*-(7-Azabenzatriazol-1-yl)-*N*,*N*,*N*',*N*'-tetramethyluronium hexafluorophosphate (38 g, 100 mmol), ammonia solution in dioxane (0.5 M, 502 mL, 250 mmol) and di-isopropylethylamine (32.6 g, 44 mL, 250 mmol) were dissolved in dry dichloromethane (500 mL) and stirred at room temperature for 50 hours under a nitrogen atmosphere. The reaction mixture was then partitioned with saturated sodium bicarbonate solution, separated and washed with saturated brine solution. The organic layer was concentrated *in vacuo*, re-dissolved in methanol and pre-adsorbed onto silica. Purification was achieved by silica flash chromatography using an ethyl acetate-cyclohexane gradient system on an ISCO sq16x machine.

1H NMR (400 MHz, DMSO-D6) δ ppm 6.47 (t, *J*=2.4 Hz, 1 H) 7.38 (t, *J*=2.8 Hz, 1H) 7.50 (bs, 1H) 7.84 (d, *J*=1.8 Hz, 1H) 7.92 (d, *J*=1.3 Hz, 1H) 8.16 (bs, 1 H) 11.28 (bs, 1H) MS m/z 237/239 (1:1 ratio) (M-1) r.t. 2.88 min.

### (8) 1,1-dimethylethyl 7-[(bis{[(1,1-dimethylethyl)oxy]carbonyl}amino)carbonyl]-5-bromo-1H-indole-1-carboxylate

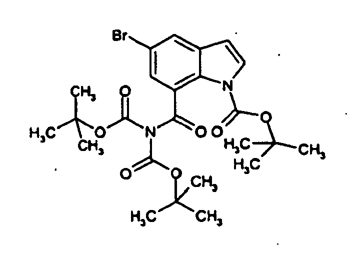

5-bromo-1H-indole-7-carboxamide (16.9 g, 70.5 mmol) was dissolved in dichloromethane (500 mL). To this was added di-tert-butylcarbonate (50.8 g, 0.233 mol) and 4-(dimethylamino)pyridine (0.86 g, 7 mmol) and the mixture stirred at room temperature for 16 hours under a nitrogen atmosphere.

Aqueous hydrochloric acid (2 M, 200 mL) was added and the organic phase separated and dried over magnesium sulfate. After filtration the dichloromethane was removed in vacuo to give the title compound as a yellow solid (40.0 g).

1 H NMR (400 MHz, DMSO-D6) δ ppm 1.32 (s, 18 H) 1.57 (s, 9 H) 6.78 (d, *J*=3.8 Hz, 1 H) 7.32 (d, *J*=1.8 Hz, 1 H) 7.77 (d, *J*=3.8 Hz, 1 H) 8.03 (d, *J*=2.0 Hz, 1 H) MS m/z 539/541 (1:1 ratio) (M+1)⁺ r.t. 4.04 min.

### (9) 1,1-dimethylethyl 7-[(bis{[(1,1-dimethylethyl)oxy]carbonyl}amino)carbonyl]-3-iodo-5-phenyl-1H-indole-1-carboxylate

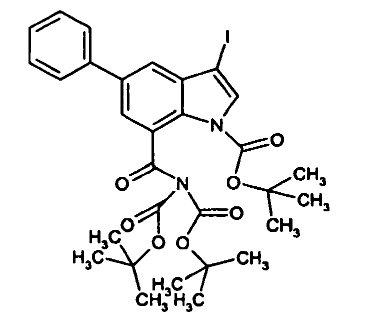

3-iodo-5-phenyl-1H-indole-7-carboxamide (1.0 g, 2.76 mmol) was dissolved in dichloromethane (120 mL) and to it was added di-tert-butylcarbonate (1.9 g, 8.56 mmol) and 4-(di-methylamino)pyridine (20 mg, 0.16 mmol) and the mixture stirred at room temperature for 16 hours under a nitrogen atmosphere. Another 1.87 g di-tert-butylcarbonate was added and the reaction left for 24 hours. Another 1.9 g di-tert-butylcarbonate and 20 mg 4-(di-methylamino)pyridine was added and the mixture stirred for 48 hours. The mixture was pre-adsorbed onto silica *in vacuo* and purified by silica flash chromatography using an ethyl acetate/cyclohexane/triethylamine elution system on an ISCO sq16x machine. The combined fractions were concentrated in vacuo to give the title compound as a white foam (1.1 g).

1H NMR (400 MHz, DMSO=D6) δ ppm 1.32 (s, 18 H) 1.59 (s, 9 H) 7.42 (t, *J*=7.4 Hz, 1 H) 7.51 (t, *J*=7.7 Hz, 2 H) 7.55 (s, 1 H) 7.69 (s, 1 H) 7.73 (d, *J*=7.8 Hz, 2 H) 8.02 (s, 1 H) MS (M+1)⁺ 663 r.t. 4.26 min.

### (10) Methyl 3-(1-methylpyrrolidin-2-yl)-5-pheny)-1H-indole-7-carboxylate

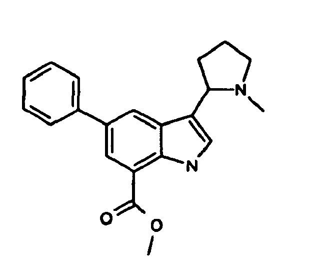

To 0.4 ml (4.0 mmol) of 1-methyl-2-pyrrolidine cooled in an ice bath was added 410.0 mg (2.6 mmol) of POCl₃ with stirring for 30min. The temperature was kept below 15°C. The mixture was stirred an additional 10min, and then a solution of 500.0 mg( 2.0 mmol) of methyl 5-phenyl-1*H*-indole-7-carboxylate in 0.4 mL of 1-methyl-2-pyrrolidine was added slowly during 1 hr. The temperature rose to 45°C and a solid precipitated. The mixture was heated at 80°C for 2 hr and purified by combiflash to get methyl (3Z)-3-(1-methyl-2-pyrrolidinylidene)-5-phenyl-3*H*-indole-7-carboxylate 200.0 mg (30%).

A solution of methyl (3Z)-3-(1-methyl-2-pyrrolidinylidene)-5-phenyl-3*H*-indole-7-carboxylate (335.0 mg, 1.0 mmol) in EtOH (10.0 mL) was treated with NaBH₄ (124.0 mg, 3.0 mmol). The resulting solution was stirred at ambient temperature overnight after which time the solvent was removed under reduced pressure and water (5.0 mL) was added. The aqueous phase was extracted with CH₂Cl₂ (3×10mL). The organic phase was dried over Na₂SO₄, filtered and concentrated and the resulting crude product purified by flash chromatography (Ethyl acetate/Hexane 10%-60% v/v) to afford the title compound 330.0 mg (90%).

1 H NMR (400 MHz, CDCl3) δ ppm 1.90 (m, 1 H) 2.09 (m, 2 H) 2.29 (m, 4 H) 3.29 (m, 1 H) 3.49 (m, 1 H) 4.03 (s, 3H) 7.38 (m, 2 H) 7.37 (m, 2 H) 7.71( m, 2H), 8.18 (d, J=2.0Hz, 1 H) 8.22(d, J=1.6Hz, 1H) 9.72(s, 1H), LC/MS m/z 335 (M+1) r.t. 1.80 min.

### (11) 3-{1-[ethyl(methyl)amino]propyl}-5-phenyl-1H-indole-7-carboxylic acid

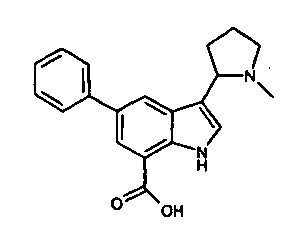

To methyl 3-(1-methylpyrrolidin-2-yl)-5-phenyl-1*H*-indole-7-carboxylate (335.0 mg, 1.0 mmol) in MeOH (15.0 mL), was added LiOH.2H₂O (200.0 mg, 5.0 mmol). The resulting solution was heated under refluxing conditions overnight. The solvent was removed *in vacuo* and the residue was acidified with aqueous hydrochloric acid (2 M, 2.5 ml) to pH=1. The resulting solution was extracted with EtOAc (4x10 mL), dried over Na₂SO₄, filtered and evaporated to afford the title compound (300.0 mg, 93%).

1H NMR (400 MHz, MeOD) δ ppm 2.34(m, 2H) 2.63 (m, 3 H) 2.88 (m, 2 H) 3.24 (m, 2 H) 3.29 (m, 1H) 3.83 (b, 1 H) 7.37 (m, 1H) 7.50 (m, 2 H) 7.74(m, 2H), 8.24 (m, 2H), LC/MS m/z 321 (M+1) r.t. 1.60 min.

### (12) Methyl 3-(2,5-dioxopyrrolidin-3-yl)-5-phenyl-1H-indole-7-carboxylate

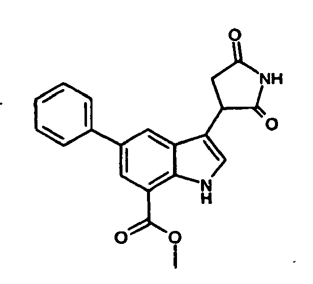

To a solution of methyl 5-phenyl-1*H*-indole-7-carboxylate(500.0 mg, 2.0 mmol) in acetric acid (10.0 ml), was added 85% H₃PO₄ (2.0 mL), 1*H*-pyrrole-2,5-dione (600.0 mg, 6.0 mmol). The reaction mixture was heated under reflux conditions for 48hr. After removing the solvent in vacuo, EtOAc (15 mL) and saturated NaHCO₃ (10 mL) were added. The aqueous phase was separated and washed with EtOAc (2x15 mL). The combined organic layers were dried over Na₂SO₄ and purified by Combiflash (Ethyl acetate/Hexane 10%-60% v/v) to afford the title compound (365.0 mg, 52%)
1H NMR (400 MHz, CDCl3) δ ppm 2.97 (dd, J=18.4, 4.2Hz, 1 H) 3.31 (m, 1 H) 3.99 (s,3 H) 4.39 (m, 1H) 7.17 (s, 1 H) 7.27 (m, 1H) 7.40 (m, 2 H) 7.46(m, 2H), 7.93 (s, 1H), 8.16(s, 1 H) 9.92 (b, 1H) LC/MS m/z 349 (M+1) r.t. 1.92 min.

### (13) (5-phenyl-3-pyrrolidin-3-yl-1H-indol-7-yl)methanol

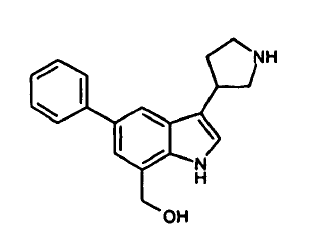

To a stirred solution of LAH in THF (1.0M, 16.0 mL, 16.0 mmol)) at 0°C was added the methyl 3-(2,5-dioxopyrrolidin-3-yl)-5-phenyl-1*H*-indole-7-carboxylate (160.0 mg, 0.41 mmol). The resulting mixture was heated to reflux under Argon for 12 hr. The resulting mixture was cooled and Na₂SO₄.10H₂O (1.5 g) was added cautiously portionwise, followed by H₂O (100 uL) and then by EtOAc (20 mL). The resulting mixture was then stirred at room temperature under argon for 4 hr. The mixture was filtered through Celite, and the filtrate was evaporated under reduced pressure and purified by combiflash (Ethyl acetate/Hexane 10%-60% v/v) to afford the title compound (79.0mg, 65%).

LC/MS m/z 294 (M+1) r.t. 1.60 min.

### (14) tert-Butyl 3-[7-(hydroxymethyl)-5-phenyl-1H-indol-3-yl]pyrrolidine-1-carboxylate

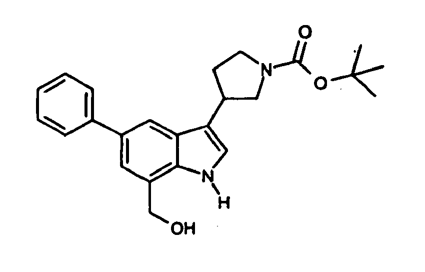

To a solution of (5-phenyl-3-pyrrolidin-3-yl-1*H*-indol-7-yl)methanol (58.6 mg, 0.2 mmol) in dichloromethane (15 mL) was added Et₃N (66.0 mg, 0.66 mmol) and bis(1,1-dimethylethyl) dicarbonate (48.4 mg, 0.22 mmol). The solution was stired overnight. Dichoromethane (50.0 mL) was added. The resulting solution was washed with H₂O (10 mL) and brine (10 mL), dried over Na₂SO₄, and purified by Combiflash (Ethyl acetate/Hexane 10%-60% v/v) to afford the title compound (48.0mg, 60%).
LC/MS m/z 394 (M+1) r.t. 2.20 min.

### (15) Methyl 3-(1-{[(1,1-dimethylethyl)oxy]carbonyl}-3-pyrrolidinyl)-5-phenyl-1H-indole-7-carboxylate

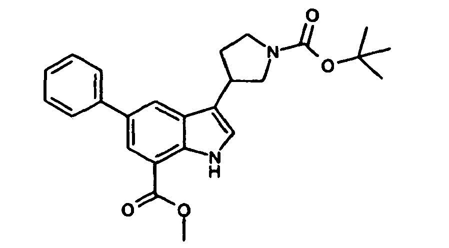

To a solution of *tert*-butyl 3-[7-(hydroxymethyl)-5-phenyl-1*H*-indol-3-yl]pyrrolidine-1-carboxylate (39.3 mg, 0.1 mmol) in THF (10 ml) was added MnO₂ (174.0 mg, 2.0 mmol). The solution was stirred for 14hr, filtered through celite and the solvent removed under reduced pressure. The crude product was dissolved in MeOH (5.0 ml) and HCl (2.0 M, 2.0 ml). To the resulting solution was added Ag₂O (23.2 mg, 0.1 mmol) and NaCN (17.1 mg, 0.3 mmol). The resulting solution was heated overnight. The solvent was removed under reduced pressure and the resulting material was used directly in the next step.
LC-MS m/z 422 (M+1) r.t. 2.72 min.

### (16) 1,1-dimethylethyl-3-[7-(aminocarbonyl)-5-phenyl-1H-indol-3-yl]-1-pyrrolidine carboxylate

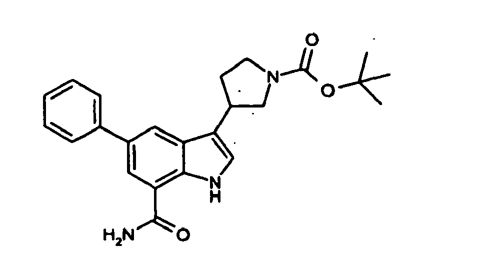

To methyl 3-(1-{[(1,1-dimethylethyl)oxy]carbonyl}-3-pyrrolidinyl)-5-phenyl-1*H*-indole-7-carboxylate (105.0 mg, 0.25 mmol) in MeOH (15.0 mL) was added LiOH.2H₂O (100.0 mg, 2.5 mmol). The resulting solution was heated at reflux overnight. The methanol was removed *in vacuo* and the residue was acidified with aqueous hydrochloric acid (2 M) to pH=1. The resulting solution was extracted with EtOAc (4x10 mL); dried over Na₂SO₄, filtered and evaporated to afford the crude acid. The crude acid, O-(7-Azabenzatriazol-1-yl)-*N*,*N*,*N*',*N*'-tetramethyluronium hexafluorophosphate (57.3 mg, 0.15 mmol) and ammonia solution in MeOH (2.0 M, 1.0 mL, 2.0 mmol) were dissolved in dry dichloromethane (10 mL) and stirred at room temperature for 50 hours under a nitrogen atmosphere. The reaction mixture was then extracted with EtOAc (4x10mL), dried over Na₂SO₄, filtered and evaporated to afford the title compound (40.0mg, 40%).
LC/MS m/z 421 (M+1) r.t. 2.60 min.

### (17) 1,1-dimethylethyl(3E)-3-{[7-(aminocarbonyl)-5-phenyl-1H-indol-3-yl] methylidene}-1-piperidinecarboxylate and 1,1-dimethylethyl(3Z)-3-{[7-(aminocarbonyl)-5-phenyl-1H-indol-3-yl] methylidene}-1-piperidinecarboxylate

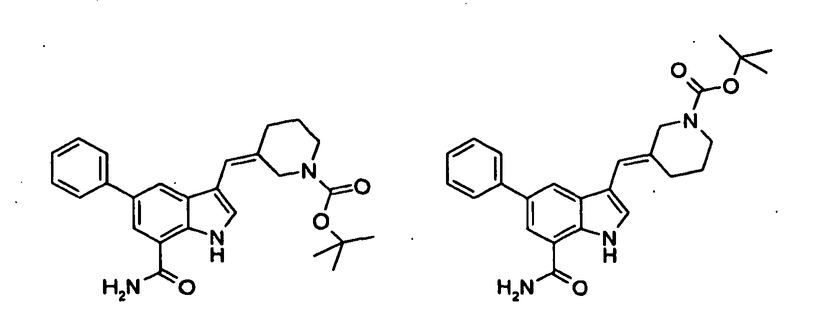

The title compound was prepared according to the procedure of example **50**. Thus, 5-phenyl-1*H*-indole-7-carboxamide (120 mg, 0.5 mmol) , 1,1-dimethylethyl 3-formyl-1-piperidinecarboxylate (227 mg, 1.0 mmol) and NaOMe (4.0 mL, 2.0 mmol) were reacted to form two isomeric compounds (175 mg).
LC/MS m/z 432.2 (M+1) r.t: 2.31; r.t: 2.40.

### (18) 1,1-dimethylethyl-3-{[7-(aminocarbonyl)-5-phenyl-1H-indol-3-yl]methyl}-1-piperidinecarboxylate

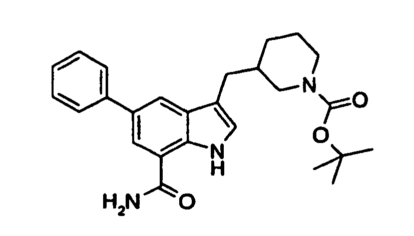

To a solution of 1,1-dimethylethyl (3Z)-3-{[7-(aminocarbonyl)-5-pheny)-1*H*-indo)-3-yl]methylidene}-1-piperidinecarboxylate (82.0 mg, 0.189 mmol) in MeOH (10 mL) was added Pd(OH)₂ (20 mg). The reaction mixture was hydrogenated under 50 Psi H₂ on a Parr-shaker for 14 hr. The metal catalyst was filtered off and the filtrate concentrated. The resulting crude product was purified by flash chromatography (Ethyl acetate/Hexane: 20-30% v/v) to give the desired product 25.0 mg. LC/MS: 334.2. r.t: 2.49. (M + 1-100)

### (19) 1,1-dimethylethyl 4-{(E)-2-[7-(aminocarbonyl)-5-phenyl-1H-indol-3-yl]ethenyl}-1-piperidinecarboxylate and 1,1-dimethylethyl 4-{(Z)-2-[7-(aminocarbonyl)-5'-phenyl-1H-indol-3-yl]ethenyl}-1-piperidinecarboxylate

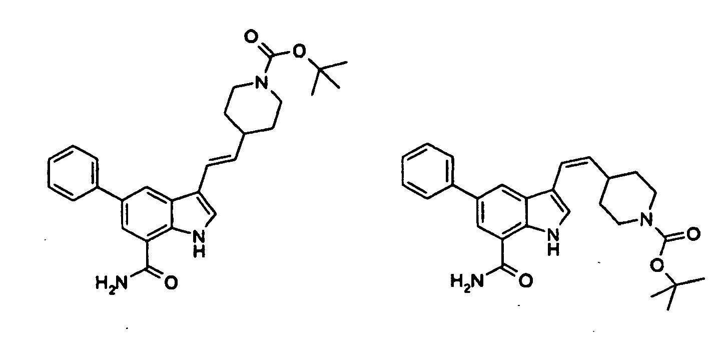

The title compound was prepared according to the procedure for example 50. Thus, 5-phenyl-1*H*-indole-7-carboxamide (830 mg, 3.5 mmol) , and 1,1-dimethylethyl 4-formyl-1-piperidinecarboxylate ( 3.2g, 14.0 mmol) were reacted to form 580.0 mg (39%) of the two isomeric (E/Z) products.
LC/MS m/z 446.6 (M+1) r.t.: 2.29; r.t: 2.81

### (20) 1,1-dimethylethyl-4-{2-[7-(aminocarbonyl)-5-phenyl-1H-indol-3-yl]ethyl}-1-piperidine carboxylate

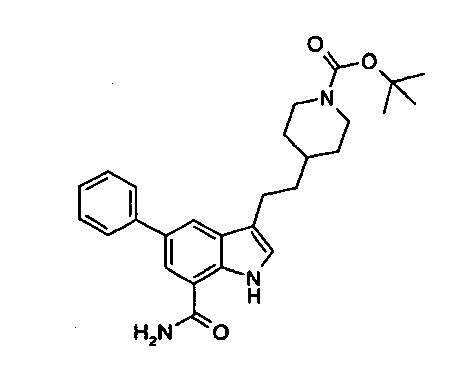

The title compound was prepared according to the procedure for intermediate **18**. Thus, 1,1-dimethylethyl 4-{(*E*)-2-[7-(aminocarbonyl)-5-phenyl-1*H*-indol-3-yl]ethenyl}-1-piperidine carboxylate (410.0 mg, 0.92 mmol) was hydrogenated to gave desired product 40.0 mg. LC/MS: 348.0 ( M+1-100). R.t: 2.85

### (21) 1,1-dimethylethyl 4-({7-(aminocarbonyl)-5-[(1E,2Z)-1-ethylidene-2,4-pentadien-1-yl]-1H-indol-3-yl)methylidene)-1-piperidinecarboxylate

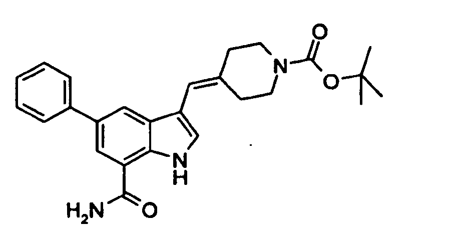

The title compound was prepared according to the procedure for example **50**. Thus, 5-phenyl-1*H*-indole-7-carboxamide (1.05 g, 4.41 mmol), 1,1-dimethylethyl 4-formyl-1-piperidinecarboxylate (3.2g, 14.0 mmol), and sodium methoxide (36 mL, 17,7 mmol) was reacted to form the desired product 660.0 mg.
LC/MS: 432.4. r.t: 2.41.

### (22) 1,1-dimethylethyl-4-{[7-(aminocarbonyl)-5-phenyl-1H-indol-3-yl]methyl}-1-piperidine carboxylate

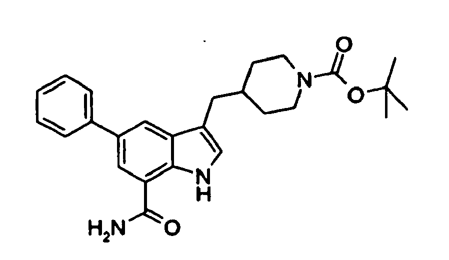

The title compound was prepared according to the procedure for Intermediate **18**. Thus, 1,1-dimethylethyl 4-({7-(aminocarbonyl)-5-[(1E,2Z)-1-ethylidene-2,4-pentadien-1-yl]-1*H-*indol-3-yl}methylidene)-1-piperidinecarboxylate (280.0 mg, 0.633 mmol) were hydrogenated to give desired product 22.0 mg.
LC/MS : 434.4. r.t: 2.61

### (23) 5-(4-chlorophenyl)-1H-indole-7-carboxylic acid

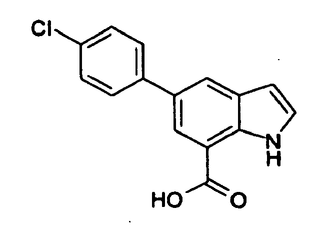

To a solution of 5-bromo-1*H*-indole-7-carboxylic acid (2.76 g, 11.5 mmol) in a mixture of dioxane (30 mL) and water (10 mL), chlorophenylboronic acid (7.19 g, 46 mmol), cesium carbonate (3.74 g, 23 mmol), palladium acetate (258 mg, 1.15 mmol) and 1,3-bis(2,4,6-trimethylphenyl)imidazolium chloride, min.(784 mg, 2.3 mmol) were added. The reaction mixture was stirred at 80°C for 16 hours. All solvent was evaporated. The residue was partitioned between ethyl acetate (50 mL) and 1 N of HCl (50 mL). The aqueous layer was extracted with ethyl acetate (50 mLx2) and the combined organic phase was dried over Mg₂SO₄ and concentrated under reduced pressure and purified by flash column chromatography (CH₂Cl₂/MeOH, 96/4) to yield the desired product (1.46 g, 47%).
LC/MS: m/z 272.0 (M+H), 1.96 min.

### (24) 5-(4-chlorophenyl)-1H-indole-7-carboxamide

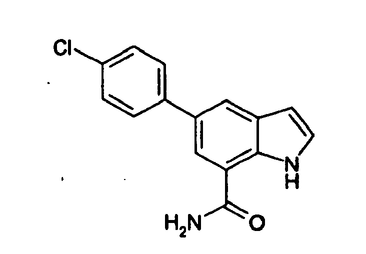

To a solution of 5-(4-chlorophenyl)-1H-indole-7-carboxylic acid (1.46g, 5.36 mmol) in DMF (10 mL), HATU (4.07 g, 10.72 mmol) and NH₃ in MeOH (2.0M, 10.72 mL) were added. The reaction mixture was stirred at room temperature for 2 hours after which time the mixture was partitioned between ethyl acetate (50 mL) and water (50 mL). The aqueous phase was extracted with ethyl acetate (50 mLx2) and the combined organic phase was dried over Mg₂SO₄ and concentrated under reduced pressure to give the title compound without further purification (1.3 g, 89%).
LC/MS: m/z 271.0 (M+H), 1.98 min.

### (25) 5-(4-chlorophenyl)-3-[1-(phenylmethyl)-1,2,3,6-tetrahydro-4-pyridinyl]-1H-indole-7-carboxamide

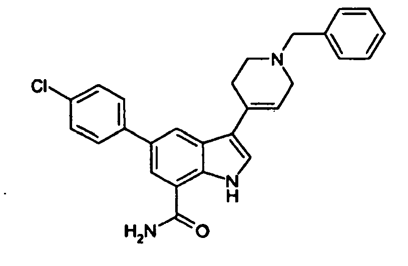

To a solution of 5-(4-chlorophenyl)-1H-indole-7-carboxamide (310 mg, 1.14 mmol) in methanol (5mL), 1-phenylmethyl-4-piperidinone (684 mg, 3.42 mmol) and sodium methoxide (0.5 M in THF, 13.7 mL, 6.84 mmol) were added. The reaction mixture was stirred at reflux temperature for 16 hours. All solvent was evaporated under reduced pressure. The residue was partitioned between ethyl acetate (25 mL) and water (25 mL), the aqueous layer was extracted with ethyl acetate (25 mLx2) and the combined organic phase was dried and concentrated under reduced pressure. The resulting residue was purified by flash column chromatography (CH₂Cl₂/MeOH, 96/4) to yield the desired product (480 mg, 93 %).
LC/MS: m/z 452.0 (M+H), 2.9 min.

### (26) 5-(4-chlorophenyl)-3-(4-piperidinyl)-1H-indole-7-carboxamide

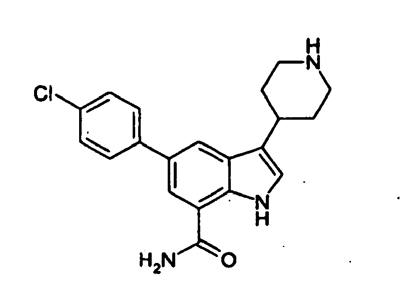

To a solution of 5-(4-chlorophenyl)-3-[1-(phenylmethyl)-1,2,3,6-tetrahydro-4-pyridinyl]-1*H-*indole-7-carboxamide (480 mg, 1.06 mmol) in ethanol (50 mL), palladium (10% on activated carbon, 55 mg) was added. The reaction mixture was hydrogenated under an atmosphere of H₂ balloon for 16 hours. The resulting mixture was filtered through celite and the filtrate concentrated to give the desired product without further purification (280 mg, 75%).
LC/MS: m/z 354.0 (M+H), 1.42 min.

### (27) 5-bromo-1H-indole-7-carboxamide

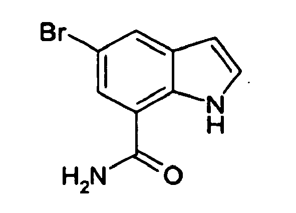

To a solution of 5-bromo-1*H*-indole-7-carboxylic acid (10.0 g, 42 mmol) in CH₂Cl₂ (100 mL) at room temperature, EDC (9.66 g, 50.4 mmol), HOBt (6.81 g, 50.4 mmol) and NH₃ (2.0 M in MeOH, 84 mL, 168 mmol) were added. The reaction mixture was stirred at room temperature for 16 hours. The solvent was evaporated and the residue partitioned between ethyl acetate (100 mL) and water (100 mL). The water layer was extracted with ethyl acetate (100 mLx2) and the combined organic phase was dried over MgSO₄ and concentrated to give the crude product (10g, 98%). This crude product was used directly in the next step without further purification.
LC/MS: m/z 240.0 (M+H), 1.95 min.

### (28) 1,1-dimethylethyl 4-[7-(aminocarbonyl)-5-bromo-1H-indol-3-yl]-3,6-dihydro-1 (2H)-pyridinecarboxylate

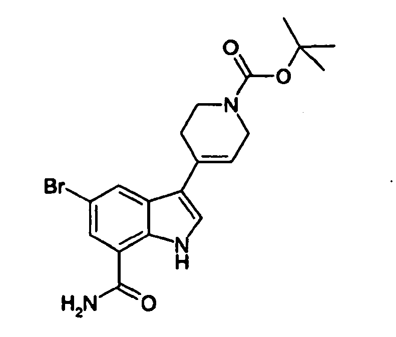

To a solution of 5-bromo-1H-indole-7-carboxamide (10 g, 41.84 mmol) in methanol (5mL), 1,1-dimethylethyl 4-oxo-1-piperidinecarboxylate (684 mg, 3.42 mmol) and sodium methoxide (0.5 M in THF, 13.7 mL, 6.84 mmol) were added. The reaction mixture was stirred at reflux temperature for 16 hours. All solvent was evaporated under reduced pressure. The residue was partitioned between ethyl acetate (100 mL) and water (100 mL). The combined organic phase was dried over MgSO₄ and concentrated under reduced pressure, and purified by flash column chromatography (ethyl acetate/hexane, 1/1) to yield the desired product (7.4 g, 43 %).
LC/MS: m/z 420.0 (M+H), 2.35 min.

### (29) 1,1-dimethylethyl 4-[7-(aminocarbonyl)-5-bromo-1H-indol-3-yl]-1-piperidine carboxylate

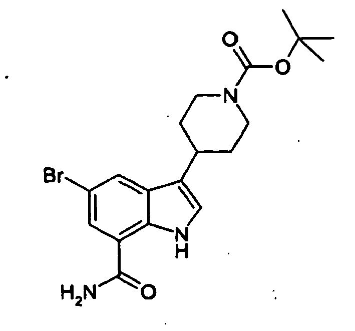

To a solution of 1,1-dimethylethyl 4-[7-(aminocarbonyl)-5-bromo-1H-indol-3-yl]-3,6-dihydro-1(2H)-pyridinecarboxylate (7.41g, 17.64 mmol) in ethanol (600 mL), platinum oxide (200 mg, 5%) was added. The reaction mixture was hydrogenated under an atmosphere of H₂ balloon for 16 hours. The resulting mixture was filtered through celite and the filtrate was concentrated. The resulting residue was purified by flash column chromatography (Ethyl acetate/Hexane, 1:4 to 2:1 v/v) to give the desired product (3.6g, 48%).
LC-MS: m/z 422.0 (M+H), 2.25 min.

### (30) 5-bromo-3-(4-piperidinyl)-1H-indole-7-carboxamide

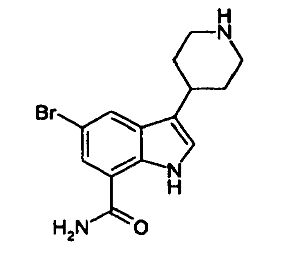

To a solution of 1,1-dimethylethyl 4-[7-(aminocarbonyl)-5-bromo-1*H*-indol-3-yl]-1-piperidinecarboxylate (1.56 g, 3.7 mmol) in methanol (10 mL), HCl in dioxane (4M, 35.5 mL) was added. The reaction mixture was stirred at room temperature for 2 hours. The solvent was evaporated under reduced pressure and the resulting residue was partitioned between ethyl acetate (50 mL) and 5% of aqueous NaOH (50 mL). The aqueous layer was washed with ethyl acetate (2x50 mL) and the combined organic phases were dried and concentrated under reduced pressure to give desired product (685 mg, 58%), which was used in the next step without further purification.
LC-MS: m/z 322.0 (M+H), 1.45 min.

### (31) 3-[1-(ethanesulfonyl)-4-piperidinyl]-5-(4,4,5,5-tetramethyl-1,3,2-dioxaborolan-2-yl)-1H-indole-7-carboxamide

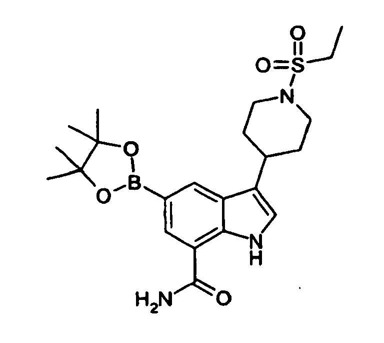

To 5-bromo-3-[1-(ethanesulfonyl)-4-piperidinyl]-1*H*-indole-7-carboxamide (100 mg, 0.24 mmol) in toluene (4 mL), bis(pinacolato) diboron (67.5 mg, 26.4 mmol) PdCl₂(dppf) (2 mg. 0.0024 mmol) and potassium acetate (47 mg, 0.48 mmol) were added. The resulting reaction mixture was heat in a Smith Synthesizer microwave at 160°C for 45 min. The solvent was removed under reduced pressure and the resulting residue was partitioned between ethyl acetate (10 ml) and water (10 ml). The combined organic phase was dried over MgSO₄ and concentrated to give a crude product (170 mg). This crude product was used directly in the next step without further purification.

LC/MS: m/z 462.0 (M+H), 2.2 min.

### (32) 1,1-dimethylethyl 4-[7-(aminocarbonyl)-5-(2-thienyl)-1H-indol-3-yl]-1-piperidinecarboxylate

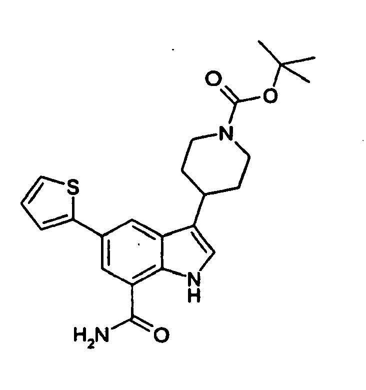

To a solution of 1,1-dimethylethyl 4-[7-(aminocarbonyl)-5-bromo-1*H*-indol-3-yl]-1-piperidinecarboxylate (95 mg, 0.23 mmol) in dioxane (1.5 mL) and water (0.5 ml), 2-thienylboronic acid (115.2 mg, 0.92 mmol), Pd(PPh₃)₄ (26.6 mg, 10%) and potassium carbonate (254 mg, 1.84 mmol) were added. The reaction mixture was heated in a Smith synthesizer microwave at 150°C for 20 min. All solvent was evaporated under reduced pressure. The residue was partitioned between ethyl acetate (10 mL) and water (10 mL). The organic phase was dried over MgSO₄ and concentrated under reduced pressure, and purified by flash column chromatography (ethyl acetate/hexane, 1/1) to yield the desired product (90 mg, 95 %).
LC/MS: m/z 426.0 (M+H), 2.47 min.

### (33) 3-(4-piperidinyl)-5-(2-thienyl)-1H-indole-7-carboxamide

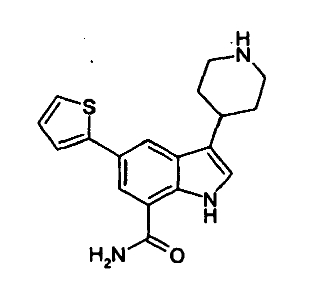

To a solution of 1,1-dimethylethyl 4-[7-(aminocarbonyl)-5-(2-thienyl)-1H-indol-3-yl]-1-piperidinecarboxylate (90 mg, 0.22 mmol) in MeOH (3 mL), HCl (4.0M in dioxane, 2.06 mL) was added. The reaction mixture was stirred at ambient temperature for 2 hours, after which time the solvent was removed under reduced pressure and the resulting residue was partitioned between ethyl acetate (10 mL) and 10% sodium hydroxide (10 mL). The aqueous layer was extracted with ethyl acetate (10 mLx2) and the combined organic phase was dried over MgSO₄ and concentrated to give a crude product (53 mg, 77%). This was used directly in the next step without further purification.
LC/MS: m/z 326.0 (M+H), 1.49 min.

### (34) 1,1-dimethylethyl 4-[7-(aminocarbonyl)-5-(3-thienyl)-1H-indol-3-yl]-1-piperidinecarboxylate

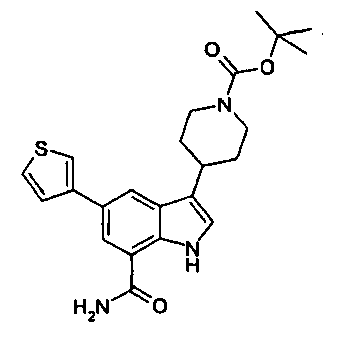

The title compound was prepared according to the procedure for intermediate **32**. Thus, 1,1-dimethylethyl 4-[7-(aminocarbonyl)-5-bromo-1H-indol-3-yl]-1-piperidinecarboxylate (500 mg, 1.18 mmol) in dioxane (1.5 mL) and water (0.5 mL), 3-thienylboronic acid (606 mg, 4.72 mmol), Pd(PPh₃)₄ (136 mg, 10%) and potassium carbonate (651 mg, 4.72 mmol) ) in dioxane (3.0 mL) and water (1.0 mL) were reacted to form the desired product which was purified by flash column chromatography (ethyl acetate/hexane, 1/1) to yield the desired product (460 mg, 92 %).
LC/MS: m/z 426.0 (M+H), 2.45 min.

### (35) 3-(4-piperidinyl)-5-(3-thienyl)-1H-indole-7-carboxamide

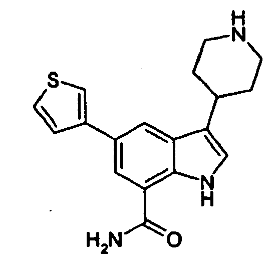

The title compound was prepared according to the procedure for intermediate **33**. Thus, 1,1-dimethylethyl 4-[7-(aminocarbonyl)-5-(3-thienyl)-1H-indol-3-yl]-1-piperidinecarboxylate (460 mg, 1.08 mmol) and HCl (4.0M in dioxane, 10 mL) in MeOH (5 mL) was reacted to form the desired product without further purification (260 mg, 74%).
LC/MS: m/z 326.0 (M+H), 1.60 min.

### (36) 4-[7-(aminocarbonyl)-1H-indol-5-yl]benzoic acid

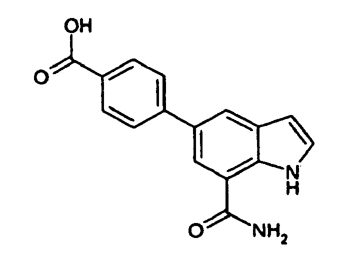

5-Bromo-1*H*-indole-7-carboxamide (2.005 g, 8.39 mmol), 4-carboxyphenyl boronic acid (4.200 g, 25.3 mmol) and potassium phosphate (3.561 g, 16.78 mmol) in 1,4-dioxan (35 mL) and water (40 mL) were treated with a solution of 2'(dimethylamino)-2-biphenyl-palladium (II) chloride Dinorbornyl phosphine complex (0.236 g, 0.420 mmol) in 1,4-doxan (5 mL). The reaction mixture was heated at 85°C for 4 hours under nitrogen. The cooled mixture was partitioned between ethyl acetate (2x80 mL), water (150 mL) and brine (10 mL). The layers were separated and the organic phase concentrated *in vacuo* to give the crude material (3.89 g). Methanol (25 mL) was added and a solid removed by filtration (1.127 g). This solid was triturated in acetonitrile (20mL) and the solid removed by filtration to give the title compound (0.994 g, 47%).

1H NMR (400 MHz, DMSO-D6) δ ppm 12.80 (v.brs, 1 H) 11.20 (br.s, 1 H) 8.28(br.s, 1 H) 8.13 (br.s, 1 H) 8.09 (br.s, 1 H) 8.02 (1/2AA'BB', 2 H) 7.92 (1/2AA'BB', 2 H) 7.45 (br.s, 1 H) 7.38 (t, 1 H) 6.56(t, 1 H)
LCMS m/z 281 (M+1)⁺, Rt. 2.91 min.

### (37) 3-[7-(aminocarbonyl)-1H-indol-5-yl]benzoic acid

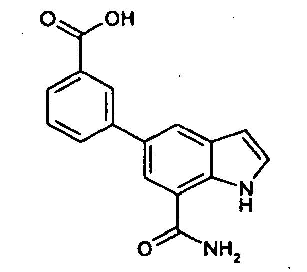

5-Bromo-1*H*-indole-7-carboxamide (2.001 g, 8.36 mmol), 3-carboxyphenyl boronic acid (4.171 g, 25.0 mmol) and potassium phosphate (3.595 g, 17.0 mmol) in 1,4-dioxan (32 mL) and water (40 mL) were treated with a solution of 2'(dimethylamino)-2-biphenylpalladium (II) chloride Dinorbornyl phosphine complex (0.234 g, 0.418 mmol)) in 1,4-doxan (8 mL). The reaction mixture was heated at 85°C for 21 hours under nitrogen. The cooled mixture was partitioned between ethyl acetate (2x80 mL), water (150 mL), brine (10 mL) and dichloromethane (70 mL). The layers were separated and the organic phase concentrated *in vacuo* to give the crude material (2.219 g). This solid was triturated in acetonitrile and the solid removed by filtration to give the title compound as a pale pink Solid (0.774 g, 33%).

1H NMR (400 MHz, DMSO-D6) δ ppm 13.05 (v.brs, 1 H) 11.17 (br.s, 1 H) 8.32(br.s + t, 2 H) 8.06,8.05 (2 x br.s, 2 H) 8.02 (dt, 1 H) 7.90 (dt, 1 H) 7.60 (t, 1 H) 7.42 (br.s, 1 H) 7.38 (t, 1 H) 6.56(dd,1 H)
LCMS m/z 281 (M+1)⁺, Rt. 2.87 min.

### (38)1,1-dimethylethyl 7-[(bis{[(1,1-dimethylethyl)oxy]carbonyl}amino)carbonyl]-5-bromo-3-(3-pyridinyl)-1H-indole-1-carboxylate

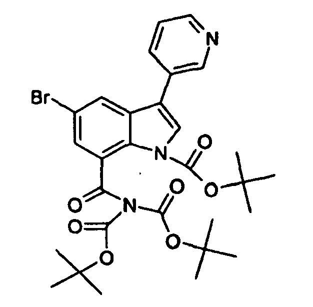

A solution of 1,1-dimethylethyl 7-[(bis{[(1,1-dimethylethyl)oxy]carbonyl}amino)carbonyl]-5-bromo-3-iodo-1*H*-indole-1-carboxylate (200.8 mg, 0.300 mmol)), 3-pyridine boronic acid (39.7 mg, 2.31 mmol, 1 eq), potassium carbonate (65.8 mg, 0.40 mmol, 1.2 eq) and [1,1'-bis(diphenylphoshino)ferocene]dichloropalladium (II) complex with dichloromethane (25.8 mg, 0.03 mmol, 0.1 eq) dissolved in dimethylformamide (2 mL) was heated at 80°C under nitrogen for 3hr. The resultant mixture was partitioned between water (20 mL), brine (5 mL) and ethylacetate (2x10 mL). The organic extracts were combined, dried and concentrated *in vacuo* to give a dark oil which was purified on a 10g silica cartridge eluting with cyclohexane/ ethylacetate, 0-20%., to give the title compound as a solid (70.4 mg).
LC/MS m/z 618.20, Rt: 3.81 min

### (39) 3-[(dimethylamino)methyl]-5-phenyl-1H-indole-7-carboxamide

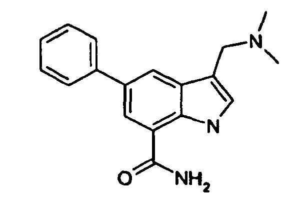

A mixture of formaldehyde (82µl, 11.01 mmol, 1.3eq) dimethylamine (1551 µl, 11.01 mmol, 1.3eq) and acetic acid (55ml) was cooled to 0°C for 25min. 5-Phenyl-1*H*-indole-7-carboxamide (2.001g, 8.47mmol) was then added to the mixture and stirring was continued at room temperature for 18hrs. The reaction mixture was brought to pH>12 with 2N NaOH and the product extracted into ethyl acetate (2x300ml). The organic layer was dried and evaporated to dryness. Dichloromethane was then added to the residue and the resultant white precipitate was filtered to give *the title compound* as a white solid (843.2mg).
LC/MS m/z = 294.3, R.t. = 2.12 min.

### (40) [7-(aminocarbonyl)-5-phenyl-1H-indol-3-yl]-N,N,N-trimethyl-methanaminium iodide

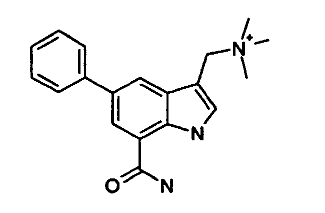

A solution of [(dimethylamino)methyl]-5-phenyl-1H-indole-7-carboxamide (821 mg, 2.77mmol) in absolute ethanol (10ml) was cooled to 0°C. lodomethane (690µl, 11.08mmol,4eq) was added and the reaction mixture was left stirring at 0°C for 2hr. The mixture was then allowed to stir at room temperature for a further 1hr. LC/MS showed the presence of product as the major component and the symmetrical quaternium salt also present. Resultant mixture was evaporated to dryness to give the *title compound* as a solid (1.24g).
LC/MS m/z = 308.3, R.t. = 2.20 min.

### (41) 1,1-dimethylethyl 4-[7-(aminocarbonyl)-5-(2,4-difluorophenyl)-1H-indol-3-yl]-1-piperidinecarboxylate

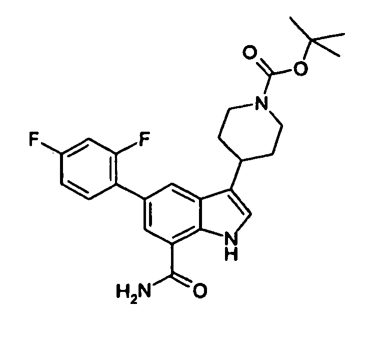

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), (2,4-difluorophenyl)boronic acid (152 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (104 mg, 97%).
LC-MS: m/z 456.2, 2.48 min.

### (42) 5-(2,4-difluorophenyl)-3-(4-piperidinyl)-1H-indole-7-carboxamide

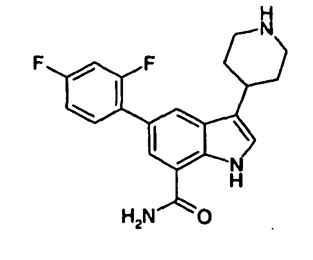

Following the general procedure for intermediate **33**, 1,1-dimethylethyl 4-[7-(aminocarbonyl)-5-(2,4-difluorophenyl)-1*H*-indol-3-yl]-1-piperidinecarboxylate (104 mg, 0.23 mmol) and HCl (4.0 M in dioxane, 2.25 mL) in methanol (5 mL) was reacted to form the desired product and was used without further purification (54.4 mg, 66%).
LC-MS: 356.4, 1.49 min.

### (43) 1,1-dimethylethyl 4-[7-(aminocarbonyl)-5-(4-methoxyphenyl)-1H-indol-3-yl]-1-piperidinecarboxylate

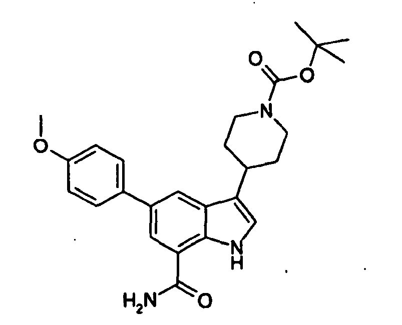

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), (4-methoxyphenyl)boronic acid (148 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (68 mg, 64%).
LC-MS: m/z 450.2, 2.42 min.

### (44) 5-(4-methoxyphenyl)-3-(4-piperidinyl)-1H-indole-7-carboxamide

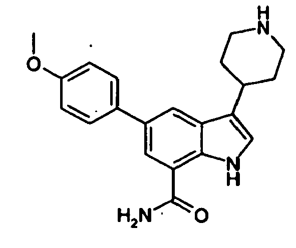

Following the general procedure for intermediate **33**, 1,1-dimethylethyl 4-[7-(aminocarbonyl)-5-(4-methoxyphenyl)-1*H*-indol-3-yl]-1-piperidinecarboxylate (68 mg, 0.15 mmol) and HCl (4.0 M in dioxane, 1.47 mL) in methanol (5 mL) was reacted to form the desired product and was used without further purification (28 mg, 52.8%).
LC-MS: 350.2, 1.52 min.

### (45) 1,1-dimethylethyl 4-[7-(aminocarbonyl)-5-(4-fluoro-2-methylphenyl)-1H-indol-3-yl]-1-piperidinecarboxylate

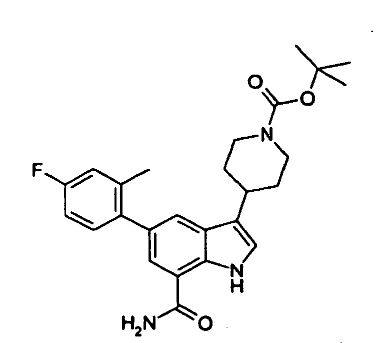

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), (4-fluoro-2-methylphenyl)boronic acid (148 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (100 mg, 95%).
LC-MS: m/z 452.2, 2.57 min.

### (46) 5-(4-fluoro-2-methylphenyl)-3-(4-piperidinyl)-1H-indole-7-carboxamide

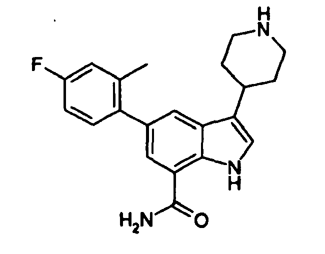

Following the general procedure for intermediate **33**, 1,1-dimethylethyl 4-[7-(aminocarbonyl)-5-(4-fluoro-2-methylphenyl)-1*H*-indol-3-yl]-1-piperidinecarboxylate (100 mg, 0.28 mmol) and HCl ( 4.0 M in dioxane, 2.17 mL) in methanol (5 mL) was reacted to form the desired product and was used without further purification (54.8 mg, 70%).
LC-MS: 351.8, 1.47 min.

### (47) 1,1-dimethylethyl 4-[7-(aminocarbonyl)-5-(4-fluorophenyl)-1H-indol-3-yl]-1-piperidinecarboxylate

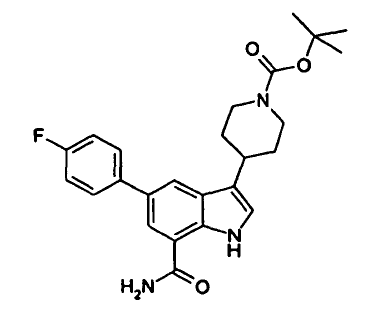

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), (4-fluorophenyl)boronic acid (134 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (92.8 mg, 90%).
LC-MS: m/z 449.4, 2.92 min.

### (48) 5-(4-fluorophenyl)-3-(4-piperidinyl)-1H-indole-7-carboxamide

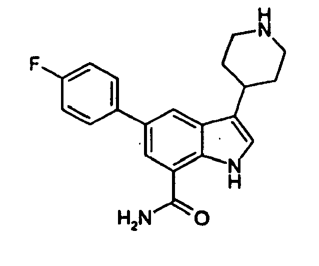

Following the general procedure for intermediate **33**, 1,1-dimethylethyl 4-[7-(aminocarbonyl)-5-(4-fluorophenyl)-1*H*-indol-3-yl]-1-piperidinecarboxylate (92.8 mg, 0.21 mmol) and HCl ( 4.0 M in dioxane, 2.1 mL) in methanol (5 mL) was reacted to form the desired product and was used without further purification (55.9 mg, 78%).
LC-MS: 338.6, 1.53 min.

### (49) 1,1-dimethylethyl 4-[7-(aminocarbonyl)-5-(4-biphenylyl)-1H-indol-3-yl]-1-piperidinecarboxylate

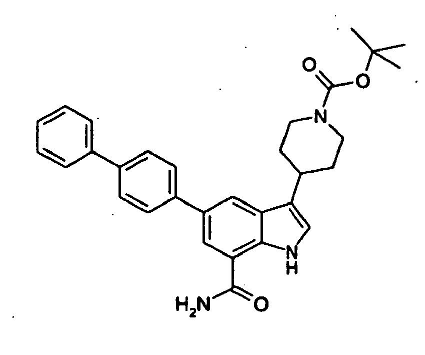

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), (4-biphenylyl)boronic acid (190 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (78.3 mg, 67%).
LC-MS: m/z 496.4, 2.88 min.

### (50) 5-(4-biphenylyl)-3-(4-piperidinyl)-1H-indole-7-carboxamide

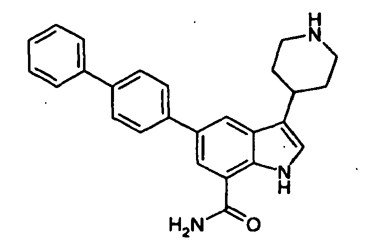

Following the general procedure for intermediate **33**, 1,1-dimethylethyl 4-[7-(aminocarbonyl)-5-(4-biphenylyl)-1*H*-indol-3-yl]-1-piperidinecarboxylate (78.3 mg, 0.16 mmol)) and HCl ( 4.0 M in dioxane, 2.01 mL) in methanol (5 mL) was reacted to form the desired product and was used without further purification (49 mg, 78.4%).
LC-MS: 396.4, 1.78 min.

### (51) 1,1-dimethylethyl 4-[7-(aminocarbonyl)-5-[4-(1,1-dimethylethyl)phenyl]-1H-indol-3-yl]-1-piperidinecarboxylate

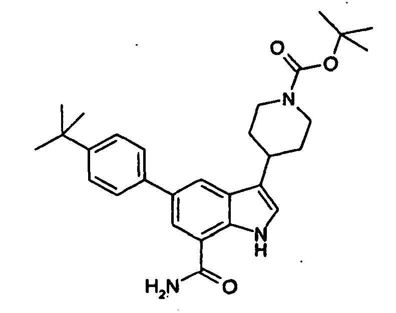

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), [4-(1,1-dimethylethyl)phenyl]boronic acid (170.9 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (105 mg, 94%).
LC-MS: m/z 476.2, 2.87 min.

### (52) 5-[4-(1,1-dimethylethyl)phenyl]-3-(4-piperidinyl)-1H-indole-7-carboxamide

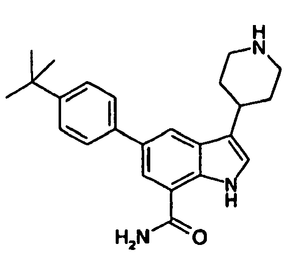

Following the general procedure for intermediate **33**, 1,1-dimethylethyl 4-[7-(aminocarbonyl)-5-[4-(1,1-dimethylethyl)phenyl]-1*H*-indol-3-yl]-1-piperidinecarboxylate (105 mg, 0.22 mmol) and HCl ( 4.0 M in dioxane, 2.27 mL) in methanol (5 mL) was reacted to form the desired product and was used without further purification (41.7 mg, 50%).
LC-MS: 376.2,1.81 min.

### (53) 1,1-dimethylethyl 4-[7-(aminocarbonyl)-5-(4-methylphenyl)-1H-indol-3-yl]-1-piperidinecarboxylate

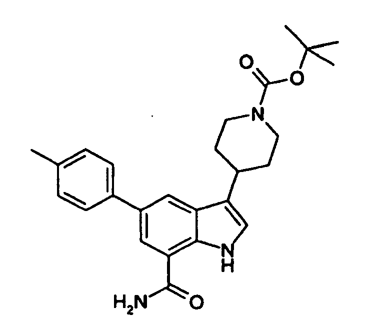

Following the general procedure for intermediate **32**, 1,1-dimethylethyl 4-[7-(aminocarbonyl)-5-bromo-1*H*-indol-3-yl]-1-piperidinecarboxylate (100 mg, 0.24 mmol), (4-phenyl)boronic acid (130 mg, 0.98 mmol), Pd(PPh₃)₄ (28 mg, 10 %) and potassium carbonate (265 mg, 1.92 mmol) in dioxane (3 mL) and water (1mL) were reacted to form the desired product which was purified by flash column chromatography (ethyl acetate/hexane, 1/1) to yield the desired product (85 mg, 84%).
LC-MS: m/z 434.2, 2.62 min.

### (54) 5-(4-methylphenyl)-3-(4-piperidinyl)-1H-indole-7-carboxamide

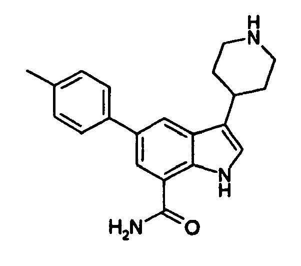

Following the general procedure for intermediate **33**, 1,1-dimethylethyl 4-[7-(aminocarbonyl)-5-(4-methylphenyl)-1*H-*indol-3-yl]-1-piperidinecarboxylate (85 mg, 0.19 mmol) and HCl ( 4.0 M in dioxane, 2.0 mL) in methanol (5 mL) was reacted to form the desired product without further purification (32 mg, 49%).
LC-MS: 334.4, 1.40 min.

### Examples

### (1) 5-phenyl-1H-indole-7-carboxamide

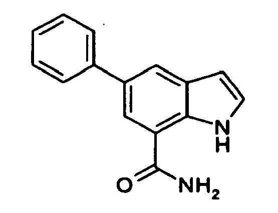

### General method A

1,1-dimethylethyl 7-[(bis{[(1,1-dimethylethyl)oxy]carbonyl}amino)carbonyl]-5-bromo-1H-indole-1-carboxylate (50 mg, 92 µmol), phenyl boronic acid (23 mg, 0.18 mmol), potassium carbonate (40 mg, 0.29 mmol) and 1,1'-bis(diphenylphosphino)ferrocenedichloropalladium (II) (4 mg, 4.9 µmol) were combined in dimethylformamide (1 mL) and heated at 80°C for 16 hours under nitrogen. The mixture was then pre-adsorbed onto silica and purified by silica flash chromatography using an ethyl acetate/cyclohexane elution system on an ISCO sq16x machine. The combined fractions were concentrated *in vacuo*, re-dissolved in ethanol (2 mL) and 15µL concentrated hydrochloric acid added. The mixture was heated in a Smith synthesizer microwave for 5 minutes at 150°C then the solvent was removed *in vacuo* to give the title compound as a pink solid (12 mg).

1H NMR (400 MHz, DMSO-D6) δ ppm 6.54 (t, *J*=2.4 Hz, 1 H) 7.32 (t, *J*=7.3 Hz, 1 H) 7.37(t, *J*=2.6 Hz, 1 H) 7.41 (bs, 1 H) 7.47 (t, *J*=7.7 Hz, 2 H) 7.78 (d, *J*=7.5 Hz, 2 H) 8.01 (s, 2 H) 8.24 (bs, 1 H) 11.13 (bs, 1 H) MS m/z 237 (M+1)⁺ r.t. 3.12 min.

### LARGER SCALE preparation of 5-phenyl-1H-indole-7-carboxamide

1,1-dimethylethyl 7-[(bis{[(1,1-dimethylethyl)oxy)carbonyl}amino)carbonyl]-5-bromo-1H-indole-1-carboxylate (4.95 g, 9.18 mmol), phenyl boronic acid (3.36 g, 27.5 mmol), potassium carbonate (5.07 g, 36.7 mmol) and 1,1'-bis(diphenylphosphino)ferrocenedichloropalladium (II) (0.49 g, 0.6 mmol) were combined in dimethylformamide (70 mL) and heated at 80 °C for 17 hours under a nitrogen atmosphere. Further portions of phenyl boronic acid (1.12 g, 9.17 mmol), potassium carbonate (1.27 g, 9.18 mmol) and 1,1'-bis(diphenylphosphino)ferrocenedichloropalladium (II) (0.25 g, 0.3 mmol) were added, and heating continued for a further 21 hours. After cooling to room temperature and pre-adsorbtion onto silica, purification by silica flash chromatography using an ethyl acetate/cyclohexane elution system on an ISCO sq16x machine yielded 1,1-dimethylethyl 7-[(bis{[(1,1-dimethylethyl)oxy]carbonyl}amino) carbonyl]-5-phenyl-1H-indole-1-carboxylate (2.86 g, 5.33 mmol) and 1,1-dimethylethyl 7-[({[(1,1-dimethylethyl)oxy]carbonyl}amino)carbonyl]-5-phenyl-1H-indole-1-carboxylate (2.05 g, 4.70 mmol).

1,1-dimethylethyl 7-[({[(1,1-dimethylethyl)oxy]carbonyl}amino)carbonyl]-5-phenyl-1H-indole-1-carboxylate (2.05 g, 4.70 mmol) was dissolved in ethanol (30 mL) and dispensed equally into ten microwave vials, with the addition of concentrated hydrochloric acid (20 µL) to each tube. After sealing the tubes, each was heated in a Smith synthesizer microwave for 5 minutes at 150 °C. The combined material was pre-adsorbed onto silica in vacuo. Purification by silica chromatography using an ethyl acetate/cyclohexane gradient system on an ISCO sq16x machine yielded the title compound (333 mg) as a yellow solid.

### (2) 5-(4-biphenylyl)-1H-indole-7-carboxamide

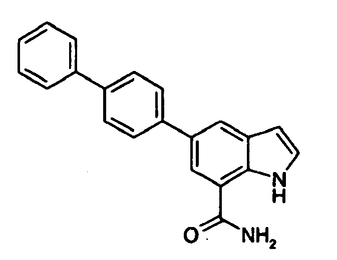

The title compound (24 mg) was prepared using general method A except that 37 mg (0.18 mmol) 4-biphenylboronic acid was used instead of phenyl boronic acid.

1H NMR (400 MHz, DMSO-D6) δ ppm 6.55 (t, *J*=2.3 Hz, 1 H) 7.38 (m, 2 H) 7.44 (bs, 1 H) 7.49 (t, *J*=7.7 Hz, 2H) 7.7-7.8 (m, 4H) 7.9 (d, *J*=8.3 Hz, 2 H) 8.09 (s, 2 H) 8.28 (bs, 1 H) 11.16 (bs, 1 H) MS m/z 311 (M-1) r.t. 3.70 min.

### (3) 5-{4-[(phenylmethyl)oxy]phenyl}-1H-indole-7-carboxamide

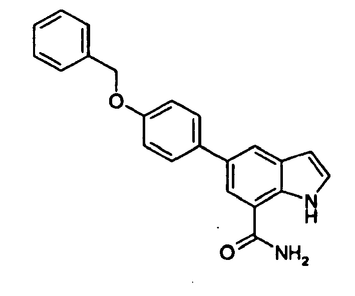

The title compound (12 mg) was prepared using general method A except that 42 mg (0.18 mmol) {4-[(phenylmethyl)oxy]phenyl}boronic acid was used instead of phenyl boronic acid.

1H NMR (400 MHz, DMSO-D6) δ ppm 5.17 (s, 2 H) 6.50 (t, *J*=2.4 Hz, 1 H) 7.11 (d, *J*=8.8 Hz, 2 H) 7.34-7.43 (m, 5 H) 7.48 (d, *J*=7.3 Hz, 2 H) 7.71 (d, *J*=8.5 Hz, 2 H) 7.96 (d, *J*=3 Hz, 2 H) 8.21 (bs, 1 H) 11.08 (bs, 1 H) MS m/z 343 (M+1)⁺ r.t. 3.57 min.

### (4) 5-{4-[(methylsulfonyl)amino]phenyl}-1H-indole-7-carboxamide

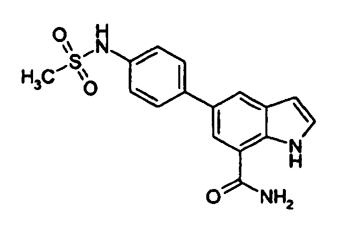

The title compound (14 mg) was prepared using general method A except that 40 mg (0.18 mmol) {4-[(methylsulfonyl)amino]phenyl}boronic acid was used instead of phenyl boronic acid.

1H NMR (400 MHz, DMSO-D6) δ ppm 3.01 (s, 3 H) 6.52 (t, *J*=2.4 Hz, 1 H) 7.31 (d, *J*=8.5 Hz, 2 H) 7.36(t, *J*=2.7 Hz, 1 H) 7.41 (bs, 1 H) 7.75 (d, *J*=8.5 Hz, 2 H) 7.98 (s, 2 H) 8.22 (bs, 1 H) 9.76 (s, 1 H) 11.12 (bs, 1 H) MS m/z 328 (M-1) r.t. 2.70 min.

### (5) 5-[4-(acetylamino)phenyl]-1H-indole-7-carboxamide

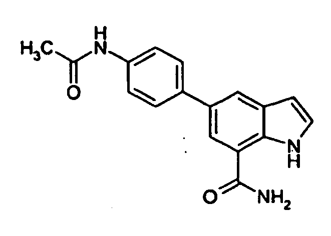

The title compound (8.2 mg) was prepared using general method A except that 33 mg (0.18 mmol) [4-(acetylamino)phenyl]boronic acid was used instead of phenyl boronic acid. The product was purified further by triturating with water, isolated by filtration and dried in a vacuum pistol.

1H NMR (400 MHz, DMSO-D6) δ ppm 2.07 (s, 3 H) 6.51 (t, *J*=2.4 Hz, 1 H) 7.35 (t, *J*=2.7 Hz, 1 H) 7.4 (bs, 1 H) 7.66 (d, *J*=8.53 Hz, 2 H) 7.71 (d, *J*=8.53 Hz, 2 H) 7.98 (s, 2 H) 8.22 (bs, 1 H) 9.99 (s, 1 H) 11.10 (s, 1 H) MS m/z 294 (M+1)⁺ r.t. 2.63 min.

### (6) 5-[3-(aminocarbonyl)phenyl]-1H-indole-7-carboxamide

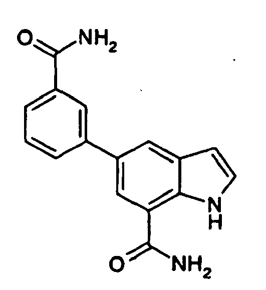

The title compound (7.9 mg) was prepared using general method A except that 31 mg (0.18 mmol) [3-(aminocarbonyl)phenyl]boronic acid was used instead of phenyl boronic acid. The product was purified further by triturating with water, isolated by filtration and dried in a vacuum pistol.

1H NMR (400 MHz, DMSO-D6) δ ppm 6.56 (s, 1 H) 7.38 (s, 1 H) 7.44 (bs, 2 H) 7.54 (t, *J*=7.7 Hz, 1 H) 7.82 (d, *J*=7.53 Hz, 1 H) 7.93 (d, *J*=7.5, 1 H) 8.05 (bs, 2 H) 8.10 (s, 1 H) 8.25 (bs, 2 H) 11.17 (bs, 1H) MS m/z 278 (M-1) r.t. 2.53 min.

### (7) 5-(4-chlorophenyl)-1H-indole-7-carboxamide

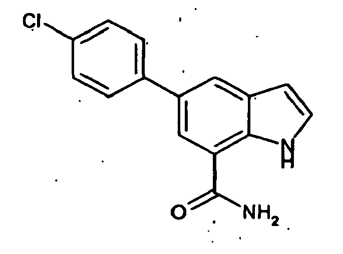

The title compound (4.5 mg) was prepared using general method A except that 29 mg (0.18 mmol) (4-chlorophenyl)boronic acid was used instead of phenyl boronic acid. The product was further purified by preparative HPLC using an acetonitrile/water elution system. The relevant fractions were blown down to dryness with nitrogen and further dried in a vacuum pistol.

1H NMR (400 MHz, DMSO-D6) δ ppm 6.54 (t, J=2.4 Hz, 1 H) 7.37 (t, *J*= 2.6 Hz, 1 H) 7.43 (bs, 1 H) 7.52 (d, *J*=8.5 Hz, 2 H) 7.81 (d, *J*=8.53 Hz, 2H) 8.02 (s, 1 H) 8.03 (s, 1 H) 8.25 (bs, 1 H) 11.16 (bs, 1H) MS m/z 269 (M-1) r.t. 3.40 min.

### (8) 5-[3-(acetylamino)phenyl]-1H-indole-7-carboxamide

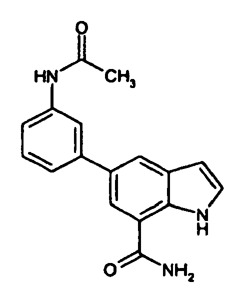

The title compound (7.3 mg) was prepared using general method A except that 33 mg (0.18 mmol) [3-(acetylamino)phenyl]boronic acid was used instead of phenyl boronic acid. The product was purified further by triturating with water, isolated by filtration and dried in a vacuum pistol.

1H NMR (400 MHz, DMSO-D6) δ ppm 2.07 (s, 3 H) 6.55 (t, *J*=2.4 Hz, 1 H) 7.36-7.44 (m, 4 H) 7.56 (d, *J*=8.0 Hz, 1 H) 7.89 (s, 1 H) 7.92 (s, 1 H) 7.96 (s, 1 H) 8.23 (bs, 1 H) 10.0 (s, 1 H) 11.14 (bs, 1 H) MS m/z 292 (M-1) r.t. 2.66 min.

### (9) 5-[3-(aminosulfonyl)phenyl]-1H-indole-7-carboxamide

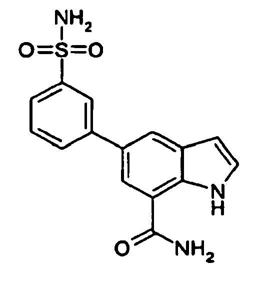

The title compound (6.4 mg) was prepared using general method A except that 53 mg (0.18 mmol) [3-(aminosulfonyl)phenyl]boronic acid was used instead of phenyl boronic acid. The product was purified further by triturating with water, isolated by filtration and dried in a vacuum pistol.

1 H NMR (400 MHz, DMSO-D6) δ ppm 6.58 (t, *J*=2.5 Hz, 1 H) 7.40 (m, 3 H) 7.47 (bs, 1H) 7.66 (t, *J*=7.8 Hz, 1 H) 7.77 (d, *J*=7.8 Hz, 1 H) 8.01 (d, *J*=8.0 Hz, 1 H) 8.05 (s, 1 H) 8.06 (s, 1 H) 8.21 (s, 1 H) 8.26 (bs, 1 H) 11.21 (s, 1 H) MS m/z 333 (M+18)⁺ r.t. 2.55 min.

### (10) 5-{3-[(dimethylamino)carbonyl]phenyl}-1H-indole-7-carboxamide

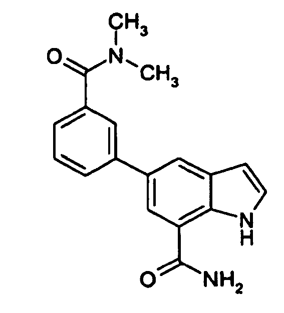

The title compound (6 mg) was prepared using general method A except that 36 mg (0.18 mmol) {3-[(dimethylamino)carbonyl]phenyl}boronic acid was used instead of phenyl boronic acid.

1 H NMR (400 MHz, DMSO-D6) δ ppm 2.97 (s, 3 H) 3.02 (s, 3 H) 6.64 (t, *J*=2.5 Hz, 1 H) 7.33 (d, *J*=7.5 Hz, 1 H) 7.37 (t, *J*=7.6, 1 H) 7.4 (bs, 1 H) 7.52 (t, *J*=7.7 Hz, 1 H) 7.8 (s, 1 H) 7.85 (d, *J*=8.0 Hz, 1 H) 8.04 (s, 1 H) 8.07 (s, 1 H) 8.29 (bs, 1 H) 11.16 (s, 1 H) MS m/z 308 (M+1)⁺ r.t. 2.6 min.

### (11) 5-(3-fluorophenyl)-1H-indole-7-carboxamide

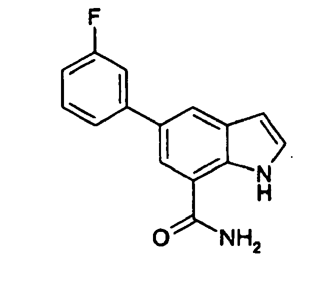

The title compound (7.8 mg) was prepared using general method A except that 26 mg (0.18 mmol) (3-fluorophenyl)boronic acid was used instead of phenyl boronic acid.

1H NMR (400 MHz, DMSO-D6) δ ppm 6.54 (t, *J*=2.5 Hz, 1 H) 7.14 (td, *J*=2.1, 8.3 Hz, 1 H) 7.38 (t, *J*=2.76, 1 H) 7.44 (bs, 1 H) 7.50 (q, *J*=7.4 Hz, 1 H) 7.65 (d, *J*=8.3, 2 H) 8.05 (s, 1 H) 8.09 (s, 1 H) 8.26 (bs, 1 H) 11.18 (s, 1 H) MS m/z 255 (M+1)⁺ r.t. 3.15 min.

### (12) 5-[3-(methyloxy)phenyl]-1H-indole-7-carboxamide

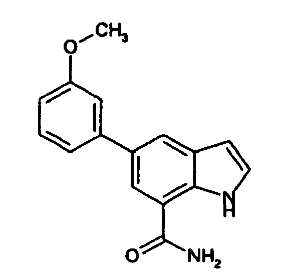

The title compound (2.2 mg) was prepared using general method A except that 28 mg (0.18 mmol) (3-methyloxy) phenyl)boronic acid was used instead of phenyl boronic acid. The crude reaction mixture was filtered through a silica solid phase extraction cartridge, concentrated *in vacuo*, re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1 H NMR (400 MHz, DMSO-D6) δ ppm 3.85 (s, 3 H) 6.53 (t, *J*=2.5 Hz, 1 H) 6.9 (dt, *J*=2.1, 7.3 Hz, 1 H) 7.32-7.37 (m, 4 H) 7.42 (bs, 1 H) 8.0 (s, 1 H) 8.03 (s, 1 H) 8.26 (bs, 1H) 11.13 (s, 1H) MS r.t. 3.09 min.

### (13) 5-(3-cyanophenyl)-1H-indole-7-carboxamide

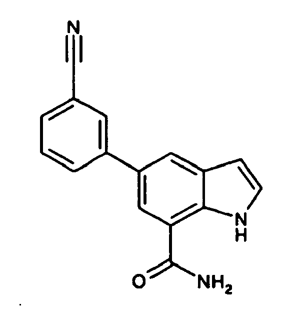

The title compound (0.9 mg) was prepared using general methods A except that 27 mg (0.18 mmol) (3-cyanophenyl)boronic acid was used instead of phenyl boronic acid. The product was purified further by triturating with water, isolated by filtration and dried in a vacuum pistol.

MS m/z 260 (M-1) r.t. 3.05 min.

### (14) 5-(3-hydroxyphenyl)-1H-indole-7-carboxamide

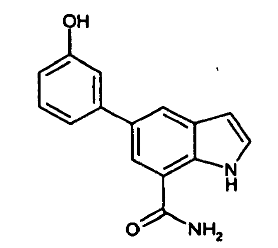

The title compound (6 mg) was prepared using general method A except that 26 mg (0.18 mmol) (3-hydroxyphenyl)boronic acid was used instead of phenyl boronic acid.

1 H NMR (400 MHz, DMSO-D6) δ ppm 6.53 (t, *J*=2.4 Hz, 1 H) 6.73(dd, *J*=1.5, 7.8 Hz, 1H) 7.13 (s, 1H) 7.17 (d, *J*=7.8 Hz, 1H) 7.25 (t, *J*=7.8 Hz, 1H) 7.35 (t, *J*=2.8 Hz, 1H) 7.38 (bs, 1H) 7.93 (s, 1H) 7.95 (s, 1H) 8.24 (bs, 1H) 9.43 (s, 1H) 11.11 (s, 1H) MS m/z 251 (M-1) r.t. 2.28 min.

### (15) 5-(3-quinolinyl)-1H-indole-7-carboxamide

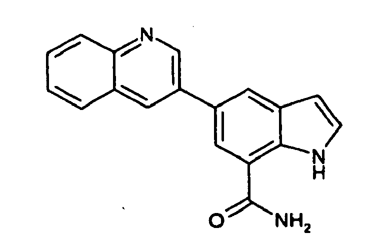

The title compound (3.6 mg) was prepared using general method A except that 32 mg (0.18 mmol) 3-quinolinylboronic acid was used instead of phenyl boronic acid. The product from the microwave reaction step was re-dissolved in 0.5 mL dimethylsulfoxide/methanol (50:50) and the title compound isolated by filtration and dried in a vacuum pistol.

1H NMR (400 MHz, DMSO-D6) δ ppm 6.63 (t, *J*=2.5 Hz, 1H) 7.45 (t, *J*=2.7 Hz, 1H) 7.59 (bs, 1H) 7.85 (t, *J*=7.5, 1H) 7.98 (t, *J*=7.5, 1H) 8.25 (t, *J*=8.3, 2H) 8.33 (s, 1H) 8.35 (bs, 1H) 8.39 (s, 1H) 9.25 (s, 1H) 9.68 (s, 1H) 11.34 (s, 1H) MS (M+1)⁺ 288 r.t. 2.90 min.

### (16) 5-(1-benzofuran-4-yl)-1H-indole-7-carboxamide

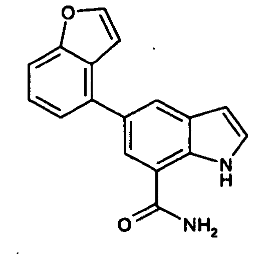

The title compound (7.4 mg) was prepared using general method A except that 45 mg (0.18 mmol) 1-benzofuran-4-ylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.58 (t, *J*=2.3 Hz, 1H) 7.11 (d, *J*=1.3 Hz, 1H) 7.4-7.47 (m, 4H) 7.58 (d, *J*=7.8, 1H) 7.95 (s, 1H) 7.99 (s, 1H) 8.07 (d, *J*=2.3, 1H) 8.19 (bs, 1H) 11.19 (s, 1H) MS (M+1)⁺ 277 r.t. 3.28 min.

### (17) 5-(1,3-benzodioxol-5-yl)-1H-indole-7-carboxamide

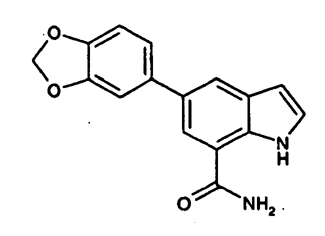

The title compound (9.1 mg) was prepared using general method A except that 31 mg (0.18 mmol) 1,3-benzodioxol-5-ylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.06 (s, 2H) 6.50 (t, *J*=2.4 Hz; 1H) 7.00 (d, *J*=8.0 Hz, 1H) 7.26 (dd, *J*=1.5, 8.2 Hz, 1H) 7.35 (t, *J*=2.7, 1H) 7.38 (d, *J*=1.5, 1H) 7.39 (bs, 1 H) 7.94 (s, 2H) 8.22 (bs, 1H) 11.09 (s, 1H) MS (M+1)⁺ 281 r.t. 3.14 min.

### (18) 5-[(E)-2-phenylethenyl]-1H-indole-7-carboxamide

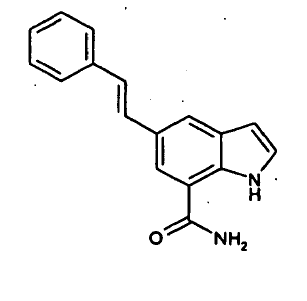

The title compound (2.6 mg) was prepared using general method A except that 27 mg (0.18 mmol) [(*E*)-2-phenylethenyl]boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.49 (t, *J*=2.4 Hz, 1H) 7.25 (t, *J*=7.4 Hz, 1H) 7.3 (d, *J*=9.0 Hz, 2H) 7.34 (t, *J*=2.8 Hz, 1H) 7.39 (t, *J*=7.7, 2H) 7.45 (bs, 1H) 7.58 (d, *J*=7.5, 2H) 7.88 (s, 1H) 8.06 (s, 1H) 8.18 (bs, 1H) 11.14 (s, 1H) MS (M+1)⁺ 263 r.t. 3.45 min.

### (19) 5-(5-pyrimidinyl)-1H-indole-7-carboxamide

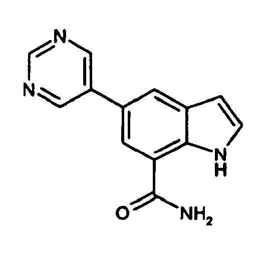

The title compound (2.5 mg) was prepared using general method A except that 23 mg (0.18 mmol) 5-pyrimidinylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.58 (t, *J*=2.4 Hz, 1H) 7.42 (t, *J*=2.8 Hz, 1H) 7.51 (bs, 1H) 8.13 (s, 1H) 8.22 (s, 1H) 8.24 (bs, 1H) 9.15 (s, 1H) 9.25 (s, 2H) 11.28 (bs, 1 H) MS (M+1)⁺ 239 r.t. 2.35 min.

### (20) 5-(3-biphenylyl-1H-indole-7-carboxamide

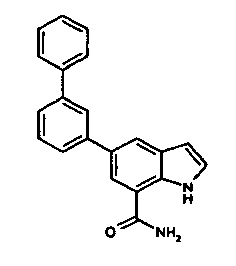

The title compound (1.4 mg) was prepared using general method A except that 37 mg (0.18 mmol) 3-biphenylylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

MS (M+1)⁺ 313 r.t. 3.61 min.

### (21) 5-(1-benzofuran-2-yl)-1H-indole-7-carboxamide

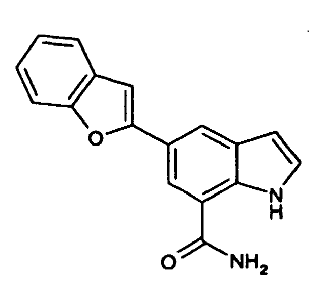

The title compound (8.7 mg) was prepared using general method A except that 30 mg (0.18 mmol) 1-benzofuran-2-ylboronic acid was used instead of phenyl boronic acid. The product from the microwave reaction step was re-dissolved in 0.5 mL dimethylsulfoxide/methanol (50:50) and purified by preparative HPLC using an acetonitrilelwater elution system. The relevant fractions were blown down to dryness with nitrogen and further dried in a vacuum pistol.

1H NMR (400 MHz, DMSO-D6) δ ppm 6.60 (t, *J*=2.3 Hz, 1H) 7.27 (m, 2 H) 7.35 (s, 1H) 7.4 (t, *J*=2.7, 1H) 7.52 (bs, 1H) 7.62 (d, *J*=7.8 Hz, 1H) 7.66 (d, *J*=7 Hz, 1H) 8.29 (s, 3 H) 11.29 (s, 1H) MS (M+1)⁺ 277 r.t. 3.56 min.

### (22) 5-(1-benzothien-2-yl)-1H-indole-7-carboxamide

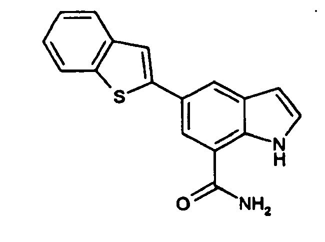

The title compound (13.6 mg) was prepared using general method A except that 33 mg (0.18 mmol) 1-benzothien-2-ylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.58 (t, *J*=2.3 Hz, 1 H) 7.3-7.4 (m, 3 H) 7.51 (bs, 1 H) 7.83 (s, 1H) 7.86 (s, 1H) 7.97 (d, *J*=7.8 Hz, 1H) 8.07 (s, 1H) 8.19 (s, 1H) 8.31 (bs, 1 H) 11.26 (s, 1H) MS (M+1)⁺ 293 r.t. 3.62 min.

### (23) 5-[3-(hydroxymethyl)phenyl]-1H-indole-7-carboxamide

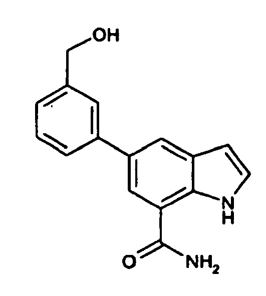

The title compound (4.8 mg) was prepared using general method A except that 28 mg (0.18 mmol) [3-(hydroxymethyl)phenyl]boronic acid was used instead of phenyl boronic acid. The crude reaction mixture was filtered through a silica solid phase extraction cartridge, concentrated *in vacuo,* redissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 4.59 (d, *J*=5.5, 2 H) 5.23 (t, *J*=5.8, 1 H) 6.54 (t, *J*=2.4 Hz, 1H) 7.28 (d, *J*=7.5 Hz, 1H) 7.36 (t, *J*=2.8, 1H) 7.42 (t, *J*=7.7, 2H) 7.64 (d, *J*=8.0 Hz, 1H) 7.71 (s, 1H) 8.00 (s, 2H) 8.25 (bs, 1H) 11.13 (s, 1H) MS (M+1)⁺ 267 r.t. 2.74 min.

### (24) 5-(2-naphthalenyl)-1H-indole-7-carboxamide

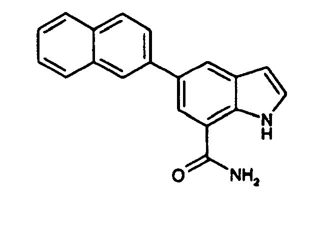

The title compound (4.3 mg) was prepared using general method A except that 32 mg (0.18 mmol) 2-naphthalenylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.58 (t, *J*=2.4, 1H) 7.39 (t, *J*=2.7, 1H) 7.47-7.56 (m, 3H) 7.94 (d, *J*=7.8 Hz, 1H) 7.98 (d, *J*=7.8, 1H) 8.01 (s, 1H) 8.19 (d, *J*=2.5, 1H) 8.31 (bs, 1H) 11.18 (s, 1H) MS (M+1)⁺ 287 r.t. 3.58 min.

### (25) 5-(4-fluorophenyl)-1H-indole-7-carboxamide

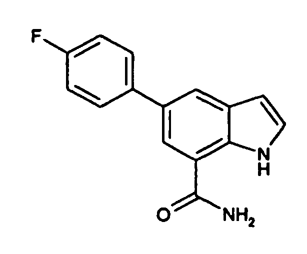

The title compound (4.1 mg) was prepared using general method A except that 26 mg (0.18 mmol) (4-fluorophenyl)boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.53 (s, 1H) 7.30 (t, *J*=8.7, 2H) 7.37 (s, 1H) 7.42 (bs, 1H) 7.81 (dd, *J*=8.1, 5.6 Hz, 2H) 7.99 (d, *J*=2.3, 2H) 8.24 (bs, 1H) 11.14 (s, 1H) MS (M+1)⁺ 296 r.t. 3.24 min.

### (26) 5-[6-(methyloxy)-3-pyridinyl]-1H-indole-7-carboxamide

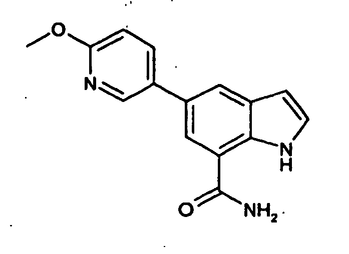

The title compound (4.9 mg) was prepared using general method A except that 28 mg (0.18 mmol) [6-(methyloxy)-3-pyridinyl]boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 3.90 (s, 3H) 6.53 (t, *J*=2.4 Hz, 1H) 6.93 (d, *J*=8.5 Hz, 1H) 7.37(t, *J*=2.7 Hz, 1H) 7.44 (bs, 1H) 7.99 (s, 1H) 8.0 (s, 1H) 8.11 (dd, *J*=8.5, 2.5 Hz, 1H) 8.21 (bs, 1H) 8.57 (d, *J*=2.5 Hz, 1H) 11.15 (s, 1H) MS (M+1)⁺ 268 r.t. 2.90 min.

### (27) 5-[4-(hydroxymethyl)phenyl]-1H-indole-7-carboxamide

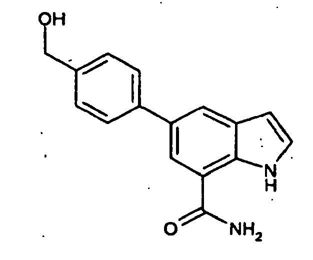

The title compound (3.3 mg) was prepared using general method A except that 28 mg (0.18 mmol) [4-(hydroxymethyl)phenyl]boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 4.55 (d, *J*=5.8 Hz, 2H) 5.19 (t, *J*=5.8 Hz, 1H) 6.53 (t, *J*=2.4 Hz, 1H) 7.36 (t, *J*=2.8 Hz, 1H) 7.4 (d, *J*=8.0 Hz, 2H) 7.74 (d, *J*=8.3 Hz, 2H) 8.01 (s, 2H) 8.24 (bs, 1H) 11.12 (s, 1H) MS (M+1)⁺ 267 r.t. 2.70 min.

### (28) 5-(3-chlorophenyl)-1H-indole-7-carboxamide

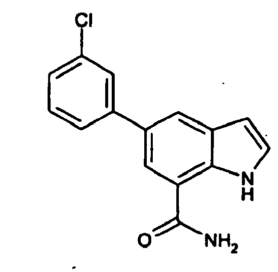

The title compound (5.8 mg) was prepared using general method A except that 29 mg (0.18 mmol) (3-chlorophenyl)boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.55 (t, *J*=2.5 Hz, 1H) 7.38 (m, 2H) 7.45 (bs, 1H) 7.49 (t, *J*=7.9 Hz, 1H) 7.76 (d, *J*=7.8 Hz, 2H) 7.88 (s, 1H) 8.04 (s, 1H) 8.08 (s, 1H) 8.28 (bs, 1H) 11.19 (s, 1H) MS (M+1)⁺ 271 r.t. 3.44 min.

### (29) 5-(2-methylphenyl)-1H-indole-7-carboxamide

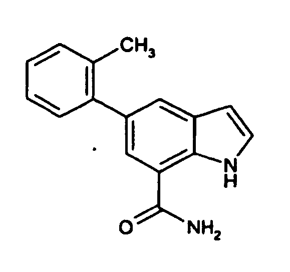

The title compound (2.9 mg) was prepared using general method A except that 25 mg (0.18 mmol (2-methylphenyl)boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 2.26 (s, 3H) 6.58 (t, *J*=2.5 Hz, 1H) 7.25-7.32 (m, 4 H) 7.35 (bs, 1H) 7.38 (t, *J*=2.7 Hz, 1H) 7.66 (s, 1H) 7.68 (s, 1H) 8.08 (bs, 1H) 11.15 (s, 1H) MS (M+1)⁺ 251 r.t. 3.25 min.

### (30) 5-{3-[(phenylmethyl)oxy]phenyl}-1H-indole-7-carboxamide

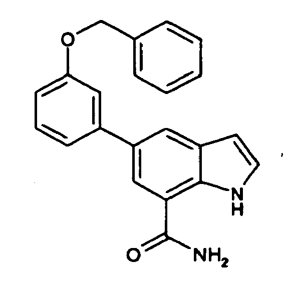

The title compound (7.8 mg) was prepared using general method A except that 42 mg (0.18 mmol {3-[(phenylmethyl)oxy]phenyl}boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 5.20 (s, 2 H) 6.53 (t, *J*=2.3 Hz, 1H) 6.97 (m, 1H) 7.32-7.43 (m, 8H) 7.51 (d, *J*=7.28 Hz, 2H) 8.01 (s, 1H) 8.04 (s, 1H) 8.26 (bs, 1H) 11.14 (s, 1H) MS (M+1)⁺ 343 r.t. 3.61 min.

### (31) 5-(2-chlorophenyl)-1H-indole-7-carboxamide

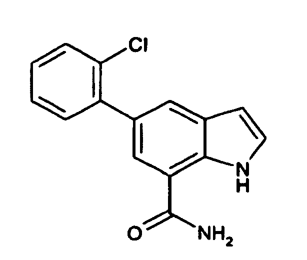

The title compound (4.1 mg) was prepared using general method A except that 29 mg (0.18 mmol (2-chlorophenyl)boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.53 (t, *J*=2.5 Hz, 1H) 7.37-7.45 (m, 4H) 7.50 (dd, *J*=7.5, 1.6 Hz, 1H) 7.58 (dd, *J*=7.7, 0.9 Hz, 1H) 7.76 (s, 1H) 7.78 (s, 1H) 8.10 (bs, 1H) 11.20 (s, 1H) MS (M+1)⁺ 271 r.t. 3.24 min.

### (32) 5-(3,5-dimethyl-4-isoxazolyl)-1H-indole-7-carboxamide

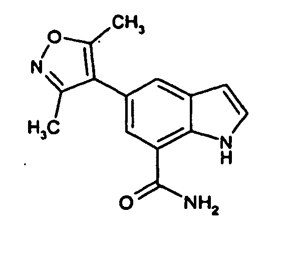

The title compound (4.1 mg) was prepared using general method A except that 26 mg (0.18 mmol) (3,5-dimethyl-4-isoxazolyl)boronic acid was used instead of phenyl boronic acid.

1H NMR (400 MHz, DMSO-D6) δ ppm 2.20 (s, 3 H) 2.40 (s, 3H) 6.51 (t, J=2.4 Hz, 1H) 7.39 (t, *J*=2.7 Hz, 1H) 7.44 (bs, 1H) 7.64 (s, 1H) 7.70 (s, 1H) 8.11 (bs, 1H) 11.22 (s, 1 H) MS m/z 256 (M+1)⁺ r.t. 2.62 min.

### (33) 5-{2-[(phenylmethyl)oxy]phenyl}-1H-indole-7-carboxamide

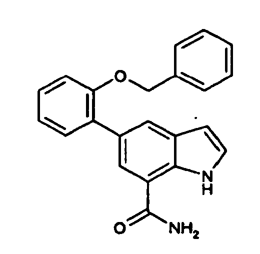

The title compound (4.1 mg) was prepared using general method A except that 42 mg (0.18 mmol) {2-[(phenylmethyl)oxy]phenyl}boronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 5.13 (s, 2H).6.48 (t. *J*=2.4 Hz, 1H) 7.07 (t. *J*=7.4 Hz, 1H) 7.20(d, *J*=8.0 Hz, 1H) 7.25-7.35 (m, 6H) 7.42 (m, 3H) 7.89 (s, 1H) 7.91 (s, 1H) 8.06 (bs, 1H) 11.09 (s, 1H) MS (M+1)⁺ 343 r.t. 3.48 min.

### (34) 5-(5-quinolinyl)-1H-indole-7-carboxamide

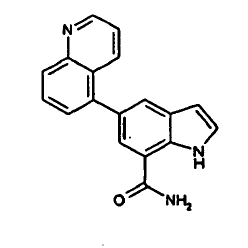

The title compound (7.1 mg) was prepared using general method A except that 32 mg (0.18 mmol) 5-quinolinylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.58 (t, *J*=2.4 Hz, 1H) 7.42 (bs, 1H) 7.45 (t, *J*=2.7 Hz, 1H) 7.67 (dd, *J*=4.3, 8.53 Hz, 1H) 7.73 (d, *J*=7.0 Hz, 1H) 7.85 (s, 2H) 7.95 (t, *J*=7.8 Hz, 1H) 8.14 (m, 2H) 8.45 (d, *J*=8.29 Hz, 1H) 9.05 (d, *J*=4.0 Hz, 1H) 11.29 (s, 1H) MS (M+1)⁺ 288 r.t. 2.61 min.

### (35) 5-(1-naphthatenyl)-1H-indole-7-carboxamide

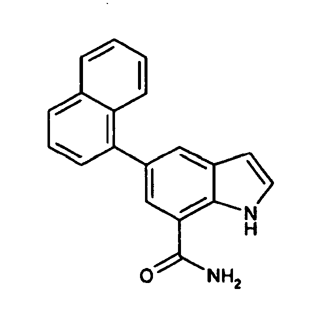

The title compound (10.4 mg) was prepared using general method A except that 32 mg (0.18 mmol 1-naphthalenylboronic acid was used instead of phenyl boronic acid. The product from the microwave reaction step was re-dissolved in 0.5 mL dimethylsulfoxide/methanol (50:50) and purified by preparative HPLC using an acetonitrile/water elution system. The relevant fractions were blown down to dryness with nitrogen and further dried in a vacuum pistol.

1H NMR (400 MHz, DMSO-D6) δ ppm 6.56 (t, *J*=2.5 Hz, 1H) 7.36 (bs, 1H) 7.43 (t, *J*=2.7 Hz, 1H) 7.47-7.52(m, 3H) 7.60 (t, *J*=7.6 Hz, 1H) 7.81 (s, 1H) 7.83 (s, 1H) 7.86 (s, 1H) 7.95 (d, *J*=8.0 Hz, 1H) 8.01 (d, *J*=8.3 Hz, 1H) 8.09 (bs, 1H) 11.23 (s, 1H) MS (M+1)⁺ 287 r.t. 3.45 min.

### (36) 3-bromo-5-phenyl-1H-indole-7-carboxamide

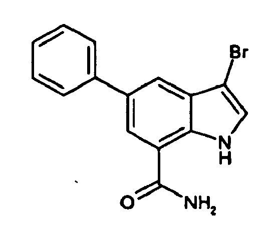

5-phenyl-1H-indole-7-carboxamide (30 mg, 0.13 mmol) and N-bromosuccinimide (25 mg, 0.14 mmol) were dissolved in dry dichloromethane (5 mL) and stirred under a nitrogen atmosphere at room temperature for 5 hours. The mixture was then pre-adsorbed onto silica and purified by silica flash chromatography using a dichloromethane/methanol elution system on an ISCO sq16x machine. The relevant fractions were combined and concentrated *in vacuo*, re-dissolved in 0.5 mL dimethylsulfoxide/methanol (50:50) and purified by preparative HPLC using an acetonitrile/water elution system. The relevant fractions were blown down to dryness with nitrogen and dried in a vacuum pistol to give 11 mg of the title compound.

1H NMR (400 MHz, DMSO-D6) δ ppm 7.36 (t, *J*=7.4 Hz, 1H) 7.50 (m, 3H) 7.53 (bs, 1H) 7.80 (m, 3H) 8.10 (d, *J*=1.5 Hz, 1H) 8.33 (bs, 1H) 11.45 (s, 1H) MS (M-1) 313/315 (1:1 ratio) r.t. 3.48 min.

### (37) 3-iodo-5-phenyl-1H-indole-7-carboxamide

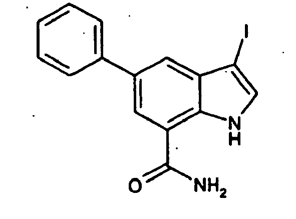

5-phenyl-1H-indole-7-carboxamide (0.80 g, 3.39 mmol) and N-iodosuccinimide (0.76 g, 3.39 mmol) were dissolved in dichloromethane (150 mL) and stirred under a nitrogen atmosphere at room temperature for 16 hours. The reaction mixture was partitioned with sodium hydroxide solution (2 M), separated and then concentrated *in vacuo* to give the title compound as a beige solid (1.16 g).

1H NMR (400 MHz, DMSO-D6) δ ppm 7.36 (t, *J*=7.3 Hz, 1H) 7.50 (m, 4H) 7.66 (s, 1H) 7.79 (d, *J*=7.5 Hz, 2 H) 8.09 (s, 1H) 8.32 (bs, 1H) 11.51 (s, 1H) MS (M-1) 361 r.t. 3.56 min.

This compound may be used in the preparation of intermediate (i)

### (38) 3,5-diphenyl-1H-indole-7-carboxamide

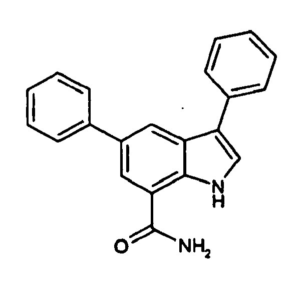

### General method B

1,1-dimethylethyl 7-[(bis{[(1,1-dimethylethyl)oxy]carbonyl}amino)carbonyl]-3-iodo-5-phenyl -1H-indole-1-carboxylate (50 mg, 75 µmol), phenyl boronic acid (18 mg, 0.15 mmol), potassium carbonate (30 mg, 0.138 mmol) and 1,1'-bis(diphenylphosphino)ferrocene dichloropalladium (II) (5 mg, 6 µmol) were combined in dimethylformamide (1 mL) and heated at 80 °C for 2 hours under nitrogen. The reaction mixture was then filtered through a 1 g silica solid phase extraction cartridge, concentrated *in vacuo* and then re-dissolved in 0.5 mL dimethylsulfoxide/methanol (50:50) and purified by preparative HPLC using an acetonitrite/water elution system. The relevant fractions were blown down to dryness with nitrogen, re-dissolved in ethanol (2 mL) and concentrated hydrochloric acid (15 µL) and then heated in a Smith synthesizer microwave for 5 minutes at 150 °C. The solvent was removed *in vacuo* to give the title compound (5.8 mg).

1H NMR (400 MHz, DMSO-D6) δ ppm 7.27 (t, *J*=7.4 Hz, 1H) 7.35 (t, *J*=7.4 Hz, 1H) 7.47(m, 5H) 7.65 (d, *J*=2.5 Hz, 1H) 7.73 (d, *J*=7.3 Hz, 2H) 7.80 (d, *J*=7.3 Hz, 2H) 8.08 (s, 1H) 8.21 (s, 1H) 8.31 (bs, 1H) 11.39 (s, 1H) MS m/z 313 (M+1)⁺ r.t. 3.61 min.

### (39) 3-{4-[(methylsulfonyl)amino]phenyl}-5-phenyl-1H-indole-7-carboxamide

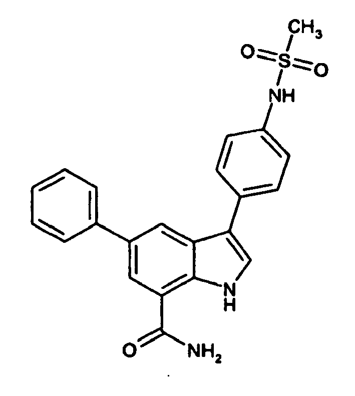

The title compound (12.3 mg) was prepared using general method B except that 39 mg (0.18 mmol) (4-[(methylsulfonyl)amino]phenyl)boronic acid was used instead of phenyl boronic acid.

1H NMR (400 MHz, DMSO-D6) δ ppm 3.01 (s, 3H) 7.3-7.36 (m, 3H) 7.48 (t, *J*=7.7 Hz, 3 H) 7.62 (d, *J*=2.5 Hz, 1H) 7.71 (d, *J*=8.5 Hz, 2H) 7.82 (d, *J*=7.5 Hz, 2H) 8.07 (s, 1H) 8.20 (s, 1H) 8.30 (bs, 1H) 9.72 (s, 1H) 11.37 (s, 1H) MS m/z 404 (M-1) r.t. 3.15 min.

### (40) 5-phenyl-3-(3-pyridinyl)-1H-indole-7-carboxamide

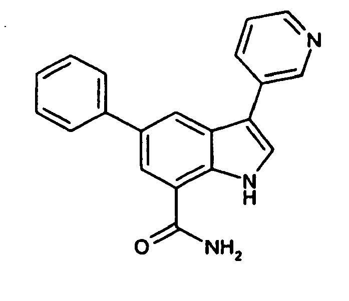

### General method C

1,1-dimethylethyl 7-[(bis{[(1,1-dimethylethyl)oxy]carbonyl}amino)carbonyl]-3-iodo-5-phenyl -1H-indole-1-carboxylate (100 mg, 0.15 µmol), 3-pyridinylboronic acid (37 mg, 0.30 mmol), potassium carbonate (60 mg, 0.28 mmol) and 1,1'-bis(diphenylphosphino)ferrocenedichloropalladium (II) (10 mg, 12 µmol) were combined in dimethylformamide (2 mL) and heated at 80 °C for 2 hours under nitrogen. The reaction mixture was pre-adsorbed onto silica *in vacuo* and purified by silica flash chromatography using an ethyl acetate/cyclohexane/triethylamine elution system on an ISCO sq16x machine. The combined fractions were concentrated *in vacuo*, re-dissolved in ethanol (2 mL) and concentrated hydrochloric acid (15 µL) and heated in a Smith synthesizer microwave for 5 minutes at 150 °C. The ethanol was removed *in vacuo* and the residue re-dissolved in 0.5 mL dimethylsulfoxide/methanol (50:50) and purified by preparative HPLC using an acetonitrile/water elution system. The relevant fractions were blown down to dryness with nitrogen to give the title compound (7 mg).

1H NMR (400 MHz, DMSO-D6) δ ppm 7.36 (m, 1H) 7.50 (t, *J*=7.7 Hz, 2H) 7.60 (bs, 1H) 7.86 (d, *J*=7.3 Hz, 2H) 7.93 (dd, *J*=5.5, 8.0 Hz, 1H) 8.05 (d, *J*=2.8 Hz, 1H) 8.14 (d, *J*=1 Hz, 1H) 8.32 (s, 1H) 8.38 (bs, 1H) 8.66 (d, *J*=5.0 Hz, 1H) 8.79 (d, *J*=8.0 Hz, 1H) 9.24 (s, 1H) 11.81 (s, 1H) MS m/z 314 (M+1)⁺ r.t. 2.63 min

### (41) 3-(4-{[(2-aminoethyl)amino]carbonyl}phenyl)-5-phenyl-1H-indole-7-carboxamide

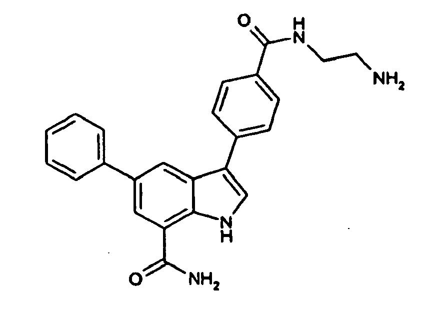

The title compound (55 mg) was prepared using general method C except that 124 mg (0.30 mmol) [4-({[2-({[(1,1-dimethylethyl)oxy]carbonyl}amino)ethyl]amino}carbonyl)phenyl] boronic acid was used instead of 3-pyridinylboronic acid.

1H NMR (400 MHz, DMSO-D6) δ ppm 3.01 (t, J=6.2 Hz, 2H) 3.55 (q, J=5.8 Hz, 2H) 7.36 (t, *J*=7.3 Hz, 1H) 7.49 (t, *J*=7.8 Hz, 2H) 7.52 (bs, 1H) 7.79 (d, J=2.5 Hz, 1H) 7.82 (d, *J*=7.5 Hz, 2H) 7.87 (d, *J*=8.5 Hz, 2H) 8.0 (d, *J*=8.3 Hz, 2H) 8.11 (s, 1H) 8.13 (bs, 2H) 8.26 (s, 1H) 8.34 (bs, 1H) 8.72 (t, *J*=5.5 Hz, 1H) 11.53 (s, 1H) MS m/z 399 (M+1)⁺ r.t. 2.39 min

### (42) 3-[4-({[4-(methyloxy)-3-(4-methyl-1-piperazinyl)phenyl]amino}carbonyl)phenyl]-5-phenyl-1H-indole-7-carboxamide formate

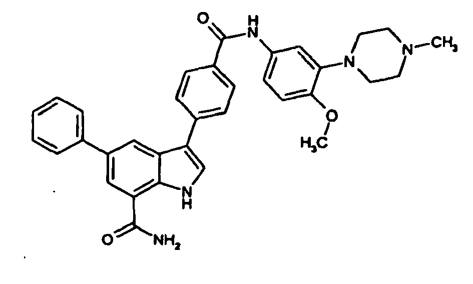

The title compound (34 mg) was prepared using general method, C except that 111 mg (0.30 mmol) [4-({[4-(methyloxy)-3-(4-methyl-1-piperazinyl)phenyl]amino}carbonyl)phenyl] boronic acid was used instead of 3-pyridinylboronic acid.

1H NMR (400 MHz, DMSO-D6) δ ppm 2.33 (bs, 3H) 2.62 (bs, 4H) 3.03 (bs, 4H) 3.78 (s, 3H) 6.92 (d, *J*=8.8 Hz, 1H) 7.36 (t, *J*=7.4 Hz, 1H) 7.41 (d, *J*=2.3 Hz, 1H) 7.46-7.53 (m, 4H) 7.82 (m, 3H) 7.9 (d, *J*=8.5 Hz, 2H) 8.05 (d, *J*=8.5 Hz, 2H) 8.11 (s, 1H) 8.15 (s, 1 H) 8.29 (s, 1H) 8.34 (bs, 1H) 10.05 (s, 1H) 11.55 (s, 1H) MS m/z 560.6 (M+1)⁺ r.t. 2.71 min

### (43) 5-phenyl-3-[3-(trifluoromethyl)phenyl]-1H-indole-7-carboxamide

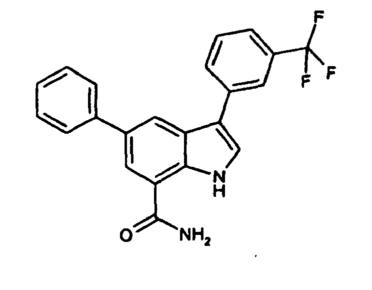

1,1-dimethylethyl 7-[(bis{[(1,1-dimethytethyl)oxy]carbonyl}amino)carbonyl]-3-iodo-5-phenyl -1H-indole-1-carboxylate (75 mg, 0.11 mmol), [3-(trifluoromethyl)phenyl]boronic acid (43 mg, 0.23 mmol), potassium carbonate (45 mg, 0.21 mmol) and 1,1' bis(diphenylphosphino)ferrocenedichloropalladium (II) (7.5 mg, 9 µmol) were combined in dimethylformamide (2 mL) and heated at 80°C for 2 hours under nitrogen. The reaction mixture was pre-adsorbed onto silica *in vacuo* and purified by silica flash chromatography using an ethyl acetate/cyclohexane/triethylamine elution system on an ISCO sq16x machine. The combined fractions were concentrated *in vacuo*, re-dissolved in ethanol (2 mL) and concentrated hydrochloric acid (15µL) and then heated in a Smith synthesizer microwave for 5 minutes at 150 °C. The ethanol was removed *in vacuo* and the residue re-dissolved in 0.5 mL dimethylsulfoxide/methanol (50:50) and purified by preparative HPLC using an acetonitrile/water elution system. The relevant fractions were blown down to dryness with nitrogen to give the title compound (5 mg).

1H NMR (400 MHz, DMSO-D6) δ ppm 7.35 (t, *J*=7.4 Hz, 1H) 7.40 (t, *J*=7.7 Hz, 2H) 7.52 (bs, 1H) 7.61 (d, *J*=7.8 Hz, 1H) 7.70 (t, *J*=7.7 Hz, 1H) 7.8 (d, *J*=7.3 Hz, 2H) 7.84 (d, *J*=2 Hz, 1H) 7.99 (s, 1H) 8.10 (m, 2H) 8.20 (s, 1H) 8.33 (bs, 1H) 11.55 (s, 1H) MS m/z 379 (M-1) r.t. 3.83 min.

### (44) 5-bromo-3-iodo-1H-indole-7-carboxamide

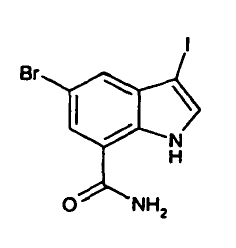

5-bromo-1H-indole-7-carboxamide (100 mg, 0.42 mmol) and N-iodosuccinimide (95 mg, 0.42 mmol) were dissolved in dichloromethane (10 mL) and stirred at room temperature for 16 hours. The reaction mixture was then partitioned with sodium hydroxide solution (2 M), separated and the organic layer pre-adsorbed onto silica *in vacuo.* Purification was achieved by silica chromatography using an ethyl acetate-cyclohexane gradient system on the ISCO sq16x machine. The relevant fractions were combined and concentrated in *vacuo* to give the title compound (96 mg).

1H NMR (400 MHz, DMSO-D6) δ ppm 7.52 (s, 1H) 7.58 (d, *J*=1.3 Hz, 1H) 7.60 (bs, 1H) 7.93 (d, *J*=1.0 Hz, 1H) 8.24 (bs, 1H) 11.64 (s, 1H) MS m/z 363/365 (1:1 ratio) (M-1) r.t. 3.47 min.

### (45) 3-(1-ethyl-3-piperidinyl)-5-phenyl-1H-indole-7-carboxamide

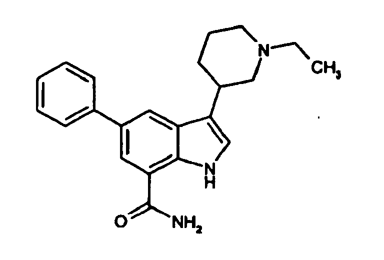

A mixture of 5-phenyl-1*H-*indole-7-carboxamide (202 mg, 0.86 mmol), sodium methoxide (10.3 mL of a 0.5 M solution in methanol, 5.15 mmol) and 1-(phenylmethyl)-3-piperidinone hydrochloride hydrate (625 mg, 2.56 mmol) was heated at reflux under nitrogen for 18 hours. 3 A molecular sieves (10) were added and reflux continued for a further 22 hours before cooling to room temperature and the addition of water (1 mL). After concentration *in vacuo* and pre-adsorbtion onto silica, purification by silica flash chromatography using a methanol/dichloromethane/ammonia gradient system on an ISCO sq16x machine yielded the intermediate alkene (59.1 mg) as a yellow-brown oil. After dissolution in ethanol/ glacial acetic acid (50:1, 15 mL) and the addition of palladium hydroxide on carbon (20 wt% palladium, 12 mg) the mixture was stirred vigorously under an atmosphere of hydrogen for 3 days. After filtration through celite and concentration *in vacuo* with pre-adsorbtion onto silica, purification by silica chromatography using a methanol/dichloromethane/ammonia gradient system on an ISCO sq16x machine yielded the title compound (5.3 mg, 0.015 mmol).

### Alternative procedure:

A mixture of 5-phenyl-1H-indole-7-carboxamide (756 mg, 3.20 mmol), potassium hydroxide (1.98 g, 35.3 mmol), and 1-ethyl-3-piperidinone hydrochloride (5.24 g, 32.0 mmol) in methanol (35 mL) was heated at reflux under nitrogen for 20 hours. A further portion of potassium hydroxide (180. mg, 3.20 mmol) was added, and reflux continued for 5 days. After concentration in vacuo with pre-adsorbtion onto silica, purification by silica chromatography using a methanol/dichloromethane/ammonia gradient system on an ISCO sq16x machine yielded a mixture of intermediate alkenes (463 mg) as a yellow-orange oil. After dissolution in ethanol/glacial acetic acid (50:1, 20 mL) and the addition of palladium hydroxide on carbon (20 wt% palladium, 93 mg) the mixture was stirred vigorously under an atmosphere of hydrogen for 3 days. The reaction mixture was filtered and concentrated in vacuo with pre-adsorbtion onto silica. Purification by preparative HPLC was followed by dissolution of the purified material in methanol (5 mL). The methanol solution was passed through an aminopropyl solid-phase extraction cartridge, rinsing with methanol (5 mL), and the combined methanol eluents concentrated in vacuo to yield the title compound (9.9 mg).

1H NMR (400 MHz, DMSO-D6) δ ppm 1.08 (t, J=7.3 Hz, 3H) 1.55 (qd, J=12.1, 4.0 Hz, 1 H) 1.67-1.84 (m, 2H) 2.02 (d, J=11.3 Hz, 1H) 2.32-2.20 (m, 2 H) 2.68-2.55 (m, 2H) 3.08 (d, J=11.3 Hz, 1H) 3.23 (d, J=10.5 Hz, 2H) 7.19 (d, J=2.0 Hz, 1H) 7.33 (t, J=7.4 Hz, 1 H) 7.40 (br s, 1H) 7.48 (t, J=7.7 Hz, 2H) 7.79 (d, J=7.5 Hz, 2H) 8.01 (s, 2H) 8.23 (s, 1 H) 10.94 (bs, 1H) MS m/z 348 (M+1)⁺ r.t. 2.26 min.

### (46) 5-phenyl-3-(3-piperidinyl)-1H-indole-7-carboxamide and 5-phenyl-3-[1-(phenylmethyl)-3-piperidinyl]-1H-indole-7-carboxamide

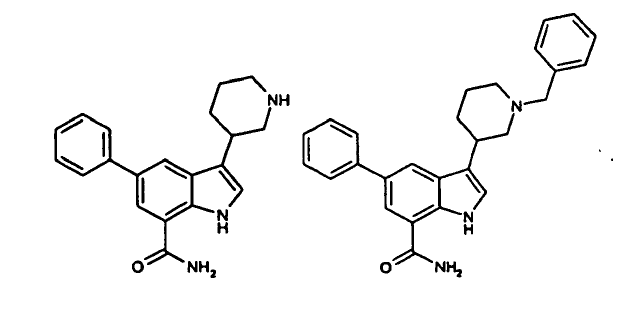

A mixture of 5-phenyl-1 H-indole-7-carboxamide (794 mg, 3.36 mmol), potassium hydroxide (2.08 g, 37.1 mmol), and 1-(phenylmethyl)-3-piperidinone hydrochloride hydrate (8.19 g, 33.60 mmol) in methanol (35 mL) was heated at reflux under nitrogen for 20 hours. A further portion of potassium hydroxide (189 mg, 3.37 mmol) was added, and reflux continued for 5 days. After concentration in vacuo with pre-adsorbtion onto silica, purification by silica chromatography using a methanol/dichloromethane/ammonia gradient system on an ISCO sq 16x machine yielded a mixture of intermediate alkenes (750 mg) as a red-orange oil. After dissolution in ethanol (25 mL) and the addition of palladium hydroxide on carbon (20 wt% palladium, 150 mg) the mixture was stirred vigorously under an atmosphere of hydrogen for 5 days. A further portion of palladium hydroxide on carbon (20 wt% palladium, 150 mg) was added, and the mixture was stirred vigorously under an atmosphere of hydrogen for 24 hours. After filtration and concentration in vacuo, the mixture was re-dissolved in ethanol/glacial acetic acid (25:1, 26 mL) and palladium hydroxide on carbon (20 wt% palladium, 150 mg) was added. After stirring vigorously under an atmosphere of hydrogen for 24 hours, the reaction mixture was filtered and concentrated in vacuo with pre-adsorbtion onto silica. Purification by preparative HPLC yielded 5-phenyl-3-(3-piperidinyl)-1H-indole-7-carboxamide (23.7 mg, 0.07 mmol) and 5-phenyl-3-[1-(phenylmethyl)-3-piperidinyl]-1H-indole-7-carboxamide (22.5 mg, 0.05 mmol).

### Data for 5-phenyl-3-(3-piperidinyl)-1H-indole-]-carboxamide

1H NMR (400 MHz, CD₃OD) δ ppm 1.85-2.04 (m, 2H) 2.09 (d, J=13.3 Hz, 1H) 2.25 (d, *J*=12.1 Hz, 1H) 3.05 (t, *J*=12.3 Hz, 2H) 3.37-3.48 (m, 2H) 3.63 (d, *J*=11.0 Hz, 1H) 7.29 (s, 1H) 7.32 (d, *J*=7.5 Hz, 1H) 7.45 (t, *J*=7.6 Hz, 2H) 7.72 (d, J=7.3 Hz, 2H) 7.98 (d, *J*=1.3 Hz, 1H) 8.03 (d, *J*=1.3 Hz, 1H) 8.38 (s, 2H)
LC/MS m/z 320 (M+1)⁺ r.t. 2.20 min.

### Data for 5-phenyl-3-[1-(phenylmethyl)-3-piperidinyl]-1H-indole-7-carboxamide

1H NMR (400 MHz, CD₃OD) δ ppm 1.96 (qd, *J*=12.3, 3.8 Hz, 1H) 1.96-2.10 (m, 2H) 2.21 (d, *J*=12.8 Hz, 1H) 2.90-3.01 (m, 2H) 3.41-3.51 (m, 2H) 3.67 (d, *J*=11.6 Hz, 1H) 4.28 (s, 2H) 7.32 (t, *J*=7.3 Hz, 1H) 7.42-7.51 (m, 6H) 7.64 (d, *J*=7.3 Hz, 2H) 7.88 (d, *J*=1.3 Hz, 1H) 7.95 (d, *J*=1.3 Hz, 1H) 8.45 (s, 2H)
LC/MS m/z 410 (M+1)⁺ r.t. 2.48 min.

### (47) 3-(1-cyclohexen-1-yl)-5-phenyl-1H-indole-7-carboxamide

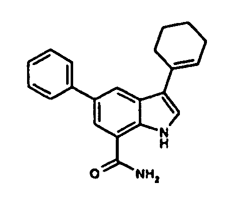

A mixture of 5-phenyl-1H-indole-7-carboxamide (509 mg, 2.15 mmol), potassium hydroxide (0.12 g, 2.15 mmol), and cyclohexanone (2.24 mL, 21.5 mmol) in methanol (2 mL) was heated at 65 °C for 24 hours before cooling to room temperature and concentration in vacuo. Purification by preparative HPLC yielded the title compound (186 mg) as a light-yellow solid.

1H NMR (400 MHz, DMSO-D6) δ ppm 1.63-1.67 (m, 2H) 1.73-1.78 (m, 2H) 2.25 (bs, 2 H) 2.42 (bs, 2H) 6.25 (s, 1H) 7.32-7.35 (m, 2H) 7.44 (bs, 1H) 7.47 (t, J=7.7 Hz, 2H) 7.77 (d, J=7.8 Hz, 2H) 8.01 (s, 1H) 8.16 (s, 1H) 8.25 (s, 1H) 11.11 (s, 1H)
LC/MS m/z 317 (M+1)⁺ r.t. 3.87 min.

### (48) 3-cyclohexyl-5-phenyl-1H-indole-7-carboxamide

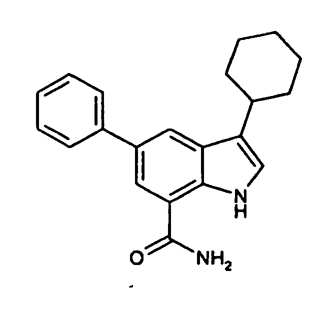

A suspension of 3-(1-cyclohexen-1-yl)-5-phenyl-1H-indole-7-carboxamide (166 mg, 0.53 mmol) and palladium hydroxide on carbon (20 wt% palladium, 33 mg) in ethanol/glacial acetic acid (50:1, 51 mL) was stirred vigorously under an atmosphere of hydrogen for 4 hours. After filtration and concentration in vacuo the resulting light-green foam was partitioned between ethyl acetate and saturated aqueous sodium hydrogencarbonate solution. After separation of the phases, the organic phase was dried over magnesium sulfate, filtered and concentrated in vacuo to yield the title compound (169 mg).

1H NMR (400 MHz, CDCl₃) δ ppm 1.29-1.38 (m, 1H) 1.47-1.53 (m, 4H) 1.80 (d, *J*=12.0 Hz, 1H) 1.83-1.91 (m, 2H) 2.11-2.18. (m, 2H) 2.90 (bs, 1H) 5.50-6.6.40 (bs, 2H) 7.12 (s, 1H) 7.37 (t, *J*=7.4 Hz, 1H) 7.49 (t, *J*=7.7 Hz, 2H) 7.58 (s, 1H) 7.65 (d, *J*=7.5 Hz, 2H) 8.02 (s, 1H) 10.01 (s, 1H)
LC/MS m/z 319 (M+1)⁺ r.t. 3.86 min.

### (49) 3-{1-[3-(methyloxy)phenyl]ethenyl}-5-phenyl-1H-indole-7-carboxamide

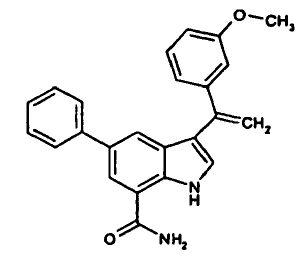

A mixture of 5-phenyl-1H-indole-7-carboxamide (115 mg, 0.49 mmol), sodium methoxide (11.7 mL of a 0.5 M solution in methanol, 5.88 mmol) and 3-methoxyacetophenone (0.40 mL, 2.94 mmol) was heated at reflux under nitrogen for 43 hours. Further portions of 3-methoxyacetophenone (0.40 mL, 2.94 mmol) and sodium methoxide (11.7 mL of a 0.5M solution in methanol, 5.88 mmol) were added, and reflux continued for a further 3 days before cooling to room temperature. Water (5 mL) was added cautiously, and the solution concentrated in vacuo. Methanol (10 mL) and potassium hydroxide (27.5 mg, 0.49 mmol) were added and the mixture heated at reflux for 24 hours before cooling to room temperature and concentration in vacuo with pre-adsorbtion onto silica. Purification by silica chromatography using an ethyl acetate/cyclohexane gradient system on an ISCO sq16x machine followed by preparative HPLC yielded the title compound (5.5 mg) as a yellow oil.

1H NMR (400 MHz, CDCl₃) δ ppm 3.80 (s, 3H) 5.52 (s, 1H) 5.61 (s, 1H) 5.80-6.50 (bs, 2 H) 6.91 (dd, J=8.0, 2.3 Hz, 1H) 7.05 (bs, 1H) 7.09 (d, J=7.5 Hz, 1H) 7.26-7.36 (m, 4H) 7.45 (t, J=7.7 Hz, 2H) 7.56 (d, J=7.3 Hz, 2H) 7.62 (s, 1H) 7.94 (s, 1H) 10.35 (bs, 1H)
LC/MS m/z 371 (M+1)⁺ r.t. 3.67 min.

### (50) 5-phenyl-3-[1-(phenylmethyl)-1,2,3,6-tetrahydro-4-pyridinyl]-1H-indole-7-carboxamide

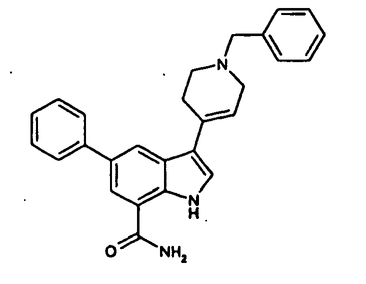

5-phenyl-1*H*-indole-7-carboxamide (500 mg, 2.12 mmol), 1-(phenylmethyl)-4-piperidinone (1.20 g, 1.18 mL. 6.35 mmol), sodium methoxide (686 mg, 12.71 mmol) in methanol (25 mL) were added together and heated at reflux under an atmosphere of nitrogen for 16 hours. The reaction was pre-adsorbed onto silica *in vacuo* and purified by silica flash chromatography using a methanol/dichloromethane/ammonia elution system on an ISCO sq16x machine. The fractions were combined and solvent removed *in vacuo* to yield the title compound (325 mg).

1H NMR (400 MHz, DMSO-D6) δ ppm 2.54 (bs, 2H) 2.68 (bt, J=5.3 Hz, 2H) 3.12 (bs, 2 H) 3.59 (s, 2H) 6.23 (bs, 1H) 7.2-7.5 (m, 10H) 7.78 (d, J=7.5 Hz, 2H) 8.02 (s, 1H) 8.19 (s, 1H) 8.27 (bs, 1H) 11.17 (bs, 1H)
LC/MS m/z 408 (M+1)⁺ r.t. 2.44 min.

### (51) 5-phenyl-3-(4-piperidinyl)-1H-indole-7-carboxamide

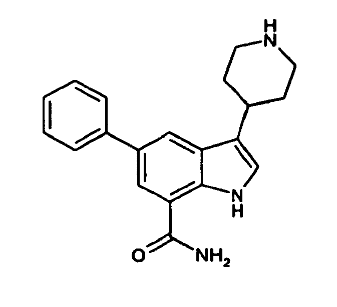

5-phenyl-3-[1-(phenylmethyl)-1,2,3,6-tetrahydro-4-pyridinyl]-1H-indole-7-carboxamide (323 mg, 0.79 mmol) was dissolved in ethanol/acetic acid (50:1, 51 mL,) and added to 20% palladium hydroxide on carbon (130 mg). The suspension was stirred at room temperature under an atmosphere of hydrogen for 70 hours. The reaction mixture was filtered and solvent removed *in vacuo.* The residue was taken up in methanol (50 mL) and pre-adsorbed onto silica *in vacuo.* The product was purified by silica flash chromatography using a methanol/dichloromethane/ammonia elution system on an ISCO sq16x machine. The desired fractions were collected and combined and solvent removed *in vacuo* to yield the title compound (189 mg).

1H NMR (400 MHz, DMSO-D6) δ ppm 1.56 (m, 2H) 1.58 (m, 2H) 2.53 (m, 2H) 3.00 (m, 3H) 7.01 (s, 1H) 7.1-7.6 (m*,* 4H) 7.78 (d, *J*=7.5 Hz, 2H) 8.02 (bs, 2H) 8.24 (bs, 1H) 10.84 (bs, 1H); LC/MS m/z 320 (M+1)⁺ r.t. 2.22 min.

### (52) 3-{1-[(4-chlorophenyl)sulfonyl]-4-piperidinyl}-5-phenyl-1H-indole-7-carboxamide

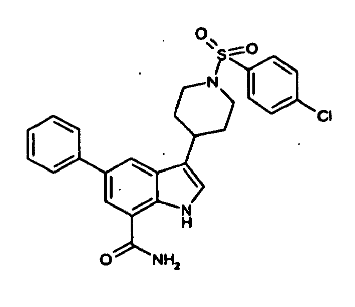

5-phenyl-3-(4-piperidinyl)-1H-indole-7-carboxamide (30 mg, 0.10 mmol) and 4-chlorobenzenesulfonyl chloride (21.8 mg, 0.10 mmol) were stirred at room temperature in pyridine (1 mL) for 1 hour. Solvent was removed *in vacuo* and the residue purified by preparative HPLC using an acetonitrile/water elution system. Removal of the solvent in *vacuo* yielded the title compound (35 mg).

1H NMR (400 MHz, DMSO-D6) δ ppm 1.68 (m, 2H) 2.05 (m, 2H) 2.44 (m, 2H) 2.90 (m, 1H) 3.75 (m, 2H) 7.11 (s, 1H) 7.31 (t, *J*=7.3 Hz, 1H) 7.38 (brs, 1H) 7.44 (t, *J*=7.8 Hz, 2 H) 7.79 (m, 6H) 7.99 (bd, *J*=3.8 Hz, 2H) 8.20 (bs, 1H) 10.89 (bs, 1H)
LC/MS m/z 492 (M-1)⁻ r.t. 3.70 min.

### (53) 5-phenyl-3-[1-(propylsulfonyl)-4-piperidinyl]-1H-indole-7-carboxamide

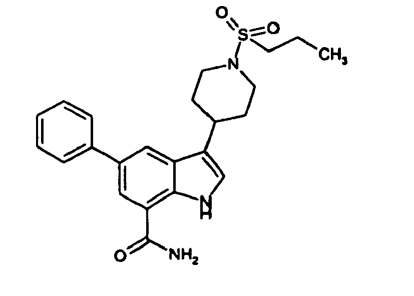

5-phenyl-3-(4-piperidinyl)-1H-indole-7-carboxamide (30 mg, 0.10 mmol) and 1-propanesulfonyl chloride (14.8 mg, 0.10 mmol) were stirred at room temperature in pyridine (1 mL) for 1 hour. Solvent was then removed *in vacuo* and the residue purified by preparative HPLC using an acetonitrile/water elution system. Removal of the solvent *in vacuo* yielded the title compound (13 mg).

1H NMR (400 MHz, DMSO-D6) δ ppm 1.01 (t, *J*=7.5 Hz, 3H) 1.70 (m, 4H) 2.07 (m, 2H) 3.00 (m, 5H) 3.70 (m, 2H) 7.15 (s, 1H) 7.33 (t, *J*=7.3 Hz, 1H) 7.39 (bs, 1H) 7.47 (t, *J*=7.5 Hz, 2H) 7.80 (d, *J*=7.8 Hz, 2H) 8.00 (d, *J*= 4.5 Hz, 2H) 8.22 (bs, 1H) 10.92 (bs, 1 H)
LC/MS m/z 424 (M-1)⁻ r.t. 3.30 min.

### (54) 3-(1-acetyl-4-piperidinyl)-5-phenyl-1H-indole-7-carboxamide

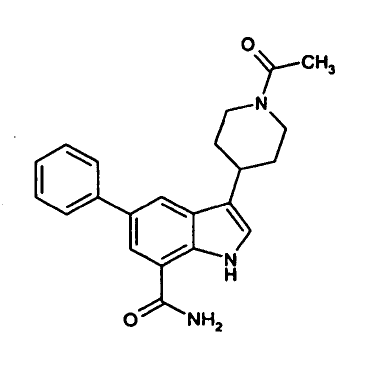

5-phenyl-3-(4-piperidinyl)-1H-indole-7-carboxamide (30 mg, 0.10 mmol) and acetyl chloride (14.8 mg, 0.10 mmol) were heated together at 40°C in pyridine (1 mL) for 16 hours. Solvent was then removed *in vacuo* and the residue purified by preparative HPLC using an acetonitrile/water elution system. Combination of the relevant fractions and removal of the solvent in vacuo yielded the title compound (13 mg).

1H NMR (400 MHz, DMSO-D6) δ ppm 1.43 (apparent q, *J*=12.3 Hz, 1H) 1.60 (apparent q, *J*=12.8 Hz, 1H) 2.02 (m, 2H) 2.03 (s, 3H) 2.12 (m, 2H) 3.90 (bd, *J*=13.6 Hz, 1H) 4.50 (bd, *J*=12.8 Hz, 1H) 7.12 (s, 1H) 7.33 (t, *J*=7.3 Hz, 1H) 7.35 (bs, 1H) 7.47 (t, *J*=7.8 Hz, 2H) 7.79 (d, *J*=7.5 Hz, 2H) 8.00 (s, 1H) 8.03 (s, 1H) 8.22 (bs, 1H) 10.88 (bs, 1H) LC/MS m/z 362 (M+1)⁺ r.t. 2.85 min.

### (55) 3-[1-(N,N-dimethyl-β-alanyl)-4-piperidinyl]-5-phenyl-1H-indole-7-carboxamide

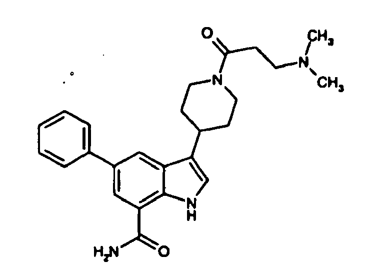

5-phenyl-3-(4-piperidinyl)-1H-indole-7-carboxamide (30 mg, 0.10 mmol), *N*,*N*-dimethyl-β-alanine (16 mg, 0.10 mmol), *N*-[(dimethylamino)(3*H*-[1,2,3]triazolo[4,5-*b*]pyridin-3-yloxy)methylidene]-*N*-methylmethanaminium hexafluorophosphate (36.5 mg, 0.10 mmol) and diisopropylethylamine (52 mg, 0.4 mmol) were suspended in dimethyl formamide (5 mL) and heated under an atmosphere of nitrogen at 80°C for 16 hours. The solvent was removed *in vacuo* and the residue purified by preparative HPLC using an acetonitrile/water elution system. The relevant fractions were collected and solvent removed *in vacuo* to yield the title compound (12 mg).

1H NMR (400 MHz, DMSO-D6) δ ppm 1.47 (apparent q, *J=*11.8 Hz, 1H) 1.64 (apparent q, *J*=12.6 Hz, 1H) 2.06 (bs, 2H) 2.77 (s, 3H) 2.78 (m, 2H) 2.79 (s, 3H) 2.92 (q, *J*=7.1 Hz, 2H) 3.28 (m, 2H) 3.48 (dt, *J*=16.0, 4.3Hz, 1H) 3.69 (dt, *J*=18.6, 4.6Hz, 1H) 3.96 (bd, *J*=13.1 Hz, 1H) 4.53 (bd, *J*=13.1 Hz, 1H) 7.12 (s, 1H) 7.33 (t, *J*=7.3 Hz, 1H) 7.40 (bs, 1H) 7.48 (t, *J*=7.8 Hz, 2H) 7.80 (d, *J*=7.5 Hz, 2H) 8.01 (s, 1H) 8.05 (s, 1H) 8.23 (bs, 1H) 10.90 (bs, 1H)
LC/MS m/z 419 (M+1)⁺ r.t. 2.28 min.

### (56) 3-(1-ethyl-4-piperidinyl)-5-phenyl-1H-indole-7-carboxamide formate

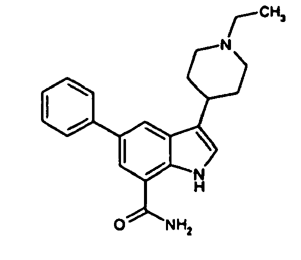

Acetaldehyde (0.5 mL) was added to 5-phenyl-3-(4-piperidinyl)-1H-indole-7-carboxamide (30 mg, 0.10 mmol) in methanol (1 mL). The mixture was stirred at room temperature for 2 minutes. Sodium triacetoxyborohydride (100 mg, 0.47 mmol) was then added and the reaction was stirred at room temperature for 16 hours. The reaction mixture was passed through a 2 g SCX cartridge eluting initially with methanol and then with an ammonia/methanol mix. The relevant fractions were collected and solvent removed in *vacuo.* The residue was purified by preparative HPLC using an acetonitrile/water elution system. The relevant fractions were collected and solvent removed *in vacuo* to yield the title compound (6 mg).

1H NMR (400 MHz, DMSO-D6) δ ppm 1.08 (t, *J*=7.3 Hz, 3H) 1.74 (m, 2H) 2.01 (bd, *J*=13.0 Hz, 2H) 2.30 (t, *J*=11.5 Hz, 2H) 2.54 (m, 1H) 2.79 (s, 3H) 2.93 (m, 2H) 3.10 (bd, *J*=10.5 Hz, 2H) 7.12 (s, 1H) 7.33 (t, *J*=7.3 Hz, 1H) 7.39 (bs, 1H) 7.47 (t, *J*=7.8 Hz, 2H) 7.78 (d, *J*=7.5 Hz, 2H) 7.99 (s, 1H) 8.01 (s, 1H) 8.22 (bs, 1H) 8.24 (s, 1H) 10.88 (bs, 1H)
LC/MS m/z 348 (M+1)⁺ r.t. 2.17 min.

### (57) 3-(1-methylpyrrolidin-2-yl)-5-phenyl-1H-indole-7-carboxamide

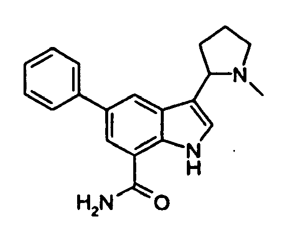

3-{1-[ethyl(methyl)amino]propyl}-5-phenyl-1H-indole-7-carboxylic acid (32.1 mg, 0.1 mmol), *O*-(7-Azabenzatriazol-1-yl)-*N*,*N*,*N',*N'-tetramethyluronium hexafluorophosphate (57.3 mg, 0.15 mmol), ammonia solution in dioxane (0.5 M, 0.8 ml, 0.4 mmol) were dissolved in dry dichloromethane (10.0 ml) and stirred at room temperature for 50 hours under nitrogen atmosphere. The reaction mixture was then extracted with ethyl acetate (4x10 ml), dried over Na₂SO₄, filtered and the solvent removed under reduced pressure to afford the title compound (25.0 mg, 80%).

1H NMR (400 MHz, MeOD) δ ppm 2.18 (m, 2H) 2.28 (m, 1H) 2.37 (m, 1H) 2.42 (s, 3H) 2.76 (b, 1H) 3.33 (m, 1H) 4.08 (m, 1H) 7.34(m, 1H), 7.47 (m, 2H), 7.76(m, 2H) 8.02 (s, 1H), 8.15 (s, 1H)
LC/MS m/z 320 (M+1) r.t. 1.50 min.

### (58) 3-[1-(ethylsulfonyl)pyrrolidin-3-yl]-5-phenyl-1H-indole-7-carboxamide

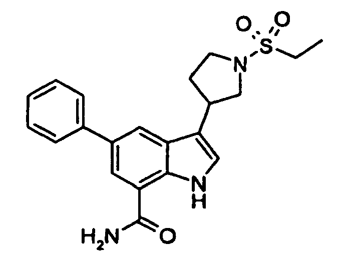

To a solution of 1,1-dimethylethyl-3-[7-(aminocarbonyl)-5-phenyl-1H-indol-3-yl]-1-pyrrolidinecarboxylate (40.0 mg, 0.1 mmol) in THF (2.0 ml), TFA (2.0 ml) was added. The resulting solution was stirred overnight. After removing the solvent in vacuo, ethane sulfonyl chloride (19.2 mg, 0.15 mmol), triethyl amine (30.3 mg, 0.3 mmol) in dichloromethane (5.0 ml) were added at 0°C**.** After 30 min the solvent was removed under reduced pressure and the resulting residue purified by Combiflash (Ethyl acetate/ Hexane 20% to 40%) to afford the title compound (6.0mg, 15%).

1H NMR (400 MHz, MeOD) δ ppm 1.50 (m, 3H) 2.28 (m, 1H) 2.52 (m, 1H) 3.18 (m, 2H) 3.53 (m, 1H) 3.65(m, 1H) 3.82(m, 1H) 3.91 (m, 1H) 7.32(m, 2H), 7.47 (m, 2H), 7.74(m, 2H) 7.98 (s, 1H), 8.05 (s, 1H)
LC/MS m/z 398 (M+1) r.t. 2.05 min.

### (59) 3-[4-(methylsulfonyl)phenyl]-5-phenyl-1H-indole-7-carboxamide

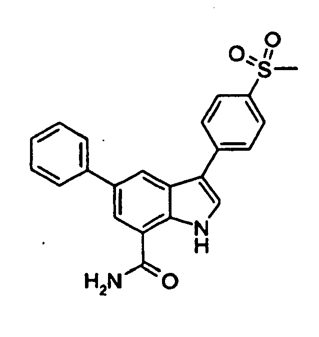

The title compound (20.0 mg) was prepared using general method B except that [4-(methylsulfonyl)phenyl]boronic acid (30.0 mg, 0.15 mmol)was used instead of phenyl boronic acid.

1H NMR (400 MHz, DMSO-D6) δ ppm 3.24 (s, 3H) 7.37 (m, 1H) 7.52 (m, 3H) 7.85(m, 3H) 7.95 (d, *J*=7.3 Hz, 2H) 8.03 (d, *J*=7.3 Hz, 2H) 8.04 (m, 2H) 8.21 (s, 1H) 11.62 (s, 1 H)
LC/MS m/z 391 (M+1)⁺ r.t. 2.04 min.

### (60) 3-[3-(acetylamino)phenyl]-5-phenyl-1H-indole-7-carboxamide

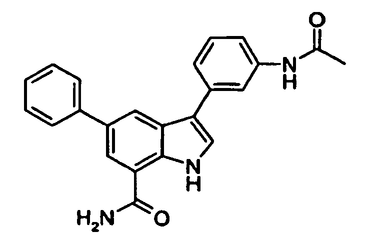

The title compound (15.4 mg) was prepared using general method B except that [3-(acetylamino)phenyl]boronic acid (27.0 mg,0.15 mmol) was used instead of phenyl boronic acid.

1H NMR (400 MHz, MeOD) δ ppm 2.17(s, 3H) 7.32 (m, 1H) 7.42 (m, 5H) 7.63 (s, 1H) 7.78 (d, J=7.3 Hz, 2H) 8.04 (m, 2H) 8.34 (s, 1H)
LC/MS m/z 370 (M+1)⁺ r.t. 2.00 min.

### (61) 3-[4-(ethylsulfonyl)phenyl]-5-phenyl-1H-indole-7-carboxamide

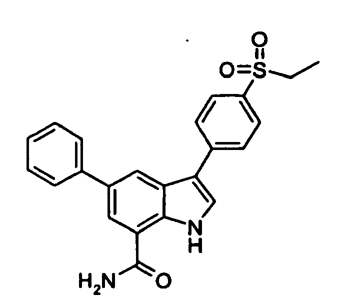

The title compound (16.0 mg) was prepared using general method B except that [4-(ethylsulfonyl)phenyl]boronic acid (33.0 mg,0.15 mmol) was used instead of phenyl boronic acid.

1H NMR (400 MHz, DMSO-D6) δ ppm 1.16 (m, 3H) 3.34 (m, 2H) 7.37 (m, 1H) 7.52 (m, 3 H) 7.85(m, 3H) 7.95 (d, *J*=7.3 Hz, 2H) 8.03 (d, *J*=7.3 Hz, 2H) 8.04 (m, 2H) 8.21 (s, 1H) 11.62 (s, 1H)
LC/MS m/z 405 (M+1)⁺ r.t. 2.16 min.

### (62) 3-[3-(methylsulfonyl)phenyl]-5-phenyl-1H-indole-7-carboxamide

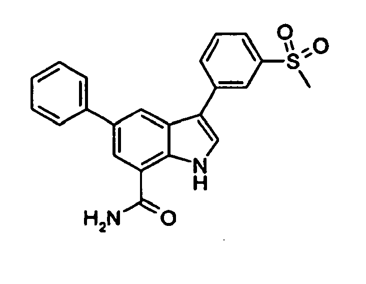

The title compound (14.0 mg) was prepared using general method B except that [3-(methylsulfonyl)phenyl]boronic acid (30.0 mg,0.15 mmol) was used instead of phenyl boronic acid.

1H NMR (400 MHz, DMSO-D6) δ ppm 3.34 (s, 3H) 7.38 (m, 1H) 7.53 (m, 3H) 7.85(m, 4H) 8.14 (m, 4H) 8.25 (s, 1H) 11.62 (s, 1H)
LC/MS m/z 391 (M+1)⁺ r.t. 2.05 min.

### (63) 3-(hexahydro-1H-azepin-4-yl)-5-phenyl-1H-indole-7-carboxamide

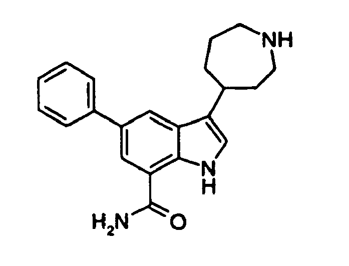

A mixture of 5-phenyl-1H-indole-7-carboxamide (237.0 mg, 1.0 mmol), aqueous sodium hydroxide (50%, 10 mL)), and 1,1-dimethylethyl 4-oxohexahydro-1H-azepine-1-carboxylate (639.0 mg, 3.0 mmol) in methanol (15 mL) was stirred under nitrogen for 60 hours. After concentration in vacuo, the aqueous phase was extracted with ethyl acetate (4x15 ml). The organic phase was dried over Na₂SO₄ and filtered. The solvent was removed in vacuo. The residue was redissolved in ethanol (25 mL). Palladium hydroxide on carbon (20 wt% palladium, 50 mg) was added. The mixture was stirred vigorously under an atmosphere of hydrogen for 24 hr. A further portion of palladium hydroxide on carbon (20 wt% palladium, 50 mg) was added, and the mixture was stirred vigorously under an atmosphere of hydrogen for 24 hours. After filtration and concentration in vacuo, the mixture was dissolved in THF (2 ml) and TFA (2 ml) was added. The solution was heated at 60°C for 12 hr. Purification by preparative HPLC (Water/ Acetonitrile 10% to 90% v/v) yielded the title compound (100.0 mg, 30%).

1H NMR (400 MHz, MeOD) δ ppm 1.80-2.50 (m, 6H) 3.42(m, 5H) 7.23 (s, 1H) 7.35 (m,1 H) 7.46(m, 3H) 7.73 (m, 2H) 7.98 (s, 1H) 8.21 (s, 1H)
LC/MS m/z 334 (M+1)⁺ r.t.1.50 min.

### (64) 3-[1-(ethylsulfonyl)hexahydro-1H-azepin-4-yl]-5-phenyl-1H-indole-7-carboxamide

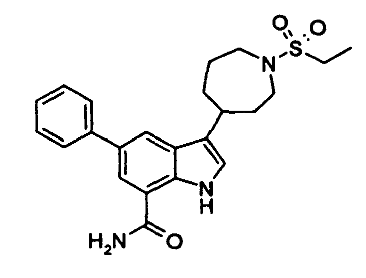

3-(hexahydro-1*H*-azepin-4-yl)-5-phenyl-1*H*-indole-7-carboxamide (33.4 mg, 0.10 mmol) and ethylsulfonyl chloride (14.1 mg, 0.11 mmol), triethyl amine (10.0 mg, 0.10 mmol) were stirred at room temperature in dichloromethane (5 mL) for 1 hour. The solvent was removed *in vacuo,* the residue was purified by reverse phase HPLC method A (water/ acetonitrile 10%-90% v/v) to yield the title compound (6.0 mg, 14%).

1 H NMR (400 MHz, MeOD) δ ppm 1.35(M, 3h) 1.80-2.30 (m, 6H) 3.24 (m, 2H) 3.42(m, 5H) 7.22 (s, 1 H) 7.35 (m,1 H) 7.46(m, 3H) 7.73 (m, 2 H) 7.96 (s, 1 H) 8.01 (s, 1 H)
LC/MS m/z 334 (M+1)⁺ r.t.1.50 min.

### (65) 5-phenyl-3-[2-(4-pyridinyl)ethyl]-1H-indole-7-carboxamide

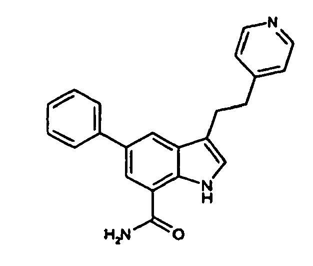

To the solution of 5-phenyl-1*H*-indole-7-carboxamide (45 mg, 0.19 mmol) in acetic acid (2 mL) was added vinyl pyridine (0.04 mL, 0.38 mmol). The reaction was heated via Smith synthesizer microwave at 230 °C for 1 h. The solution was then extracted with 1 N KOH solution (20 mL), NaHCO₃ (sat. 10 mL) and brine (10 mL). The organic layer was evaporated and purified by reverse phase HPLC methoad A (water/ acetonitrile 10%-90% v/v). 10 mg of the desired product was isolated.
LC/MS m/z 342 (MH⁺), Rt 1.64 min.

### (66) 3-{[1-(ethylsulfonyl)-4-piperidinylidene]methyl}-5-phenyl-1H-indole-7-carboxamide

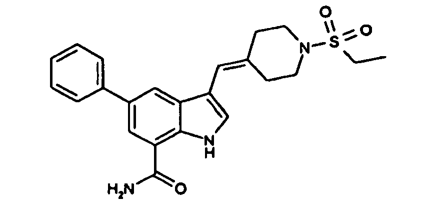

To the solution of 5-phenyl-1*H*-indole-7-carboxamide (50 mg, 0.21 mmol) in MeOH (10 mL) was added NaOMe (0.5 M, 1.7 mL) at rt. The solution was stirred for 0.5 h and 1,1-dimethylethyl 4-formyl-1-piperidinecarboxylate (90 mg, 0.42 mmol) was added. The solution was heated at reflux for 4 h after which time the solvent was removed under reduced pressure. The residue was re-dissolved in EtOAc and washed with water. The organic layer was evaporated and the residue was dissolved in methylene chloride (10 mL) and TFA (1 mL) was added at rt. The reaction was stirred for 1 h and quenched by addition of 1N NaOH solution (20 mL). The organic layer was washed with brine and evaporated. The residue was dissolved in metheylene chloride again at 0 °C. To this solution were added Hunig's base (0.14 mL, 0.84 mmol) and ethane sulfonyl chloride (0.03 mL, 0.32 mmol). The resulting solution was stirred at 0 °C for 2 h and purified by column chromatography (Ethyl Acetate/ Hexane, 50% to 100%). 15 mg of the desired product was isolated.
LC/MS m/z 424 (MH⁺), Rt 2.34 min.

### (67) 3-[1-(ethylsulfonyl)-4-piperidinyl]-5-[4-(hydroxymethyl)phenyl]-1H-indole-7-carboxamide

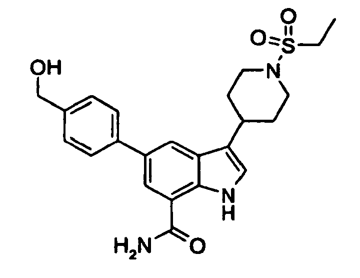

To a solution of 5-bromo-3-[1-(ethylsulfonyl)-4-piperidinyl]-1*H*-indole-7-carboxamide (example **90**) (20.0 mg, 0.048 mmol), K₃PO₄ (21.0 mg, 0.096 mmol) and [4-(hydroxymethyl) phenyl] boronic acid (30.0 mg, 0.193 mmol) in dioxane/H₂O (2 mL/0.7 mL) was bubbled argon for 5 minutes prior to addition of Pd(PPh₃)₄ (5.0 mg, 0.0048 mmol). The reaction mixture was heated in a microwave reactor (Smith synthesizer) for 20 minutes at 160°C. The solvent was evaporated, and the residue was partitioned between ethyl acetate and water. The organic layer was washed with brine (10 mL), dried over Mg₂SO₄, concentrated, and purified by reverse phase HPLC method A (water/ CH₃CN, 0.1 % TFA 10-90%) to give the title compound 6.4 mg (30%).
1 H NMR (400 MHz, DMSO-D6) δ ppm 1.25 (t, *J*=7.2 Hz, 3H), 1.65 (m, 2H), 2.02 (m, 2H), 2.99 (m, 7H), 3.71 (m, 2H), 7.16 (s, 1 H), 7.42 (m, 3H), 7.77 (m, 2H), 8.02 (m, 2H), 8.22 (m, 1H), 10.91 (s, 1H).
LC/MS: 442.4. r.t: 1.73.

### (68) 5-phenyl-3-(3-piperidinylmethyl)-1H-indole-7-carboxamide

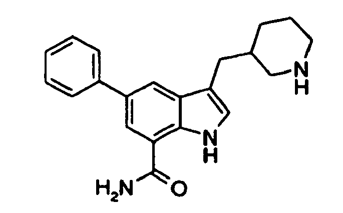

To a solution of 1,1-dimethylethyl 3-{[7-(aminocarbonyl)-5-phenyl-1*H*-indol-3-yl]methyl}-1-piperidinecarboxylate (intermediate 18) (20.0 mg, 0.046 mmol) in dioxane/MeOH (0.7 mL/2.0mL) was added HCl (37%, 0.1 mL). The reaction mixture was refluxed at 60°C for 1 hr, and solvent removed in vacuo. The resulting residue was dried over MgSO₄, concentrated to give title compound 14.0 mg.
1H NMR (400 MHz, CD₃OD) δ ppm 1.42 (m, 1H), 1.72 (m, 1H), 1.98 (m, 2H), 2.20 (m, 1H), 2.88 (m, 4H), 3.30 (m, 2H), 7.26 (s, 1H), 7.33 (m, 1H), 7.47 (m, 2H), 7.74 (m, 2H), 8.02 (m, 2H).
LC/MS: 334.4. r.t: 1.57

### (69) 5-phenyl-3-[2-(4-piperidinyl)ethyl]-1H-indole-7-carboxamide

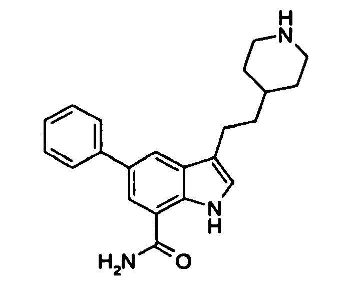

The title compound was prepared according to the procedure for example **68**. Thus, 1,1-dimethylethyl 4-{2-[7-(aminocarbonyl)-5-phenyl-1*H*-indol-3-yl]ethyl}-1-piperidine carboxylate (11.0 mg, 0.024 mmol) and concentrated HCl (0.05 mL) were reacted to give 8.4 mg (100%) of the title compound.

1H NMR (400 MHz, CD₃OD) δ ppm 1.48 (m, 2H), 1.72 (m, 3H), 2.10 (m, 2H), 2.89 (m, 4H), 3.40 (m, 2H), 7.22 (s, 1H), 7.31 (m, 1H), 7.44 (m, 2H), 7.71 (m, 2H), 7.98 (m, 2H).
LC/MS: 348.2. r.t: 1.54.

### (70) 3-{2-[1-(ethylsulfonyl)-4-piperidinyl]ethyl}-5-phenyl-1H-indole-7-carboxamide

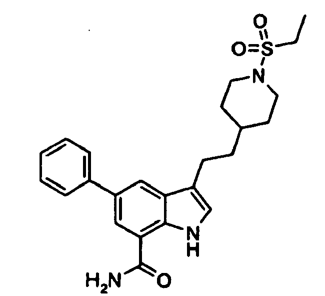

1,1-dimethylethyl 4-{2-[7-(aminocarbonyl)-5-phenyl-1*H*-indol-3-yl]ethyl}-1-piperidine carboxylate (40.0 mg, 0.088 mmol) was reacted with HCl (37%. 0.22 mL) to give crude intermediate, which was dissolved in DMF (2 mL). Et₃N (0.028 mL, 0.2 mmol) was added, followed by a catalytic amount of DMAP (1.0 mg), and ethanesulfonyl chloride (0.010mL, 0.088 mmol). After 3 hrs, the solution was concentrated by vacuum pump, and the resulting residue purified via solid phase extraction on a 500 mg aminopropyl column (Intemational Sorbent Technologies) eluting with dichloromethane (20 mL), ethyl acetate/ hexane (20%, 20 mL), ethyl acetate/ hexane (50%, 20 mL), ethyl acetate/ hexane (90%. 20 mL). 4.0 mg (11%) of the title compound was obtained.

1H NMR (400 MHz, CD₃OD) δ ppm 1.28 (t, *J*=7.2 Hz, 3H), 1.52 (m, 2H), 1.78 (m, 2H), 1.90 (m, 2H), 2.82 (m, 5H), 2.98 (q, *J*=7.2 Hz, 2H), 3.69 (m, 2H), 7.16 (s, 1H), 7.29 (m, 1H), 7.47 (m, 2H), 7.75 (m, 2H), 7.97 (m, 2H).
LC/MS: 440.4. r.t: 2.36.

### (71) 3-{[1-(ethylsulfonyl)-3-piperidinyl]methyl}-5-phenyl-1H-indole-7-carboxamide

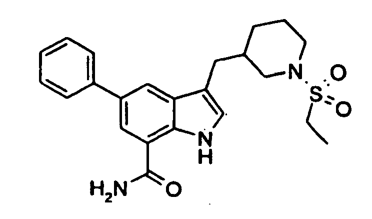

The title compound was prepared according to the procedure for example **70**. Thus, 1,1-dimethylethyl 3-{([7-(aminocarbonyl)-5-phenyl-1*H*-indol-3-yl]methyl}-1-piperidine carboxylate (25.0 mg, 0.056 mmol) was reacted with HCl (37%, 0.150 mL) and the resulting product reacted with ethanesulfonyl chloride (0.006mL, 0.058 mmol) in the presence of Et₃N (0.028 mL, 0.2 mmol), and a catalytic amount of DMAP (cal 1.0 mg) to give 9.5 mg (39%) of the title compound.

1H NMR (400 MHz, CD₃OD) δ ppm 1.28 (t, *J*=7.2 Hz, 3 H), 1.52 (m, 1H), 1.77 (m, 1H), 1.84 (m, 1H), 1.98 (m, 1H), 2.67 (m, 2H), 2.89 (m, 3H), 2.98 (q, *J*=7.2 Hz, 2H), 3.69 (m, 2H), 7.14 (s, 1H), 7.30 (m, 1H), 7.48 (m, 2H), 7.74 (m, 2H), 7.97 (m, 2H), 10.53 (s, 1H).
LC/MS: 426.2 r.t: 2.41.

### (72) 3-{[1-(ethylsulfonyl)-4-piperidinyl]methyl}-5-phenyl-1H-indole-7-carboxamide

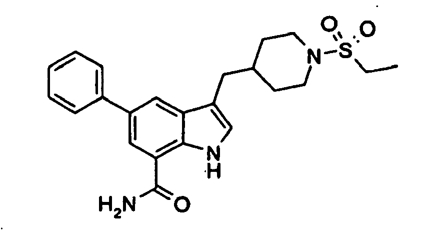

The title compound was prepared according to the procedure for example 70. Thus, 1,1-dimethylethyl 4-{[7-(aminocarbonyl)-5-phenyl-1*H*-indol-3-yl]methyl}-1-piperidine carboxylate (20.0 mg , 0.045 mmol) was reacted with concentrated HCl (0.100 mL) and the resulting product reacted with ethanesulfonyl chloride (0.01 mL, 0.060 mmol) in the presence of Et₃N (0.025 mL, 0.19 mmol) and a catalytic amount of DMAP (cal 1.0 mg) to give 2.8 mg (14%) of the title compound in 2 steps.
1H NMR (400 MHz, CD₃OD) δ ppm 1.28 (t, J=7.2 Hz, 3 H), 1.79 (m, 3H), 2.76 (m, 4H), 2.99 (m, 4H), 3.75 (m, 2H), 7.21 (s, 1H), 7.35 (m, 1H), 7.46 (m, 2H), 7.74 (m, 2H), 7.95 (m, 2H). LC/MS: 426.2. r.t: 2.20

### (73) 3-{1-[(2)sulfonyl]-4-piperidinyl}-5-phenyl-1H-indole-7-carboxamide

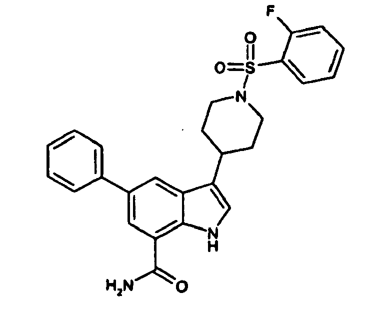

To 5-phenyl-3-(4-piperidinyl)-1*H-*indole-7-carboxamide (38.9 mg, 0.12 mmol) in CH₂Cl₂ (5mL) at 0°C, 2-fluorobenzenesulfonyl chloride (36 mg, 0.18 mmol) and triethylamine (0.07 mL, 0.48 mmol) were added. The reaction mixture was stirred at 0°C for 30 min. after which time the mixture was partitioned between CH₂Cl₂ and water. The aqueous phase was extracted with CH₂Cl₂ (25 mLx2) and the combined organic phase dried over MgSO₄ and concentrated under reduced pressure. The resulting residue was purified by reverse phase HPLC method B (CH₃CN/Water, 0.1 % TFA) to give the title compound (40 mg. 69%)
LC/MS: m/z 478.2 (M+H), 2.55 min

### (74) 3-{1-[(4-fluorophenyl)sulfonyl]-4-piperidinyl}-5-phenyl-1H-indole-7-carboxamide

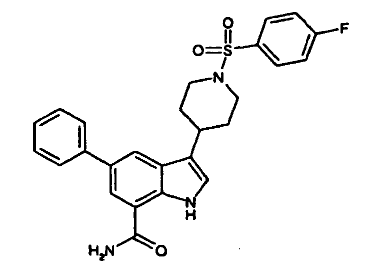

Following the general procedure in example **73**, 5-(phenyl)-3-(4-piperidinyl)-1*H*-indole-7-carboxamide (41 mg, 0.13 mmol), 4-fluorobenzenesulfonyl chloride (38 mg, 0.195 mmol) and triethylamine (0.09 mL, 0.65 mmol) were reacted to form the desired product which was purified by reverse phase HPLC method B (CH₃CN/Water, 0.1 % TFA) (20.3 mg, 33 %).
LC/MS: m/z 478.2 (M+H), 2.52 min.

### (75) 3-{1-[(4-methylphenyl)sulfonyl]-4-piperidinyl}-5-phenyl-1H-indole-7-carboxamide

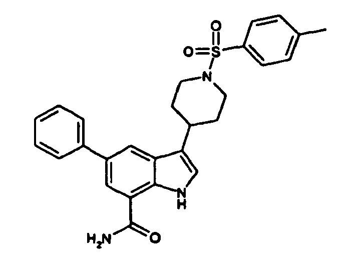

Following the general procedure in example **73**, 5-(phenyl)-3-(4-piperidinyl)-1*H*-indole-7-carboxamide (41 mg, 0.13 mmol), 4-methylbenzenesulfonyl chloride (37 mg, 0.195 mmol) and triethylamine (0.07 mL, 0.48 mmol) were reacted to form the desired product which was purified by reverse phase HPLC method B (CH₃CN/Water, 0.1 % TFA) (30 mg, 49%).
LC/MS: m/z 474.2 (M+H), 2.65 min.

### (76) 3-{phenylsulfonyl-4-piperidinyl}-5-phenyl-1H-indole-7-carboxamide

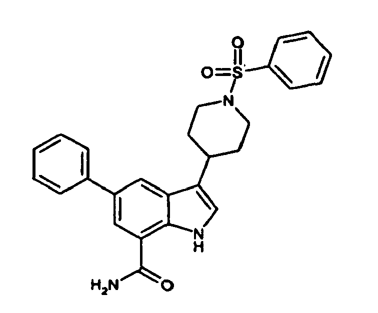

Following the general procedure in example **73**, 5-(phenyl)-3-(4-piperidinyl)-1*H-*indole-7-carboxamide (41 mg, 0.13 mmol), benzenesulfonyl chloride (34.5 mg, 0.195 mmol) and triethylamine (0.07 mL, 0.48 mmol) were reacted to form the desired product which was purified by reverse phase HPLC method B (CH₃CN/Water, 0.1 % TFA) (15.4 mg, 26%).
LC/MS: m/z 460.2 (M+H), 2.62 min.

### (77) 3-(1-{[4-(methyloxy)phenyl]sulfonyl}-4-piperidinyl)-5-phenyl-1H-indole-7-carboxamide

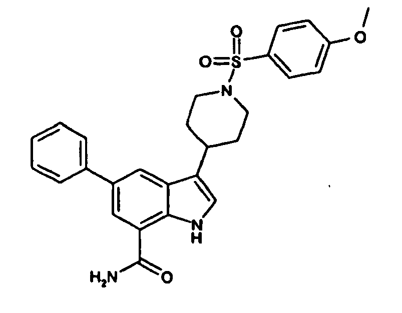

Following the general procedure in example **73**, 5-(phenyl)-3-(4-piperidinyl)-1*H*-indole-7-carboxamide (39 mg, 0.12 mmol), 4-methoxybenzenesulfonyl chloride (38 mg, 0.18 mmol) and triethylamine (0.07 mL, 0.48 mmol) were reacted to form the desired product which was purified by reverse phase HPLC method B (CH₃CN/Water, 0.1 % TFA) (17.9 mg, 30%).
LC/MS: m/z 490.2 (M+H), 2.45 min.

### (78) 3-[1-(ethanesulfonyl)-4-piperidinyl]-5-phenyl-1H-indole-7-carboxamide

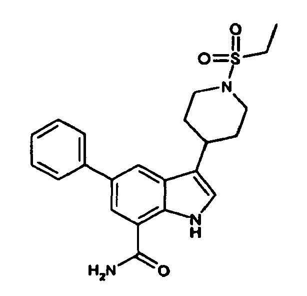

Following the general procedure in example **73**, 5-(phenyl)-3-(4-piperidinyl)-1*H-*indole-7-carboxamide (16 mg, 0.04 mmol), ethanesulfonyl chloride (0.004 mL, 0.08 mmol) and triethylamine (0.016 mL, 0.12 mmol) were reacted to form the desired product which was purified by reverse phase HPLC method B (CH₃CN/Water, 0.1 % TFA) (12.8 mg, 62%).
LC/MS: m/z 412.0 (M+H), 2.18 min.

### (79) 3-{1-[(2-propanesulfonyl]-4-piperidinyl}-5-phenyl-1H-indole-7-carboxamide

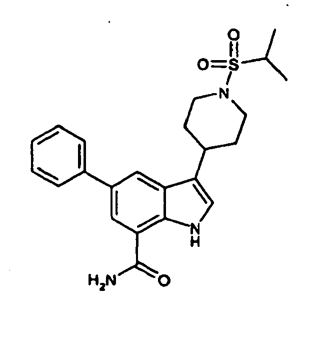

Following the general procedure in example **73**, 5-(phenyl)-3-(4-piperidinyl)-1*H*-indole-7-carboxamide (34 mg, 0.11 mmol), 2-propanesulfonyl chloride (0.012 mL, 0.11 mmol) and triethylamine (0.08 mL, 0.65 mmol) were reacted to form the desired product which was purified by reverse phase HPLC method B (CH₃CN/Water, 0.1 % TFA) (9.6 mg, 21 %). LC/MS: m/z 426.0, 2.19 min.

### (80) 5-phenyl-3-[1-(propanesulfonyl)-1,2,3,6-tetrahydro-4-pyridinyl]-1H-indole-7-carboxamide

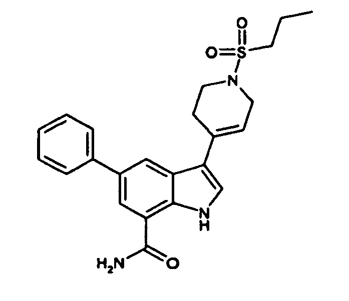

Following the general procedure in example **73**, 5-phenyl-3-(1,2,3,6-tetrahydro-4-pyridinyl)-1H-indole-7 carboxamide (100 mg, 0.32 mmol), 2-propanesulfonyl chloride (0.043 mL, 0.384 mmol) and triethylamine (0.178 mL, 1.28 mmol) were reacted to form the desired product which was purified by reverse phase HPLC method B (CH₃CN/Water, 0.1 % TFA) (20 mg, 15%).
LC/MS: m/z 424.0 (M+H), 1.87 min.

### (81) 5-phenyl-3-(1-{[4-(trifluoromethyl)phenyl]sulfonyl}-4-piperidinyl)-1H-indole-7-carboxamide

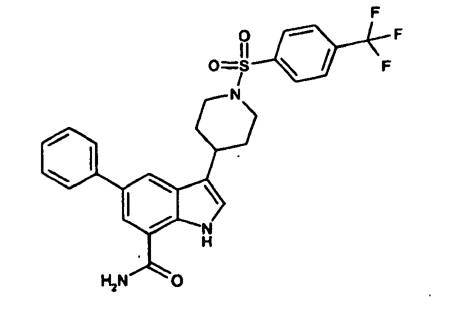

Following the general procedure in example **73**, 5-phenyl-3-(4-piperidinyl)-1*H*-indole-7-carboxamide (32 mg, 0.1 mmol), 4-(trifluoromethyl)benzenesulfonyl chloride (36.7 mg, 0.15 mmol) and triethylamine (0.06 mL, 0.4 mmol) were reacted to form the desired product which was purified by reverse phase HPLC method B (CH₃CN/Water, 0.1 % TFA) (16.2 mg, 31%).
LC/MS: m/z 528.4 (M+H), 2.69 min.

### (82) 3-{1-[(2,4-dichlorophenyl)sulfonyl]-4-piperidinyl}-5-phenyl-1H-indole-7-carboxamide

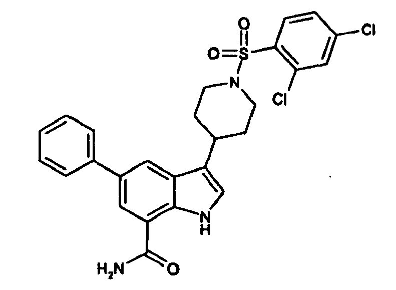

Following the general procedure in example **73**, 5-phenyl-3-(4-piperidinyl)-1*H-*indole-7-carboxamide (32 mg, 0.1 mmol), 2,4-dichlorobenzene sulfonyl chloride (37 mg, 0.15 mmol) and triethylamine (0.06 mL, 0.4 mmol) were reacted to form the desired product which was purified by reverse phase HPLC method B (CH₃CN/Water, 0.1 % TFA) (12.6 mg, 24%).
LC/MS: m/z 528.2 (M+H), 2.60 min.

### (83) 3-{1-[(3,4-dichlorophenyl)sulfonyl]-4-piperidinyl}-5-phenyl-1H-indole-7-carboxamide

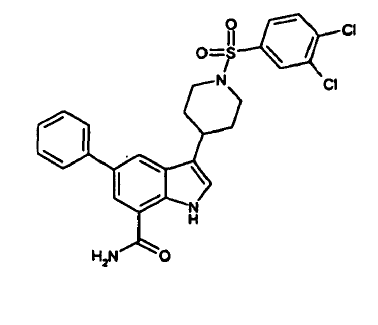

Following the general procedure in example **73**, 5-phenyl-3-(4-piperidinyl)-1*H*-indole-7-carboxamide (32 mg, 0.1 mmol), 3,4-dichlorobenzenesulfonyl chloride (37 mg, 0.15 mmol) and triethylamine (0.06 mL, 0.4 mmol) were reacted to form the desired product which was purified by reverse phase HPLC method B (CH₃CN/Water, 0.1 % TFA) (10 mg, 17%).
LC/MS: m/z 528.2 (M+H), 2.75 min.

### (84) 3-{1-[(ethylamino)carbonyl]-4-piperidinyl}-5-phenyl-1H-indole-7-carboxamide

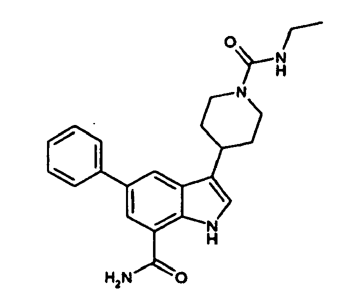

Following the general procedure in example **73**, 5-phenyl-3-(4-piperidinyl)-1*H*-indole-7-carboxamide (50 mg, 0.16 mmol), ethylisocyanate (11.4 mg, 0.16 mmol) and triethylamine (0.07 mL, 0.48 mmol) were reacted to form the desired product which was purified by reverse phase HPLC method B (CH₃CN/Water, 0.1 % TFA) (20 mg, 33%). LC/MS: m/z 391.0 (M+H), 1.94 min.

### (85) 3-{1-[(4-1-piperazinyl)carbonyl]-4-piperidinyl}-5-phenyl-1H-indole-7-carboxamide

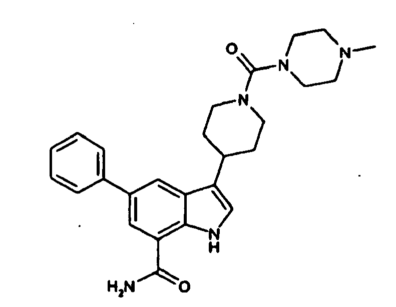

Following the general procedure in example **73**, 5-phenyl-3-(4-piperidinyl)-1*H*-indole-7-carboxamide (50 mg, 0.16 mmol), 4-methyl-1-piperazinecarbonyl chloride (37.3 mg, 0.192 mmol) and triethylamine (0.07 mL, 0.48 mmol) were reacted to form the desired product which was purified by reverse phase HPLC method B (CH₃CN/Water, 0.1 % TFA) (28 mg, 8%).
LC/MS: m/z 446.6 (M+H), 1.63 min.

### (86) 5-(4-chlorophenyl)-3-[1-(propanesulfonyl)-4-piperidinyl]-1H-indole-7-carboxamide

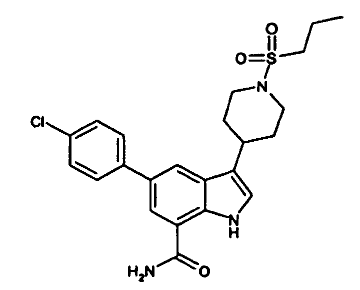

Following the general procedure in example **73**, 5-(4-chlorophenyl)-3-(4-piperidinyl)-1*H-*indole-7-carboxamide (50 mg, 0.14 mmol), 1-propanesulfonyl chloride (0.015 ml, 0.14 mmol) and triethylamine (0.07 mL, 0.48 mmol) were reacted to form the desired product which was purified by reverse phase HPLC method B (CH₃CN/Water, 0.1 % TFA) (15.7 mg, 24%).
LC/MS: m/z 460.4 (M+H), 2.29 min.

### (87) 3-{1-[(4-fluorophenyl)sulfonyl]-4-piperidinyl}-5-(4-chlorophenyl)-1H-indole-7-carboxamide

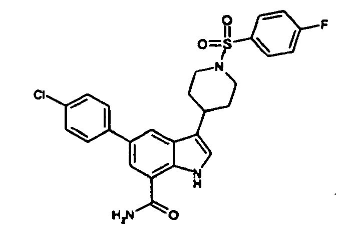

Following the general procedure in example **73**, 5-(4-chlorophenyl)-3-(4-piperidinyl)-1*H-*indole-7-carboxamide (53 mg, 0.14 mmol), 4-fluorobenzenesulfonyl chloride (41 mg, 0.21 mmol) and triethylamine (0.07 mL, 0.48 mmol) were reacted to form the desired product which was purified by reverse phase HPLC method B (CH₃CN/Water, 0.1 % TFA) (6.5 mg, 8%).
LC/MS: m/z 512.2,(M+H), 2.66 min.

### (88) 5-{4-[(methylsulfonyl)amino]phenyl}-3-[1-(phenylmethyl)-1,2,3,6-tetrahydro-4-pyridinyl]-1H-indole-7-carboxamide

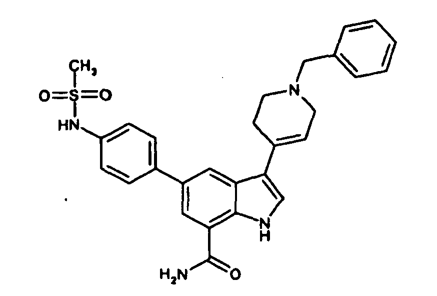

Following the general procedure in example **50**, 5-{4-[(methylsulfonyl)amino]phenyl}-1H-indole-7-carboxamide (488 mg, 1.48 mmol), 1-(phenylmethyl)-4-piperidinone (0.79 mL, 4.44 mmol) and sodium methoxide (0.5 M in THF, 17.76 mL, 8.88 mmol) were reacted to form the desired product which was purified by flash column chromatography (CH₂Cl₂:MeOH:NH₄OH, 94:5:1) (580 mg, 78.4%).
LC/MS: m/z 501.2 (M+H), 1.57 min.

### (89) 5-{4-[(methylsulfonyl)amino]phenyl}-3-(4-piperidinyl)-1H-indole-7-carboxamide

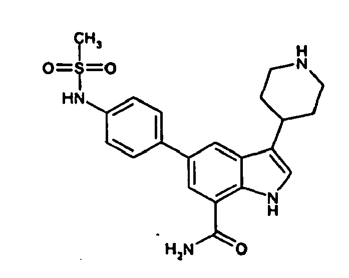

Following the general procedure in example **51**, 5-{45-{4-[(methylsulfonyl)amino] phenyl}-3-[1-(phenylmethyl)-1,2,3,6-tetrahydro-4-pyridinyl]-1H-indole-7-carboxamide (580 mg, 1.16 mmol), palladium hydroxide (154 mg) in a mixture of ethanol and acetic acid (75/1.5 mL) were reacted to form the desired product (163 mg), 20 mg of crude product was purified by reverse phase HPLC method B (CH₃CN/Water, 0.1 % TFA) to give 6 mg (30%) of pure product.
LC/MS: m/z 413.4 (M+H), 1.30 min.

### (90) 5-bromo-3-[1-(ethanesulfonyl)-4-piperidinyl]-1H-indole-7-carboxamide

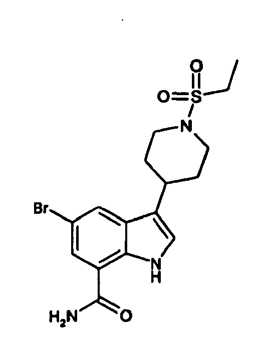

Following the general procedure in example **73**, 5-bromo-3-(4-piperidinyl)-1H-indole-7-carboxamide (900 mg, 2.8 mmol), ethanesulfonyl chloride (0.8 mg, 8.4 mmol) and triethylamine (1.6 mL, 11.2 mmol) were reacted to form the desired product which was purified via solid phase extraction on a 500 mg aminopropyl column (International Sorbent Technologies) eluting with chloroform (30 mLx2) and ethyl acetate (50 mL) (800 mg, 69%), LC/MS: m/z 414.0 (M+H), 2.2 min.

### (91) 3-[1-(ethanesulfonyl)-4-piperldlnyl]-5-{4-[(methylsulfonyl)amino]phenyl}-1H-indole-7-carboxamide

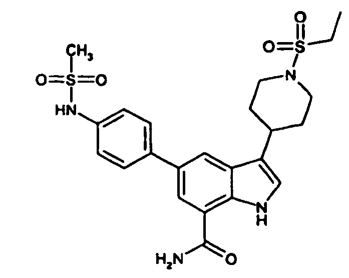

To a solution of 5-bromo-3-[1-(ethanesulfonyl)-4-piperidinyl]-1H-indole-7-carboxamide (50 mg, 0.12 mmol) in a mixture of dioxane and water (1.5mL/0.5 mL), (4-[(methylsulfonyl)amino] phenyl}boronic acid (104 mg, 0.48 mmol), cesium carbonate (78.2 mg, 0.24 mmol) and palladium acetate (5.4 mg, 0.24 mmol) were added. The reaction mixture was heated at 160 °C for 20 min. in a Smith Synthesizer microwave reator. The solvent was removed under reduced pressure and the residue was partitioned between ethyl acetate (50 mL) and water (50 mL). The aqueous layer was extracted with ethyl acetate (50 mLx2) and the combined organic phase was dried over magnesium sulfate, filtered, concentrated under reduced pressure and purified by reverse phase HPLC method B (CH₃CN/Water, 0.1 % TFA) (6.6 mg, 11%).
LC/MS: m/z 505.0 (M+H), 1.58 min.

### (92) 3-[1-(ethanesulfonyl)-4-piperidinyl]-5-(3-methylphenyl)-1H-indole-7-carboxamide

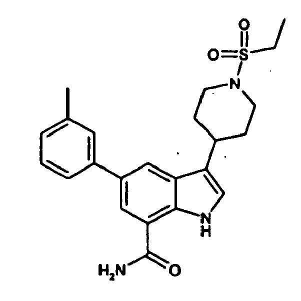

To a solution of 3-[1-(ethanesulfonyl)-4-piperidinyl]-5-(4,4,5,5-tetramethyl-1,3,2-dioxaborolan-2-yl)-1H-indole-7-carboxamide (118 mg, 0.26 mmol) in a mixture of dioxane and water (3.0mL/1.0 mL), 1-bromo-3-methylbenzene (88.94 mg, 0.52 mmol) and cesium carbonate (169.4 mg, 0.52 mmol), palladium acetate (11.7 mg, 0.052 mmol) were added. The resulting reaction mixture was heated via microwave at 160°C for 30 min. All the solvent was removed under reduced pressure. The residue was partitioned between ethyl acetate (10 mL) and water (10 mL). The aqueous layer was extracted with ethyl acetate (10 mLx2) and the combined organic phase was dried over Mg₂SO₄ and concentrated to give a crude product which was purified by reverse phase HPLC method B (CH₃CN/Water, 0.1 % TFA) to provide pure product (10 mg, 10%). LC/MS: m/z 426.0 (M+H), 2.10 min.

### (93) 3-[1-(ethylsulfonyl)-4-piperidinyl]-5-(2-thienyl)-1H-indole-7-carboxamide

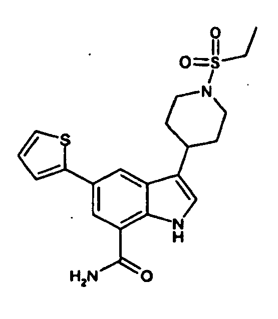

Following the general procedure in example **73**, 3-(4-piperidinyl)-5-(2-thienyl)-1H-indole-7-carboxamide (53 mg, 0.16 mmol), ethanesulfonyl chloride (0.022 mL, 0.24 mmol) and triethylamine (0.07 mL, 0.48 mmol) were reacted to form the desired product which was purified by reverse phase HPLC method B (CH₃CN/Water, 0.1 % TFA) (16.5 mg, 24%).
LC/MS: m/z 418.2 (M+H), 2.21 min.

### (94) 3-[1-(ethylsulfonyl)-4-piperidinyl]-5-(3-thienyl)-1H-indole-7-carboxamide

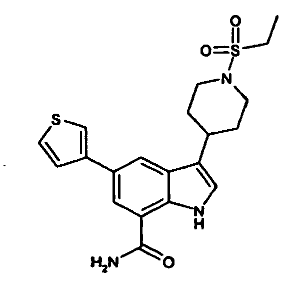

Following the general procedure in example **73**, 3-(4-piperidinyl)-5-(3-thienyl)-1H-indole-7-carboxamide (65 mg, 0.16 mmol), ethanesulfonyl chloride (0.03 mL, 0.3 mmol) and triethylamine (0.09 mL, 0.6 mmol) were reacted to form the desired product which was purified by reverse phase HPLC method B (CH₃CN/Water, 0.1 % TFA) (11.3 mg, 14%).
LC/MS: m/z 418.2 (M+H), 1.90 min.

### (95) 3-[4-(methylsulfonyl)phenyl]-5-phenyl-1H-indole-7-carboxamide

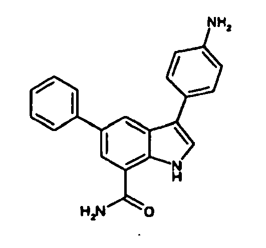

The title compound (15.0 mg) was prepared using general method B except that N-[4-(4,4,5,5-tetramethyl-1,3,2-dioxaborolan-2-yl)phenyl]acetamide (40.0 mg, 0.15 mmol)was used instead of phenyl boronic acid.
1H NMR (400 MHz, MeOD-D4) δ ppm 7.47 (m, 1 H) 7.58 (m, 4 H) 7.85(s, 1H) 7.95 (d, J=7.3 Hz, 2 H) 8.05 (d, J=7.3 Hz, 2 H) 8.07 (2, 1 H) 8.24 (s, 1 H) LC/MS m/z 328 (M+1)+ r.t. 1.74 min.

### (96) 3-{4-[(dimethylamino)sulfonyl]phenyl}-5-phenyl-1H-indole-7-carboxamide

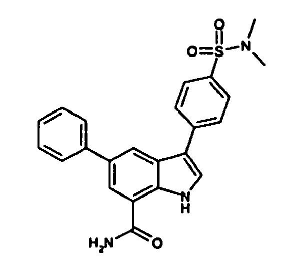

The title compound (10.0 mg) was prepared using general method B except that N,N-dimethyl-4-(4,4,5,5-tetramethyl-1,3,2-dioxaborolan-2-yl)benzenesulfonamide (50.0 mg, 0.15 mmol) was used instead of phenyl boronic acid.
1 H NMR (400 MHz, MeOD-D4) δ ppm 2.70 (s, 6H) 7.57 (m, 1 H) 7.68 (m, 4 H) 7.87(s, 1 H) 7.98 (d, J=7.3 Hz, 2 H) 8.04 (d, J=7.3 Hz, 2 H) 8.07 (2, 1H) 8.24 (s, 1 H) LC/MS m/z 421 (M+1)+ r.t. 2.24 min.

### (97) 3-{3-[(methylsulfonyl)amino]phenyl}-5-phenyl-1H-indole-7-carboxamide

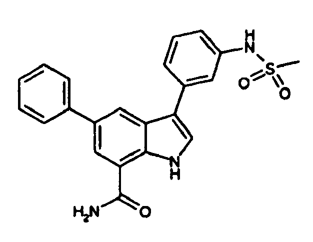

The title compound (13.0 mg) was prepared using general method B except that {3-[(methylsulfonyl)amino]phenyl)boronic acid (35.0 mg, 0.15 mmol) was used instead of phenyl boronic acid.
1H NMR (400 MHz, MeOD-D4) δ ppm 3.05 (s, 3H) 7.27 (m, 1 H) 7.41 (m, 1 H) 7.68 (m, 4 H) 7.87(s, 1 H) 7.98 (s, 1 H) 8.04 (d, J=7.3 Hz, 2 H) 8.07 (s, 1 H) 8.24 (s, 1 H) LC/MS m/z 406 (M+1)+ r.t. 2.04 min.

### (98) 3-[1-(ethylsulfonyl)-4-piperidinyl]-5-{3-[(methylsulfonyl)amino]phenyl}-1H-indole-7-carboxamide

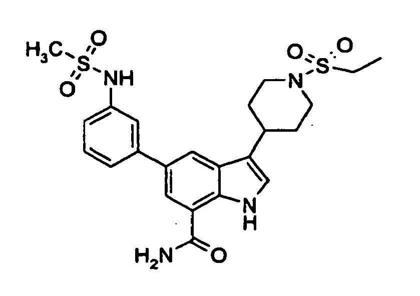

To a solution of 5-bromo-3-[1-(ethylsulfonyl)-4-piperidinyl]-1*H*-indole-7-carboxamide (example **90**) (50.0 mg, 0.120 mmol), cesium carbonate (150.0 mg, 0.480 mmol) and {3-[(methylsulfonyl)amino]phenyl}boronic acid (105.0 mg, 0.480 mmol) in dioxane/H₂O ( 2 mL/0.7 mL) was bubbled argon for 5 minutes prior to the addition of Pd(PPh₃)₄ (12.5 mg, 0.012 mmol). The reaction mixture was heated in a microwave reactor (Smith synthesizer) for 20 minutes at 160°C. The solvent was evaporated, and the residue was dissolved in 1.2 mL DMSO. The resulting suspension was filtered and the organic solution was purified by reverse phase HPLC method A (water/ CH₃CN, 0.1 % TFA) to give the title compound 8.0 mg (13%).
LC/MS: 505.2. r.t: 1.77.

### (99) 5-[4-(acetylamino)phenyl]-3-[1-(ethylsulfonyl)-4-piperidinyl]-1H-indole-7-carboxamide

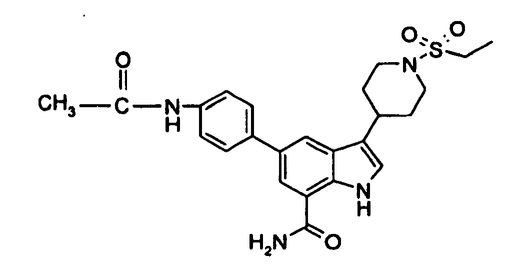

Following the general procedure of example **98**, 5-bromo-3-[1-(ethylsulfonyl)-4-piperidinyl]-1*H*-indole-7-carboxamide (example **90**) (50.0 mg, 0.120 mmol), cesium carbonate (150.0 mg, 0.480 mmol) and *N-*[4-(4,4,5,5-tetramethyl-1,3,2-dioxaborolan-2-yl)phenyl]acetamide (125.0 mg, 0.480 mmol) as well as Pd(PPh₃)₄ (12.5 mg, 0.012 mmol) were reacted to give 19.0 mg (34%) of title compound.
LC/MS: 469.4. r.t: 1.78.

### (100) 5-{4-[(dimethylamino)sulfonyl]phenyl}-3-[1-(ethylsulfonyl)-4-piperidinyl]-1H-indole-7-carboxamide

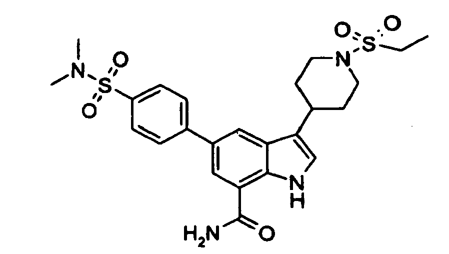

Following the general procedure of example **98** except reaction time was set to 60 minutes instead of 20 minutes, 5-bromo-3-[1-(ethylsulfonyl)-4-piperidinyl]-1*H*-indole-7-carboxamide (example **90**) (50.0 mg, 0.120 mmol), cesium carbonate ( 150.0 mg, 0.480 mmol) and *N*,*N*-dimethyl-4-(4,4,5,5-tetramethyl-1,3,2-dioxaborolan-2-yl)benzenesulfonamide (150.0 mg, 0.480 mmol) as well as Pd(PPh₃)₄ (12.5 mg, 0.012 mmol) were reacted to give 20.0 mg (32%) of title compound.
LC/MS: 519.4. r.t: 1.97.

### (101) 5-[3-(acetylamino)phenyl]-3-[1-(ethylsulfonyl)-4-piperidinyl]-1H-indole-7-carbox-amide

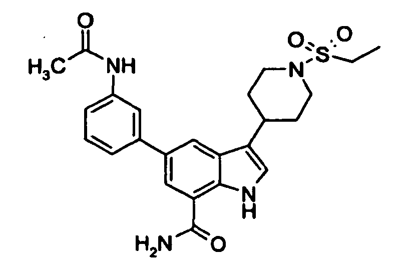

Following the general procedure of example **98**, 5-bromo-3-[1-(ethylsulfonyl)-4-piperidinyl]-1*H*-indole-7-carboxamide (example **90**) (50.0 mg, 0.120 mmol), cesium carbonate (150.0 mg, 0.480 mmol) and [3-(acetylamino)phenyl]boronic acid (100.0 mg, 0.480 mmol) as well as Pd(PPh₃)₄ (12.5 mg, 0.012 mmol) were reacted to give 21.0 mg (38%) of title compound.
LC/MS: 469.4. r.t: 1.76.

### (102) 3-[1-(ethylsulfonyl)-4-piperidinyl]-5-(1H-pyrazol-4-yl)-1H-indole-7-carboxamide

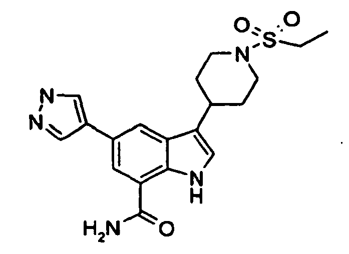

Following the general procedure of example **98**, 5-bromo-3-[1-(ethylsulfonyl)-4-piperidinyl]-1*H*-indole-7-carboxamide (example **90**) (50.0 mg, 0.120 mmol), cesium carbonate (150.0 mg, 0.480 mmol) and 4-(4,4,5,5-tetramethyl-1,3,2-dioxaborolan-2-yl)-1*H*-pyrazole (96.0 mg, 0.480 mmol) as well as Pd(PPh₃)₄ (12.5 mg, 0.012 mmol) were reacted to give 3.0 mg (6%) of title compound.
LC/MS: 402.2. r.t: 1.55.

### (103) 3-[1-(ethylsulfonyl)-4-piperidinyl]-5-[3-(hydroxymethyl)phenyl]-1H-indole-7-carboxamide

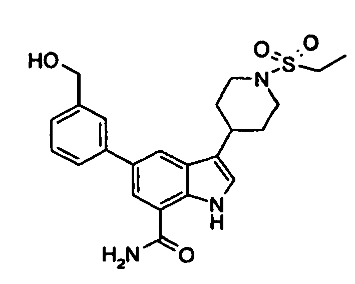

Following the general procedure of example **98**, 5-bromo-3-[1-(ethylsulfonyl)-4-piperidinyl]-1*H*-indole-7-carboxamide (example **90**) (50.0 mg, 0.120 mmol), cesium carbonate (150.0 mg, 0.480 mmol) and [3-(hydroxymethyl)phenyl]boronic acid (72.0 mg, 0.480 mmol) as well as Pd(PPh₃)₄ (12.5 mg, 0.012 mmol) were reacted to give 32.3 mg (61%) of title compound.
LC/MS: 442.4. r.t: 1.75

### (104) 5-(2,4-difluorophenyl)-3-[1-(ethylsulfonyl)-4-piperidinyl]-1H-indole-7-carboxamide

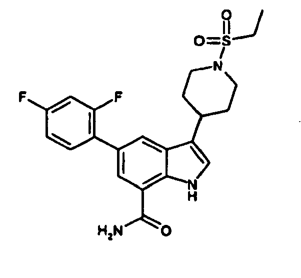

Following the general procedure in example **73**, 5-(2,4-difluorophenyl)-3-(4-piperidinyl)-1*H*-indole-7-carboxamide (15 mg, 0.042 mmol), ethanesulfonyl chloride (0.084 mL, 0.08 mmol) and triethylamine (0.126 mL, 0.02 mmol) were reacted to form the desired product which was purified by reverse phase HPLC method B (CH₃CN/Water, 0.1 % TFA) (6.0 mg, 32%).
LC/MS: m/z 448.2 (M+H), 2.1 min.

### (105) 3-[1-(ethylsulfonyl)-4-piperidinyl]-5-[4-(methyloxy)phenyl]-1H-indole-7-carboxamide;

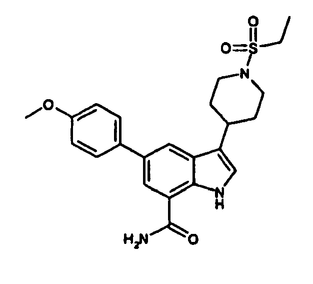

Following the general procedure in example **73**, 5-[4-(methyloxy)phenyl]-3-(4-piperidinyl)-1*H*-indole-7-carboxamide (15 mg, 0.043 mmol), ethanesulfonyl chloride (0.086 mL, 0.09 mmol) and triethylamine (0.129 mL, 0.03 mmol) were reacted to form the desired product which was purified by reverse phase HPLC method B (CH₃CN/Water, 0.1 % TFA) (8.5 mg, 45%).
LC/MS: m/z 442.2 (M+H), 1.96 min.

### (106) 3-[1-(ethylsulfonyl)-4-piperidinyl]-5-(4-fluoro-2-methylphenyl)-1H-indole-7-carboxamide

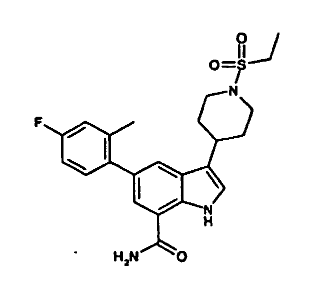

Following the general procedure in example **73**, 5-(4-fluoro-2-methylphenyl)-3-(4-piperidinyl)-1*H*-indole-7-carboxamide (15 mg, 0.042 mmol), ethanesulfonyl chloride (0.084 mL, 0.08 mmol) and triethylamine (0.126 mL, 0.02 mmol)) were reacted to form the desired product which was purified by reverse phase HPLC method B (CH₃CN/Water, 0.1 % TFA) (10.9 mg, 57%).
LC/MS: m/z 444.6 (M+H), 2.06 min.

### (107) 3-[1-(ethylsulfonyl)-4-piperidinyl]-5-(4-fluorophenyl)-1H-indole-7-carboxamide

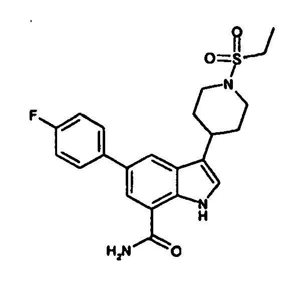

Following the general procedure in example **73**, 5-(4-fluorophenyl)-3-(4-piperidinyl)-1*H-*indole-7-carboxamide (15 mg, 0.044 mmol), ethanesulfonyl chloride (0.09 mL, 0.09 mmol) and triethylamine (0.129 mL, 0.03 mmol) were reacted to form the desired product which was purified by reverse phase HPLC method B (CH₃CN/Water, 0.1 % TFA) (6.8 mg, 36%).
LC/MS: m/z 430.2 (M+H), 2.00 min.

### (108) 5-(4-biphenylyl)-3-[1-ethylsulfonyl)-4-piperidinyl]-1H-indole-7-carboxamide

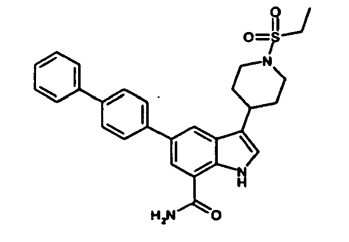

Following the general procedure in example **73**, 5-(4-biphenylyl)-3-(4-piperidinyl)-1*H-*indole-7-carboxamide (15 mg, 0.038 mmol), ethanesulfonyl chloride (0.07 mL, 0.076 mmol) and triethylamine (0.114 mL, 0.015 mmol) were reacted to form the desired product which was purified by reverse phase HPLC method B (CH₃CN/Water, 0.1 % TFA) (8.0 mg, 43%). LC/MS: m/z 488.0 (M+H), 2.36 min.

### (109) 5-[4-(1,1-dimethylethyl)phenyl]-3-[1-(ethylsulfonyl)-4-piperidinyl]-1H-indole-7-carboxamide

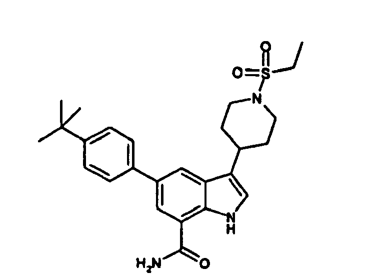

Following the general procedure in example **73**, 5-[4-(1,1-dimethylethyl)phenyl]-3-(4-piperidinyl)-1*H*-indole-7-carboxamide (15 mg, 0.038 mmol), ethanesulfonyl chloride (0.07 mL, 0.076 mmol) and triethylamine (0.114 mL, 0.015 mmol) were reacted to form the desired product which was purified by reverse phase HPLC method B (CH₃CN/Water, 0.1 % TFA) (9.6 mg, 51%).
LC/MS: m/z 468.2 (M+H), 2.45 min.

### (110) 3-[1-(ethylsulfonyl)-4-piperldinyl]-5-(4-methylphenyl)-1H-indole-7-carboxamide

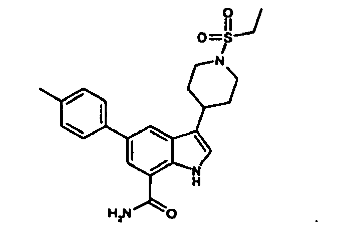

Following the general procedure in example **73**, 5-(4-methylphenyl)-3-(4-piperidinyl)-1*H-*indole-7-carboxamide (15 mg, 0.044 mmol), ethanesulfonyl chloride (0.09 mL, 0.09 mmol) and triethylamine (0.129 mL, 0.03 mmol) were reacted to form the desired product which was purifed by reverse phase HPLC method B (CH₃CN/Water, 0.1 % TFA) (7.9 mg, 41%).
LC/MS: m/z 426.4 (M+H), 2.15 min

### (111) 3-[1-(ethylsulfonyl)-4-piperidinyl]-5-(4-pyridinyl)-1H-indole-7-carboxamide

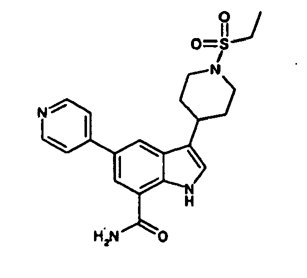

To a solution of 5-bromo-3-[1-(ethanesulfonyl)-4-piperidinyl]-1H-indole-7-carboxamide (50 mg, 0.12 mmol) in a mixture of dioxane and water (1.5mL/0.5 mL), 4-pyridinylboronic acid (60 mg, 0.48 mmol), potassium carbonate (132 mg, 0.96 mmol), tetrakis(triphenyl phosphine)palladium(0) (14 mg, 0.012 mmol) were added. Then it was run microwave reaction at 150°C for 20 min. All solvent was evaporated under reduce pressure. The residue was partitioned between ethyl acetate (10 mL) and water (10 mL). The aqueous layer was washed with ethyl acetate (2x10 mL). The combined organic phase was dried and concentrated under reduce pressure, and purified by reverse phase HPLC method B (CH₃CN/Water, 0.1 % TFA) (12.7 mg, 25%).
LC/MS: m/z 413.4 (M+H), 1.42 min.

### (112) 3-[1-(ethylsulfonyl)-4-piperidinyl]-5-(6-fluoro-3-pyridinyl)-1H-indole-7-carboxamide

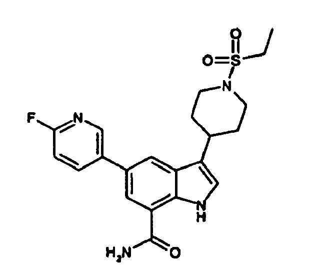

Following the general procedure in example **111**, 5-bromo-3-[1-(ethanesulfonyl)-4-piperidinyl]-1H-indole-7-carboxamide (28.6 mg, 0.07 mmol), (4-fluoro-3-pyridinyl)boronic acid (39.2 mg, 0.28 mmol) and potassium carbonate (75.5 mg, 0.56 mmol), tetrakis(triphenylphosphine)palladium(0) (8 mg, 0.007 mmol) in a mixture of dioxane and water (1.5mL/0.5 mL) were reacted to form the desired product which was purified by reverse phase HPLC method B (CH₃CN/Water, 0.1 % TFA) (11.5 mg, 38%).
LC/MS: m/z 431.2 (M+H), 1.88 min.

### (113) 3-[1-(ethylsulfonyl)-4-piperidinyl]-5-(6-methyl-3-pyridinyl)-1H-indole-7-carboxamide

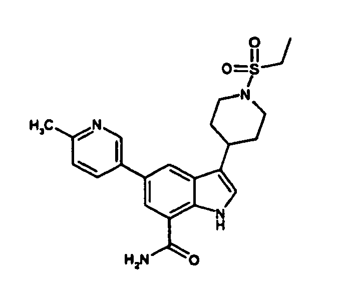

Following the general procedure in example **111**, 5-bromo-3-[1-(ethanesulfonyl)**-**4-piperidinyl]-1H-indole-7-carboxamide (34 mg, 0.08 mmol), (4-methyl-3-pyridinyl)boronic acid (43.82 mg, 0.32 mmol) and potassium carbonate (88.34 mg, 0.64 mmol), tetrakis(triphenylphosphine)palladium(0) (9.52 mg, 0.008 mmol) in a mixture of dioxane and water (1.5mL/0.5 mL) were reacted to form the desired product which was purified by reverse phase HPLC method B (CH₃CN/Water, 0.1 % TFA) (21.7 mg, 62%).
LC/MS: m/z 427.2 (M+H), 1.43 min.

### (114) 3-[1-(ethylsulfonyl)-4-piperidinyl]-5-(4-methyl-3-pyridinyl)-1H-indole-7-carboxamide

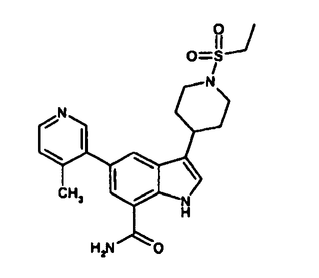

Following the general procedure in example **111**, 5-bromo-3-[1-(ethanesulfonyl)-4-piperidinyl]-1H-indole-7-carboxamide (34 mg, 0.08 mmol), (6-methyl-3-pyridinyl)boronic acid (43.82 mg, 0.32 mmol) and potassium carbonate (88.34 mg, 0.64 mmol), tetrakis(triphenylphosphine)palladium(0) (9.52 mg, 0.008 mmol) in a mixture of dioxane and water (1.5mL/0.5 mL) were reacted to form the desired product which was purified by reverse phase HPLC method B (CH₃CN/Water, 0.1 % TFA) (23 mg, 65%).
LC/MS: m/z 427.2 (M+H), 1.32 min.

### (115) 3-[1-(ethylsulfonyl)-4-piperldinyl]-5-[6-(methyloxy)-3-pyridinyl]-1H-indole-7-carboxamide

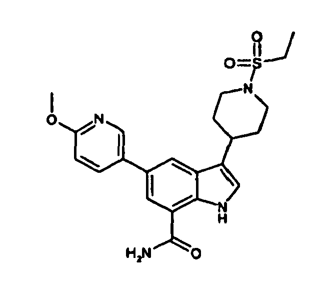

Following the general procedure in example **111**, 5-bromo-3-[1-(ethanesulfonyl)**-**4-piperidinyl]-1H-indole-7-carboxamide (34 mg, 0.08 mmol), (4-methoxy-3-pyridinyl)boronic acid (48.94 mg, 0.32 mmol) and potassium carbonate (88.34 mg, 0.64 mmol), tetrakis(triphenylphosphine)palladium(0) (9.52 mg, 0.008 mmol) in a mixture of dioxane and water (1.5mL/0.5 mL) were reacted to form the desired product which was purified by reverse phase HPLC method B (CH₃CN/Water, 0.1 % TFA) (16.6 mg, 45%).
LC/MS: m/z 443.0 (M+H), 1.84 min.

### (116) 5-phenyl-3-(N-acetyl-3-piperidinylmethyl)-1H-indole-7-carboxamide

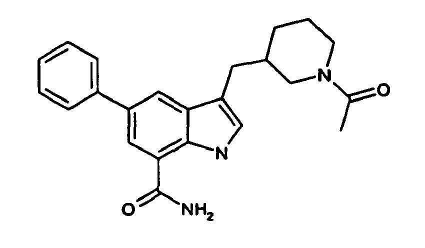

To a solution of 5-phenyl-3-(3-piperidinylmethyl)-1*H*-indole-7-carboxamide (example **68**) (50 mg, 150 µmol) in CH₂Cl₂ (20mL) at 0°C was added Hunig's base (100 µL, 574 µmol). The reaction mixture was stirred for 5 minutes then acetyl chloride (10.6 µL, 150 µmol) was added dropwise. After one hour, the reaction mixture was quenched with water (10 mL) and warmed to rt. The aqueous layer was extracted with CH₂Cl₂ (2x10 mL) and the combined organic phase was concentrated *in vacuo.* The residue was purified by reverse phase HPLC method A (acetonitrile/water) to yield the title compound (8.3 mg, 15%).
LC/MS: 376.2. r.t: 2.11

### (117) 5-[3-(ethyloxy)phenyl]-3-[1-(ethylsulfonyl)-4-piperidinyl]-1H-indole-7-carboxamide

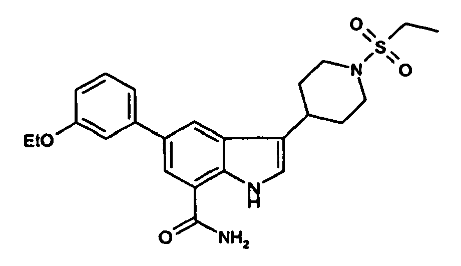

A solution of K₂CO₃ (40 mg, 0.289 mmol) in H₂O (1.2 mL) was added to a slurry of 5-bromo-3-[1-(ethanesulfonyl)-4-piperidinyl]-1*H*-indole-7-carboxamide (40 mg, 0.0965 mmol) in 1,4-dioxane (2.8 mL) in a CEM microwave vial. 3-Ethoxyphenylboronic acid (65 mg, 0.386 mmol) was then added, followed by chloro(di-2-norbomylphosphino)(2-dimethyl-aminomethylferrocen-1-yl)palladium (II) (1 mg, 0.00165 mmol). The vial was capped and heated in a CEM microwave for 10 min at 160 °C. The reaction was determined to be complete by LC/MS analysis. The reaction mixture was concentrated under a stream of nitrogen at 80 °C, and the crude product was dissolved in 1 mL DMSO. This solution was filtered through a 0.45 µM PTFE membrane (Acrodisc) and then purified via preparative HPLC. Samples were purified using an Agilent 1100 Series LC with UV and MSD detection and fraction collection. DMSO solutions of crude products were injected onto a ZORBAX Eclipse XDB-C18 column (21.2 x 50 mm) and eluted over 10.6 min at a flow rate of 20 mL/min. Fraction collection was triggered by absorption at 214 nm. 7.9 mg (18%) of the title compound was recovered.
LC/MS ESI R_{T} 2.19 min MH⁺, 456

### (118) 3-[1-(ethylsulfonyl)-4-piperidinyl]-5-(2-fluorophenyl)-1H-indole-7-carboxamide

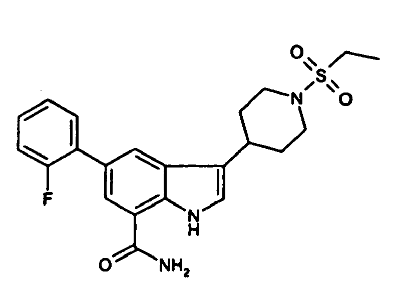

The title compound was prepared according to the procedure in example **117**, except that 2-fluorophenylboronic acid (55 mg, 0.386 mmol) was used instead of 3-ethoxyphenylboronic acid.
LC/MS ESI R_{T} 2.04 min MH⁺ 430

### (119) 3-[1-ethylsulfonyl)-4-piperidinyl]-5-[3-(trifluoromethyl)phenyl]-1H-indole-7-carboxamide

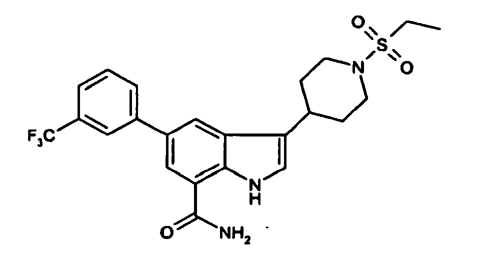

The title compound was prepared according to the procedure in example **117**, except that (3-trifluoromethyl)phenylboronic acid (74 mg, 0.386 mmol) was used instead of 3-ethoxyphenylboronic acid.
LC/MS ESI R_{T} 2.27 min MH⁺ 480

### (120) 3-[1-(ethylsulfonyl)-4-piperidinyl]-5-[4-(trifluoromethyl)phenyl]-1H-indole-7-carboxamide

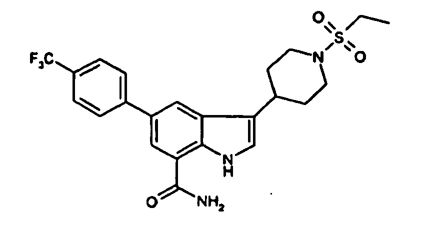

The title compound was prepared according to the procedure in example **117**, except that (4-trifluoromethyl)phenylboronic acid (74 mg, 0.386 mmol) was used instead of 3-ethoxyphenylboronic acid.
LC/MS ESI R_{T} 2.28 min MH⁺ 480

### (121) 3-[1-(ethylsulfonyl)-4-piperidinyl)-5-(3-fluorophenyl)-1H-indole-7-carboxamide

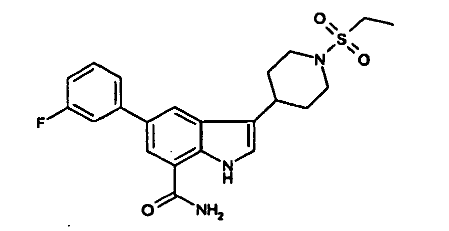

The title compound was prepared according to the procedure in example **117**, except that 3-fluorophenylboronic acid (55 mg, 0.386 mmol) was used instead of 3-ethoxyphenylboronic acid.
LC/MS ESI R_{T} 2.11 min MH⁺ 430

### (122) 5-(3,5-dichlorophenyl)-3-[1-(ethylsulfonyl)-4-piperidinyl]-1H-indole-7-carboxamide

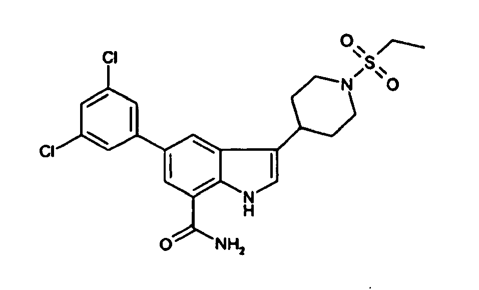

The title compound was prepared according to the procedure in example **117**, except that 3,5-dichlorophenylboronic acid (75 mg, 0.386 mmol) was used instead of 3-ethoxyphenylboronic acid, and the crude product was purified by reverse phase HPLC method A (CH₃CN/Water, 0.1 % TFA, 10/90 v/v)
LC/MS ESI R_{T} 2.26 min MH⁺ 480

### (123) 5-(3,4-difluorophenyl)-3-[1-(ethylsulfonyl)-4-piperidinyl]-1H-indole-7-carboxamide

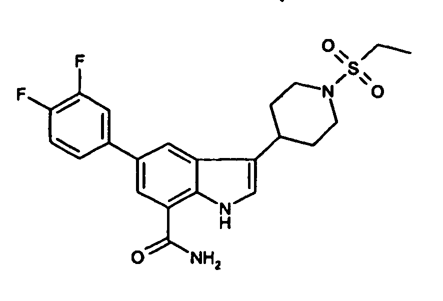

The title compound was prepared according to the procedure outlined in the general method, except that 3,4-difluorophenylboronic acid (62 mg, 0.386 mmol) was used instead of 3-ethoxyphenylboronic acid, and the crude product was purified by reverse phase HPLC method A (CH₃CN/Water, 0.1 % TFA, 10/90 v/v,) LC/MS ESI R_{T} 2.17 min MH⁺ 448

Using the same procedure as example **73**, examples **124-155** were synthesized. Compounds were purified via solid phase extraction on a 500 mg aminopropyl column (International Sorbent Technologies) eluting with chloroform (2 mL) then ethyl acetate (2 mL) to give the desired compound.

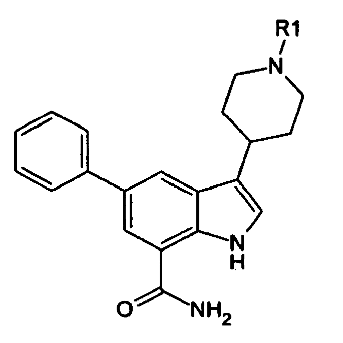

**Table 1**

| **Example** | **R** | **MS [M+]** | **Rt (min)** |
|---|---|---|---|
| 124 | 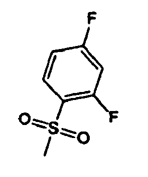 | 496 | 2.45 |
| 125 | 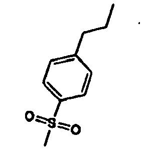 | 502 | 2.71 |
| 126 | 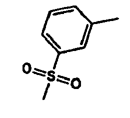 | 474 | 2.56 |
| 127 | 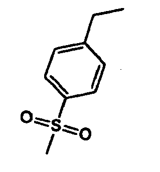 | 487 | 2.57 |
| 128 | 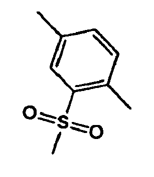 | 487 | 2.37 |
| 129 | 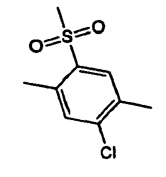 | 522 | 2.75 |
| 130 | 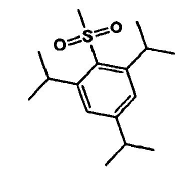 | 586 | 3.1 |
| 131 | 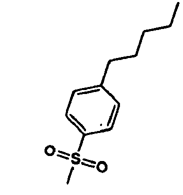 | 530 | 2.97 |
| 132 | 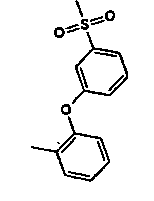 | 566 | 2.79 |
| 133 | 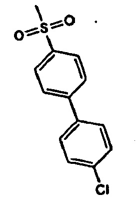 | 570 | 2.99 |
| 134 | 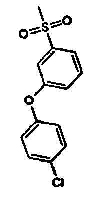 | 586 | 2.8 |
| 135 | 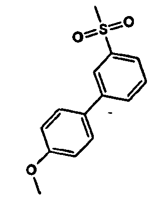 | 566 | 2.69 |
| 136 | 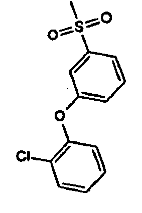 | 586 | 2.76 |
| 137 | 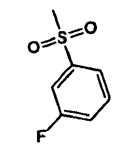 | 478 | 2.42 |
| 138 | 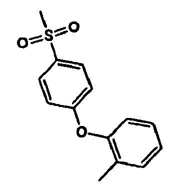 | 566 | 2.81 |
| 139 | 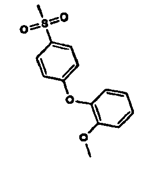 | 582 | 2.68 |
| 140 | 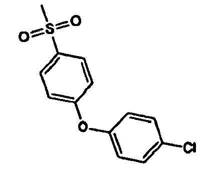 | 586 | 2.83 |
| 141 | 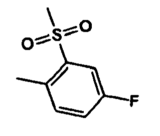 | 492 | 2.48 |
| 142 | 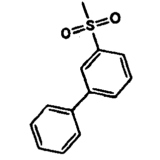 | 536 | 2.72 |
| 143 | 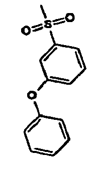 | 552 | 2.64 |
| 144 | 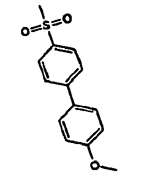 | 566 | 2.71 |
| 145 | 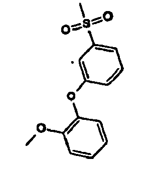 | 582 | 2.62 |
| 146 | 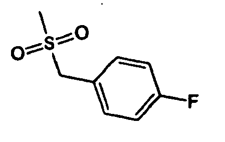 | 493 | 3.06 |
| 147 | 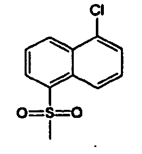 | 544 | 2.8 |
| 148 | 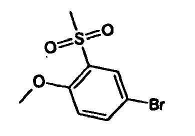 | 568 | 2.52 |
| 149 | 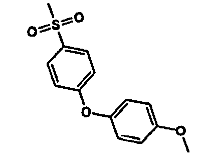 | 582 | 2.47 |
| 150 | 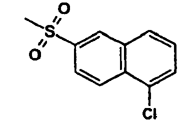 | 544 | 2.84 |
| 151 | 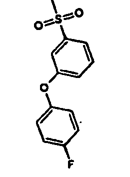 | 570 | 3.07 |
| 152 | 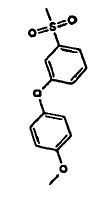 | 582 | 3.15 |
| 153 | 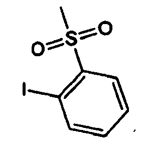 | 586 | 2.47 |
| 154 | 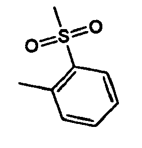 | 474 | 2.43 |
| 155 | 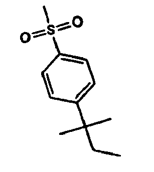 | 530 | 2.86 |

Using the general procedure as following, examples **156-166** were synthesized. Chloro(di-2-norbomylphosphino)(2'-dimethylamino-1,1'-biphenyl-2-yl) palladium (II). (3 mg, 5 mol%) and a solution of K₃PO₄ (2 equiv) in H₂O (0.1 mL) were added to a solution of the 5-bromo-indolecarboxamide (24 mg, 0.1 mmol) in dioxane (1 mL). The aryl-boronic acid (3 equiv) was added and the mixture was stirred at 90 °C for 18 h and then at rt for 10 min. Chloroform (1.5 mL) and H₂O (0.5 mL) were added, and the layers were separated. The organic layer was concentrated under reduced pressure, and the crude residue was taken up in chloroform (1 mL). Polymer bound pyridine-hydrobromide-perbromide (55 mg, 0.11 mmol, Fluka) was added, and the mixture was agitated for 2 h. The reaction was filtered, and the resin was washed with chloroform (1.5 mL) and MeOH (1 mL). The filtrate was concentrated, and the crude product was purified via mass-directed reverse phase HPLC to give the desired compound.
Mass-Directed HPLC conditions:
Samples were purified using an Agilent 1100 Series LC with UV and MSD detection and fraction collection. Crude samples were dissolved in a 1:1 mixture of DMSO/methanol (600 µL) for injection onto a ZORBAX Eclipse XDB-C18 column (21.2 x 50 mm) and eluted over ~10 min. at a flow rate of 20 mL/min. Fraction collection was triggered by absorption at 230 nm and triggering of the MSD at the MH+ of the desired compound.

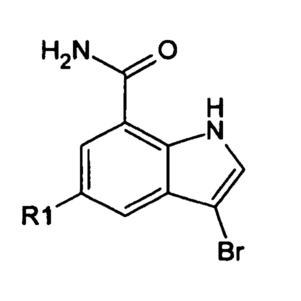

**Table 2**

| **Example** | **R** | **MS [M+]** | **Rt (min)** |
|---|---|---|---|
| 156 | 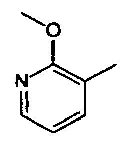 | 345 | 3.19 |
| 157 | 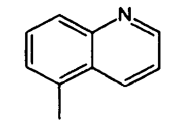 | 365 | 3.07 |
| 158 | 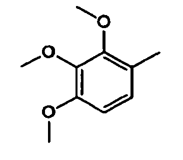 | 404 | 3.31 |
| 159 | 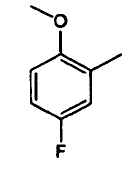 | 362 | 3.49 |
| 160 | 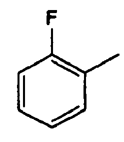 | 332 | 3.51 |
| 161 | 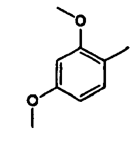 | 374 | 3.39 |
| 162 | 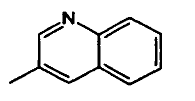 | 365 | 3.41 |
| 163 | 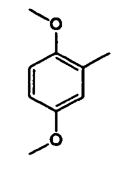 | 374 | 3.38 |
| 164 | 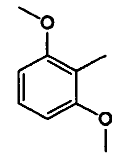 | 374 | 3.37 |
| 165 | 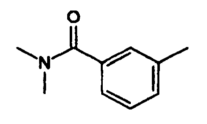 | 385 | 3.03 |
| 166 | 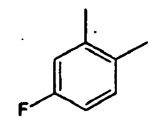 | 346 | 3.75 |

### (167) 5-(2-fluorophenyl)-1H-indole-7-carboxamide

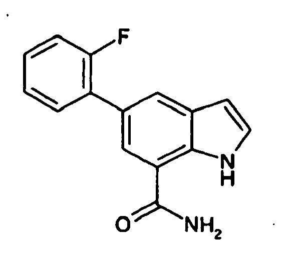

5-Bromo-1*H*-indole-7-carboxamide (0.050 g, 0.21 mmol), 2-fluorophenyl boronic acid (0.088g, 0.63mmol) and potassium phosphate (0.089 g, 0.42 mmol) were treated with a solution of 2'(dimethylamino)-2-biphenyl-palladium (II) chloride Dinorbornyl phosphine complex (0.006 g, 0.0107 mmol) in 1,4-doxan (1.5 mL). Water (1.5 mL) was added and the reaction mixture was heated at 85 °C for 17 hours under nitrogen. The cooled mixture was pre-absorbed onto silica gel *in* vacuo and purified on a silica gel SPE cartridge [2g] eluting with a cyclohexane / ethyl acetate gradient system to give the title compound (0.043 g, 81%).

1H NMR (400 MHz, DMSO-D6) δ ppm 11.2 (v.brs, 1 H) 8.16 (br.s, 1 H) 7.90,7.86(2 x br.s , 2 H) 7.62(br.t, 1 H) 7.45-7.35 (br.s + m, 3 H) 7.35-7.27 (m, 2 H) 6.55 (dd, 1 H) MS m/z 255 (M+1)⁺ r.t. 3.06 min.

Using the general procedure as following, examples **168-172** were synthesized. 4-[7-(aminocarbonyl)-1*H*-indol-5-yl]benzoic acid or 3-[7-(aminocarbonyl)-1*H*-indol-5-yl]benzoic acid (67 umol, 18.7 mg) was dissolved in DMF (1 mL) and treated with HATU (1.12 eq, 75 umol, 28.5 mg) and DIPEA (3 eq, 0.2 mmol, 35.5 uL). After shaking for 5mins a solution was obtained which was treated with an amine (1.1 eq. 74 umol) and allowed to stand at rt overnight. Excess solvent was removed using a genevac and the crudes purified using aminopropyl SPE (500 mg) load: CHCl3 (500 uL), elute: CHCl3 (1500 uL), ethyl acetate (1500 uL) and 20% methanol: ethyl acetate (1500 uL). The ethyl acetate and methanol: ethyl acetate fractions were combined, concentrated and further purified using MDAP.

Samples were purified using an Agilent 1100 Series LC with UV and MSD detection and fraction collection. Crude samples were dissolved in a 1:1 mixture of DMSO/methanol (600 uL) for injection onto a ZORBAX Eclipse XDB-C18 column (21.2 x 50 mm) and eluted over -10 min. at a flow rate of 20 mL/min. Fraction collection was triggered by absorption at 230 nm and triggering of the MSD at the MH+ of the desired compound.

**Table 3**

| **Example** | **Structure** | **MS [M+]** | **Rt (min)** |
|---|---|---|---|
| 168 | 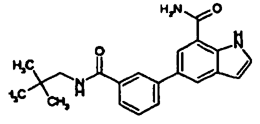 | 350 | 3.24 |
| 169 | 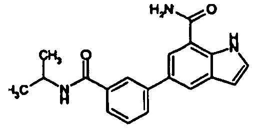 | 322 | 2.98 |
| 170 | 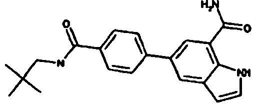 | 350 | 3.21 |
| 171 | 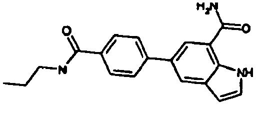 | 322 | 2.96 |
| 172 | 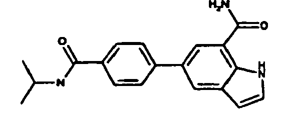 | 322 | 2.94 |

### (173) 5-{4-[(diethylamino)carbonyl]phenyl}-1H-indole-7-carboxamide

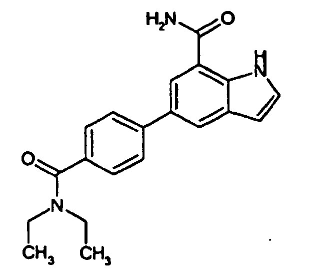

4-[7-(aminocarbonyl)-1*H*-indol-5-yl]benzoic acid (67 umol, 18.7 mg) was dissolved in DMF (1mL) and treated with HATU (1.12 eq, 75 umol, 28.5 mg) and DIPEA (3 eq, 0.2 mmol, 35.5 uL). After shaking for 5mins a solution was obtained which was treated with an amine (1.1eq, 74umol) and allowed to stand at rt overnight. Excess solvent was removed using a genevac and the crudes purified using aminopropyl SPE (500 mg) load: CHCl3 (500 uL), elute: CHCl3 (1500 uL), ethyl acetate (1500 uL) and 20% methanol: ethyl acetate (1500 uL). The fractions were concentrated and the largest used for characterisation.
LC/MS m/z 336 (M+1)⁺ Rt. 3.03min..

### (174) 3-[1-(methylsulfonyl)-1,2,3,6-tetrahydro-4-pyridinyl]-5-phenyl-1H-indole-7-carboxamide

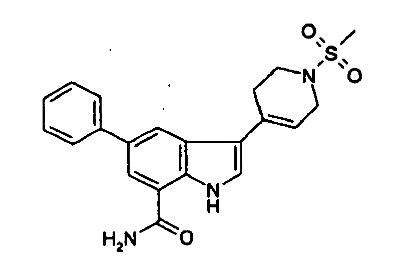

5-Phenyl-1H-indole-7-carboxamide (47 mg, 0.0002 mole),1-(methylsulfonyl)-4-piperidinone (35 mg, 0.0002 mole) and bismuth (III) triflate (4 mg) were combined in acetonitrile (8 mL), and the resulting mixture stirred at room temperature for 24hr. The mixture was pre absorbed onto silica, purification by silica flash chromatography using an ethyl acetate/cyclohexane elution system yielded the title compound (7 mg) as an off white solid.
MS m/z 395 (M+1)⁺ Rt. 3.21 min.

### (175) 3-(3-oxocyclopentyl)-5-phenyl-1H-indole-7-carboxamide

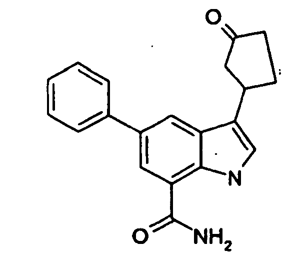

To a solution of 5-phenyl-1H-indole-7-carboxamide (1.0 g, 4.2 mmol) and cyclopentenone (4.24 mmol, 348 mg) in dry acetonitrile (20 mL) was added bismuth triflate (3 mol%, 0.127 mmol, 83 mg). The resultant mixture was stirred at room temperature for 18hr. The mixture was pre-absorbed onto silica and purified on a 20 g silica Bondelut cartridge, eluting with dichloromethane, then, cyclohexane: ethylacetate 2:1 to *give the title compound* as a white powder (600 mg, 45%)
LC/MS m/z 401, R.t. 3.2 min.

### (176) 5-phenyl-3-{3-[(phenylmethyl)amino]cyclopentyl}-1H-indole-7-carboxamide

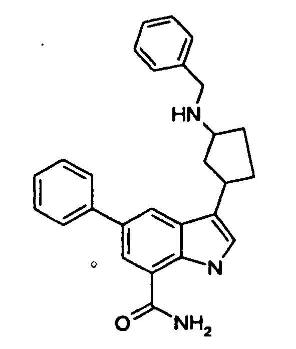

A solution of 3-(3-oxocyclopentyl)-5-phenyl-1H-indole-7-carboxamide (370 mg, 1.16 mmol) in dry dichloromethane containing activated molecular sieves was treated with glacial acetic acid (1eq, 1.16mmol, 70mg) followed by benzylamine (5eq, 5.8 mmol, 622 mg). After stirring for 20min at RT under nitrogen, sodium triacetoxyborohydride (5 eq, 5.8 mmol, 1.23 g) was added and stirring was continued overnight. Methanol (2 mL), dichloromethane (10 mL) and 2N aqueous sodium hydroxide (10 mL) were added and the layers were separated and the aqueous layer was extracted with dichloromethane. The combined organic layers were dried by filtering through a hydrophobic frit and evaporated to dryness to give a yellow oil which was purified on a 20g silica Bondelute cartridge, eluting with dichloromethane: ammonia methanol(2 M) 100:1, then 60:1, to give the separation of isomers, Isomer 1 (135.4 mg) and isomer 2 (185 mg)). Isomer 2 is the title compound as a solid (185 mg).
LC/MS m/z 410, R.t. 2.7 min.

### (177) 3-(3-aminocyclopentyl)-5-phenyl-1H-indole-7-carboxamide

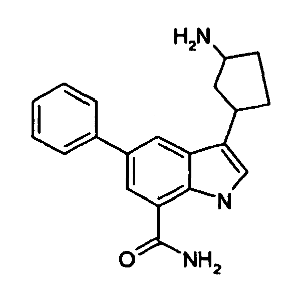

A mixture containing 5-phenyl-3-{3-[(phenylmethyl)amino]cyclopentyl}-1H-indole-7-carboxamide 170 mg, 0.42 mmol) ammonium formate (7 eq, 2.9 mmol, 183 mg) and 10% palladium on carbon (90 mg) in ethanol (4 mL) was stirred under nitrogen for 4hrs. Reaction was incomplete hence ammonium formate (90 mg) and 10% palladium on carbon (50 mg) in ethanol (2 mL) were added to the mixture and stirring was continued for a further 15hr. Reaction mixture was filtered through fibre glass filter paper. The filtrate was evaporated to dryness to give *the title compound* as an off-white solid. (76 mg).
LC/MS: m/z 320, Rt. 2.3 min.

### (178) 3-{3-[(ethylsulfonyl)amino]cyclopentyl}-5-phenyl-1H-indole-7-carboxamide

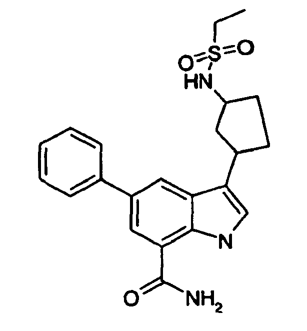

To 3-(3-aminocyclopentyl)-5-phenyl-1*H*-indole-7-carboxamide (62 mg 0.19 mmol) was added pyridine (2 mL) and ethanesulfonyl chloride (1.2 eq, 0.23 mmol,30 mg). Resultant orange solution was stirred at room temperature for 5hrs. The resultant mixture was quenched with saturated sodium bicarbonate and extracted with ethylacetate, then dried using hydrophobic frit and evaporated to dryness to give yellow oil which was then purified using MDAP to give the title compound as an orange solid (10 mg)
1HNMR (400MHz, DMSO-d6) 10.85 (1H, br.s) 8.20 (1H, br.s) 7.99 (2H, s) 7.78(2H, m) 7.45 (2H, t) 7.38 (1H, br.s) 7.35 (1H, tt) 7.24 (1H, d), 7.14 (1 H, s) 3.80 (1H,m) 3.35 (1H, m[+H₂O]) 3.00 (2H, q) 2.5-1.6 (6H, 3xm, 3xCH2) 1.5 (3H, t)
LC/MS m/z 412, Rt. 3.12 min

### (179) 5-bromo-3-[1-(propylsulfonyl)-4-piperidinyl]-1H-indole-7-carboxamide

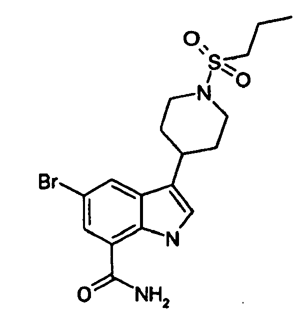

To a solution of 5-bromo-3-(4-piperidinyl)-1*H* indole-7-carboxamide (406 mg, 1.26 mmol) in pyridine (20 mL) was added propanesulfonyl chloride (200 uL, 2.04 mmol, 1.8 eq). Resultant mixture was heated at 90°C for 90hr. The mixture was evaporated to dryness and partitioned between 2N NaOH (200 mL), brine (15 mL) and dichloromethane (2 x 80 mL) and ethylacetate (2 x 70 mL). The combined organic extract was dried and evaporated to dryness to give a yellow oil which was purified by MDAP to give *the title compound* as a solid (6.7 mg).
1HNMR (400MHz, DMSO-d6) 11.05 (1H, br. s) 8.15 (1H, br s) 7.95 (1H, br.s) 7.83 (1H,br.s) 7.45(1H, br s) 7.18 (1 H, s) 3.70 (2H, br.d) 3.05 (2H, m) 2.95 (3H, m) 2.05 (2H, br.d) 1.75 (2H, m) 1.60 (2H, dq) 1.05 (3H, t)
LC/MS m/z 426.1, Rt. 3.13 min

### (180) 5-bromo-3-(3-pyridinyl)-1H-indole-7-carboxamide

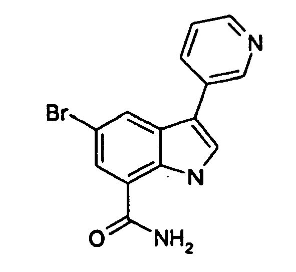

A solution of 1,1-dimethylethyl 7-[(bis{[(1,1-dimethylethyl)oxy]carbonyl}amino)carbonyl]-5-bromo-3-(3-pyridinyl)-1*H*-indole-1-carboxylate (70 mg, 0.11 mmol) in HCl in acetic acid (10 mL, 10 mmol, 90 eq) was stirred at room temperature for 2hr 30min. The resultant mixture was partitioned between 2N NaOH (30 mL), ethylacetate (2x10 mL), dichloromethane (2x10 mL) and brine (5 mL). The organic extracts were combined, dried and concentrated *in vacuo* to give *the title compound* as a solid (12.2 mg).
1HNMR (400MHz, DMSO-d6) 11.70(1H, br.s) 8.88 (1H,d) 8.48 (1H,br.d) 8.28 (1H, br.s) 8.13 (1H,s) 8.08 (1 H,dt) 7.92 (1H,br.s) 7.80 (1H, s) 7.60 (1H, br.s) 7.45 (1H,dd)
LC/MS m/z 318.1, Rt 2.44 min

### (181)5-bromo-3-[1-(methylsulfonyl)-1,2,3,6-tetrahydro-4-pyridinyl]-1H-indole-7-carboxamide

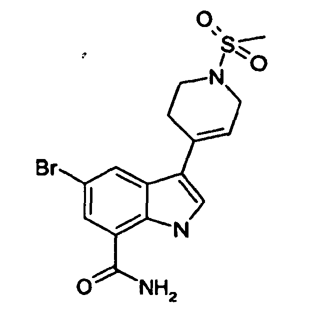

A mixture of 5-bromo-1H-indole-7-carboxamide (55 mg, 0.21 mmol) with 1-(methylsulfonyl)-4-piperidinone (112 mg, 0.678 mmol, 3 eq) in sodium methoxide in methanol,(3 mL, 1.5 mmol, 6 eq) was heated at reflux under nitrogen for 18hrs. The reaction mixture was evaporated to dryness and partitioned between 2N-NaOH (40 mL), brine (5 mL), dichloromethane (2x20 mL) and ethyl acetate (2x15 mL). The combined organic extract was dried, then evaporated to dryness. Purification by preparative chromatography gave the title compound as a solid (4.69 mg).
1HNMR (400MHz, DMSO-d6) 11.40 (1H, br, s) 8.20 ( 1H, br.s) 8.15 (1H, br.s) 7.90 (1H,br.s) 7.55 (1H,br.s) 7.45 (1H,s) 6.15 (1H, t) 3.90 (2H,t) 2.92 (3H,s) 2.60 (2H,br t)
LC/MS m/z 398.1, Rt. 2.87 min.

### (182) 3-[(4-hydroxyphenyl)methyl]-5-phenyl-1H-indole-7-carboxamide

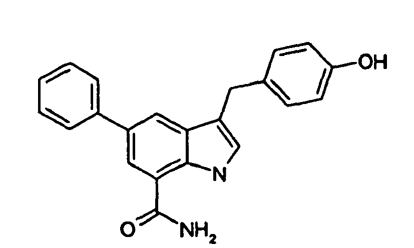

A mixture of [7-(aminocarbonyl)-5-phenyl-1*H*-indol-3-yl]-*N*,*N*,*N*-trimethylmethanaminium iodide (54.3 mg, 0.130 mmol), with sodium phenoxide (150 mg, 0.89 mm0l, 6.8 eq) in acetonitrile (3 mL) was stirred at room temperature for 18hrs. The resultant mixture was evaporated to dryness and purified on a 5g silica Bondelute cartridge, eluting with ethyl acetate, followed by purification on MDAP to give *the title compound* as a solid (4.37 mg). 1HNMR (400MHz, DMSO-d6) 10.88 (1H, br s) 9.15 (1H, br.s) 7.98 (1H, br.s) 7.88 (1H, br.s) 7.83 (2H, dd) 7.45 (2H, t) 7.30 (1H, tt) 7.90 (3H, m) 6.65 (2H, 1/2AA'BB') 3.98 (2H, s)
LC/MS m/z 343.28, Rt. 3.33min.

### BIOLOGICAL DATA

### IKK2 Assay

Recombinant human IKK2 (residues 1-737) was expressed in baculovirus as a C-terminal GST-tagged fusion protein, and its activity was assessed using a time-resolved fluorescence resonance energy transfer (TR-FRET) assay. Briefly, IKK2 (5 nM final) diluted in assay buffer (50 mM HEPES, 10 mM MgCl₂, 1 mM CHAPS pH 7.4 with 1 mM DTT and 0.01% w/v BSA) was added to wells containing various concentrations of compound or DMSO vehicle (3% final). The reaction was initiated by the addition of GST-IκBα substrate (25 nM final)/ATP (1 µM final), in a total volume of 30 µl. The reaction was incubated for 30 minutes at room temperature, then terminated by the addition of 15 µl of 50 mM EDTA. Detection reagent (15 µl) in buffer (100 mM HEPES pH 7.4, 150 mM NaCl and 0.1 % w/v BSA) containing antiphosphoserine-IκBα-32/36 monoclonal antibody 12C2 (Cell Signalling Technology, Beverly Massachusetts, USA) labelled with W-1024 europium chelate (Wallac OY, Turku, Finland), and an APC-labelled anti-GST antibody (Prozyme, San Leandro, California, USA) was added and the reaction was further incubated for 60 minutes at room temperature. The degree of phosphorylation of GST-IκBα was measured using a Packard Discovery plate reader (Perkin-Elmer Life Sciences, Pangboume, UK) as a ratio of specific 665 nm energy transfer signal to reference europium 620 nm signal.

Examples 1-182 (with the exception of Examples 3,32,33,35, 65, 81, 83, 154, 157, 158, 159, 161, 163 and 166) and compounds labelled as Intermediates 7, 24 and 30 were all found to have activity >4.8 in the above identified assay.

## Claims

1. A compound of Formula (I): 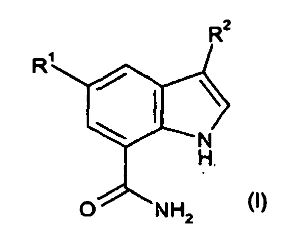 wherein R¹ represents H, halogen, or a group -YZ;
Y represents a bond (i.e. is absent), C₁₋₆ alkylene or C₂₋₆ alkenylene;
Z represents an aryl or heteroaryl group each comprising 5-14 ring members, said aryl or heteroaryl being optionally substituted by one or more substituents independently selected from halogen, OH, C₁₋₆ alkyl, C₁₋₆ haloalkyl, C₁₋₆ alkoxy, C₁₋₆ haloalkoxy, CN, C₁₋₆ hydroxyalkyl, phenyl, O-(CH₂)₁₋₆-phenyl, NHSO₂R³, NHCOR³, CONR⁴R⁵, SO₂NR⁴R⁵;
R³, R⁴ and R⁵ independently represent H or C₁₋₆ alkyl;
R² represents H, halogen or a group -Y¹Z¹;
Y¹ represents a bond (i.e. is absent), C₁₋₆ alkylene, C₂₋₆ alkenylene;
Z¹ represents a 6 membered aryl, 5 or 6 membered heteroaryl, 5 - 7 membered heterocyclyl, C₅₋₇ cycloalkyl, C₅₋₇ cycloalkenyl, each of which may be optionally substituted by one or more substituents independently selected from SO₂R⁶ NHSO₂R⁶, 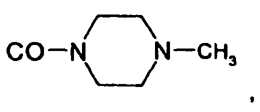 COR⁷, NR⁷R⁸, SO₂NR⁷R⁸, C₁₋₆ alkyl, C₁₋₆ haloalkyl, C₁₋₆ alkoxy, C₁₋₆ haloalkoxy, halogen, CONR⁷R⁸, NHCOR⁷, or phenyl (directly attached or attached by a C₁₋₆alkylene, CONH, C₂₋₆ alkenylene spacer and optionally substituted by one or more substituent selected from 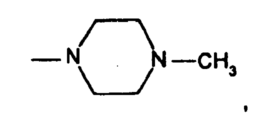 C₁₋₆ alkyl, C₁₋₆ alkoxy, C₁₋₆ haloalkyl, C₁₋₆ haloalkoxy, OH, halogen);
R⁶ represents H, C₁₋₆ alkyl, -(CH₂)ₙ phenyl or -(CH₂)ₙ napthyl (where n is 0 or 1 and each of which phenyl or naphthyl may be optionally substituted by one or more substitutents independently selected from C₁₋₆ alkyl, C₁₋₆ alkoxy, halogen, NR⁷R⁸, C₁₋₆ haloalkyl, C₁₋₆ haloalkoxy), CN or -(O)ₚ phenyl (where p is 0 or 1 and the phenyl is optionally substituted by one or more substitutents independently selected from halogen, C₁₋₆ alkyl or C₁₋₆ alkoxy)):
R⁷ and R³ independently represents C₁₋₆ alkyl, H, C₁₋₆ alkylene NR⁹R¹⁰;
R⁹ and R¹⁰ independently represents C₁₋₆ alkyl, H;
with the proviso R¹ and R² do not both represent H;
or a salt thereof.

2. A compound according to claim 1 wherein R¹ is YZ.

3. A compound according to claim 2 wherein Y is a bond or -CH = CH-.

4. A compound according to claim 3 wherein Y is a bond.

5. A compound according to claims 1- 4 wherein Z is phenyl (which may be unsubstituted or substituted once or twice by substituents independently selected from C₁₋₃ alkoxy, CN, OH, phenyl, -OCH₂ phenyl NHSO₂R³, NHCOR³, CONR⁴R⁵, SO₂NR⁴R⁵, halogen, C₁₋₃ hydroxyalkyl, C₁₋₄ alkyl) or a heteroaryl group selected from benzofuranyl, quinolinyl, 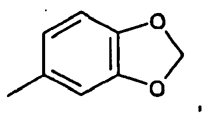 pyrimidinyl, thiophenyl, isoxazolyl, pyridinyl (each of which may be optionally substituted by one or two groups independently selected from C₁₋₃ alkyl, C₁₋₃ alkoxy, halogen.

6. A compound according to claim 5 wherein Z is phenyl (which is unsubstituted or substituted once by a substituent selected from phenyl, OCH₂ phenyl, NHSO₂CH₃, NHCOCH₃, CONH₂, CON(CH₃)₂, CI, F, OCH₃, CN, OH, CH₂OH, CH₃, C(CH₃)₃) or a heterocyclic group selected from benzofuranyl, quinolinyl, 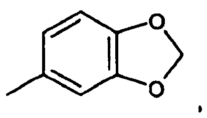 pyrimidinyl, thiophenyl, benzothiophenyl, isoxazolyl, pyridinyl (each of which is substituted or is substituted once by a group selected from -OCH₃, CH₃, F).

7. A compound according to claim 6 wherein Z is phenyl (which is unsubstituted or substituted once by a substituent selected from phenyl, OCH₂ phenyl, NHSO₂CH₃, NHCOCH₃, CONH₂, CON(CH₃)₂, CI, F, OCH₃, CN, OH, CH₂OH, CH₃, C(CH₃)₃).

8. A compound according to claim 7 wherein Z is phenyl.

9. A compound according to any of claims 1 - 8 wherein R² is H or Y¹Z¹.

10. A compound according to claim 9 wherein R² is Y¹Z¹.

11. A compound according to claim 10 wherein Y¹ is a bond or C₁₋₃ alkylene.

12. A compound according to claims 10 - 11 wherein Z¹ is phenyl (unsubstituted or substituted by one substituent selected from NHSO₂R⁶, CONR⁷R⁸, CF₃, C₁₋₃ alkoxy, SO₂R⁶, NHCOR⁷, SO₂NR⁷R⁸, NR⁷R⁸) or a 6 membered heterocyclic group which contains one nitrogen atom (which is unsubstituted or substituted one time by a group selected from C₁₋₃ alkyl, CH₂ phenyl, SO₂R⁶, CONR⁷R⁸).

13. A compound according to claim 12 wherein Z¹ is a 6 membered heterocycle substituted by SO₂R⁸.

14. A compound according to claim 13 wherein the 6 membered heterocycle is 4-piperidyl.

15. A compound according to claim 1 wherein R¹ is YZ and R² is H or Br.

16. A compound according to claim 1 wherein R¹ is phenyl or Br and R² is Y¹Z¹.

17. A compound according to claim 1 wherein R¹ is YZ and R² is Y¹Z¹.

18. A compound as claimed in claim 1, selected from the group consisting of:
5-phenyl-1 H-indole-7-carboxamide;
5-(4-biphenylyl)-1H-indole-7-carboxamide;
5-{4-[(phenylmethyl)oxy]phenyl}-1H-indole-7-carboxamide;
5-{4-[(methylsulfonyl)amino]phenyl}-1H-indole-7-carboxamide;
5-[4-(acetylamino)phenyl]-1H-indole-7-carboxamide;
5-[3-(aminocarbonyl)phenyl]-1H-indole-7-carboxamide;
5-(4-chlorophenyl)-1H-indole-7-carboxamide;
5-[3-(acetylamino)phenyl]-1H-indole-7-carboxamide;
5-[3-(aminosulfonyl)phenyl]-1H-indole-7-carboxamide;
5-{3-[(dimethylamino)carbonyl]phenyl}-1H-indole-7-carboxamide;
5-(3-fluorophenyl)-1H-indole-7-carboxamide;
5-[3-(methyloxy)phenyl]-1H-indole-7-carboxamide;
5-(3-cyanophenyl)-1H-indole-7-carboxamide;
5-(3-hydroxyphenyl)-1H-indole-7-carboxamide;
5-(3-quinolinyl)-1H-indole-7-carboxamide;
5-(1-benzofuran-4-yl)-1H-indole-7-carboxamide;
5-(1,3-benzodioxol-5-yl)-1H-indole-7-carboxamide;
5-[(E)-2-phenylethenyl]-1H-indole-7-carboxamide;
5-(5-pyrimidinyl)-1H-indole-7-carboxamide;
5-(3-biphenylyl)-1H-indole-7-carboxamide;
5-(1-benzofuran-2-yl)-1H-indole-7-carboxamide;
5-(1-benzothien-2-yl)-1*H*-indole-7-carboxamide;
5-[3-(hydroxymethyl)phenyl]-1H-indole-7-carboxamide;
5-(2-naphthalenyl)-1H-indole-7-carboxamide;
5-(4-fluorophenyl)-1H-indole-7-carboxamide;
5-[6-(methyloxy)-3-pyridinyl]-1H-indole-7-carboxamide;
5-[4-(hydroxymethyl)phenyl]-1H-indole-7-carboxamide;
5-(3-chlorophenyl)-1H-indole-7-carboxamide;
5-(2-methylphenyl)-1 H-indole-7-carboxamide;
5-{3-[(phenylmethyl)oxy]phenyl}-1H-indole-7-carboxamide;
5-(2-chlorophenyl)-1H-indole-7-carboxamide;
5-(3,5-dimethyl-4-isoxazolyl)-1H-indole-7-carboxamide;
5-{2-[(phenylmethyl)oxy]phenyl}-1H-indole-7-carboxamide;
5-(5-quinolinyl)-1 H-indole-7-carboxamide;
5-(1-naphthalenyl)-1H-indole-7-carboxamide;
3-bromo-5-phenyl-1H-indole-7-carboxamide;
3-iodo-5-phenyl-1H-indole-7-carboxamide;
3,5-diphenyl-1 H-indole-7-carboxamide;
3-{4-[(methylsulfonyl)amino]phenyl}-5-phenyl-1H-indole-7-carboxamide;
5-phenyl-3-(3-pyridinyl)-1*H*-indole-7-carboxamide;
3-(4-{[(2-aminoethyl)amino]carbonyl}phenyl)-5-phenyl-1*H*-indole-7-carboxamide;
3-[4-({[4-(methyloxy)-3-(4-methyl-1-piperazinyl)phenyl]amino}carbonyl)phenyl]-5-phenyl-1H-indole-7-carboxamide formate;
5-phenyl-3-[3-(trifluoromethyl)phenyl]-1H-indole-7-carboxamide;
5-bromo-3-iodo-1H-indole-7-carboxamide;
3-(1-ethyl-3-piperidinyl)-5-phenyl-1H-indole-7-carboxamide;
5-phenyl-3-(3-piperidinyl)-1H-indole-7-carboxamide;
5-phenyl-3-[1-(phenylmethyl)-3-piperidinyl]-1H-indole-7-carboxamide;
3-(1-cyclohexen-1-yl)-5-phenyl-1H-indole-7-carboxamide;
3-cyclohexyl-5-phenyl-1H-indole-7-carboxamide;
3-{1-[3-(methyloxy)phenyl]ethenyl}-5-phenyl-1H-indole-7-carboxamide;
5-phenyl-3-[1-(phenylmethyl)-1,2,3,6-tetrahydro-4-pyridinyl]-1H-indole-7-carbox-amide;
5-phenyl-3-(4-piperidinyl)-1H-indole-7-carboxamide;
3-{1-[(4-chlorophenyl)sulfonyl]-4-piperidinyl}-5-phenyl-1H-indole-7-carboxamide;
5-phenyl-3-[1-(propylsulfonyl)-4-piperidinyl]-1H-indole-7-carboxamide;
3-(1-acetyl-4-piperidinyl)-5-phenyl-1H-indole-7-carboxamide;
3-[1-(N,N-dimethyl-β-alanyl)-4-piperidinyl]-5-phenyl-1H-indole-7-carboxamide;
3-(1-ethyl-4-piperidinyl)-5-phenyl-1H-indole-7-carboxamide formate;
3-(1-methylpyrrolidin-2-yl)-5-phenyl-1*H*-indole-7-carboxamide;
3-[1-(ethylsulfonyl)pyrrolidin-3-yl]-5-phenyl-1*H*-indole-7-carboxamide;
3-[4-(methylsulfonyl)phenyl]-5-phenyl-1*H*-indole-7-carboxamide;
3-[3-(acetylamino)phenyl]-5-phenyl-1*H*-indole-7-carboxamide;
3-[4-(ethylsulfonyl)phenyl]-5-phenyl-1*H*-indole-7-carboxamide;
3-[3-(methylsulfonyl)phenyl]-5-phenyl-1*H*-indole-7-carboxamide;
3-(hexahydro-1*H*-azepin-4-yl)-5-phenyl-1*H*-indole-7-carboxamide;
3-[1-(ethylsulfonyl)hexahydro-1*H*-azepin-4-yl]-5-phenyl-1*H*-indole-7-carbox-amide;
5-phenyl-3-[2-(4-pyridinyl)ethyl]-1*H*-indole-7-carboxamide;
3-{[1-(ethylsulfonyl)-4-piperidinylidene]methyl}-5-phenyl-1*H*-indole-7-carboxamide;
3-[1-(ethylsulfonyl)-4-piperidinyl]-5-[4-(hydroxymethyl)phenyl]-1*H*-indole-7-carboxamide;
5-phenyl-3-(3-piperidinylmethyl)-1*H*-indole-7-carboxamide;
5-phenyl-3-[2-(4-piperidinyl)ethyl]-1*H*-indole-7-carboxamide;
3-{2-[1-(ethylsulfonyl)-4-piperidinyl]ethyl}-5-phenyl-1*H*-indole-7-carboxamide;
3-{[1-(ethylsulfonyl)-3-piperidinyl]methyl}-5-phenyl-1*H*-indole-7-carboxamide;
3-{[1-(ethylsulfonyl)-4-piperidinyl]methyl}-5-phenyl-1*H*-indole-7-carboxamide;
3-{1-[(2)sulfonyl]-4-piperidinyl}-5-phenyl-1*H*-indole-7-carboxamide;
3-{1-[(4-fluorophenyl)sulfonyl]-4-piperidinyl}-5-phenyl-1*H*-indole-7-carboxamide;
3-{1-[(4-methylphenyl)sulfonyl]-4-piperidinyl}-5-phenyl-1*H*-indole-7-carboxamide;
3-{phenylsulfonyl-4-piperidinyl}-5-phenyl-1*H*-indole-7-carboxamide;
3-(1-{[4-(methyloxy)phenyl]sulfonyl}-4-piperidinyl)-5-phenyl-1*H*-indole-7-carboxamide;
3-[1-(ethanesulfonyl)-4-piperidinyl]-5-phenyl-1*H*-indole-7-carboxamide;
3-{1-[(2-propanesulfonyl]-4-piperidinyl}-5-phenyl-1*H*-indole-7-carboxamide;
5-phenyl-3-[1-(propanesulfonyl)-1,2,3,6-tetrahydro-4-pyridinyl]-1*H*-indole-7-carboxamide;
5-phenyl-3-(1-{[4-(trifluoromethyl)phenyl]sulfonyl}-4-piperidinyl)-1*H*-indole-7-carboxamide;
3-{1-[(2,4-dichlorophenyl)sulfonyl]-4-piperidinyl}-5-phenyl-1*H*-indole-7-carboxamide;
3-{1-[(3,4-dichlorophenyl)sulfonyl]-4-piperidinyl}-5-phenyl-1*H*-indole-7-carboxamide;
3-{1-[(ethylamino)carbonyl]-4-piperidinyl}-5-phenyl-1*H*-indole-7-carboxamide;
3-{1-[(4-1-piperazinyl)carbonyl]-4-piperidinyl}-5-phenyl-1*H*-indole-7-carboxamide;
5-(4-chlorophenyl)-3-[1-(propanesulfonyl)-4-piperidinyl]-1*H*-indole-7-carboxamide;
3-{1-[(4-fluorophenyl)sulfonyl]-4-piperidinyl}-5-(4-chlorophenyl)-1*H*-indole-7-carboxamide;
5-{4-[(methylsulfonyl)amino]phenyl}-3-[1-(phenylmethyl)-1,2,3,6-tetrahydro-4-pyridinyl]-1*H*-indole-7-carboxamide;
5-{4-[(methylsulfonyl)amino]phenyl}-3-(4-piperidinyl)-1*H*-indole-7-carboxamide;
5-bromo-3-[1-(ethanesulfonyl)-4-piperidinyl]-1*H*-indole-7-carboxamide;
3-[1-(ethanesulfonyl)-4-piperidinyl]-5-{4-[(methylsulfonyl)amino]phenyl}-1*H*-indole-7-carboxamide;
3-[1-(ethanesulfonyl)4-piperidinyl]-5-(3-methylphenyl)-1*H*-indole-7-carboxamide;
3-[1-(ethylsulfonyl)-4-piperidinyl]-5-(2-thienyl)-1*H*-indole-7-carboxamide;
3-[1-(ethylsulfonyl)-4-piperidinyl]-5-(3-thienyl)-1*H*-indole-7-carboxamide;
3-[4-(methylsulfonyl)phenyl]-5-phenyl-1H-indole-7-carboxamide;
3-{4-[(dimethylamino)sulfonyl]phenyl}-5-phenyl-1H-indole-7-carboxamide;
3-{3-[(methylsulfonyl)amino]phenyl}-5-phenyl-1H-indole-7-carboxamide;
3-[1-(ethylsulfonyl)-4-piperidinyl]-5-{3-[(methylsulfonyl)amino]phenyl}-1*H*-indole -7-carboxamide;
5-[4-(acetylamino)phenyl]-3-[1-(ethylsulfonyl)-4-piperidinyl]-1*H*-indole-7-carboxamide;
5-{4-[(dimethylamino)sulfonyl]phenyl}-3-[1-(ethylsulfonyl)-4-piperidinyl]-1*H*-indole-7-carboxamide;
5-[3-(acetylamino)phenyl]-3-[1-(ethylsulfonyl)-4-piperidinyl]-1*H*-indole-7-carbox-amide;
3-[1-(ethylsulfonyl)-4-piperidinyl]-5-(1*H*-pyrazol-4-yl)-1*H*-indole-7-carboxamide;
3-[1-(ethylsulfonyl)-4-piperidinyl]-5-[3-(hydroxymethyl)phenyl]-1*H*-indole-7-carboxamide;
5-(2,4-difluorophenyl)-3-[1-(ethylsulfonyl)-4-piperidinyl]-1*H*-indole-7-carboxamide;
3-[1-(ethylsulfonyl)-4-piperidinyl]-5-[4-(methyloxy)phenyl]-1*H*-indole-7-carboxamide;
3-[1-(ethylsulfonyl)-4-piperidinyl]-5-(4-fluoro-2-methylphenyl)-1*H*-indole-7-carboxamide;
3-[1-(ethylsulfonyl)-4-piperidinyl]-5-(4-fluorophenyl)-1*H*-indole-7-carboxamide;
5-(4-biphenylyl)-3-[1-(ethylsulfonyl)-4-piperidinyl]-1*H*-indole-7-carboxamide;
5-[4-(1,1-dimethylethyl)phenyl]-3-[1-(ethylsulfonyl)-4-piperidinyl]-1*H*-indole-7-carboxamide;
3-[1-(ethylsulfonyl)-4-piperidinyl]-5-(4-methylphenyl)-1*H*-indole-7-carboxamide;
3-[1-(ethylsulfonyl)-4-piperidinyl]-5-(4-pyridinyl)-1*H*-indole-7-carboxamide;
3-[1-(ethylsulfonyl)-4-piperidinyl]-5-(6-fluoro-3-pyridinyl)-1*H*-indole-7-carboxamide;
3-[1-(ethylsulfonyl)-4-piperidinyl]-5-(6-methyl-3-pyridinyl)-1*H*-indole-7-carboxamide;
3-[1-(ethylsulfonyl)-4-piperidinyl]-5-(4-methyl-3-pyridinyl)-1*H*-indole-7-carboxamide;
3-[1-(ethylsulfonyl)-4-piperidinyl]-5-[6-(methyloxy)-3-pyridinyl]-1*H*-indole-7-carboxamide;
5-phenyl-3-(N-acetyl-3-piperidinylmethyl)-1*H*-indole-7-carboxamide;
5-[3-(ethyloxy)phenyl]-3-[1-(ethylsulfonyl)-4-piperidinyl]-1*H*-indole-7-carboxamide;
3-[1-(ethylsulfonyl)-4-piperidinyl]-5-(2-fluorophenyl)-1*H*-indole-7-carboxamide;
3-[1-(ethylsulfonyl)-4-piperidinyl]-5-[3-(trifluoromethyl)phenyl]-1*H*-indole-7-carboxamide;
3-[1-(ethylsulfonyl)-4-piperidinyl]-5-[4-(trifluoromethyl)phenyl]-1*H*-indole-7-carboxamide;
3-[1-(ethylsulfonyl)-4-piperidinyl]-5-(3-fluorophenyl)-1*H*-indole-7-carboxamide;
5-(3,5-dichlorophenyl)-3-[1-(ethylsulfonyl)-4-piperidinyl]-1*H*-indole-7-carboxamide;
5-(3,4-difluorophenyl)-3-[1-(ethylsulfonyl)-4-piperidinyl]-1*H*-indole-7-carboxamide;
3-bromo-5-{3-[(dimethylamino)carbonyl]phenyl}-1*H*-indole-7-carboxamide;
5-[2,6-bis(methyloxy)phenyl]-3-bromo-1*H*-indole-7-carboxamide;
3-bromo-5-(4-fluoro-2-methylphenyl)-1*H*-indole-7-carboxamide;
3-bromo-5-[5-fluoro-2-(methyloxy)phenyl]-1*H*-indole-7-carboxamide;
3-bromo-5-(3-quinolinyl)-1*H*-indole-7-carboxamide trifluoroacetate;
3-bromo-5-(5-quinolinyl)-1*H*-indole-7-carboxamide trifluoroacetate;
5-[2,5-bis(methyloxy)phenyl]-3-bromo-1*H*-indole-7-carboxamide;
3-bromo-5-(2-fluorophenyl)-1*H*-indole-7-carboxamide;
5-[2,4-bis(methyloxy)phenyl]-3-bromo-1*H*-indole-7-carboxamide;
3-bromo-5-[2-(methyloxy)-3-pyridinyl]-1*H*-indole-7-carboxamide trifluoroacetate;
3-bromo-5-[2,3,4-tris(methyloxy)phenyl]-1*H*-indole-7-carboxamide;
3-{1-[(4-chloro-2,5-dimethylphenyl)sulfonyl]-4-piperidinyl}-5-phenyl-1*H*-indole-7-carboxamide;
3-(1-{(5-bromo-2-(methyloxy)phenyl]sulfonyl}-4-piperidinyl)-5-phenyl-1*H*-indole-7-carboxamide;
3-{1-[(5-fluoro-2-methylphenyl)sulfonyl]-4-piperidinyl}-5-phenyl-1*H*-indole-7-carboxamide;
3-{1-[(3-fluorophenyl)sulfonyl]-4-piperidinyl}-5-phenyl-1*H*-indole-7-carboxamide;
5-phenyl-3-(1-{[2,4,6-tris(1-methylethyl)phenyl]sulfonyl}-4-piperidinyl)-1*H*-indole-7-carboxamide;
3-(1-{[4-(1,1-dimethylpropyl)phenyl]sulfonyl}-4-piperidinyl)-5-phenyl-1*H*-indole-7-carboxamide;
3-{1-[(2-methylphenyl)sulfonyl]-4-piperidinyl}-5-phenyl-1*H*-indole-7-carboxamide;
3-{1-[(2-iodophenyl)sulfonyl]-4-piperidinyl}-5-phenyl-1*H*-indole-7-carboxamide;
3-{1-[(4-pentylphenyl)sulfonyl]-4-piperidinyl}-5-phenyl-1*H*-indole-7-carboxamide;
5-phenyl-3-{1-[(4-propylphenyl)sulfonyl]-4-piperidinyl}-1*H*-indole-7-carboxamide;
3-{1-[(2,4-difluorophenyl)sulfonyl]-4-piperidinyl}-5-phenyl-1*H*-indole-7-carboxamide;
3-{1-[(2,5-dimethylphenyl)sulfonyl]-4-piperidinyl}-5-phenyl-1*H*-indole-7-carboxamide;
3-{1-[(4-ethylphenyl)sulfonyl]-4-piperidinyl}-5-phenyl-1*H*-indole-7-carboxamide;
3-{1-[(3-methylphenyl)sulfonyl]-4-piperidinyl}-5-phenyl-1*H*-indole-7-carboxamide;
3-{1-[(4-{[2-(methyloxy)phenyl]oxy}phenyl)sulfonyl]-4-piperidinyl}-5-phenyl-1*H*-indole-7-carboxamide;
3-{1-[(4-{[4-(methyloxy)phenyl]oxy}phenyl)sulfonyl]-4-piperidinyl}-5-phenyl-1*H*-indole-7-carboxamide;
3-[1-({3-[(4-fluorophenyl)oxy]phenyl}sulfonyl)-4-piperidinyl]-5-phenyl-1*H*-indole-7-carboxamide;
3-{1-[(3-{[2-(methyloxy)phenyl]oxy}phenyl)sulfonyl]-4-piperidinyl}-5-phenyl-1*H*-indole-7-carboxamide; .
3-[1-({4-[(4-chlorophenyl)oxy]phenyl}sulfonyl)-4-piperidinyl]-5-phenyl-1*H*-indole-7-carboxamide;
3-{1-[(3-{[4-(methyloxy)phenyl]oxy}phenyl)sulfonyl]-4-piperidinyl}-5-phenyl-1*H*-indole-7-carboxamide;
5-phenyl-3-(1-{[3-(phenyloxy)phenyl]sulfonyl}-4-piperidinyl)-1*H*-indole-7-carboxamide;
3-[1-({3-[(4-chlorophenyl)oxy]phenyl}sulfonyl)-4-piperidinyl]-5-phenyl-1*H*-indole-7-carboxamide;
3-[1-({4-[(2-methylphenyl)oxy]phenyl}sulfonyl)-4-piperidinyl]-5-phenyl-1*H*-indole-7-carboxamide;
3-{1-[(4'-chloro-4-biphenylyl)sulfonyl]-4-piperidinyl}-5-phenyl-1*H*-indole-7-carboxamide;
3-[1-({3-[(2-methylphenyl)oxy]phenyl}sulfonyl)-4-piperidinyl]-5-phenyl-1*H*-indole-7-carboxamide;
3-[1-({3-[(2-chlorophenyl)oxy]phenyl}sulfonyl)-4-piperidinyl]-5-phenyl-1*H*-indole-7-carboxamide;
3-{1-[(5-chloro-1-naphthalenyl)sulfonyl]-4-piperidinyl}-5-phenyl-1*H*-indole-7-carboxamide;
3-(1-{[4'-(methyloxy)-3-biphenylyl]sulfonyl}-4-piperidinyl)-5-phenyl-1*H*-indole-7-carboxamide;
3-[1-(3-biphenylylsulfonyl)-4-piperidinyl]-5-phenyl-1*H*-indole-7-carboxamide;
3-(1-{[(4-fluorophenyl)methyl]sulfonyl}-4-piperidinyl)-5-phenyl-1*H*-indole-7-carboxamide;
3-{1-[(5-chloro-2-naphthalenyl)sulfonyl]-4-piperidinyl}-5-phenyl-1*H*-indole-7-carboxamide;
3-(1-{[4'-(methyloxy)-4-biphenylyl]sulfonyl}-4-piperidinyl)-5-phenyl-1*H*-indole-7-carboxamide;
5-(2-fluorophenyl)-1*H*-indole-7-carboxamide;
5-(3-{[(2,2-dimethylpropyl)amino]carbonyl}phenyl)-1*H*-indole-7-carboxamide;
5-(3-{[(1-methylethyl)amino]carbonyl}phenyl)-1*H*-indole-7-carboxamide;
5-(4-{[(2,2-dimethylpropyl)amino]carbonyl}phenyl)-1*H*-indole-7-carboxamide;
5-{4-[(propylamino)carbonyl]phenyl}-1*H*-indole-7-carboxamide;
5-(4-{[(1-methylethyl)amino]carbonyl}phenyl)-1*H*-indole-7-carboxamide;
5-{4-[(diethylamino)carbonyl]phenyl}-1*H*-indole-7-carboxamide;
3-[1-(methylsulfonyl)-1,2,3,6-tetrahydro-4-pyridinyl]-5-phenyl-1*H*-indole-7-carboxamide;
3-(3-oxocyclopentyl)-5-phenyl-1*H*-indole-7-carboxamide;
5-phenyl-3-{3-[(phenylmethyl)amino]cyclopentyl}-1*H*-indole-7-carboxamide;
3-(3-aminocyclopentyl)-5-phenyl-1*H*-indole-7-carboxamide;
3-{3-[(ethylsulfonyl)amino]cyclopentyl}-5-phenyl-1*H*-indole-7-carboxamide;
5-bromo-3-[1-(propylsulfonyl)-4-piperidinyl]-1*H*-indole-7-carboxamide;
5-bromo-3-(3-pyridinyl)-1*H*-indole-7-carboxamide;
5-bromo-3-[1-(methylsulfonyl)-1,2,3,6-tetrahydro-4-pyridinyl]-1*H*-indole-7-carboxamide;
3-[(4-hydroxyphenyl)methyl]-5-phenyl-1H-indole-7-carboxamide;
5-bromo-1H-indole-7-carboxamide;
5-(4-chlorophenyl)1H-indole-7-carboxamide;
5-bromo-3(4-piperidinyl)1H-indole-7-carboxamide;
or a salt thereof.

19. A compound as claimed in claim 1 selected from the group consisting of:
3-{1-[(4-chlorophenyl)sulfonyl]-4-piperidinyl}-5-phenyl-1H-indole-7-carboxamide;
3-{1-[(2)sulfonyl]-4-piperidinyl}-5-phenyl-1*H*-indole-7-carboxamide;
3-{1-[(4-fluorophenyl)sulfonyl]-4-piperidinyl}-5-phenyl-1*H*-indole-7-carboxamide;
3-{1-[(4-methylphenyl)sulfonyl]-4-piperidinyl}-5-phenyl-1*H*-indole-7-carboxamide;
3-{phenylsulfonyl-4-piperidinyl}-5-phenyl-1*H*-indole-7-carboxamide;
3-(1-{[4-(methyloxy)phenyl]sulfonyl}-4-piperidinyl)-5-phenyl-1*H*-indole-7-carboxamide;
5-phenyl-3-(1-{[4-(trifluoromethyl)phenyl]sulfonyl}-4-piperidinyl)-1*H*-indole-7-carboxamide;
3-{1-[(2,4-dichlorophenyl)sulfonyl]-4-piperidinyl}-5-phenyl-1*H*-indole-7-carboxamide;
3-{1-[(3,4-dichlorophenyl)sulfonyl]-4-piperidinyl}-5-phenyl-1*H*-indole-7-carboxamide;
3-{1-[(4-fluorophenyl)sulfonyl]-4-piperidinyl}-5-(4-chlorophenyl)-1*H*-indole-7-carboxamide;
3-{1-[(4-chloro-2,5-dimethylphenyl)sulfonyl]-4-piperidinyl}-5-phenyl-1*H*-indole-7-carboxamide;
3-(1-{[5-bromo-2-(methyloxy)phenyl]sulfonyl}-4-piperidinyl)-5-phenyl-1*H*-indole-7-carboxamide;
3-{1-[(5-fluoro-2-methylphenyl)sulfonyl]-4-piperidinyl}-5-phenyl-1*H*-indole-7-carboxamide;
3-{1-[(3-fluorophenyl)sulfonyl]-4-piperidinyl}-5-phenyl-1*H*-indole-7-carboxamide;
5-phenyl-3-(1-{[2,4,6-tris(1-methylethyl)phenyl]sulfonyl}-4-piperidinyl)-1*H*-indole-7-carboxamide;
3-(1-{[4-(1,1-dimethylpropyl)phenyl]sulfonyl}-4-piperidinyl)-5-phenyl-1*H*-indole-7-carboxamide;
3-{1-[(2-methylphenyl)sulfonyl]-4-piperidinyl}-5-phenyl-1*H*-indole-7-carboxamide;
3-{1-[(2-iodophenyl)sulfonyl]-4-piperidinyl}-5-phenyl-1*H*-indole-7-carboxamide;
3-{1-[(4-pentylphenyl)sulfonyl]-4-piperidinyl}-5-phenyl-1*H*-indole-7-carboxamide;
5-phenyl-3-{1-[(4-propylphenyl)sulfonyl]-4-piperidinyl}-1*H*-indole-7-carboxamide;
3-{1-[(2,4-difluorophenyl)sulfonyl]-4-piperidinyl}-5-phenyl-1*H*-indole-7-carboxamide;
3-{1-[(2,5-dimethylphenyl)sulfonyl]-4-piperidinyl}-5-phenyl-1*H*-indole-7-carboxamide;
3-{1-[(4-ethylphenyl)sulfonyl]-4-piperidinyl}-5-phenyl-1*H*-indole-7-carboxamide;
3-{1-[(3-methylphenyl)sulfonyl]-4-piperidinyl}-5-phenyl-1*H*-indole-7-carboxamide;
3-{1-[(4-{[2-(methyloxy)phenyl]oxy}phenyl)sulfonyl]-4-piperidinyl}-5-phenyl-1*H*-indole-7-carboxamide;
3-{1-[(4-{[4-(methyloxy)phenyl]oxy}phenyl)sulfonyl]-4-piperidinyl}-5-phenyl-1*H*-indole-7-carboxamide;
3-[1-({3-[(4-fluorophenyl)oxy]phenyl}sulfonyl)-4-piperidinyl]-5-phenyl-1*H*-indole-7-carboxamide;
3-{1-[(3-{[2-(methyloxy)phenyl]oxy}phenyl)sulfonyl]-4-piperidinyl}-5-phenyl-1*H*-indole-7-carboxamide;
3-[1-({4-[(4-chlorophenyl)oxy]phenyl}sulfonyl)-4-piperidinyl]-5-phenyl-1*H*-indole-7-carboxamide;
3-{1-[(3-{[4-(methyloxy)phenyl]oxy}phenyl)sulfonyl]-4-piperidinyl}-5-phenyl-1*H*-indole-7-carboxamide;
5-phenyl-3-(1-{[3-(phenyloxy)phenyl]sulfonyl}-4-piperidinyl)-1*H*-indole-7-carboxamide;
3-[1-({3-[(4-chlorophenyl)oxy]phenyl}sulfonyl)-4-piperidinyl]-5-phenyl-1*H*-indole-7-carboxamide;
3-[1-({4-[(2-methylphenyl)oxy]phenyl}sulfonyl)-4-piperidinyl]-5-phenyl-1*H*-indole-7-carboxamide;
3-{1-[(4'-chloro-4-biphenylyl)sulfonyl]-4-piperidinyl}-5-phenyl-1*H*-indole-7-carboxamide;
3-[1-({3-[(2-methylphenyl)oxy]phenyl}sulfonyl)-4-piperidinyl]-5-phenyl-1*H*-indole-7-carboxamide;
3-[1-({3-[(2-chlorophenyl)oxy]phenyl}sulfonyl)-4-piperidinyl]-5-phenyl-1*H*-indole-7-carboxamide;
3-{1-[(5-chloro-1-naphthalenyl)sulfonyl]-4-piperidinyl}-5-phenyl-1*H*-indole-7-carboxamide;
3-(1-{[4'-(methyloxy)-3-biphenylyl]sulfonyl}-4-piperidinyl)-5-phenyl-1*H*-indole-7-carboxamide;
3-[1-(3-biphenylylsulfonyl)-4-piperidinyl]-5-phenyl-1*H*-indole-7-carboxamide;
3-(1-{[(4-fluorophenyl)methyl]sulfonyl}-4-piperidinyl)-5-phenyl-1*H*-indole-7-carboxamide;
3-(1-[(5-chloro-2-naphthalenyl)sulfonyl]-4-piperidinyl)-5-phenyl-1*H*-indole-7-carboxamide; and
3-(1-{[4'-(methyloxy)-4-biphenylyl]sulfonyl}-4-piperidinyl)-5-phenyl-1*H*-indole-7-carboxamide;
or a salt thereof.

20. A compound as claimed in claim 1 selected from the group consisting of:
5-phenyl-3-[1-(propylsulfonyl)-4-piperidinyl]-1H-indole-7-carboxamide;
3-[1-(ethylsulfonyl)-4-piperidinyl]-5-[4-(hydroxymethyl)phenyl]-1*H*-indole-7-carboxamide;
3-[1-(ethanesulfonyl)-4-piperidinyl]-5-phenyl-1*H*-indole-7-carboxamide;
3-{1-[(2-propanesulfonyl]-4-piperidinyl}-5-phenyl-1*H*-indole-7-carboxamide;
5-phenyl-3-[1-(propanesulfonyl)-1,2,3,6-tetrahydro-4-pyridinyl]-1*H*-indole-7-carboxamide;
5-(4-chlorophenyl)-3-[1-(propanesulfonyl)-4-piperidinyl]-1*H*-indole-7-carboxamide;
3-[1-(ethanesulfonyl)-4-piperidinyl]-5-{4-[(methylsulfonyl)amino]phenyl}-1*H*-indole-7-carboxamide;
3-[1-(ethanesulfonyl)-4-piperidinyl]-5-(3-methylphenyl)-1*H*-indole-7-carboxamide;
3-[1-(ethylsulfonyl)-4-piperidinyl]-5-{3-[(methylsulfonyl)amino]phenyl}-1*H*-indole -7-carboxamide;
5-[4-(acetylamino)phenyl]-3-[1-(ethylsulfonyl)-4-piperidinyl]-1*H*-indole-7-carboxamide;
5-{4-[(dimethylamino)sulfonyl]phenyl}-3-[1-(ethylsulfonyl)-4-piperidinyl]-1*H*-indole-7-carboxamide;
5-[3-(acetylamino)phenyl]-3-[1-(ethylsulfonyl)-4-piperidinyl]-1*H*-indole-7-carboxamide;
3-[1-(ethylsulfonyl)-4-piperidinyl]-5-[3-(hydroxymethyl)phenyl]-1*H*-indole-7-carboxamide;
5-(2,4-difluorophenyl)-3-[1-(ethylsulfonyl)-4-piperidinyl]-1*H*-indole-7-carboxamide;
3-[1-(ethylsulfonyl)-4-piperidinyl]-5-[4-(methyloxy)phenyl]-1*H*-indole-7-carboxamide;
3-[1-(ethylsulfonyl)-4-piperidinyl-5-(4-fluoro-2-methylphenyl)-1*H*-indole-7-carboxamide;
3-[1-(ethylsulfonyl)-4-piperidinyl]-5-(4-fluorophenyl)-1*H*-indole-7-carboxamide;
5-(4-biphenylyl)-3-[1-(ethylsulfonyl)-4-piperidinyl]-1*H*-indole-7-carboxamide;
5-[4-(1,1-dimethylethyl)phenyl]-3-[1-(ethylsulfonyl)-4-piperidinyl]-1*H*-indole-7-carboxamide;
3-[1-(ethylsulfonyl)-4-piperidinyl]-5-(4-methylphenyl)-1*H*-indole-7-carboxamide;
5-[3-(ethyloxy)phenyl]-3-[1-(ethylsulfonyl)-4-piperidinyl]-1*H*-indole-7-carboxamide;
3-[1-(ethylsulfonyl)-4-piperidinyl]-5-(2-fluorophenyl)-1*H-*indole-7-carboxamide;
3-[1-(ethylsulfonyl)-4-piperidinyl]-5-[3-(trifluoromethyl)phenyl]-1*H*-indole-7-carboxamide;
3-[1-(ethylsulfonyl)-4-piperidinyl]-5-[4-(trifluoromethyl)phenyl]-1*H*-indole-7-carboxamide;
3-[1-(ethylsulfonyl)-4-piperidinyl]-5-(3-fluorophenyl)-1*H*-indole-7-carboxamide;
5-(3,5-dichlorophenyl)-3-[1-(ethylsulfonyl)-4-piperidinyl]-1*H*-indole-7-carboxamide; and
5-(3,4-difluorophenyl)-3-[1-(ethylsulfonyl)-4-piperidinyl]-1*H*-indole-7-carboxamide;
or a salt thereof.

21. A compound as claimed in claim 1 selected from the group consisting of:
3-[1-(ethylsulfonyl)-4-piperidinyl]-5-(2-thienyl)-1*H*-indole-7-carboxamide;
3-[1-(ethylsulfonyl)-4-piperidinyl]-5-(3-thienyl)-1*H-*indole-7-carboxamide;
3-[1-(ethylsulfonyl)-4-piperidinyl]-5-(1*H*-pyrazol-4-yl)-1*H*-indole-7-carboxamide;
3-[1-(ethylsulfonyl)-4-piperidinyl]-5-(4-pyridinyl)-1*H*-indole-7-carboxamide;
3-[1-(ethylsulfonyl)-4-piperidinyl]-5-(6-fluoro-3-pyridinyl)-1*H*-indole-7-carboxamide;
3-[1-(ethylsulfonyl)-4-piperidinyl]-5-(6-methyl-3-pyridinyl)-1*H*-indole-7-carboxamide;
3-[1-(ethylsulfonyl)-4-piperidinyl]-5-(4-methyl-3-pyridinyl)-1*H*-indole-7-carboxamide; and
3-[1-(ethylsulfonyl)-4-piperidinyl]-5-[6-(methyloxy)-3-pyridinyl]-1*H*-indole-7-carboxamide;
or a salt thereof.

22. A compound as claimed in claim 1 which is 5-bromo-3-[1-(ethanesulfonyl)-4-piperidinyl]-1*H*-indole-7-carboxamide or a salt thereof.

23. A compound as claimed in claim 1 selected from the group consisting of:
3-[1-(methylsulfonyl)-1,2,3,6-tetrahydro-4-pyridinyl]-5-phenyl-1*H*-indole-7-carboxamide; and
5-bromo-3-[1-(methylsulfonyl)-1,2,3,6-tetrahydro-4-pyridinyl]-1*H*-indole-7-carboxamide;
or a salt thereof.

24. A pharmaceutical composition, comprising a compound as claimed in any one of claims 1 - 23, or a salt thereof and one or more of pharmaceutically acceptable carriers, diluents and excipients.

25. A compound as claimed in any of claims 1 - 23, or a salt thereof for use in therapy.

26. A compound according to claims 1 - 23 for use in the treatment of a disorder mediated by inappropriate kinase activity.

27. A compound according to claims 1-23 for use in the treatment of a disorder mediated by inappropriate IKK2 activity.

28. Use of a compound as claimed in any of claims 1 - 23, or a salt thereof in the preparation of a medicament for use in the treatment of a disorder mediated by inappropriate kinase activity.

29. Use according to claim 28 wherein the inappropriate kinase activity is inappropriate IKK2 activity.

30. Use according to claim 29 wherein disorder mediated by inappropriate IKK2 activity is inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, ankylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

31. Use according to claim 30 wherein the IKK2 disorder is an inflammatory or tissue repair disorder.

32. Use according to claim 31 wherein the IKK2 disorder is rheumatoid arthritis, inflammatory bowel disease, asthma or COPD.

33. Use according to claim 32 wherein the IKK2 disorder is asthma.

34. Use according to claim 32 wherein the IKK2 disorder is COPD.

35. Use according to claim 32 wherein the IKK2 disorder is rheumatoid arthritis.

36. Use according to claim 30 wherein the IKK2 disorder is selected from the group consisting of autoimmune diseases; tissue or organ rejection, Alzheimer's disease, stroke atherosclerosis, restenosis, diabetes, glomerulonephritis, osteoarthritis, osteoporosis, and Ataxia Telangiestasia.

37. Use according to claim 36 wherein said disease is an autoimmune disease.

38. Use according to claim 37 wherein the autoimmune disease is systemic lupus eythematosus, multiple sclerosis, psoriatic arthritis, or ankylosing spondylitis, diabetes.

39. Use according to claim 30 wherein the disease is cancer and or cachexia.

40. Use according to claim 39 wherein the cancer is Hodgkins disease.

## Patentansprüche

1. Verbindung der Formel (I): 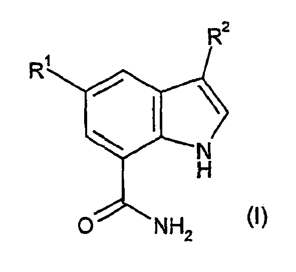
worin R¹ H, Halogen oder eine Gruppe -YZ darstellt;
Y eine Bindung (d.h. fehlt), C₁₋₆-Alkylen oder C₂₋₆-Alkenylen darstellt;
Z eine Aryl- oder Heteroaryl-Gruppe darstellt, jeweils 5 bis 14 Ringglieder umfassend, wobei das Aryl oder Heteroaryl gegebenenfalls mit einem oder mehreren Substituenten substituiert ist, der/die unabhängig ausgewählt ist/sind aus Halogen, OH, C₁₋₆-Alkyl, C₁₋₆-Halogenalkyl, C₁₋₆-Alkoxy, C₁₋₆-Halogenalkoxy, CN, C₁₋₆-Hydroxyalkyl, Phenyl, O-(CH₂)₁₋₆-Phenyl, NHSO₂R³, NHCOR³, CONR⁴R⁵, SO₂NR⁴R⁵;
R³, R⁴ und R⁵ unabhängig H oder C₁₋₆-Alkyl darstellen;
R² H, Halogen oder eine Gruppe -Y¹Z¹ darstellt;
Y¹ eine Bindung (d.h. fehlt), C₁₋₆-Alkylen, C₂₋₆-Alkenylen darstellt;
Z¹ ein 6-gliedriges Aryl, 5- oder 6-gliedriges Heteroaryl, 5- bis 7-gliedriges Heterocyclyl, C₅₋₇-Cycloalkyl, C₅₋₇-Cycloalkenyl darstellt, wobei jedes gegebenenfalls mit einem oder mehreren Substituenten substituiert sein kann, der/die unabhängig ausgewählt ist/sind aus SO₂R⁶, NHSO₂R⁶, 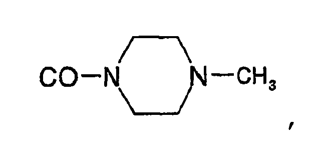 COR⁷, NR⁷R⁸, SO₂NR⁷R⁸, C₁₋₆-Alkyl, C₁₋₆-Halogenalkyl, C₁₋₆-Alkoxy, C₁₋₆-Halogenalkoxy, Halogen, CONR⁷R⁸, NHCOR⁷ oder Phenyl (direkt gebunden oder gebunden durch einen C₁₋₆-Alkylen-, CONH-, C₂₋₆-Alkenylen-Spacer und gegebenenfalls mit einem oder mehreren Substituenten substituiert ist, der/die ausgewählt ist/sind aus 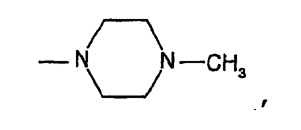 C₁₋₆-Alkyl, C₁₋₆-Alkoxy, C₁₋₆-Halogenalkyl, C₁₋₆-Halogenalkoxy, OH, Halogen);
R⁶ H, C₁₋₆-Alkyl, -(CH₂)ₙ-Phenyl oder -(CH₂)ₙ-Naphthyl (wobei n 0 oder 1 ist und jedes Phenyl oder Naphthyl gegebenenfalls mit einem oder mehreren Substituenten substituiert sein kann, der/die unabhängig ausgewählt ist/sind aus C₁₋₆-Alkyl, C₁₋₆-Alkoxy, Halogen, NR⁷R⁸, C₁₋₆-Halogenalkyl, C₁₋₆-Halogenalkoxy), CN oder -(O)p-Phenyl (wobei p 0 oder
1 ist und das Phenyl gegebenenfalls mit einem oder mehreren Substituenten substituiert ist, der/die unabhängig ausgewählt ist/sind aus Halogen, C₁₋₆-Alkyl oder C₁₋₆-Alkoxy));
R⁷ und R⁸ unabhängig C₁₋₆-Alkyl, H, C₁₋₆-Alkylen, NR⁹R¹⁰ darstellen;
R⁹ und R¹⁰ unabhängig C₁₋₆-Alkyl, H darstellen;
mit der Maßgabe, daß R¹ und R² nicht beide H darstellen;
oder ein Salz davon.

2. Verbindung gemäß Anspruch 1, worin R¹ YZ ist.

3. Verbindung gemäß Anspruch 2, worin Y eine Bindung oder -CH=CH- ist.

4. Verbindung gemäß Anspruch 3, worin Y eine Bindung ist.

5. Verbindung gemäß Ansprüchen 1 bis 4, worin Z Phenyl (das unsubstituiert oder ein- oder zweimal mit Substituenten substituiert sein kann, der/die unabhängig ausgewählt ist/sind aus C₁₋₃-Alkoxy, CN, OH, Phenyl, -OCH₂-Phenyl, NHSO₂R³, NHCOR³, CONR⁴R⁵, SO₂NR⁴R⁵, Halogen, C₁₋₃-Hydroxyalkyl, C₁₋₄-Alkyl) oder eine Heteroaryl-Gruppe ist, die ausgewählt ist aus Benzofuranyl, Chinolinyl, 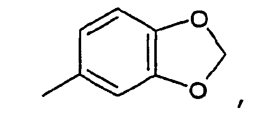 Pyrimidinyl, Thiophenyl, Isoxazolyl, Pyridinyl (wobei jedes gegebenenfalls mit einer oder zwei Gruppen substituiert sein kann, die unabhängig ausgewählt ist/sind aus C₁₋₃-Alkyl, C₁₋₃-Alkoxy, Halogen).

6. Verbindung gemäß Anspruch 5, worin Z Phenyl (das unsubstituiert oder einmal mit einem Substituenten substituiert ist, der ausgewählt ist aus Phenyl, OCH₂-Phenyl, NHSO₂CH₃, NHCOCH₃, CONH₂, CON(CH₃)₂, Cl, F, OCH₃, CN, OH, CH₂OH, CH₃, C(CH₃)₃) oder eine Heterocyclyl-Gruppe ist, die ausgewählt ist aus Benzofuranyl, Chinolinyl, 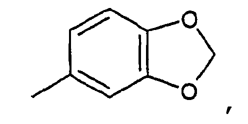 Pyrimidinyl, Thiophenyl, Benzothiophenyl, Isoxazolyl, Pyridinyl (wobei jedes substituiert ist oder einmal mit einer Gruppe substituiert ist, die ausgewählt ist aus -OCH₃, CH₃, F).

7. Verbindung gemäß Anspruch 6, worin Z Phenyl ist (das unsubstituiert oder einmal mit einem Substituenten substituiert ist, der ausgewählt ist aus Phenyl, OCH₂-Phenyl, NHSO₂CH₃, NHCOCH₃, CONH₂, CON(CH₃)₂, Cl, F, OCH₃, CN, OH, CH₂OH, CH₃, C(CH₃)₃).

8. Verbindung gemäß Anspruch 7, worin Z Phenyl ist.

9. Verbindung gemäß einem der Ansprüche 1 bis 8, worin R² H oder Y¹Z¹ ist.

10. Verbindung gemäß Anspruch 9, worin R² Y¹Z¹ ist.

11. Verbindung gemäß Anspruch 10, worin Y¹ eine Bindung oder C₁₋₃-Alkylen ist.

12. Verbindung gemäß Ansprüchen 10 bis 11, worin Z¹ Phenyl (unsubstituiert oder mit einem Substituenten substituiert, der ausgewählt ist aus NHSO₂R⁶, CONR⁷R⁸, CF₃, C₁₋₃-Alkoxy, SO₂R⁶, NHCOR⁷, SO₂NR⁷R⁸, NR⁷R⁸) oder eine 6-gliedrige Heterocyclyl-Gruppe ist, die ein Stickstoffatom enthält (das unsubstituiert oder einmal mit einer Gruppe substituiert ist, die ausgewählt ist aus C₁₋₃-Alkyl, CH₂-Phenyl, SO₂R⁶, CO_{NR}7_{R}8).

13. Verbindung gemäß Anspruch 12, worin Z¹ ein 6-gliedriger Heterocyclus ist, der mit SO₂R⁶ substituiert ist.

14. Verbindung gemäß Anspruch 13, worin der 6-gliedrige Heterocyclus 4-Piperidyl ist.

15. Verbindung gemäß Anspruch 1, worin R¹ YZ ist und R² H oder Br ist.

16. Verbindung gemäß Anspruch 1, worin R¹ Phenyl oder Br ist und R² Y¹Z¹ ist.

17. Verbindung gemäß Anspruch 1, worin R¹ YZ ist und R² Y¹Z¹ ist.

18. Verbindung gemäß Anspruch 1, die aus der Gruppe ausgewählt ist, bestehend aus:
5-Phenyl-1H-indol-7-carboxamid;
5-(4-Biphenyl)-1H-indol-7-carboxamid;
5-{4-[(Phenylmethyl)oxy]phenyl}-1H-indol-7-carboxamid;
5-{4-[(methylsulfonyl)amino]phenyl}-1H-indol-7-carboxamid;
5-[4-(Acetylamino)phenyl]-1H-indol-7-carboxamid;
5-[3-(Aminocarbonyl)phenyl]-1H-indol-7-carboxamid;
5-(4-Chlorphenyl)-1H-indol-7-carboxamid;
5-[3-(Acetylamino)phenyl]-1H-indol-7-carboxamid;
5-[3-(Aminosulfonyl)phenyl]-1H-indol-7-carboxamid;
5-{3-[(Dimethylamino)carbonyl]phenyl}-1H-indol-7-carboxamid;
5-(3-Fluorphenyl)-1H-indol-7-carboxamid;
5-[3-(Methyloxy)phenyl]-1H-indol-7-carboxamid;
5-(3-Cyanophenyl)-1H-indol-7-carboxamid;
5-(3-Hydroxyphenyl)-1H-indol-7-carboxamid;
5-(3-Chinolinyl)-1H-indol-7-carboxamid;
5-(1-Benzofuran-4-yl)-1H-indol-7-carboxamid;
5-(1,3-Benzodioxol-5-yl)-1H-indol-7-carboxamid;
5-[(E)-2-Phenylethenyl]-1H-indol-7-carboxamid;
5-(5-Pyrimidinyl)-1H-indol-7-carboxamid;
5-(3-Biphenylyl)-1H-indol-7-carboxamid;
5-(1-Benzofuran-2-yl)-1H-indol-7-carboxamid;
5-(1-Benzothien-2-yl)-1H-indol-7-carboxamid;
5-[3-(Hydroxymethyl)phenyl]-1H-indol-7-carboxamid;
5-(2-Naphthalenyl)-1H-indol-7-carboxamid;
5-(4-Fluorphenyl)-1H-indol-7-carboxamid;
5-[6-(Methyloxy)-3-pyridinyl]-1H-indol-7-carboxamid;
5-[4-(Hydroxymethyl)phenyl]-1H-indol-7-carboxamid;
5-(3-Chlorphenyl)-1H-indol-7-carboxamid;
5-(2-Methylphenyl)-1H-indol-7-carboxamid;
5-{3-[(Phenylmethyl)oxy]phenyl}-1H-indol-7-carboxamid;
5-(2-Chlorphenyl)-1H-indol-7-carboxamid;
5-(3,4-Dimethyl-4-isoxazolyl)-1H-indol-7-carboxamid;
5-{2-[(Phenylmethyl)oxy]phenyl}-1H-indol-7-carboxamid;
5-(5-Chinolinyl)-1H-indol-7-carboxamid;
5-(1-Naphthalenyl)-1H-indol-7-carboxamid;
3-Brom-5-phenyl-1H-indol-7-carboxamid;
3-Iod-5-phenyl-1H-indol-7-carboxamid;
3,5-Diphenyl-1H-indol-7-carboxamid;
3-{4-[(Methylsulfonyl)amino]phenyl}-5-phenyl-1H-indol-7-carboxamid;
5-Phenyl-3-(3-pyridinyl)-1H-indol-7-carboxamid;
3-(4-{[(2-Aminoethyl)amino]carbonyl}phenyl)-5-phenyl-1H-indol-7-carboxamid;
3-[4-({[4-(Methyloxy)-3-(4-methyl-1-piperazinyl)phenyl]amino}carbonyl)phenyl]-5-phenyl-1H-indol-7-carboxamidformiat;
5-Phenyl-3-[3-(trifluormethyl)phenyl]-1H-indol-7-carboxamid;
5-Brom-3-Iod-1H-indol-7-carboxamid;
3-(1-Ethyl-3-piperidinyl)-5-phenyl-1H-indol-7-carboxamid;
5-Phenyl-3-(3-piperidinyl)-1H-indol-7-carboxamid;
5-Phenyl-3-[1-(phenylmethyl)-3-piperidinyl]-1H-indol-7-carboxamid;
3-(1-Cyclohexen-1-yl)-5-phenyl-1H-indol-7-carboxamid;
3-Cyclohexyl-5-phenyl-1H-indol-7-carboxamid;
3-{1-[3-(Methyloxy)phenyl]ethenyl}-5-phenyl-1H-indol-7-carboxamid;
5-Phenyl-3-[1-(phenylmethyl)-1,2,3,6-tetrahydro-4-pyridinyl]-1H-indol-7-carboxamid;
5-Phenyl-3-(4-piperidinyl)-1H-indol-7-carboxamid;
3-{1-[(4-Chlorphenyl)sulfonyl]-4-piperidinyl}-5-phenyl-1H-indol-7-carboxamid;
5-Phenyl-3-[1-(propylsulfonyl)-4-piperidinyl]-1H-indol-7-carboxamid;
3-(1-Acetyl-4-piperidinyl)-5-phenyl-1H-indol-7-carboxamid;
3-[1-(N,N-Dimethyl-β-alanyl)-4-piperidinyl]-5-phenyl-1H-indol-7-carboxamid;
3-(1-Ethyl-4-piperidinyl)-5-phenyl-1H-indol-7-carboxamidformiat;
3-(1-Methylpyrrolidin-2-yl)-5-phenyl-1H-indol-7-carboxamid;
3-[1-(Ethylsulfonyl)pyrrolidin-3-yl]-5-phenyl-1H-indol-7-carboxamid;
3-[4-(Methylsulfonyl)phenyl]-5-phenyl-1H-indol-7-carboxamid;
3-[3-(Acetylamino)phenyl]-5-phenyl-1H-indol-7-carboxamid;
3-[4-(Ethylsulfonyl)phenyl]-5-phenyl-1H-indol-7-carboxamid;
3-[3-(Methylsulfonyl)phenyl]-5-phenyl-1H-indol-7-carboxamid;
3-(Hexahydro-1H-azepin-4-yl)-5-phenyl-1H-indol-7-carboxamid;
3-[1-(Ethylsulfonyl)hexahydro-1H-azepin-4-yl]-5-phenyl-1H-indol-7-carboxamid;
5-Phenyl-3-[2-(4-pyridinyl)ethyl]-1H-indol-7-carboxamid;
3-{[1-(Ethylsulfonyl)-4-piperidinyliden]methyl}-5-phenyl-1H-indol-7-carboxamid;
3-[1-(Ethylsulfonyl)-4-piperidinyl]-5-[4-(hydroxymethyl)phenyl]-1H-indol-7-carboxamid;
5-Phenyl-3-(3-piperidinylmethyl)-1H-indol-7-carboxamid;
5-Phenyl-3-[2-(4-piperidinyl)ethyl]-1H-indol-7-carboxamid;
3-{2-[1-(Ethylsulfonyl)-4-piperidinyl]ethyl}-5-phenyl-1H-indol-7-carboxamid;
3-{[1-(Ethylsulfonyl)-3-piperidinyl]methyl}-5-phenyl-1H-indol-7-carboxamid;
3-{[1-(Ethylsulfonyl)-4-piperidinyl]methyl}-5-phenyl-1H-indol-7-carboxamid;
3-{1-[(2)Sulfonyl]-4-piperidinyl}-5-phenyl-1H-indol-7-carboxamid;
3-{1-[(4-Fluorphenyl)sulfonyl]-4-piperidinyl}-5-phenyl-1H-indol-7-carboxamid;
3-{1-[(4-(Methylphenyl)sulfonyl]-4-piperidinyl}-5-phenyl-1H-indol-7-carboxamid;
3-{Phenylsulfonyl-4-piperidinyl}-5-phenyl-1H-indol-7-carboxamid;
3-(1-{[4-(Methyloxy)phenyl]sulfonyl}-4-piperidinyl)-5-phenyl-1H-indol-7-carboxamid;
3-[1-(Ethansulfonyl)-4-piperidinyl}-5-phenyl-1H-indol-7-carboxamid;
3-{1-[(2-Propansulfonyl]-4-piperidinyl}-5-phenyl-1H-indol-7-carboxamid;
5-Phenyl-3-[1-(propansulfonyl)-1,2,3,6-tetrahydro-4-pyridinyl]-1H-indol-7-carboxamid;
5-Phenyl-3-(1-{[4-(trifluormethyl)phenyl]-sulfonyl}-4-piperidinyl)-1H-indol-7-carboxamid;
3-{1-[(2,4-Dichlorphenyl)sulfonyl]-4-piperidinyl}-5-phenyl-1H-indol-7-carboxamid;
3-{1-[(3,4-Dichlorphenyl)sulfonyl]-4-piperidinyl}-5-phenyl-1H-indol-7-carboxamid;
3-{1-[(Ethylamino)carbonyl]-4-piperidinyl}-5-phenyl-1H-indol-7-carboxamid;
3-{1-[(4-1-Piperazinyl)carbonyl]-4-piperidinyl}-5-phenyl-1H-indol-7-carboxamid;
5-(4-Chlorphenyl)-3-[1-(propansulfonyl)-4-piperidinyl]-1H-indol-7-carboxamid;
3-{1-[(4-Fluorphenyl)sulfonyl]-4-piperidinyl}-5-(4-chlorphenyl)-1H-indol-7-carboxamid;
5-{4-[(Methylsulfonyl)amino]phenyl}-3-[1-(phenylmethyl)-1,2,3,6-tetrahydro-4-pyridinyl]-1H-indol-7-carboxamid;
5-{4-[(Methylsulfonyl)amino]phenyl}-3-(4-piperidinyl)-1H-indol-7-carboxamid;
5-Brom-3-[1-(Ethansulfonyl)-4-piperidinyl]-1H-indol-7-carboxamid;
3-[1-(Ethansulfonyl)-4-piperidinyl]-5-{4-[(methylsulfonyl)amino]phenyl}-1H-indol-7-carboxamid;
3-[1-(Ethansulfonyl)-4-piperidinyl]-5-(3-methylphenyl)-1H-indol-7-carboxamid;
3-[1-(Ethylsulfonyl)-4-piperidinyl]-5-(2-thienyl)-1H-indol-7-carboxamid;
3-[1-(Ethylsulfonyl)-4-piperidinyl]-5-(3-thienyl)-1H-indol-7-carboxamid;
3-[4-(Methylsulfonyl)phenyl]-5-phenyl-1H-indol-7-carboxamid;
3-{4-[(Dimethylamino)sulfonyl]phenyl}-5-phenyl-1H-indol-7-carboxamid;
3-{3-[(Methylsulfonyl)amino]phenyl}-5-phenyl-1H-indol-7-carboxamid;
3-[1-(Ethylsulfonyl)-4-piperidinyl]-5-{3-[(methylsulfonyl)amino]phenyl}-1H-indol-7-carboxamid;
5-[4-(Acetylamino)phenyl]-3-[1-(ethylsulfonyl)-4-piperidinyl]-1H-indol-7-carboxamid;
5-{4-[(Dimethylamino)sulfonyl]phenyl}-3-[1-(ethylsulfonyl)-4-piperidinyl]-1H-indol-7-carboxamid;
5-[3-(Acetylamino)phenyl]-3-[1-(ethylsulfonyl)-4-piperidinyl]-1H-indol-7-carboxamid;
3-[1-(Ethylsulfonyl)-4-piperidinyl]-5-(1H-pyrazol-4-yl)-1H-indol-7-carboxamid;
3-[1-(Ethylsulfonyl)-4-piperidinyl]-5-[3-(hydroxymethyl)phenyl]-1H-indol-7-carboxamid;
5-(2,4-Difluorphenyl)-3-[1-(ethylsulfonyl)-4-piperidinyl]-1H-indol-7-carboxamid;
3-[1-(Ethylsulfonyl)-4-piperidinyl]-5-[4-(methyloxy)phenyl]-1H-indol-7-carboxamid;
3[1-(Ethylsulfonyl)-4-piperidinyl]-5-(4-fluor-2-methylphenyl)-1H-indol-7-carboxamid;
3-[1-(Ethylsulfonyl)-4-piperidinyl]-5-(4-fluorphenyl)-1H-indol-7-carboxamid;
5-(4-biphenylyl)-3-[1-(ethylsulfonyl)-4-piperidinyl]-1H-indol-7-carboxamid;
5-[4-(1,1-Dimethylethyl)phenyl]-3-[1-ethylsulfonyl)-4-piperidinyl]-1H-indol-7-carboxamid;
3-[1-(Ethylsulfonyl)-4-piperidinyl]-5-(4-methylphenyl)-1H-indol-7-carboxamid;
3-[1-(Ethylsulfonyl)-4-pyridinyl)-1H-indol-7-carboxamid;
3-[1-(Ethylsulfonyl)-4-piperidinyl]-5-(6-fluor-3-pyridinyl)-1H-indol-7-carboxamid;
3-[1-(Ethylsulfonyl)-4-piperidinyl]-5-(6-methyl-3-pyridinyl)-1H-indol-7-carboxamid;
3-[1-(Ethylsulfonyl)-4-piperidinyl]-5-(4-methyl-3-pyridinyl)-1H-indol-7-carboxamid;
3-[1-(Ethylsulfonyl)-4-piperidinyl]-5-[6-(methyloxy)-3-pyridinyl]-1H-indol-7-carboxamid;
5-Phenyl-3-(N-acetyl-3-piperidinylmethyl)-1H-indol-7-carboxamid;
5-[3-(Ethyloxy)phenyl]-3-[1-(ethylsulfonyl)-4-piperidinyl]-1H-indol-7-carboxamid;
3-[1-(Ethylsulfonyl)-4-piperidinyl]-5-(2-fluorphenyl)-1H-indol-7-carboxamid;
3-[1-(Ethylsulfonyl)-4-piperidinyl]-5-[3-trifluormethyl)phenyl]-1H-indol-7-carboxamid;
3-[1-(Ethylsulfonyl)-4-piperidinyl]-5-[4-(trifluormethyl)phenyl]-1H-indol-7-carboxamid;
3-[1-(Ethylsulfonyl)-4-piperidinyl]-5-(3-fluorphenyl)-1H-indol-7-carboxamid;
5-(3,5-Dichlorphenyl)-3-[1-(ethylsulfonyl)-4-piperidinyl]-1H-indol-7-carboxamid;
5-(3,4-Difluorphenyl)-3-[1-(ethylsulfonyl)-4-piperidinyl]-1H-indol-7-carboxamid;
3-Brom-5-{3-[(dimethylamino)carbonyl]phenyl}-1H-indol-7-carboxamid;
5-[2,6-Bis(methyloxy)phenyl]-3-brom-1H-indol-7-carboxamid;
3-Brom-5-(4-fluor-2-methylphenyl)-1H-indol-7-carboxamid;
3-Brom-5-[5-fluor-2-(methyloxy)phenyl]-1H-indol-7-carboxamid;
3-Brom-5-(3-chinolinyl)-1H-indol-7-carboxamidtrifluoracetat;
3-Brom-5-(5-chinolinyl)-1H-indol-7-carboxamidtrifluoracetat;
5-[2,5-Bis(methyloxy)phenyl]-3-brom-1H-indol-7-carboxamid;
3-Brom-5-(2-fluorphenyl)-1H-indol-7-carboxamid;
5-[2,4-Bis(methyloxy)phenyl]-3-brom-1H-indol-7-carboxamid;
3-Brom-5-[2-(methyloxy)-3-pyridinyl]-1H-indol-7-carboxamidtrifluoracetat;
3-Brom-5-[2,3,4-tris(methyloxy)phenyl]-1H-indol-7-carboxamid;
3-{1-[(4-Chlor-2,5-Dimethylphenyl)sulfonyl]-4-piperidinyl}-5-phenyl-1H-indol-7-carboxamid;
3-(1-{[5-Brom-2-(methyloxy)phenyl]sulfonyl}-4-piperidinyl)-5-phenyl-1H-indol-7-carboxamid;
3-{1-[(5-Fluor-2-methylphenyl)sulfonyl]-4-piperidinyl}-5-phenyl-1H-indol-7-carboxamid;
3-{1-[(3-Fluorphenyl)sulfonyl]-4-piperidinyl}-5-phenyl-1H-indol-7-carboxamid;
5-Phenyl-3-(1-{[2,4,6-tris(1-methylethyl)-phenyl]sulfonyl}-4-piperidinyl)-1H-indol-7-carboxamid;
3-(1-{[4-(1,1-Dimethylpropyl)phenyl]sulfonyl}-4-piperidinyl)-5-phenyl-1H-indol-7-carboxamid;
3-{1-[(2-Methylphenyl)sulfonyl]-4-piperidinyl}-5-phenyl-1H-indol-7-carboxamid;
3-{1-[(2-Iodphenyl)sulfonyl]-4-piperidinyl}-5-phenyl-1H-indol-7-carboxamid;
3-{1-[(4-Pentylphenyl)sulfonyl]-4-piperidinyl}-5-phenyl-1H-indol-7-carboxamid;
5-Phenyl-3-{1-[(4-propylphenyl)sulfonyl]-4-piperidinyl}-1H-indol-7-carboxamid;
3-{1-[(2,4-Difluorphenyl)sulfonyl]-4-piperidinyl}-5-phenyl-1H-indol-7-carboxamid;
3-1-[(2,5-Dimethylphenyl)sulfonyl]-4-piperidinyl}-5-phenyl-1H-indol-7-carboxamid;
3-{1-[(4-Ethylphenyl)sulfonyl]-4-piperidinyl}-5-phenyl-1H-indol-7-carboxamid;
3-{1-[(3-Methylphenyl)sulfonyl]-4-piperidinyl}-5-phenyl-1H-indol-7-carboxamid;
3-{1-[(4-{[2-(Methyloxy)phenyl]oxy}-phenyl)sulfonyl]-4-piperidinyl}-5-phenyl-1H-indol-7-carboxamid;
3-{1-[(4-{[4-(Methyloxy)phenyl]oxy}phenyl)-sulfonyl]-4-piperidinyl}-5-phenyl-1H-indol-7-carboxamid;
3-[1-({3-[(4-Fluorphenyl)oxy]phenyl}sulfonyl)-4-piperidinyl]-5-phenyl-1H-indol-7-carboxamid;
3-{1-[(3-{[2-(Methyloxy)phenyl]oxy}-phenyl)sulfonyl]-4-piperidinyl}-5-phenyl-1H-indol-7-carboxamid;
3-[1-({4-[(4-Chlorphenyl)oxy]phenyl}sulfonyl)-4-piperidinyl]-5-phenyl-1H-indol-7-carboxamid;
3-{1-[(3-{[4-(Methyloxy)phenyl]oxy}phenyl)-sulfonyl]-4-piperidinyl}-5-phenyl-1H-indol-7-carboxamid;
5-Phenyl-3-(1-{[3-(phenyloxy)phenyl]sulfonyl}-4-piperidinyl)-1H-indol-7-carboxamid;
3-[1-({3-[(4-Chlorphenyl)oxy]phenyl}sulfonyl)-4-piperidinyl]-5-phenyl-1H-indol-7-carboxamid;
3-[1-({4-[(2-Methylphenyl)oxy]phenyl}-sulfonyl)-4-piperidinyl]-5-phenyl-1H-indol-7-carboxamid;
3-{1-[(4'-Chlor-4-biphenylyl)sulfonyl]-4-piperidinyl}-5-phenyl-1H-indol-7-carboxamid;
3-[1-({3-[(2-Methylphenyl)oxy]phenyl}sulfonyl-4-piperidinyl]-5-phenyl-1H-indol-7-carboxamid;
3-[1-({3-[(2-Chlorphenyl)oxy]phenyl}sulfonyl)-4-piperidinyl]-5-phenyl-1H-indol-7-carboxamid;
3-{1-[(5-Chlor-1-naphthalinyl)sulfonyl]-4-piperidinyl}-5-phenyl-1H-indol-7-carboxamid;
3-(1-{[4'-(Methyloxy)-3-biphenylyl]sulfonyl}-4-piperidinyl)-5-phenyl-1H-indol-7-carboxamid;
3-[1-(3-Biphenylylsulfonyl)-4-piperidinyl]-5-phenyl-1H-indol-7-carboxamid;
3-(1-{[(4-Fluorphenyl)methyl]sulfonyl}-4-piperidinyl)-5-phenyl-1H-indol-7-carboxamid;
3-{1-[(5-Chlor-2-naphthalinyl)sulfonyl]-4-piperidinyl}-5-phenyl-1H-indol-7-carboxamid;
3-{1-{[4'-(Methyloxy)-4-biphenylyl]sulfonyl}-4-piperidinyl)-5-phenyl-1H-indol-7-carboxamid;
5-(2-Fluorphenyl)-1H-indol-7-carboxamid;
5-(3-{[(2,2-Dimethylpropyl)amino]carbonyl}-phenyl)-1H-indol-7-carboxamid;
5-(3-{[(1-Methylethyl)amino]carbonyl}phenyl)-1H-indol-7-carboxamid;
5-(4-{[(2,2-Dimethylpropyl)amino]carbonyl}-phenyl)-1H-indol-7-carboxamid;
5-{4-[(Propylamino)carbonyl]phenyl}-1H-indol-7-carboxamid;
5-(4-{[(1-Methylethyl)amino]carbonyl}phenyl)-1H-indol-7-carboxamid;
5-{4-[(Diethylamino)carbonyl]phenyl}-1H-indol-7-carboxamid;
3-[1-(Methylsulfonyl)-1,2,3,6-tetrahydro-4-pyridinyl]-5-phenyl-1H-indol-7-carboxamid;
3-(3-Oxocyclopentyl)-5-phenyl-1H-indol-7-carboxamid;
5-Phenyl-3-{3-[(phenylmethyl)amino]-cyclopentyl}-1H-indol-7-carboxamid;
3-(3-Aminocyclopentyl)-5-phenyl-1H-indol-7-carboxamid;
3-{3-[(Ethylsulfonyl)amino]cyclopentyl}-5-phenyl-1H-indol-7-carboxamid;
5-Brom-3-[1-(propylsulfonyl)-4-piperidinyl]-1H-indol-7-carboxamid;
5-Brom-3-(3-pyridinyl)-1H-indol-7-carboxamid;
5-Brom-3-[1-(methylsulfonyl)-1,2,3,6-tetrahydro-4-pyridinyl]-1H-indol-7-carboxamid;
3-[(4-Hydroxyphenyl)methyl]-5-phenyl-1H-indol-7-carboxamid;
5-Brom-1H-indol-7-carboxamid;
5-(4-Chlorphenyl)-1H-indol-7-carboxamid;
5-Brom-3(4-piperidinyl)-1H-indol-7-carboxamid;
oder ein Salz davon.

19. Verbindung gemäß Anspruch 1, die aus der Gruppe ausgewählt ist, bestehend aus:
3-{1-[(4-Chlorphenyl)sulfonyl]-4-piperidinyl}-5-phenyl-1H-indol-7-carboxamid;
3-{1-[(2)-Sulfonyl]-4-piperidinyl}-5-phenyl-1H-indol-7-carboxamid;
3-{1-[(4-Fluorphenyl)sulfonyl]-4-piperidinyl}-5-phenyl-1H-indol-7-carboxamid;
3-{1-[(4-Methylphenyl)sulfonyl]-4-piperidinyl}-5-phenyl-1H-indol-7-carboxamid;
3-{Phenylsulfonyl-4-piperidinyl}-5-phenyl-1H-indol-7-carboxamid;
3-(1-{[4-(Methyloxy)phenyl]sulfonyl}-4-piperidinyl)-5-phenyl-1H-indol-7-carboxamid;
5-Phenyl-3-(1-{[4-(trifluormethyl)phenyl]-sulfonyl}-4-piperidinyl)-1H-indol-7-carboxamid;
3-{1-[(2,4-Dichlorphenyl)sulfonyl]-4-piperidinyl}-5-phenyl-1H-indol-7-carboxamid;
3-{1-[(3,4-Dichlorphenyl)sulfonyl]-4-piperidinyl}-5-phenyl-1H-indol-7-carboxamid;
3-{1-[(4-Fluorphenyl)sulfonyl]-4-piperidinyl}-5-(4-chlorphenyl)-1H-indol-7-carboxamid;
3-{1-[(4-Chlor-2,5-dimethylphenyl)sulfonyl]-4-piperidinyl}-5-phenyl-1H-indol-7-carboxamid;
3-(1-{[5-Brom-2-(methyloxy)phenyl]sulfonyl}-4-piperidinyl)-5-phenyl-1H-indol-7-carboxamid;
3-{1-[(5-Fluor-2-methylphenyl)sulfonyl]-4-piperidinyl}-5-phenyl-1H-indol-7-carboxamid;
3-{1-[(3-Fluorphenyl)sulfonyl]-4-piperidinyl}-5-phenyl-1H-indol-7-carboxamid;
5-Phenyl-3-(1-{[2,4,6-tris(1-methylethyl)-phenyl]sulfonyl}-4-piperidinyl)-1H-indol-7-carboxamid;
3-(1-{[4-(1,1-Dimethylpropyl)phenyl]sulfonyl}-4-piperidinyl)-5-phenyl-1H-indol-7-carboxamid;
3-{1-[(2-Methylphenyl)sulfonyl]-4-piperidinyl}-5-phenyl-1H-indol-7-carboxamid;
3-{1-[(2-Iodphenyl)sulfonyl]-4-piperidinyl}-5-phenyl-1H-indol-7-carboxamid;
3-{1-[(4-Pentylphenyl)sulfonyl]-4-piperidinyl}-5-phenyl-1H-indol-7-carboxamid;
5-Phenyl-3-{1-[(4-propylphenyl)sulfonyl]-4-piperidinyl}-1H-indol-7-carboxamid;
3-{1-[(2,4-Difluorphenyl)sulfonyl]-4-piperidinyl}-5-phenyl-1H-indol-7-carboxamid;
3-{1-[(2,5-Dimethylphenyl)sulfonyl]-4-piperidinyl}-5-phenyl-1H-indol-7-carboxamid;
3-{1-[(4-Ethylphenyl)sulfonyl]-4-piperidinyl}-5-phenyl-1H-indol-7-carboxamid;
3-{1-(3-Methylphenyl)sulfonyl]-4-piperidinyl}-5-phenyl-1H-indol-7-carboxamid;
3-{1-[(4-{[2-(Methyloxy)phenyl]oxy}phenyl)-sulfonyl]-4-piperidinyl}-5-phenyl-1H-indol-7-carboxamid;
3-{1-[(4-{[4-(Methyloxy)phenyl]oxy}-phenyl)sulfonyl]-4-piperidinyl}-5-phenyl-1H-indol-7-carboxamid;
3-[1-({3-[(4-fluorphenyl)oxy]phenyl}sulfonyl)-4-piperidinyl]-5-phenyl-1H-indol-7-carboxamid;
3-{1-[(3-{[2-Methyloxy)phenyl]oxy}phenyl)-sulfonyl]-4-piperidinyl}-5-phenyl-1H-indol-7-carboxamid;
3-[1-({4-[(4-Chlorphenyl)oxy]phenyl}sulfonyl]-4-piperidinyl]-5-phenyl-1H-indol-7-carboxamid;
3-{1-[(3-{[4-(Methyloxy)phenyl]oxy}-phenyl)sulfonyl]-4-piperidinyl}-5-phenyl-1H-indol-7-carboxamid;
5-Phenyl-3-(1-{[3-(phenyloxy)phenyl]sulfonyl}-4-piperidinyl)-1H-indol-7-carboxamid;
3-[1-({3-[(4-Chlorphenyl)oxy]phenyl}sulfonyl)-4-piperidinyl]-5-phenyl-1H-indol-7-carboxamid;
3-[1-({4-[(2-Methylphenyl)oxy]phenyl}-sulfonyl)-4-piperidinyl]-5-phenyl-1H-indol-7-carboxamid;
3-{1-[(4'-Chlor-4-biphenylyl)sulfonyl]-4-piperidinyl}-5-phenyl-1H-indol-7-carboxamid;
3-[1-({3-[(2-Methylphenyl)oxy]phenyl}-sulfonyl)-4-piperidinyl]-5-phenyl-1H-indol-7-carboxamid;
3-[1-({3-[(2-Chlorphenyl)oxy]phenyl}sulfonyl)-4-piperidinyl]-5-phenyl-1H-indol-7-carboxamid:
3-{1-[(5-Chlor-1-naphthalinyl)sulfonyl]-4-piperidinyl}-5-phenyl-1H-indol-7-carboxamid;
3-(1-{[4'-(Methyloxy)-3-biphenylyl]sulfonyl}-4-piperidinyl)-5-phenyl-1H-indol-7-carboxamid;
3-[1-(3-biphenylylsulfonyl)-4-piperidinyl]-5-phenyl-1H-indol-7-carboxamid;
3-(1-{[(4-Fluorphenyl)methyl]sulfonyl}-4-piperidinyl)-5-phenyl-1H-indol-7-carboxamid;
3-{1-[(5-Chlor-2-naphthalinyl)sulfonyl]-4-piperidinyl}-5-phenyl-1H-indol-7-carboxamid; und
3-(1-{[4'-(Methyloxy)-4-biphenylyl]sulfonyl}-4-piperidinyl)-5-phenyl-1H-indol-7-carboxamid;
oder ein Salz davon.

20. Verbindung gemäß Anspruch 1, die aus der Gruppe ausgewählt ist, bestehend aus:
5-Phenyl-3-[1-(propylsulfonyl)-4-piperidinyl]-1H-indol-7-carboxamid;
3-[1-(Ethylsulfonyl)-4-piperidinyl]-5-[4-(hydroxymethyl)phenyl]-1H-indol-7-carboxamid;
3-[1-(Ethansulfonyl)-4-piperidinyl]-5-phenyl-1H-indol-7-carboxamid;
3-{1-[(2-Propansulfonyl]-4-piperidinyl}-5-phenyl-1H-indol-7-carboxamid;
5-Phenyl-3-[1-(propansulfonyl)-1,2,3,6-tetrahydro-4-pyridinyl]-1H-indol-7-carboxamid;
5-(4-Chlorphenyl)-3-[1-(propansulfonyl)-4-piperidinyl]-1H-indol-7-carboxamid;
3-[1-(Ethansulfonyl)-4-piperidinyl]-5-{4-[(methylsulfonyl)amino]phenyl}-1H-indol-7-carboxamid;
3-[1-Ethansulfonyl)-4-piperidinyl]-5-(3-methylphenyl)-1H-indol-7-carboxamid;
3-[1-(Ethylsulfonyl)-4-piperidinyl]-5-{3-[(methylsulfonyl)amino]phenyl}-1H-indol-7-carboxamid;
5-[4-(Acetylamino)phenyl]-3-[1-(ethylsulfonyl)-4-piperidinyl]-1H-indol-7-carboxamid;
5-{4-[(Dimethylamino)sulfonyl]phenyl}-3-[1-(ethylsulfonyl)-4-piperidinyl]-1H-indol-7-carboxamid;
5-[3-(Acetylamino)phenyl]-3-[1-(ethylsulfonyl)-4-piperidinyl]-1H-indol-7-carboxamid;
3-[1-(Ethylsulfonyl)-4-piperidinyl]-5-[3-(hydroxymethyl)phenyl]-1H-indol-7-carboxamid;
5-(2,4-Difluorphenyl)-3-[1-(ethylsulfonyl)-4-piperidinyl]-1H-indol-7-carboxamid;
3-[1-(Ethylsulfonyl)-4-piperidinyl]-5-[4-(methyloxy)phenyl]-1H-indol-7-carboxamid;
3-[1-(Ethylsulfonyl)-4-piperidinyl]-5-(4-fluor-2-methylphenyl)-1H-indol-7-carboxamid;
3-[1-(Ethylsulfonyl)-4-piperidinyl]-5-(4-fluorphenyl)-1H-indol-7-carboxamid;
5-(4-Biphenylyl)-3-[1-(ethylsulfonyl)-4-piperidinyl]-1H-indol-7-carboxamid;
5-[4-(1,1-Dimethylethyl)phenyl]-3-[1-(ethylsulfonyl)-4-piperidinyl]-1H-indol-7-carboxamid;
3-[1-(Ethylsulfonyl)-4-piperidinyl]-5-(4-methylphenyl)-1H-indol-7-carboxamid;
5-[3-(Ethyloxy)phenyl]-3-[1-(ethylsulfonyl)-4-piperidinyl]-1H-indol-7-carboxamid;
3-[1-(Ethylsulfonyl)-4-piperidinyl]-5-(2-fluorphenyl)-1H-indol-7-carboxamid;
3-[1-(Ethylsulfonyl)-4-piperidinyl]-5-[3-(trifluormethyl)phenyl]-1H-indol-7-carboxamid;
3-[1-(Ethylsulfonyl)-4-piperidinyl]-5-[4-(trifluormethyl)phenyl]-1H-indol-7-carboxamid;
3-[1-(Ethylsulfonyl)-4-piperidinyl]-5-(3-fluorphenyl)-1H-indol-7-carboxamid;
5-(3,5-Dichlorphenyl)-3-[1-(ethylsulfonyl)-4-piperidinyl]-1H-indol-7-carboxamid; und
5-(3,4-Difluorphenyl)-3-[1-(ethylsulfonyl)-4-piperidinyl]-1H-indol-7-carboxamid;
oder ein Salz davon.

21. Verbindung gemäß Anspruch 1, die aus der Gruppe ausgewählt ist, bestehend aus:
3-[1-(Ethylsulfonyl)-4-piperidinyl]-5-(2-thienyl)-1H-indol-7-carboxamid;
3-[1-(Ethylsulfonyl)-4-piperidinyl]-5-(3-thienyl)-1H-indol-7-carboxamid;
3-[1-(Ethylsulfonyl)-4-piperidinyl]-5-(1H-pyrazol-4-yl)-1H-indol-7-carboxamid;
3-[1-(Ethylsulfonyl)-4-piperidinyl]-5-(4-pyridinyl)-1H-indol-7-carboxamid;
3-[1-(Ethylsulfonyl)-4-piperidinyl]-5-(6-fluor-3-pyridinyl)-1H-indol-7-carboxamid;
3-[1-(Ethylsulfonyl)-4-piperidinyl]-5-(6-methyl-3-pyridinyl)-1H-indol-7-carboxamid;
3-[1-(Ethylsulfonyl)-4-piperidinyl]-5-(4-methyl-3-pyridinyl)-1H-indol-7-carboxamid; und
3-[1-(Ethylsulfonyl)-4-piperidinyl]-5-[6-(methyloxy)-3-pyridinyl]-1H-indol-7-carboxamid;
oder ein Salz davon.

22. Verbindung gemäß Anspruch 1, die 5-Brom-3-[1-(ethansulfonyl)-4-piperidinyl]-1H-indol-7-carboxamid oder ein Salz davon ist.

23. Verbindung gemäß Anspruch 1, die aus der Gruppe ausgewählt ist, bestehend aus:
3-[1-(Methylsulfonyl)-1,2,3,6-tetrahydro-4-pyridinyl]-5-phenyl-1H-indol-7-carboxamid und
5-Brom-3-[1-(methylsulfonyl)-1,2,3,6-tetrahydro-4-pyridinyl]-1H-indol-7-carboxamid;
oder ein Salz davon.

24. Pharmazeutische Zusammensetzung, die eine Verbindung gemäß einem der Ansprüche 1 bis 23 oder ein Salz davon und einen oder mehrere aus pharmazeutisch annehmbaren Trägern, Verdünnungsstoffen und Exzipienten umfaßt.

25. Verbindung gemäß einem der Ansprüche 1 bis 23 oder ein Salz davon zur Verwendung in der Therapie.

26. Verbindung gemäß Anspruch 1 bis 23 zur Verwendung in der Behandlung einer Störung, die durch unangemessene Kinaseaktivität vermittelt wird.

27. Verbindung gemäß Anspruch 1 bis 23 zur Verwendung in der Behandlung einer Störung, die durch unangemessene IKK2-Aktivität vermittelt wird.

28. Verwendung einer Verbindung gemäß einem der Ansprüche 1 bis 23 oder eines Salzes davon in der Herstellung eines Medikaments zur Verwendung in der Behandlung einer Störung, die durch unangemessene Kinase-Aktivität vermittelt wird.

29. Verwendung gemäß Anspruch 28, worin die unangemessene Kinase-Aktivität unangemessene IKK2-Aktivität ist.

30. Verwendung gemäß Anspruch 29, worin die Störung, die durch unangemessene IKK2-Aktivität vermittelt wird, Entzündungs- und Gewebereparaturstörungen ist, insbesondere rheumatoide Arthritis, entzündliche Darmerkrankung, Asthma und COPD (chronischobstruktive Lungenerkrankung); Osteoarthritis, Osteoporose und fibrotische Erkrankungen; Dermatose, einschließlich Psoriasis, atopische Dermatitis und durch ultraviolette Strahlung (UV)-induzierte Hautschädigung; Autoimmunerkrankungen, einschließlich systemischer Lupus erythematodes; Multiple Sklerose, psoriatische Arthritis, Spondylitis ankylosans, Gewebe- und Organabstoßung; Alzheimer-Erkrankung, Schlaganfall, Atherosklerose, Restenose, Diabetes, Glomerulonephritis, Krebs, einschließlich Hodgkins-Erkrankung, Kachexie, Entzündung, die mit Infektion und bestimmten viralen Infektionen assoziiert ist, einschließlich erworbenem Immundefizienzsyndrom (AIDS), Atemwegsblockade des Erwachsenen und Ataxia Telagiestasia.

31. Verwendung gemäß Anspruch 30, worin die IKK2-Störung eine Entzündungs- oder Gewebereparaturstörung ist.

32. Verwendung gemäß Anspruch 31, worin die IKK2-Störung rheumatoide Arthritis, entzündliche Darmerkrankung, Asthma oder COPD ist.

33. Verwendung gemäß Anspruch 32, worin die IKK2-Störung Asthma ist.

34. Verwendung gemäß Anspruch 32, worin die IKK2-Störung COPD ist.

35. Verwendung gemäß Anspruch 32, worin die IKK2-Störung rheumatoide Arthritis ist.

36. Verwendung gemäß Anspruch 30, worin die IKK2-Störung aus der Gruppe ausgewählt ist, die aus Autoimmunerkrankung, Gewebe- oder Organabstoßung, Alzheimer-Erkrankung, Schlaganfall, Atherosklerose, Restenose, Diabetes, Glomerulonephritis, Osteoarthritis, Osteoporose und Ataxia Telangiestasia besteht.

37. Verwendung gemäß Anspruch 36, worin die Erkrankung eine Autoimmunerkrankung ist.

38. Verwendung gemäß Anspruch 37, worin die Autoimmunerkrankung systemischer Lupus erythematodes, Multiple Sklerose, psoriatische Arthritis oder Spondylitis ankylosans oder Diabetes ist.

39. Verwendung gemäß Anspruch 30, worin die Störung Krebs und/oder Kachexie ist.

40. Verwendung gemäß Anspruch 39, worin der Krebs Hodgkin-Erkrankung ist.

## Revendications

1. Composé de formule (I) : 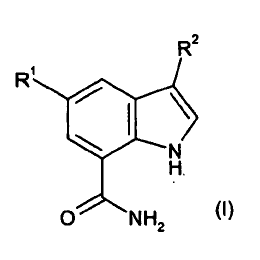
dans laquelle R¹ représente H, un atome d'halogène ou un groupe -YZ ;
Y représente une liaison (c'est-à-dire, est absent), un groupe alkylène en C₁ à C₆ ou alcénylène en C₂ à C₆ ;
Z représente un groupe aryle ou hétéroaryle comprenant chacun 5 à 14 chaînons de cycle, ledit groupe aryle ou hétéroaryle étant éventuellement substitué par un ou plusieurs substituants choisis indépendamment parmi un atome d'halogène, OH, un groupe alkyle en C₁ à C₆, halogénoalkyle en C₁ à C₆, alcoxy en C₁ à C₆, halogénoalcoxy en C₁ à C₆, CN, hydroxyalkyle en C₁ à C₆ , phényle, O-(CH₂)₁₋₆-phényle, NHSO₂R³, NHCOR³, CONR⁴R⁵ , SO_{2NR}⁴_{R}5 _{;}
R³, R⁴ et R⁵ représentent indépendamment H ou un groupe alkyle en C₁ à C₆ ;
R² représente H, un atome d'halogène ou un groupe -Y¹Z¹;
Y¹ représente une liaison (c'est-à-dire, est absent), un groupe alkylène en C₁ à C₆ ou alcénylène en C₂ à C₆ ;
Z¹ représente un groupe aryle à 6 chaînons, hétéroaryle à 5 ou 6 chaînons, hétérocyclyle à 5 à 7 chaînons, cycloalkyle en C₅ à C₇, cycloalcényle en C₅ à C₇, chacun d'entre eux pouvant être éventuellement substitué par un ou plusieurs substituants choisis indépendamment parmi SO₂R⁶, NHSO₂R⁶, 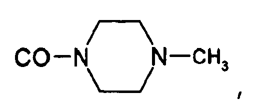 COR⁷, NR⁷R⁸, SO₂NR⁷R⁸, un groupe alkyle en C₁ à C₆, halogénoalkyle en C₁ à C₆, alcoxy en C₁ à C₆, halogénoalcoxy en C₁ à C₆ , halogène, CONR⁷R⁸, NHCOR⁷, ou phényle (fixé directement ou fixé par un espaceur alkylène en C₁ à C₆, CONH, alcénylène en C₂ à C₆ et éventuellement substitué par un ou plusieurs substituants choisis parmi 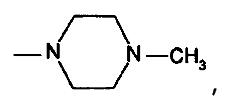 un groupe alkyle en C₁ à C₆, alcoxy en C₁ à C₆, halogénoalkyle en C₁ à C₆, halogénoalcoxy en C₁ à C₆, OH, halogène);
R⁶ représente H, un groupe alkyle en C₁ à C₆, -(CH₂)ₙ-phényle ou -(CH₂)ₙ-naphtyle (où n vaut 0 ou 1 et chacun des groupes phényle ou naphtyle peut être éventuellement substitué par un ou plusieurs substituants choisis indépendamment parmi un groupe alkyle en C₁ à C₆, alcoxy en C₁ à C₆, halogène, NR⁷R⁸, halogénoalkyle en C₁ à C₆, halogénoalcoxy en C₁ à C₆), CN ou -(O)p-phényle (où p vaut 0 ou 1 et le groupe phényle est éventuellement substitué par un ou plusieurs substituants choisis indépendamment parmi un atome d'halogène, un groupe alkyle en C₁ à C₆ ou alcoxy en C₁ à C₆)) ;
R⁷ et R⁸ représentent indépendamment un groupe alkyle en C₁ à C₆, H, alkylène en C₁ à C₆, NR⁹R¹⁰;
R⁹ et R¹⁰ représentent indépendamment un groupe alkyle en C₁ à C₆, H ;
à condition que R¹ et R² ne représentent pas tous les deux H ;
ou un sel de celui-ci.

2. Composé selon la revendication 1, où R¹ représente YZ.

3. Composé selon la revendication 2, où Y représente une liaison ou -CH = CH-.

4. Composé selon la revendication 3, où Y représente une liaison.

5. Composé selon les revendications 1 à 4, où Z représente un groupe phényle (qui peut être non substitué ou substitué une fois ou deux fois par des substituants choisis indépendamment parmi un groupe alcoxy en C₁ à C₃, CN, OH, phényle , -OCH₂-phényle, NHSO₂R³, NHCOR³ , CONR⁴R⁵, SO₂NR⁴R⁵, halogène, hydroxyalkyle en C₁ à C₃, alkyle en C₁ à C₄) ou un groupe hétéroaryle choisi parmi un groupe benzofuranyle, quinoléinyle, 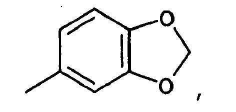 pyrimidinyle, thiophényle, isoxazolyle, pyridinyle (chacun d'entre eux pouvant être substitué par un ou deux groupes choisis indépendamment parmi un groupe alkyle en C₁ à C₃, alcoxy en C₁ à C₃, halogène.

6. Composé selon la revendication 5, où Z représente un groupe phényle (qui est non substitué ou substitué une fois par un substituant choisi parmi un groupe phényle, OCH₂-phényle, NHSO₂CH₃, NHCOCH₃, CONH₂, CON(CH₃)₂, Cl, F, OCH₃ , CN, OH, CH₂OH, CH₃, C(CH₃)₃) ou un groupe hétérocyclique choisi parmi un groupe benzofuranyle, quinoléinyle, 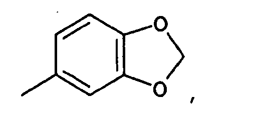 pyrimidinyle, thiophényle, benzothiophényle, isoxazolyle, pyridinyle (chacun d'entre eux étant substitué ou étant substitué une fois par un groupe choisi parmi -OCH₃, CH₃, F).

7. Composé selon la revendication 6, où Z représente un groupe phényle (qui est non substitué ou substitué une fois par un substituant choisi parmi un groupe phényle, OCH₂-phényle, NHSO₃CH3 , NHCOCH₃ , CONH₂, CON(CH₃)₂, Cl, F, OCH₃ , CN, OH, CH₂OH CH₃, C(CH₃)₃).

8. Composé selon la revendication 7, où Z représente un groupe phényle.

9. Composé selon l'une quelconque des revendications 1 à 8, où R² représente H ou Y¹Z¹.

10. Composé selon la revendication 9, où R² représente Y¹Z¹.

11. Composé selon la revendication 10, où Y¹ représente une liaison ou un groupe alkylène en C₁ à C₃.

12. Composé selon les revendications 10 et 11, où Z¹ représente un groupe phényle (non substitué ou substitué par un substituant choisi parmi NHSO₂R⁶, CONR⁷R⁸, CF₃, alcoxy en C₁ à C₃, SO₂R⁶, NHCOR⁷, SO₂NR⁷R⁸, NR⁷R⁸) ou un groupe hétérocyclique à 6 chaînons qui contient un atome d'azote (qui est non substitué ou substitué une fois par un groupe choisi parmi un groupe alkyle en C₁ à C₃, CH₂-phényle, SO₂R⁶, CONR⁷R⁸).

13. Composé selon la revendication 12, où Z¹ représente un hétérocycle à 6 chaînons substitué par SO₂R⁶.

14. Composé selon la revendication 13, où l'hétérocycle à 6 chaînons représente un groupe 4-pipéridyle.

15. Composé selon la revendication 1, où R¹ représente YZ et R² représente H ou Br.

16. Composé selon la revendication 1, où R¹ représente un groupe phényle ou Br et R² représente Y¹Z¹.

17. Composé selon la revendication 1, où R¹ représente YZ et R² représente Y¹Z¹.

18. Composé selon la revendication 1, choisi dans le groupe constitué de :
5-phényl-1*H*-indole-7-carboxamide ;
5-(4-biphénylyl)-1*H*-indole-7-carboxamide ;
5-{4-[(phénylméthyl)oxy]phényl}-1*H*-indole-7-carboxamide ;
5-{4-[(méthylsulfonyl)amino]phényl}-1*H*-indole-7-carboxamide ;
5-[4-(acétylamino)phényl]-1*H*-indole-7-carboxamide ;
5-[3-(aminocarbonyl)phényl]-1*H*-indole-7-carboxamide ;
5-(4-chlorophényl)-1*H*-indole-7-carboxamide ;
5-[3-(acétylamino)phényl]-1*H*-indole-7-carboxamide ;
5-[3-(aminosulfonyl)phényl]-1*H*-indole-7-carboxamide ;
5-{3-[(diméthylamino)carbonyl]phényl]1*H*-indole-7-carboxamide ;
5-(3-fluorophényl)-1*H*-indole-7-carboxamide ;
5-[3-(méthyloxy)phenyl]-1*H*-indole-7-carboxamide ;
5-(3-cyanophényl)-1*H*-indole-7-carboxamide ;
5-(3-hydroxyphényl)-1*H*-indole-7-carboxamide ;
5-(3-quinoléinyl)-1*H*-indole-7-carboxamide ;
5-(1-benzofuran-4-yl)-1*H*-indole-7-carboxamide ;
5-(1,3-benzodioxol-5-yl)-1*H*-indole-7-carboxamide ;
5-[(E)-2-phényléthényl]-1*H*-indole-7-carboxamide ;
5-(5-pyrimidinyl)-1*H*-indole-7-carboxamide ;
5-(3-biphénylyl)-1*H*-indole-7-carboxamide ;
5-(1-benzofuran-2-yl)-1*H*-indole-7-carboxamide ;
5-(1-benzothién-2-yl)-1*H*-indole-7-carboxamide ;
5-[3-(hydroxyméthyl)phényl]-1*H*-indole-7-carboxamide ;
5-(2-naphtalényl)-1*H*-indole-7-carboxamide ;
5-(4-fluorophényl)-1*H*-indole-7-carboxamide ;
5-[6-(méthyloxy)-3-pyridinyl]-1*H*-indole-7-carboxamide ;
5-[4-(hydroxyméthyl)phényl-1*H*-indole-7-carboxamide ;
5-(3-chlorophényl)-1*H*-indole-7-carboxamide ;
5-(2-méthylphényl)-1*H*-indole-7-carboxamide ;
5-{3-[(phénylméthyl)oxy]phényl}-1*H*-indole-7-carboxamide ;
5-(2-chlorophényl)-1*H*-indole-7-carboxamide ;
5-(3,5-diméthyl-4-isoxazolyl)-1*H*-indole-7-carboxamide ;
5-{2-[(phénylmethyl)oxy]phényl)-1*H*-indole-7-carboxamide ;
5-(5-quinoléinyl)-1*H*-indole-7-carboxamide ;
5-(1-naphtalényl)-1*H*-indole-7-carboxamide ;
3-bromo-5-phényl-1*H*-indole-7-carboxamide ;
3-iodo-5-phényl-1*H*-indole-7-carboxamide ;
3,5-diphényl-1*H*-indole-7-carboxamide ;
3-{4-[(méthylsulfonyl)amino]phényl}-5-phényl-1*H*-indole-7-carboxamide ;
5-phényl-3-(3-pyridinyl)-1*H*-indole-7-carboxamide ;
3-(4-{[(2-aminoéthyl)amino]carbonyl}phényl)-5-phényl-1*H-*indole-7-carboxamide ;
formiate de 3-4-({[4-(méthyloxy)-3-(4-méthyl-1-pipérazinyl)phényl]amino}carbonyl)phényl]-5-phényl-1*H*-indole-7-carboxamide ;
5-phényl-3-[3-(trifluoromethyl)phényl]-1*H*-indole-7-carboxamide ;
5-bromo-3-iodo-1*H*-indole-7-carboxamide ;
3-(1-éthyl-3-pipéridinyl)-5-phényl-1*H*-indole-7-carboxamide ;
5-phényl-3-(3-pipéridinyl)-1*H*-indole-7-carboxamide ;
5-phenyl-3-[1-(phénylméthyl)-3-pipéridinyl]-1*H*-indole-7-carboxamide ;
3-(1-cyclohexén-1-yl)-5-phényl-1*H*-indole-7-carboxamide ;
3-cyclohexyl-5-phényl-1*H*-indole-7-carboxamide ;
3-{1-[3-(méthyloxy)phényl]éthényl}-5-phényl-1*H*-indole-7-carboxamide ;
5-phényl-3-[1-(phénylméthyl)-1,2,3,6-tétrahydro-4-pyridinyl]-1*H*-indole-7-carboxamide ;
5-phényl-3-(4-pipéridinyl)-1*H*-indole-7-carboxamide ;
3-{1-[(4-chlorophényl)sulfonyl]-4-pipéridinyl}-5-phényl-1*H*-indole-7-carboxamide ;
5-phenyl-3-[1-(propylsulfonyl)-4-pipéridinyl]-1*H*-indole-7-carboxamide ;
3-(1-acétyl-4-pipéridinyl)-5-phényl-1*H*-indole-7-carboxamide ;
3-[1-(N,N-diméthyl-β-alanyl)-4-pipéridinyl]-5-phényl-1*H-*indole-7-carboxamide ;
formiate de 3-(1-éthyl-4-pipéridinyl)-5-phényl-1*H*-indole-7-carboxamide ;
3-(1-méthylpyrrolidin-2-yl)-5-phényl-1*H*-indole-7-carboxamide ;
3-[1-(éthylsulfonyl)pyrrolidin-3-yl]-5-phényl-1*H*-indole-7-carboxamide ;
3-[4-(méthylsulfonyl)phényl]-5-phényl-1*H*-indole-7-carboxamide ;
3-[3-(acétylamino)phényl]-5-phényl-1*H*-indole-7-carboxamide ;
3-[4-(éthylsulfonyl)phényl]-5-phényl-1*H*-indole-7-carboxamide ;
3-[3-(méthylsulfonyl)phényl]-5-phényl-1*H*-indole-7-carboxamide ;
3-(hexahydro-1*H*-azépin-4-yl)-5-phényl-1*H*-indole-7-carboxamide ;
3-[1-(éthylsulfonyl)hexahydro-1*H*-azépin-4-yl]-5-phényl-1*H*-indole-7-carboxamide ;
5-phényl-3-[2-(4-pyridinyl)éthyl]-1*H*-indole-7-carboxamide ;
3-{[1-(éthylsulfonyl)-4-pipéridinylidène]méthyl)-5-phényl-1*H*-indole-7-carboxamide ;
3-[1-(éthylsulfonyl)-4-pipéridinyl]-5-[4-(hydroxyméthyl)phényl]-1*H*-indole-7-carboxamide ;
5-phényl-3-(3-pipéridinylméthyl)-1*H*-indole-7-carboxamide ;
5-phényl-3-[2-(4-pipéridinyl)éthyl]-1*H*-indole-7-carboxamide ;
3-{2-[1-(éthylsulfonyl)-4-pipéridinyl]éthyl}-5-phényl-1*H-*indole-7-carboxamide ;
3-{[1-(éthylsulfonyl)-3-pipéridinyl]méthyl}-5-phényl-1*H-*indole-7-carboxamide ;
3-{[1-(éthylsulfonyl)-4-pipéridinyl]méthyl}-5-phényl-1*H-*indole-7-carboxamide ;
3-{1-[(2)sulfonyl]-4-pipéridinyl}-5-phényl-1*H*-indole-7-carboxamide ;
3-{1-[(4-fluorophényl)sulfonyl]-4-pipéridinyl}-5-phényl-1*H*-indole-7-carboxamide ;
3-{1-[(4-méthylphényl)sulfonyl]-4-pipéridinyl}-5-phényl-1*H*-indole-7-carboxamide ;
3-{phénylsulfonyl-4-pipéridinyl}-5-phényl-1*H*-indole-7-carboxamide ;
3-(1-{[4-(méthyloxy)phényl]sulfonyl}-4-pipéridinyl)-5-phényl-1*H*-indole-7-carboxamide ;
3-[1-(éthanesulfonyl)-4-pipéridinyl]-5-phényl-1*H*-indole-7-carboxamide ;
3-{1-[(2-propanesulfonyl]-4-pipéridinyl}-5-phényl-1*H-*indole-7-carboxamide ;
5-phényl-3-[1-(propanesulfonyl)-1,2,3,6-tétrahydro-4-pyridinyl]-1*H*-indole-7-carboxamide ;
5-phényl-3-(1-{[4-(trifluorométhyl)phényl]sulfonyl}-4-pipéridiny1)-1*H*-indole-7-carboxamide ;
3-{1-[(2,4-dichlorophényl)sulfonyl]-4-pipéridinyl)-5-phényl-1*H*-indole-7-carboxamide ;
3-{1-[(3,4-dichlorophényl)sulfonyl]-4-pipéridinyl}-5-phényl-1*H*-indole-7-carboxamide ;
3-{1-[(éthylamino)carbonyl]-4-pipéridinyl}-5-phényl-1*H-*indole-7-carboxamide ;
3-{1-[(4-1-pipérazinyl)carbonyl]-4-pipéridinyl}-5-phényl-1*H*-indole-7-carboxamide ;
5-(4-chlorophényl)-3-[1-(propanesulfonyl)-4-pipéridinyl]-1*H*-indole-7-carboxamide ;
3-{1-[(4-fluorophényl)sulfonyl]-4-pipéridinyl}-5-(4-chlorophényl)-1*H*-indole-7-carboxamide ;
5-{4-[(méthylsulfonyl)amino]phényl}-3-[1-(phénylméthyl)-1,2,3,6-tétrahydro-4-pyridinyl]-1*H*-indole-7-carboxamide ;
5-{4-[(méthylsulfonyl)amino]phényl}-3-(4-pipéridinyl)-1*H*-indole-7-carboxamide ;
5-bromo-3-[1-(éthanesulfonyl)-4-pipéridinyl]-1*H-*indole-7-carboxamide ;
3-[1-(éthanesulfonyl)-4-pipéridinyl]-5-{4-[(méthylsulfonyl)amino]phényl}-1*H*-indole-7-carboxamide ;
3-[1-(éthanesulfonyl)-4-pipéridinyl]-5-(3-méthylphényl)-1*H*-indole-7-carboxamide ;
3-[1-(éthylsulfonyl)-4-pipéridinyl]-5-(2-thiényl) - 1*H*-indole-7-carboxamide ;
3-[1-(éthylsulfonyl)-4-pipéridinyl]-5-(3-thiényl)-1*H*-indole-7-carboxamide ;
3-[4-(méthylsulfonyl)phényl]-5-phényl-1*H*-indole-7-carboxamide ;
3-{4-[(diméthylamino)sulfonyl]phényl}-5-phényl-1*H-*indole-7-carboxamide ;
3-{3-[(méthylsulfonyl)amino]phényl}-5-phényl-1*H-*indole-7-carboxamide ;
3-[1-(éthylsulfonyl)-4-pipéridinyl]-5-{3-[(méthylsulfonyl)amino]phényl}-1*H*-indole-7-carboxamide;
5-[4-(acétylamino)phényl]-3-[1-(éthylsulfonyl)-4-pipéridinyl]-1*H*-indole-7-carboxamide ;
5-{4-[(diméthylamino)sulfonyl]phényl}-3-[1-(éthylsulfonyl)-4-pipéridinyl]-1*H*-indole-7-carboxamide ;
5-[3-(acétylamino)phényl]-3-[1-(éthylsulfonyl)-4-pipéridinyl]-1*H*-indole-7-carboxamide ;
3-[1-(éthylsulfonyl)-4-pipéridinyl]-5-(1*H*-pyrazol-4-yl)-1*H*-indole-7-carboxamide ;
3-[1-(éthylsulfonyl)-4-pipéridinyl]-5-[3-(hydroxyméthyl)phényl]-1*H*-indole-7-carboxamide ;
5-(2,4-difluorophényl)-3-[1-(éthylsulfonyl)-4-pipéridinyl]-1*H*-indole-7-carboxamide ;
3-[1-(éthylsulfonyl)-4-pipéridinyl]-5-[4-(méthyloxy) - phényl]-1*H*-indole-7-carboxamide ;
3-[1-(éthylsulfonyl)-4-pipéridinyl]-5-(4-fluoro-2-méthylphényl)-1*H*-indole-7-carboxamide ;
3-[1-(éthylsulfonyl)-4-pipéridinyl]-5-(4-fluorophényl)-1*H*-indole-7-carboxamide ;
5-(4-biphénylyl)-3-[1-(éthylsulfonyl)-4-pipéridinyl]-1*H*-indole-7-carboxamide ;
5-[4-(1,1-diméthyléthyl)phényl]-3-[1-(éthylsulfonyl) - 4-pipéridinyl]-1*H*-indole-7-carboxamide ;
3-[1-(éthylsulfonyl)-4-pipéridinyl]-5-(4-méthylphényl)-1*H*-indole-7-carboxamide ;
3-[1-(éthylsulfonyl)-4-pipéridinyl]-5-(4-pyridinyl) - 1*H*-indole-7-carboxamide ;
3-[1-(éthylsulfonyl)-4-pipéridinyl]-5-(6-fluoro-3-pyridinyl)-1*H*-indole-7-carboxamide ;
3-[1-(éthylsulfonyl)-4-pipéridinyl]-5-(6-méthyl-3-pyridinyl)-1*H*-indole-7-carboxamide ;
3-[1-(éthylsulfonyl)-4-pipéridinyl]-5-(4-méthyl-3-pyridinyl)-1*H*-indole-7-carboxamide ;
3-[1-(éthylsulfonyl)-4-pipéridinyl]-5-[6-(méthyloxy)-3-pyridinyl]-1*H*-indole-7-carboxamide ;
5-phényl-3-(*N*-acétyl-3-pipéridinylméthyl)-1*H*-indole-7-carboxamide ;
5-[3-(éthyloxy)phényl]-3-[1-(éthylsulfonyl)-4-pipéridinyl]-1*H*-indole-7-carboxamide ;
3-[1-(éthylsulfonyl)-4-pipéridinyl]-5-(2-fluorophényl)-1*H*-indole-7-carboxamide ;
3-[1-(éthylsulfonyl)-4-pipéridinyl]-5-[3-(trifluorométhyl)phényl]-1*H*-indole-7-carboxamide ;
3-[1-(éthylsulfonyl)-4-pipéridinyl]-5-[4-(trifluorométhyl)phényl]-1*H*-indole-7-carboxamide ;
3-[1-(éthylsulfonyl)-4-pipéridinyl]-5-(3-fluorophényl)-1*H*-indole-7-carboxamide ;
5-(3,5-dichlorophényl)-3-[1-(éthylsulfonyl)-4-pipéridinyl]-1*H*-indole-7-carboxamide ;
5-(3,4-difluorophenyl)-3-[1-(éthylsulfonyl)-4-pipéridinyl]-1*H*-indole-7-carboxamide ;
3-bromo-5-{3-[(diméthylamino)carbonyl]phényl}-1*H-*indole-7-carboxamide ;
5-[2,6-bis(méthyloxy)phényl]-3-bromo-1*H*-indole-7-carboxamide ;
3-bromo-5-(4-fluoro-2-méthylphenyl)-1*H*-indole-7-carboxamide ;
3-bromo-5-[5-fluoro-2-(méthyloxy)phényl]-1*H*-indole-7-carboxamide ;
trifluoroacétate de 3-bromo-5-(3-quinoléinyl)-1*H-*indole-7-carboxamide ;
trifluoroacétate de 3-bromo-5-(5-quinoléinyl)-1*H-*indole-7-carboxamide ;
5-[2,5-bis(méthyloxy)phényl]-3-bromo-1*H*-indole-7-carboxamide ;
3-bromo-5-(2-fluorophényl)-1*H*-indole-7-carboxamide ;
5-[2,4-bis(méthyloxy)phényl]-3-bromo-1*H*-indole-7-carboxamide ;
trifluoroacétate de 3-bromo-5-[2-(méthyloxy)-3-pyridinyl]-1*H*-indole-7-carboxamide ;
3-bromo-5-[2,3,4-tris(méthyloxy)phényl]-1*H*-indole-7-carboxamide ;
3-{1-[(4-chloro-2,5-diméthylphényl)sulfonyl]-4-pipéridinyl}-5-phényl-1*H*-indole-7-carboxamide ;
3-(1-{[5-bromo-2-(méthyloxy)phényl]sulfonyl}-4-pipéridinyl)-5-phényl-1*H*-indole-7-carboxamide ;
3-{1-[(5-fluoro-2-méthylphényl)sulfonyl]-4-pipéridinyl}-5-phényl-1*H*-indole-7-carboxamide ;
3-{1-[(3-fluorophényl)sulfonyl]-4-pipéridinyl}-5-phényl-1*H*-indole-7-carboxamide ;
5-phényl-3-(1-{[2,4,6-tris(1-méthyléthyl)phényl]-sulfonyl}-4-pipéridinyl)-1*H*-indole-7-carboxamide ;
3-(1-{[4-(1,1-diméthylpropyl)phényl]sulfonyl}-4-pipéridinyl)-5-phényl-1*H*-indole-7-carboxamide ;
3-{1-[(2-méthylphényl)sulfonyl]-4-pipéridinyl}-5-phényl-1*H*-indole-7-carboxamide ;
3-{1-[(2-iodophényl)sulfonyl]-4-pipéridinyl}-5-phényl-1*H*-indole-7-carboxamide ;
3-{1-[(4-pentylphényl)sulfonyl]-4-pipéridinyl}-5-phényl-1*H*-indole-7-carboxamide ;
5-phényl-3-{1-[(4-propylphényl)sulfonyl]-4-pipéridinyl}-1*H*-indole-7-carboxamide ;
3-{1-[(2,4-difluorophényl)sulfonyl]-4-pipéridinyl}-5-phényl-1*H*-indole-7-carboxamide ;
3-{1-[(2,5-diméthylphényl)sulfonyl]-4-pipéridinyl}-5-phényl-1*H*-indole-7-carboxamide ;
3-{1-[(4-éthylphényl)sulfonyl]-4-pipéridinyl}-5-phényl-1*H*-indole-7-carboxamide ;
3-{1-[(3-méthylphényl)sulfonyl]-4-pipéridinyl}-5-phényl-1*H*-indole-7-carboxamide ;
3-{1-[(4-([2-(méthyloxy)phényl]oxy}phényl)sulfonyl]-4-pipéridinyl}-5-phényl-1*H*-indole-7-carboxamide ;
3-{1-[(4-{[4-(méthyloxy)phényl]oxy}phényl)sulfonyl]-4-pipéridinyl}-5-phényl-1*H*-indole-7-carboxamide ;
3-[1-({3-[(4-fluorophényl)oxy]phényl}sulfonyl)-4-pipéridinyl]-5-phényl-1*H*-indole-7-carboxamide ;
3-{1-[(3-{[2-(méthyloxy)phényl]oxy}phényl)sulfonyl]-4-pipéridinyl}-5-phényl-1*H*-indole-7-carboxamide ;
3-[1-({4-[(4-chlorophényl)oxy]phényl}sulfonyl)-4-pipéridinyl]-5-phényl-1*H*-indole-7-carboxamide ;
3-{1-[(3-{[4-(méthyloxy)phényl]oxy}phényl)sulfonyl]-4-pipéridinyl)-5-phényl-1*H*-indole-7-carboxamide ;
5-phényl-3-(1-{[3-(phényloxy)phényl]sulfonyl}-4-pipéridinyl)-1*H*-indole-7-carboxamide ;
3-[1-({3-[(4-chlorophényl)oxy]phényl)sulfonyl)-4-pipéridinyl]-5-phényl-1*H*-indole-7-carboxamide ;
3-[1-({4-[(2-méthylphényl)oxy]phényl}sulfonyl)-4-pipéridinyl]-5-phényl-1*H*-indole-7-carboxamide ;
3-{1-[(4'-chloro-4-biphénylyl)sulfonyl]-4-pipéridinyl}-5-phényl-1*H*-indole-7-carboxamide ;
3-[1-({3-[(2-méthylphenyl)oxy]phényl}sulfonyl)-4-pipéridinyl]-5-phényl-1*H*-indole-7-carboxamide ;
3-[1-({3-[(2-chlorophényl)oxy]phényl}sulfonyl)-4-pipéridinyl]-5-phényl-1*H*-indole-7-carboxamide ;
3-(1-[(5-chloro-1-naphtalényl)sulfonyl]-4-pipéridinyl}-5-phényl-1*H*-indole-7-carboxamide ;
3-(1-{[4'-(méthyloxy)-3-biphénylyl]sulfonyl}-4-pipéridinyl)-5-phényl-1*H*-indole-7-carboxamide ;
3-[1-(3-biphénylylsulfonyl)-4-pipéridinyl]-5-phényl-1*H-*indole-7-carboxamide ;
3-(1-{[(4-fluorophényl)méthyl]sulfonyl}-4-pipéridinyl)-5-phényl-1*H*-indole-7-carboxamide ;
3-{1-[(5-chloro-2-naphtalényl)sulfonyl]-4-pipéridinyl}-5-phényl-1*H*-indole-7-carboxamide ;
3-(1-{[4'-(méthyloxy)-4-biphénylyl]sulfonyl}-4-pipéridinyl)-5-phényl-1*H*-indole-7-carboxamide ;
5-(2-fluorophényl)-1*H*-indole-7-carboxamide ;
5-(3-{[(2,2-diméthylpropyl)amino]carbonyl}phényl)-1*H-*indole-7-carboxamide ;
5-(3-{[(1-méthyléthyl)amino]carbonyl)phényl)-1*H*-indole-7-carboxamide ;
5-(4-{[(2,2-diméthylpropyl)amino]carbonyl}phényl)-1*H-*indole-7-carboxamide ;
5-{4-[(propylamino)carbonyl]phényl}-1*H*-indole-7-carboxamide ;
5-(4-{[(1-méthyléthyl)amino]carbonyl)phényl)-1*H*-indole-7-carboxamide ;
5-{4-[(diéthylamino)carbonyl]phényl}-1*H*-indole-7-carboxamide ;
3-[1-(méthylsulfonyl)-1,2,3,6-tétrahydro-4-pyridinyl]-5-phényl-1*H*-indole-7-carboxamide ;
3-(3-oxocyclopentyl)-5-phényl-1*H*-indole-7-carboxamide ;
5-phényl-3-{3-[(phénylméthyl)amino]cyclopentyl}-1*H-*indole-7-carboxamide ;
3-(3-aminocyclopentyl)-5-phényl-1*H*-indole-7-carboxamide ;
3-{3-[(éthylsulfonyl)amino]cyclopentyl}-5-phenyl-1*H-*indole-7-carboxamide ;
5-bromo-3-[1-(propylsulfonyl)-4-pipéridinyl]-1*H*-indole-7-carboxamide ;
5-bromo-3-(3-pyridinyl)-1*H*-indole-7-carboxamide ;
5-bromo-3-[1-(méthylsulfonyl)-1,2,3,6-tétrahydro-4-pyridinyl]-1*H*-indole-7-carboxamide ;
3-[(4-hydroxyphényl)méthyl]-5-phényl-1*H*-indole-7-carboxamide ;
5-bromo-1*H*-indole-7-carboxamide ;
5-(4-chlorophényl)-1*H*-indole-7-carboxamide ;
5-bromo-3-(4-pipéridinyl)-1*H*-indole-7-carboxamide ;
ou un sel de celui-ci.

19. Composé selon la revendication 1 choisi dans le groupe de :
3-{1-[(4-chlorophényl)sulfonyl]-4-pipéridinyl}-5-phényl-1*H*-indole-7-carboxamide ;
3-{1-[(2)sulfonyl]-4-pipéridinyl}-5-phényl-1*H*-indole-7-carboxamide ;
3-{1-[(4-fluorophényl)sulfonyl]-4-pipéridinyl}-5-phényl-1*H*-indole-7-carboxamide ;
3-{1-[(4-méthylphényl)sulfonyl]-4-pipéridinyl}-5-phényl-1*H*-indole-7-carboxamide ;
3-{phénylsulfonyl-4-pipéridinyl}-5-phényl-1*H*-indole-7-carboxamide ;
3-(1-([4-(méthyloxy)phényl]sulfonyl}-4-pipéridinyl)-5-phényl-1*H*-indole-7-carboxamide ;
5-phényl-3-(1-{[4-(trifluorométhyl)phényl]sulfonyl}-4-pipéridinyl)-1*H*-indole-7-carboxamide ;
3-{1[(2,4-dichlorophényl)sulfonyl]-4-pipéridinyl}-5-phényl-1*H*-indole-7-carboxamide ;
3-{1[(3,4-dichlorophényl)sulfonyl]-4-pipéridinyl}-5-phényl-1*H*-indole-7-carboxamide ;
3-{1[(4-fluorophényl)sulfonyl]-4-pipéridinyl}-5-(4-chlorophényl)-1*H*-indole-7-carboxamide ;
3-{1-[(4-chloro-2,5-diméthylphényl)sulfonyl]-4-pipéridinyl}-5-phényl-1*H*-indole-7-carboxamide ;
3-(1-([5-bromo-2-(méthyloxy)phényl]sulfonyl-1-4-pipéridinyl)-5-phényl-1*H*-indole-7-carboxamide ;
3-{1-[(5-fluoro-2-méthylphényl)sulfonyl]-4-pipéridinyl}-5-phényl-1*H*-indole-7-carboxamide ;
3-{1-[(3-fluorophényl)sulfonyl]-4-pipéridinyl}-5-phényl-1*H*-indole-7-carboxamide ;
5-phényl-3-(1-([2,4,6-tris(1-méthyléthyl)phényl]-sulfonyl)-4-pipéridinyl)-1*H*-indole-7-carboxamide ;
3-(1-{[4-(1,1-diméthylpropyl)phényl]sulfonyl}-4-pipéridinyl)-5-phényl-1*H*-indole-7-carboxamide ;
3-{1-[(2-méthylphényl)sulfonyl]-4-pipéridinyl}-5-phényl-1*H*-indole-7-carboxamide ;
3-{1-[(2-iodophényl)sulfonyl]-4-pipéridinyl}-5-phényl-1*H*-indole-7-carboxamide ;
3-{1-[(4-pentylphényl)sulfonyl]-4-pipéridinyl}-5-phényl-1*H*-indole-7-carboxamide ;
5-phényl-3-{1-[(4-propylphényl)sulfonyl]-4-pipéridinyl}-1*H*-indole-7-carboxamide ;
3-{1-[(2,4-difluorophényl)sulfonyl]-4-pipéridinyl}-5-phényl-1*H*-indole-7-carboxamide ;
3-{1-[(2,5-diméthylphényl)sulfonyl]-4-pipéridinyl}-5-phényl-1*H*-indole-7-carboxamide ;
3-{1-[(4-éthylphényl)sulfonyl]-4-pipéridinyl}-5-phényl-1*H*-indole-7-carboxamide ;
3-{1-[(3-méthylphényl)sulfonyl]-4-pipéridinyl}-5-phényl-1*H*-indole-7-carboxamide ;
3-{1-[(4-{[2-(méthyloxy)phényl]oxy}phényl)sulfonyl]-4-pipéridinyl}-5-phényl-1*H*-indole-7-carboxamide ;
3-{1-[(4-{[4-(méthyloxy)phényl]oxy}phényl)sulfonyl]-4-pipéridinyl}-5-phényl-1*H*-indole-7-carboxamide ;
3-[1-({3-[(4-fluorophényl)oxy]phényl}sulfonyl)-4-pipéridinyl]-5-phényl-1*H*-indole-7-carboxamide ;
3-{1-[(3-{[2-(méthyloxy)phenyl]oxy}phényl)sulfonyl]-4-pipéridinyl)-5-phényl-1*H*-indole-7-carboxamide ;
3-[1-({4-[(4-chlorophényl)oxy]phényl}sulfonyl)-4-pipéridinyl]-5-phényl-1*H*-indole-7-carboxamide ;
3-{1-[(3-{[4-(méthyloxy)phényl]oxy}phényl)sulfonyl]-4-pipéridinyl}-5-phényl-1*H*-indole-7-carboxamide ;
5-phényl-3-(1-{[3-(phényloxy)phényl]sulfonyl1-4-pipéridinyl)-1*H*-indole-7-carboxamide ;
3-[1-({3-[(4-chlorophényl)oxy]phényl}sulfonyl)-4-pipéridinyl}-5-phényl-1*H*-indole-7-carboxamide ;
3-[1-({4-[(2-méthylphenyl)oxy]phényl}sulfonyl)-4-pipéridinyl1-5-phényl-1*H*-indole-7-carboxamide ;
3-{1-[(4'-chloro-4-biphénylyl)sulfonyl]-4-pipéridinyl}-5-phényl-1*H*-indole-7-carboxamide ;
3-[1-({3-[(2-méthylphényl)oxy]phényl}sulfonyl)-4-pipéridinyl]-5-phényl-1*H*-indole-7-carboxamide ;
3-[1-({3-[(2-chlorophényl)oxy]phényl}sulfonyl)-4-pipéridinyl]-5-phényl-1*H*-indole-7-carboxamide ;
3-{1-[(5-chloro-1-naphtalényl)sulfonyl]-4-pipéridinyl}-5-phényl-1*H*-indole-7-carboxamide ;
3-(1-{[4'-(méthyloxy)-3-biphénylyl]sulfonyl}-4-pipéridinyl)-5-phényl-1*H*-indole-7-carboxamide ;
3-[1-(3-biphénylylsulfonyl)-4-pipéridinyl]-5-phényl-1*H-*indole-7-carboxamide ;
3-(1-{[(4-fluorophényl)méthyl]sulfonyl}-4-pipéridinyl)-5-phényl-1*H*-indole-7-carboxamide ;
3-{1-[(5-chloro-2-naphtalényl)sulfonyl]-4-pipéridinyl}-5-phényl-1*H*-indole-7-carboxamide ; et
3-(1-{[4'-(méthyloxy)-4-biphénylyl]sulfonyl}-4-pipéridinyl)-5-phényl-1*H*-indole-7-carboxamide ;
ou un sel de celui-ci.

20. Composé selon la revendication 1 choisi dans le groupe constitué de :
5-phényl-3-[1-(propylsulfonyl)-4-pipéridinyl]-1*H*-indole-7-carboxamide ;
3-[1-(éthylsulfonyl)-4-pipéridinyl]-5-[4-(hydroxyméthyl)phényl]-1*H*-indole-7-carboxamide ;
3-[1-(éthanesulfonyl)-4-pipéridinyl]-5-phényl-1*H*-indole-7-carboxamide ;
3-{1-[(2-propanesulfonyl]-4-pipéridinyl}-5-phényl-1*H-*indole-7-carboxamide ;
5-phényl-3-[1-(propanesulfonyl)-1,2,3,6-tétrahydro-4-pyridinyl]-1*H*-indole-7-carboxamide ;
5-(4-chlorophényl)-3-[1-(propanesulfonyl)-4-pipéridinyl]-1*H*-indole-7-carboxamide ;
3-[1-(éthanesulfonyl)-4-pipéridinyl]-5-{4-[(méthylsulfonyl)amino]phényl)-1*H*-indole-7-carboxamide ;
3-[1-(éthanesulfonyl)-4-pipéridinyl]-5-(3-méthylphényl) - 1*H*-indole-7-carboxamide ;
3-[1-(éthylsulfonyl)-4-pipéridinyl]-5-{3-[(méthylsulfonyl)amino]phényl}-1*H*-indole-7-carboxamide ;
5-[4-(acétylamino)phényl]-3-[1-(éthylsulfonyl)-4-pipéridinyl]-1*H*-indole-7-carboxamide ;
5-{4-[(diméthylamino)sulfonyl]phényl}-3-[1-(éthylsulfonyl)-4-pipéridinyl]-1*H*-indole-7-carboxamide ;
5-[3-(acétylamino)phényl]-3-[1-(éthylsulfonyl)-4-pipéridinyl]-1*H*-indole-7-carboxamide ;
3-[1-(éthylsulfonyl)-4-pipéridinyl]-5-[3-(hydroxyméthyl)-phényl]-1*H*-indole-7-carboxamide ;
5-(2,4-difluorophényl)-3-[1-(éthylsulfonyl)-4-pipéridinyl]-1*H*-indole-7-carboxamide ;
3-[1-(éthylsulfonyl)-4-pipéridinyl]-5-[4-(méthyloxy)-phényl]-1*H*-indole-7-carboxamide ;
3-[1-(éthylsulfonyl)-4-pipéridinyl]-5-(4-fluoro-2-méthylphényl)-1*H*-indole-7-carboxamide ;
3-[1-(éthylsulfonyl)-4-pipéridinyl]-5-(4-fluorophényl)-1*H*-indole-7-carboxamide ;
5-(4-biphénylyl)-3-[1-(éthylsulfonyl)-4-pipéridinyl]-1*H-*indole-7-carboxamide ;
5-[4-(1,1-diméthyléthyl)phényl]-3-[1-(éthylsulfonyl)-4-pipéridinyl]-1*H*-indole-7-carboxamide ;
3-[1-(éthylsulfonyl)-4-pipéridinyl]-5-(4-méthylphényl)-1*H*-indole-7-carboxamide ;
5-[3-(éthyloxy)phényl]-3-[1-(éthylsulfonyl)-4-pipéridinyl]-1*H*-indole-7-carboxamide ;
3-[1-(éthylsulfonyl)-4-pipéridinyl]-5-(2-fluorophényl) - 1*H*-indole-7-carboxamide ;
3-[1-(éthylsulfonyl)-4-pipéridinyl]-5-[3-(trifluorométhyl)phényl]-1*H*-indole-7-carboxamide ;
3-[1-(éthylsulfonyl)-4-pipéridinyl]-5-[4-(trifluorométhyl)phényl]-1*H*-indole-7-carboxamide ;
3-[1-(éthylsulfonyl)-4-pipéridinyl]-5-(3-fluorophényl)-1*H*-indole-7-carboxamide ;
5-(3,5-dichlorophényl)-3-[1-(éthylsulfonyl)-4-pipéridinyl]-1*H*-indole-7-carboxamide ; et
5-(3,4-difluorophényl)-3-[1-(éthylsulfonyl)-4-pipéridinyl]-1*H*-indole-7-carboxamide ;
ou un sel de celui-ci.

21. Composé selon la revendication 1 choisi dans le groupe constitué de :
3-[1-(éthylsulfonyl)-4-pipéridinyl]-5-(2-thiényl)-1*H-*indole-7-carboxamide ;
3-[1-(éthylsulfonyl)-4-pipéridinyl]-5-(3-thiényl)-1*H-*indole-7-carboxamide ;
3-[1-(éthylsulfonyl)-4-pipéridinyl]-5-(1*H*-pyrazol-4-yl) - 1*H*-indole-7-carboxamide ;
3-[1-(éthylsulfonyl)-4-pipéridinyl]-5-(4-pyridinyl)-1*H-*indole-7-carboxamide ;
3-[1-(éthylsulfonyl)-4-pipéridinyl]-5-(6-fluoro-3-pyridinyl)-1*H*-indole-7-carboxamide ;
3-[1-(éthylsulfonyl)-4-pipéridinyl]-5-(6-méthyl-3-pyridinyl)-1*H*-indole-7-carboxamide ;
3-[1-(éthylsulfonyl)-4-pipéridinyl]-5-(4-méthyl-3-pyridinyl)-1*H*-indole-7-carboxamide ; et
3-[1-(éthylsulfonyl)-4-pipéridinyl]-5-[6-(méthyloxy)-3-pyridinyl]-1*H*-indole-7-carboxamide ;
ou un sel de celui-ci.

22. Composé selon la revendication 1 qui est le 5-bromo-3-[1-(éthanesulfonyl)-(4-pipéridinyl]-1*H*-indole-7-carboxamide ou un sel de celui-ci.

23. Composé selon la revendication 1 choisi dans le groupe constitué de :
3-[1-(méthylsulfonyl)-1,2,3,6-tétrahydro-4-pyridinyl]-5-phényl-1*H*-indole-7-carboxamide ; et
5-bromo-3-[1-(méthylsulfonyl)-1,2,3,6-tétrahydro-4-pyridinyl]-1*H*-indole-7-carboxamide ;
ou un sel de celui-ci.

24. Composition pharmaceutique comprenant un composé selon l'une quelconque des revendications 1 à 23, ou un sel de celui-ci et un ou plusieurs supports, diluants et excipients pharmaceutiquement acceptables.

25. Composé selon l'une quelconque des revendications 1 à 23, ou un sel de celui-ci pour une utilisation en thérapie.

26. Composé selon l'une quelconque des revendications 1 à 23 pour une utilisation dans le traitement d'un trouble médié par une activité kinase inappropriée.

27. Composé selon l'une quelconque des revendications 1 à 23 pour une utilisation dans le traitement d'un trouble médié par une activité IKK2 inappropriée.

28. Utilisation d'un composé selon l'une quelconque des revendications 1 à 23, ou d'un sel de celui-ci dans la préparation d'un médicament pour une utilisation dans le traitement d'un trouble médié par une activité kinase inappropriée.

29. Utilisation selon la revendication 28, où l'activité kinase inappropriée est une activité IKK2 inappropriée.

30. Utilisation selon la revendication 29, où le trouble médié par une activité IKK2 inappropriée concerne des troubles inflammatoires et de réparation tissulaire, en particulier la polyarthrite rhumatoïde, la maladie inflammatoire des intestins, l'asthme et la BPCO (bronchopneumopathie chronique obstructive) ; l'arthrose, l'ostéoporose et des maladies fibrotiques ; une dermatose, y compris le psoriasis, la dermite atopique et les lésions cutanées provoquées par les rayons ultraviolets (UV) ; des maladies auto-immunes y compris le lupus érythémateux systémique, la sclérose en plaques, l'arthrite psoriasique, la spondylarthrite ankylosante, le rejet de tissu et d'organe, la maladie d'Alzheimer, un accident vasculaire cérébral, l'athérosclérose, une resténose, le diabète, la glomérulonéphrite, le cancer, y compris la maladie d'Hodgkin, la cachexie, une inflammation associée à une infection et certaines infections virales, y compris le syndrome d'immunodéficience acquise (SIDA), le syndrome de détresse respiratoire de l'adulte, et l'ataxie télangiectasique.

31. Utilisation selon la revendication 30, où le trouble de l'IKK2 est un trouble inflammatoire ou de réparation tissulaire.

32. Utilisation selon la revendication 31, où le trouble de l'IKK2 est la polyarthrite rhumatoïde, la maladie inflammatoire des intestins, l'asthme ou la BPCO.

33. Utilisation selon la revendication 32, où le trouble de l'IKK2 est l'asthme.

34. Utilisation selon la revendication 32, où le trouble de l'IKK2 est la BPCO.

35. Utilisation selon la revendication 32, où le trouble de l'IKK2 est la polyarthrite rhumatoïde.

36. Utilisation selon la revendication 30, où le trouble de l'IKK2 est choisi dans le groupe constitué par des maladies auto-immunes ; le rejet de tissu ou d'organe, la maladie d'Alzheimer, un accident vasculaire cérébral, l'athérosclérose, une resténose, le diabète, la glomérulonéphrite, l'arthrose, l'ostéoporose et l'ataxie télangiectasique.

37. Utilisation selon la revendication 36, où ladite maladie est une maladie auto-immune.

38. Utilisation selon la revendication 37, où la maladie auto-immune est le lupus érythémateux systémique, la sclérose en plaques, l'arthrite psoriasique ou la spondylarthrite ankylosante, le diabète.

39. Utilisation selon la revendication 30, où la maladie est le cancer ou la cachexie.

40. Utilisation selon la revendication 39, où le cancer est la maladie de Hodgkin.
